# EUROPEAN PATENT SPECIFICATION

(11) **EP 2 121 949 B1**
(45) Date of publication and mention of the grant of the patent: **08.05.2013**
(21) Application number: 07871733.7
(22) Date of filing: 21.12.2007
(51) Int. Cl.: C12N 9/88, C12N 9/00, C12N 9/02, C12P 7/16

(54) **BUTANOL PRODUCTION BY METABOLICALLY ENGINEERED YEAST**
BUTANOLHERSTELLUNG MITTELS METABOLISCH MANIPULIERTER HEFE
PRODUCTION DE BUTANOL PAR UNE LEVURE MÉTABOLIQUEMENT MODIFIÉE

(30) Priority: 21.12.2006 US 871427 P; 02.02.2007 US 888016 P; 08.05.2007 US 928283 P
(43) Date of publication of application: 25.11.2009
(73) Proprietor: GEVO, Inc., Centennial, CO 80112 (US)
(72) Inventor: GUNAWARDENA, Uvini, Irvine, CA 92602 (US); MEINHOLD, Peter, Denver, CO 80209 (US); PETERS, Matthew W., Highlands Ranch, CO 80216 (US); URANO, Jun, Culver City, CA 90024 (US); FELDMAN, Reid M. Renny, Denver, CO 80209 (US)
(74) Representative: Walton, Seán Malcolm
(86) International application number: PCT/US2007/088705
(87) International publication number: WO 2008/080124

(56) References cited:
- WO-A2-02/42471
- WO-A2-2005/073364
- WO-A2-2007/024718
- WO-A2-2007/041269
- US-B1- 6 358 717
- TER SCHURE EELKO G ET AL: "Pyruvate decarboxylase catalyzes decarboxylation of branched-chain 2-oxo acids but is not essential for fusel alcohol production by Saccharomyces cerevisiae" APPLIED AND ENVIRONMENTAL MICROBIOLOGY, vol. 64, no. 4, April 1998 (1998-04), pages 1303-1307, XP002578980 ISSN: 0099-2240
- REMIZE F. ET AL.: 'Engineering of the pyruvate dehydrogenase bypass in Saccharomyces cerevisiae: role of cytosolic Mg21 and mitochondrial K1 acetaldehyde dehydrogenases Ald6p and Ald4p in acetate formation during alcoholic fermentation' APPL. ENVIRON. MICROBIOL. vol. 66, no. 8, August 2000, pages 3151 - 3159, XP008110150
- KRESNOWATI M.T.A.P. ET AL.: 'When transcriptome meets metabolome: fast cellular responses of yeast to sudden relief of glucose limitation' MOL. SYS. BIOL. vol. 2, no. 49, 12 September 2006, pages 1 - 16, XP008110553
- ZEEMAN A.M. ET AL.: 'Inactivation of the Kluyveromyces lactis KIPDA1 gene leads to loss of pyruvate dehydrogenase activity, impairs growth on glucose and triggers aerobic alcoholic fermentation' MICROBIOL. vol. 144, no. 12, December 1998, pages 3437 - 3446, XP008110115
- SONDEREGGER M. ET AL.: 'Metabolic engineering of a phosphoketolase pathway for pentose catabolism in Saccharomyces cerevisiae' APPL. ENVIRON. MICROBIOL. vol. 70, no. 5, May 2004, pages 2892 - 2897, XP002355573

### SEQUENCE LISTING

<110> Gunawardena, Uvini Meinhold, Peter Peters, Matthew W. Urano, Jun Feldman, Reid M. Renny
<120> BUTANOL PRODUCTION BY METABOLICALLY ENGINEERED YEAST
<130> 56836.830003.US1
<150> 60/871,427
   <151> 2006-12-21
<150> 60/888,016
   <151> 2007-02-02
<150> 60/928,283
   <151> 2007-05-08
<160> 190
<170> PatentIn version 3.4
<210> 1
   <211> 1179
   <212> DNA
   <213> Ca-thl-co
<400> 1
   atgaaagaag ttgtaatagc tagcgcggtg cgtaccgcca ttggctctta tggtaaaagt      60
   ctgaaggatg ttccggcagt cgacttaggg gctacggcga tcaaagaagc cgtaaaaaag     120
   gcaggaatta aaccagagga tgtgaatgaa gttatcctgg gcaacgtcct gcaggctggt     180
   ttagggcaaa atcctgcgcg ccaggcctca tttaaagcag gactgccggt agagattcca     240
   gctatgacta tcaacaaggt gtgcggctcc ggtctgcgga cagtttcgtt agcggcccaa     300
   attatcaaag caggcgacgc tgatgtcatt atcgcgggtg ggatggaaaa tatgagccgt     360
   gccccttacc tggcaaacaa tgcgcgctgg ggatatcgta tgggcaacgc taaattcgtg     420
   gacgaaatga ttaccgatgg tctgtgggat gcctttaatg actaccatat gggcatcacg     480
   gcagagaaca ttgcggaacg ctggaatatc tctcgggagg aacaggatga gttcgcttta     540
   gccagtcaga agaaagcaga ggaagcgatt aaatcaggtc aatttaagga cgagatcgta     600
   ccggttgtga ttaaagggcg taaaggagaa actgtcgttg atacagacga acacccgcgc     660
   ttcggctcca ccattgaggg tctggctaag ctgaaaccag cctttaaaaa ggatgggacg     720
   gtaaccgcag gcaacgcgtc gggtttaaat gattgtgccg cagtgctggt catcatgagc     780
   gcggaaaaag ctaaagagct gggagttaag cctctggcca aaattgtgtc ttatggcagt     840
   gcgggtgtag acccggctat catggggtac ggcccgttct atgcaactaa agccgcgatt     900
   gaaaaggctg gttggacagt cgatgaatta gacctgatcg agtcaaacga agcatttgcc     960
   gcgcagtccc tggctgttgc aaaagattta aaattcgata tgaataaggt gaacgtaaat    1020
   ggaggcgcca ttgcgctggg tcatccaatc ggggcttcgg gagcacgtat tctggttacg 1080
   ttagtgcacg ccatgcaaaa acgcgacgcg aaaaagggcc tggctaccct gtgcatcggt 1140
   gggggccagg gtactgcaat attgctagaa aagtgctag 1179
<210> 2
   <211> 849
   <212> DNA
   <213> Ca-hbd-co
<400> 2
   atgaaaaagg tatgtgttat aggcgcggga accatgggta gcggtattgc ccaggcattt 60
   gctgcaaaag gtttcgaagt ggttctgcgt gatatcaagg acgagtttgt cgatcgcggc 120
   ttagacttca ttaataaaaa cctgtctaaa ctggtaaaga aagggaaaat cgaagaggcg 180
   acgaaggtgg aaattttaac tcggatcagt ggaacagttg atctgaatat ggccgctgac 240
   tgcgatctgg tcattgaagc ggccgtagag cgtatggata tcaaaaaaca aatttttgca 300
   gacttagata acatctgtaa gccggaaacc attctggctt caaatacgtc ctcgctgagc 360
   atcactgagg tggcgtctgc cacaaaacgc ccagacaaag ttattggcat gcatttcttt 420
   aaccctgcac cggtcatgaa gttagtggaa gtaatccgtg ggattgctac cagtcaggaa 480
   acgttcgatg cggttaaaga gacctcaatc gccattggaa aagacccagt ggaagtcgca 540
   gaggcgcctg gctttgttgt aaatcgcatt ctgatcccga tgattaacga agctgtggga 600
   atcctggccg aaggaattgc atccgtcgag gatatcgaca aggcgatgaa attaggcgct 660
   aatcacccga tgggtccact ggaactgggc gacttcattg gtctggatat ctgcttagcc 720
   attatggacg ttctgtattc ggagactggg gatagcaaat accggcctca tacactgtta 780
   aagaaatatg tgcgtgcagg atggctgggc cgcaaatctg gtaagggttt ctacgattat 840
   tcaaaataa 849
<210> 3
   <211> 786
   <212> DNA
   <213> Ca-crt-co
<400> 3
   atggaactaa acaatgtcat cctggaaaaa gagggcaagg tggcggttgt caccattaat      60
   cgtccgaaag ccttaaacgc actgaatagc gatacgctga aagaaatgga ctatgtaatc     120
   ggtgagattg aaaacgattc tgaagtgtta gctgttatcc tgactggggc gggagagaag     180
   agttttgtcg ccggcgcaga catttcagaa atgaaagaga tgaatacaat cgaaggtcgc     240
   aaattcggga ttctgggaaa caaggtattt cggcgtttag aactgctgga gaaaccagtg     300
   atcgctgcgg ttaatggctt cgccttaggt ggcggttgcg aaattgcaat gtcctgtgat     360
   atccgcattg cttcgagcaa cgcgcgtttt gggcagcctg aggtcggact gggcatcaca     420
   ccgggtttcg gcggtacgca acgcctgtct cggttagtgg ggatgggaat ggccaaacag     480
   ctgattttta ctgcacaaaa tatcaaggct gacgaagcgc tgcgtattgg cctggtaaac     540
   aaagttgtgg aaccaagtga gttaatgaat acagccaaag aaatcgcaaa caagattgtc     600
   tcaaatgcgc ctgttgctgt aaaactgtcc aaacaggcca ttaaccgcgg tatgcagtgc     660
   gatatcgaca ccgcactggc gttcgagtcg gaagcttttg gggaatgttt cagcacggag     720
   gaccaaaagg atgccatgac cgcatttatt gaaaaacgta aaattgaagg cttcaaaaat     780
   agatag                                                                786
<210> 4
   <211> 1140
   <212> DNA
   <213> Ca-bcd-co
<400> 4
   atggatttta atttaacaag agaacaggaa ctggtccgtc agatggtacg tgaatttgca     60
   gaaaacgagg ttaaaccgat tgctgcagag attgatgaga ctgaacgctt cccgatggaa    120
   aacgtcaaaa agatgggtca gtatggcatg atgggcattc cgttctctaa agagtacggc    180
   ggtgcgggtg gcgacgttct gtcttatatc atcgctgtag aggaactgtc caaagtatgt    240
   ggcaccacgg gcgtgatcct gtccgcgcac acctctctgt gcgcaagcct gatcaacgaa    300
   cacggcaccg aggaacagaa gcaaaaatac ctggtcccgc tggccaaagg tgaaaagatc    360
   ggtgcatacg gtctgacgga accgaacgca ggtacggaca gcggcgcaca acagacggtt    420
   gcggtactgg aaggcgacca ctacgttatt aacggtagca aaatcttcat cacgaacggt    480
   ggcgtggctg acacctttgt tatcttcgcg atgaccgacc gcactaaagg cactaaaggt    540
   atctctgcgt tcatcatcga gaagggtttc aagggttttt ctatcggcaa agtggaacag    600
   aagctgggta tccgtgcctc ctctactacc gagctggttt tcgaagacat gattgtgccg    660
   gttgaaaata tgatcggcaa agaaggcaaa ggcttcccga tcgctatgaa aaccctggat    720
   ggcggccgta tcggcattgc agcacaggca ctgggtatcg cagaaggcgc tttcaacgaa    780
   gcacgtgcgt acatgaaaga acgtaaacag tttggccgtt ctctggataa atttcaaggc    840
   ctggcgtgga tgatggcaga catggacgta gcgattgaat ctgcgcgcta cctggtctat    900
   aaagcagctt acctgaaaca ggcaggtctg ccttacaccg ttgacgcagc acgtgcgaaa    960
   ctgcacgcgg ccaacgttgc catggatgtt accaccaaag ccgtgcaact gtttggcggt   1020
   tacggctata ctaaggatta tccggttgaa cgtatgatgc gtgacgcgaa aatcaccgaa   1080
   atctatgaag gtacttccga agtgcagaaa ctggtcattt caggaaaaat ttttagttaa   1140
<210> 5
   <211> 1011
   <212> DNA
   <213> Ca-eftA-co
<400> 5
   atgaataaag cagattacaa gggcgtttgg gtctttgcgg aacagcgtga tggtgaactg      60
   cagaaagtgt ccctggaact gctgggcaaa ggcaaggaga tggcagaaaa actgggtgtt     120
   gaactgaccg cagttctgct gggtcacaac actgaaaaga tgtccaaaga cctgctgtcc     180
   catggcgcag acaaggtgct ggctgcggac aacgaactgc tggctcactt tagcaccgac     240
   ggttatgcaa aagtaatctg cgacctggtt aacgagcgca agccggaaat cctgttcatc     300
   ggcgccactt ttattggtcg cgacctgggc cctcgtattg ctgcgcgtct gtccactggc     360
   ctgactgcgg attgcacctc cctggacatt gatgttgaaa accgtgatct gctggcaact     420
   cgcccggcat tcggcggcaa cctgatcgcc accatcgtat gttccgacca ccgtccgcaa     480
   atggctactg tacgtccggg cgtatttgaa aagctgccgg tgaacgacgc aaacgtttcc     540
   gacgacaaaa tcgaaaaagt tgctatcaag ctgaccgcta gcgatatccg taccaaagtt     600
   tctaaagtag tgaaactggc gaaggacatc gcagatattg gtgaagcaaa agttctggtg     660
   gcaggcggtc gtggcgtcgg ttccaaagag aacttcgaaa aactggagga actggcgtct     720
   ctgctgggcg gtactattgc agcgtcccgt gcagcaatcg aaaaagaatg ggtggacaag     780
   gatctgcagg tgggccagac tggtaaaacc gttcgtccga ccctgtacat cgcctgcggc     840
   atctccggtg ctattcagca cctggccggc atgcaggaca gcgactacat catcgccatc     900
   aacaaagacg ttgaagctcc gatcatgaaa gtggcggacc tggcaatcgt tggtgacgtg     960
   aacaaagttg ttccggaact gatcgcgcag gttaaagctg ctaataatta a             1011
<210> 6
   <211> 780
   <212> DNA
   <213> Ca-eftB-co
<400> 6
   atgaatatag ttgtttgttt aaaacaggtc ccggacaccg cagaagttcg tattgatcca 60
   gtaaagggta cgctgattcg cgagggcgtg ccgtctatca tcaacccaga tgacaagaac 120
   gccctggaag aagcactggt cctgaaagat aattacggcg ctcacgtaac tgttatctct 180
   atgggtccgc cgcaagcgaa aaatgcgctg gttgaagctc tggcgatggg cgctgacgag 240
   gctgttctgc tgactgatcg tgctttcggt ggtgcggaca ccctggccac ttcccacact 300
   atcgcggcag gtatcaagaa actgaaatat gacattgtgt ttgctggtcg tcaggctatt 360
   gacggtgaca cggcacaggt aggcccggaa atcgccgaac acctgggtat tccgcaggtg 420
   acctacgtag aaaaagtaga agtagacggt gataccctga aaatccgcaa agcatgggaa 480
   gatggctacg aggtggttga agtaaaaacc ccggtactgc tgaccgctat caaagagctg 540
   aatgtaccgc gttacatgtc tgttgagaaa atcttcggcg cgttcgacaa ggaagtaaag 600
   atgtggaccg ctgatgatat tgacgttgac aaagcgaatc tgggcctgaa gggctcccca 660
   actaaagtta agaagtcctc tactaaagaa gtgaagggtc agggtgaggt gattgataaa 720
   cctgttaaag aagctgctgc gtacgtggtt tctaagctga aagaagaaca ctatatttaa 780
<210> 7
   <211> 2577
   <212> DNA
   <213> Ca-adhE2-co
<400> 7
   atgaaagtta caaatcaaaa agaactgaaa cagaagttaa atgagctgcg tgaggcgcaa 60
   aaaaaatttg ccacctatac gcaggaacaa gtggataaga ttttcaaaca gtgcgcaatc 120
   gctgcggcca aagaacgcat taacctggca aagttagctg ttgaagagac tggcatcggt 180
   ctggtcgagg acaaaattat caaaaatcat tttgcggccg agtacattta taacaagtac 240
   aaaaacgaga aaacctgtgg gatcattgac cacgatgata gcctgggaat cacaaaggta 300
   gcagaaccga ttggcatcgt ggctgcgatt gttccaacga ctaatcctac atctaccgcc 360
   atcttcaaaa gtttaatttc actgaaaacg cggaatgcaa tctttttctc cccgcatcca 420
   cgtgctaaga aatcgaccat tgcggccgca aaactgattt tagacgcggc tgtcaaggcc 480
   ggtgcaccta aaaacatcat tgggtggatc gacgaaccga gcattgaact gtctcaggat 540
   ctgatgagtg aggcggacat cattttagct actggaggcc cgtcaatggt aaaagccgca 600
   tattcctcgg gtaagccagc gatcggcgtg ggtgctggga atactcctgc cattatcgac 660
   gaaagcgcag acattgatat ggcggtttct agtatcattc tgtcaaaaac gtacgacaac 720
   ggagtcatct gcgcctccga acagtcgatt ctggtgatga atagcatcta tgagaaagta 780
   aaggaagagt ttgttaaacg cggctcttac attctgaacc agaatgaaat tgcaaaaatc 840
   aaggaaacca tgttcaaaaa cggtgcgatt aatgctgata tcgtgggcaa aagtgcctat 900
   attatcgcga agatggctgg tattgaggtc ccgcaaacta caaaaatctt aattggggaa 960
   gttcagtcag tagaaaaatc cgagctgttt agccacgaaa agctgtcgcc ggtgttagca 1020
   atgtataaag tcaaagattt cgacgaggcc ctgaagaaag cgcagcgtct gatcgaatta 1080
   ggaggctctg gtcataccag ttcactgtac attgatagcc aaaacaataa agacaaggtt 1140
   aaagaatttg ggctggctat gaaaacgtcc cgcaccttta tcaacatgcc atcgtctcag 1200
   ggcgcaagtg gtgatttata taatttcgcc attgcgccta gctttactct gggatgtggc 1260
   acatggggtg ggaactcagt gtcccaaaat gtagagccga agcatctgct gaacatcaaa 1320
   tcggtcgctg aacggcgtga gaatatgtta tggttcaaag ttccacagaa gatttacttt 1380
   aaatatggct gcctgcgctt cgcactgaaa gaattaaagg atatgaacaa aaaacgtgcc 1440
   tttatcgtga cggacaagga tctgttcaaa ctgggttacg taaataaaat taccaaggtt 1500
   ttagacgaaa ttgatatcaa atattctatt tttactgaca tcaaaagcga tccgacaatt 1560
   gatagtgtga agaaaggagc gaaagagatg ctgaacttcg aacctgacac gatcatttca 1620
   atcggcggtg ggtccccgat ggatgctgca aaggtcatgc atctgttata cgagtatcca 1680
   gaagccgaaa ttgagaatct ggcgatcaac tttatggaca ttcgcaaacg gatctgtaat 1740
   tttccgaaac tgggaaccaa ggctattagc gttgcaatcc ctactacggc cggcaccggt 1800
   tcggaagcga caccgttcgc tgtgattacc aacgatgaga ctgggatgaa atatccactg 1860
   acatcttacg aattaacgcc gaatatggca atcattgata ccgaactgat gctgaacatg 1920
   cctcgtaaat taactgccgc gacgggcatt gacgcactgg tacacgccat cgaggcgtat 1980
   gtcagtgtta tggcaaccga ttacacagac gaactggcgt tacgcgctat taagatgatc 2040
   tttaaatatc tgccacgtgc ctacaaaaat ggtactaacg atattgaagc gcgcgagaag 2100
   atggctcatg catcaaatat cgccggaatg gcgttcgcta acgcatttct gggcgtgtgc 2160
   cacagcatgg cccataaatt aggtgcgatg caccatgtac cgcatgggat tgcttgtgca 2220
   gtcctgatcg aagaggttat taaatataat gccacggact gccctaccaa gcagacagcg 2280
   ttcccgcaat acaaatcccc aaacgctaaa cggaagtatg cagaaatcgc cgaatatctg 2340
   aatctgaaag gcacttcgga tacggagaaa gtgaccgcgt taattgaagc tatctctaag 2400
   ctgaaaattg atctgagtat cccgcagaac atttcagcag ccggtattaa taaaaaggac 2460
   ttttacaaca ccttagataa aatgagcgag ctggcgttcg acgatcaatg tacaactgct 2520
   aatcctcgtt atccgctgat ctccgaatta aaagatatct atataaaatc attttaa 2577
<210> 8
   <211> 1152
   <212> DNA
   <213> Me-bcd-co
<400> 8
   atggatttta acttaacaga tattcagcaa gacttcctga agctggcaca cgactttggt      60
   gaaaagaaac tggcccctac tgttaccgaa cgcgaccaca aaggtatcta cgataaagaa     120
   ctgattgacg aactgctgtc tctgggtatc accggcgcat acttcgaaga aaaatacggc     180
   ggtagcggtg acgacggtgg cgatgtactg tcttatatcc tggccgtaga agaactggcg     240
   aaatacgacg ctggtgttgc tatcactctg tctgccaccg taagcctgtg tgcgaatccg     300
   atttggcagt ttggtactga ggctcagaaa gaaaagtttc tggttccact ggtcgaaggt     360
   actaaactgg gtgcgtttgg tctgaccgaa ccgaacgcgg gcactgatgc gagcggccag     420
   caaactattg ctactaaaaa cgatgacggc acgtacaccc tgaacggtag caaaatcttc     480
   atcaccaacg gtggcgctgc cgatatctac atcgtatttg cgatgaccga caaaagcaag     540
   ggtaaccatg gcatcaccgc gttcatcctg gaagatggca ctccgggttt cacctacggc     600
   aaaaaggaag ataaaatggg tatccacacc tctcagacta tggaactggt tttccaggac     660
   gttaaggtcc cggccgagaa catgctgggc gaagaaggca aaggcttcaa gattgcaatg     720
   atgaccctgg acggcggtcg cattggcgtt gcggcccagg cactgggcat cgcagaggca     780
   gcgctggccg acgctgttga atacagcaaa cagcgtgttc agtttggcaa acctctgtgc     840
   aaattccaat ccattagctt taagctggcc gatatgaaaa tgcagatcga agccgcacgc     900
   aacctggtat ataaagctgc atgcaagaaa caagaaggta aaccgttcac cgtagacgct     960
   gcgatcgcga aacgtgtagc cagcgatgtg gcaatgcgcg tgactaccga agcagttcag    1020
   attttcggtg gctatggtta ctctgaagaa tacccggtgg ctcgccacat gcgcgacgca    1080
   aaaatcactc agatctacga gggtacgaac gaagtgcagc tgatggtcac cggcggtgct    1140
   ctgttaagtt aa                                                         1152
<210> 9
   <211> 1017
   <212> DNA
   <213> Me-eftA-co
<400> 9
   atggatttag cagaatacaa aggcatctac gtgatcgcag agcagttcga aggtaaactg 60
   cgtgacgttt ctttcgaact gctgggtcaa gcgcgcatcc tggcggacac gatcggcgac 120
   gaagtaggcg caatcctgat tggcaaagat gtaaaaccac tggcgcagga actgatcgcg 180
   catggtgctc ataaagtgta cgtctatgac gacccgcagc tggaacatta caacacgact 240
   gcctatgcca aagtgatttg cgacttcttt catgaagaga aaccaaacgt tttcctggtt 300
   ggtgcaacta acatcggtcg tgacctgggt ccacgtgtag cgaacagcct gaaaaccggt 360
   ctgactgcgg attgtaccca gctgggtgtt gatgatgata agaaaaccat cgtttggacc 420
   cgtccggcac tgggcggcaa catcatggcg gaaattatct gtccagataa ccgcccgcag 480
   atgggcactg tgcgtcctca tgtcttcaaa aagccggaag ccgacccgag cgcaactggt 540
   gaagtcattg aaaagaaagc gaacctgtct gacgctgatt tcatgactaa gttcgtagaa 600
   ctgatcaaac tgggtggtga aggcgttaaa atcgaggatg ccgatgttat tgttgctggt 660
   ggccgtggca tgaatagcga agagcctttt aaaaccggta tcctgaaaga gtgcgcggac 720
   gtactgggcg gtgctgtcgg tgccagccgt gccgccgtgg acgcgggctg gatcgacgct 780
   ctgcaccagg tcggccagac tggcaaaacc gttggtccga aaatctacat tgcttgtgcg 840
   attagcggtg ctatccagcc gctggcaggc atgacgggct ctgattgtat tatcgcaatt 900
   aacaaagatg aagacgcgcc tattttcaag gtgtgcgact atggcattgt gggcgatgtg 960
   ttcaaagtgc tgccactgct gactgaggcg atcaagaaac agaaaggcat tgcataa 1017
<210> 10
   <211> 813
   <212> DNA
   <213> Me-eftB-co
<400> 10
   atggaaatat tggtatgtgt caaacaagtg ccggatactg cagaagtcaa aattgatccg 60
   gttaaacaca ccgtgattcg tgcgggtgtg ccgaatatct tcaacccgtt cgaccaaaac 120
   gcgctggaag cggcgctggc gctgaaggac gcggataaag acgttaagat tactctgctg 180
   tctatgggcc cggaccaggc aaaagatgtt ctgcgtgaag gcctggccat gggcgctgat    240
   gacgcgtacc tgctgtccga tcgtaaactg ggtggctccg acactctggc caccggttat    300
   gctctggccc aggctattaa gaaactggct gcggacaagg gtattgagca attcgacatc    360
   atcctgtgtg gtaagcaagc gattgacggt gataccgctc aggtaggtcc acagatcgct    420
   tgtgagctgg gcatcccgca gatcacttat gctcgtgaca tcaaggttga gggcgataag    480
   gttactgtgc agcaggaaaa cgaagagggt tacatcgtga ccgaagcgca gttcccggtt    540
   ctgatcaccg cggttaaaga cctgaacgaa cctcgtttcc cgaccatccg tggcaccatg    600
   aaggcgaagc gtcgtgaaat cccgaacctg gacgcagctg cagttgccgc ggacgacgcg    660
   cagatcggcc tgtccggttc tccgaccaaa gtacgcaaaa ttttcacccc accgcagcgt    720
   tccggcggtc tggtactgaa agtggaagac gacaacgaac aggccattgt cgaccaggtt    780
   atggaaaaac tggttgccca gaaaatcatt taa                                 813
<210> 11
   <211> 5024
   <212> DNA
   <213> pGV1090
<400> 11
   ctagtgcttg gattctcacc aataaaaaac gcccggcggc aaccgagcgt tctgaacaaa 60
   tccagatgga gttctgaggt cattactgga tctatcaaca ggagtccaag cgagctcgat 120
   atcaaattac gccccgccct gccactcatc gcagtactgt tgtaattcat taagcattct 180
   gccgacatgg aagccatcac agacggcatg atgaacctga atcgccagcg gcatcagcac 240
   cttgtcgcct tgcgtataat atttgcccat ggtgaaaacg ggggcgaaga agttgtccat 300
   attggccacg tttaaatcaa aactggtgaa actcacccag ggattggctg agacgaaaaa 360
   catattctca ataaaccctt tagggaaata ggccaggttt tcaccgtaac acgccacatc 420
   ttgcgaatat atgtgtagaa actgccggaa atcgtcgtgg tattcactcc agagcgatga 480
   aaacgtttca gtttgctcat ggaaaacggt gtaacaaggg tgaacactat cccatatcac 540
   cagctcaccg tctttcattg ccatacgaaa ctccggatga gcattcatca ggcgggcaag 600
   aatgtgaata aaggccggat aaaacttgtg cttatttttc tttacggtct ttaaaaaggc 660
   cgtaatatcc agctgaacgg tctggttata ggtacattga gcaactgact gaaatgcctc 720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccgaa ttcaaaattg 1080
   aaggcttcaa aaatagatag gaggtaagtt tatatggatt ttaatttaac aagagaacag 1140
   gaactggtcc gtcagatggt acgtgaattt gcagaaaacg aggttaaacc gattgctgca 1200
   gagattgatg agactgaacg cttcccgatg gaaaacgtca aaaagatggg tcagtatggc 1260
   atgatgggca ttccgttctc taaagagtac ggcggtgcgg gtggcgacgt tctgtcttat 1320
   atcatcgctg tagaggaact gtccaaagta tgtggcacca cgggcgtgat cctgtccgcg 1380
   cacacctctc tgtgcgcaag cctgatcaac gaacacggca ccgaggaaca gaagcaaaaa 1440
   tacctggtcc cgctggccaa aggtgaaaag atcggtgcat acggtctgac ggaaccgaac 1500
   gcaggtacgg acagcggcgc acaacagacg gttgcggtac tggaaggcga ccactacgtt 1560
   attaacggta gcaaaatctt catcacgaac ggtggcgtgg ctgacacctt tgttatcttc 1620
   gcgatgaccg accgcactaa aggcactaaa ggtatctctg cgttcatcat cgagaagggt 1680
   ttcaagggtt tttctatcgg caaagtggaa cagaagctgg gtatccgtgc ctcctctact 1740
   accgagctgg ttttcgaaga catgattgtg ccggttgaaa atatgatcgg caaagaaggc 1800
   aaaggcttcc cgatcgctat gaaaaccctg gatggcggcc gtatcggcat tgcagcacag 1860
   gcactgggta tcgcagaagg cgctttcaac gaagcacgtg cgtacatgaa agaacgtaaa 1920
   cagtttggcc gttctctgga taaatttcaa ggcctggcgt ggatgatggc agacatggac 1980
   gtagcgattg aatctgcgcg ctacctggtc tataaagcag cttacctgaa acaggcaggt 2040
   ctgccttaca ccgttgacgc agcacgtgcg aaactgcacg cggccaacgt tgccatggat 2100
   gttaccacca aagccgtgca actgtttggc ggttacggct atactaagga ttatccggtt 2160
   gaacgtatga tgcgtgacgc gaaaatcacc gaaatctatg aaggtacttc cgaagtgcag 2220
   aaactggtca tttcaggaaa aatttttagt taattaaagg aggttaagag gatgaatata 2280
   gttgtttgtt taaaacaggt cccggacacc gcagaagttc gtattgatcc agtaaagggt 2340
   acgctgattc gcgagggcgt gccgtctatc atcaacccag atgacaagaa cgccctggaa 2400
   gaagcactgg tcctgaaaga taattacggc gctcacgtaa ctgttatctc tatgggtccg 2460
   ccgcaagcga aaaatgcgct ggttgaagct ctggcgatgg gcgctgacga ggctgttctg 2520
   ctgactgatc gtgctttcgg tggtgcggac accctggcca cttcccacac tatcgcggca 2580
   ggtatcaaga aactgaaata tgacattgtg tttgctggtc gtcaggctat tgacggtgac 2640
   acggcacagg taggcccgga aatcgccgaa cacctgggta ttccgcaggt gacctacgta 2700
   gaaaaagtag aagtagacgg tgataccctg aaaatccgca aagcatggga agatggctac 2760
   gaggtggttg aagtaaaaac cccggtactg ctgaccgcta tcaaagagct gaatgtaccg 2820
   cgttacatgt ctgttgagaa aatcttcggc gcgttcgaca aggaagtaaa gatgtggacc 2880
   gctgatgata ttgacgttga caaagcgaat ctgggcctga agggctcccc aactaaagtt 2940
   aagaagtcct ctactaaaga agtgaagggt cagggtgagg tgattgataa acctgttaaa 3000
   gaagctgctg cgtacgtggt ttctaagctg aaagaagaac actatattta agttaggagg 3060
   gatttttcaa tgaataaagc agattacaag ggcgtttggg tctttgcgga acagcgtgat 3120
   ggtgaactgc agaaagtgtc cctggaactg ctgggcaaag gcaaggagat ggcagaaaaa 3180
   ctgggtgttg aactgaccgc agttctgctg ggtcacaaca ctgaaaagat gtccaaagac 3240
   ctgctgtccc atggcgcaga caaggtgctg gctgcggaca acgaactgct ggctcacttt 3300
   agcaccgacg gttatgcaaa agtaatctgc gacctggtta acgagcgcaa gccggaaatc 3360
   ctgttcatcg gcgccacttt tattggtcgc gacctgggcc ctcgtattgc tgcgcgtctg 3420
   tccactggcc tgactgcgga ttgcacctcc ctggacattg atgttgaaaa ccgtgatctg 3480
   ctggcaactc gcccggcatt cggcggcaac ctgatcgcca ccatcgtatg ttccgaccac 3540
   cgtccgcaaa tggctactgt acgtccgggc gtatttgaaa agctgccggt gaacgacgca 3600
   aacgtttccg acgacaaaat cgaaaaagtt gctatcaagc tgaccgctag cgatatccgt 3660
   accaaagttt ctaaagtagt gaaactggcg aaggacatcg cagatattgg tgaagcaaaa 3720
   gttctggtgg caggcggtcg tggcgtcggt tccaaagaga acttcgaaaa actggaggaa 3780
   ctggcgtctc tgctgggcgg tactattgca gcgtcccgtg cagcaatcga aaaagaatgg 3840
   gtggacaagg atctgcaggt gggccagact ggtaaaaccg ttcgtccgac cctgtacatc 3900
   gcctgcggca tctccggtgc tattcagcac ctggccggca tgcaggacag cgactacatc 3960
   atcgccatca acaaagacgt tgaagctccg atcatgaaag tggcggacct ggcaatcgtt 4020
   ggtgacgtga acaaagttgt tccggaactg atcgcgcagg ttaaagctgc taataattaa 4080
   ggatcccatg gtacgcgtgc tagaggcatc aaataaaacg aaaggctcag tcgaaagact 4140
   gggcctttcg ttttatctgt tgtttgtcgg tgaacgctct cctgagtagg acaaatccgc 4200
   cgccctagac ctaggcgttc ggctgcggcg agcggtatca gctcactcaa aggcggtaat 4260
   acggttatcc acagaatcag gggataacgc aggaaagaac atgtgagcaa aaggccagca 4320
   aaaggccagg aaccgtaaaa aggccgcgtt gctggcgttt ttccataggc tccgcccccc 4380
   tgacgagcat cacaaaaatc gacgctcaag tcagaggtgg cgaaacccga caggactata 4440
   aagataccag gcgtttcccc ctggaagctc cctcgtgcgc tctcctgttc cgaccctgcc 4500
   gcttaccgga tacctgtccg cctttctccc ttcgggaagc gtggcgcttt ctcaatgctc 4560
   acgctgtagg tatctcagtt cggtgtaggt cgttcgctcc aagctgggct gtgtgcacga 4620
   accccccgtt cagcccgacc gctgcgcctt atccggtaac tatcgtcttg agtccaaccc 4680
   ggtaagacac gacttatcgc cactggcagc agccactggt aacaggatta gcagagcgag 4740
   gtatgtaggc ggtgctacag agttcttgaa gtggtggcct aactacggct acactagaag 4800
   gacagtattt ggtatctgcg ctctgctgaa gccagttacc ttcggaaaaa gagttggtag 4860
   ctcttgatcc ggcaaacaaa ccaccgctgg tagcggtggt ttttttgttt gcaagcagca 4920
   gattacgcgc agaaaaaaag gatctcaaga agatcctttg atcttttcta cggggtctga 4980
   cgctcagtgg aacgaaaact cacgttaagg gattttggtc atga 5024
<210> 12
   <211> 3206
   <212> DNA
   <213> pGV1095
<400> 12
   ctagtgcttg gattctcacc aataaaaaac gcccggcggc aaccgagcgt tctgaacaaa 60
   tccagatgga gttctgaggt cattactgga tctatcaaca ggagtccaag cgagctcgat 120
   atcaaattac gccccgccct gccactcatc gcagtactgt tgtaattcat taagcattct 180
   gccgacatgg aagccatcac agacggcatg atgaacctga atcgccagcg gcatcagcac 240
   cttgtcgcct tgcgtataat atttgcccat ggtgaaaacg ggggcgaaga agttgtccat 300
   attggccacg tttaaatcaa aactggtgaa actcacccag ggattggctg agacgaaaaa 360
   catattctca ataaaccctt tagggaaata ggccaggttt tcaccgtaac acgccacatc 420
   ttgcgaatat atgtgtagaa actgccggaa atcgtcgtgg tattcactcc agagcgatga 480
   aaacgtttca gtttgctcat ggaaaacggt gtaacaaggg tgaacactat cccatatcac 540
   cagctcaccg tctttcattg ccatacgaaa ctccggatga gcattcatca ggcgggcaag 600
   aatgtgaata aaggccggat aaaacttgtg cttatttttc tttacggtct ttaaaaaggc 660
   cgtaatatcc agctgaacgg tctggttata ggtacattga gcaactgact gaaatgcctc 720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccgaa ttcaacagga 1080
   ggggttaaag tggttgattt cgaatattca ataccaacta gaattttttt cggtaaagat 1140
   aagataaatg tacttggaag agagcttaaa aaatatggtt ctaaagtgct tatagtttat 1200
   ggtggaggaa gtataaagag aaatggaata tatgataaag ctgtaagtat acttgaaaaa 1260
   aacagtatta aattttatga acttgcagga gtagagccaa atccaagagt aactacagtt 1320
   gaaaaaggag ttaaaatatg tagagaaaat ggagttgaag tagtactagc tataggtgga 1380
   ggaagtgcaa tagattgcgc aaaggttata gcagcagcat gtgaatatga tggaaatcca 1440
   tgggatattg tgttagatgg ctcaaaaata aaaagggtgc ttcctatagc tagtatatta 1500
   accattgctg caacaggatc agaaatggat acgtgggcag taataaataa tatggataca 1560
   aacgaaaaac taattgcggc acatccagat atggctccta agttttctat attagatcca 1620
   acgtatacgt ataccgtacc taccaatcaa acagcagcag gaacagctga tattatgagt 1680
   catatatttg aggtgtattt tagtaataca aaaacagcat atttgcagga tagaatggca 1740
   gaagcgttat taagaacttg tattaaatat ggaggaatag ctcttgagaa gccggatgat 1800
   tatgaggcaa gagccaatct aatgtgggct tcaagtcttg cgataaatgg acttttaaca 1860
   tatggtaaag acactaattg gagtgtacac ttaatggaac atgaattaag tgcttattac 1920
   gacataacac acggcgtagg gcttgcaatt ttaacaccta attggatgga gtatatttta 1980
   aataatgata cagtgtacaa gtttgttgaa tatggtgtaa atgtttgggg aatagacaaa 2040
   gaaaaaaatc actatgacat agcacatcaa gcaatacaaa aaacaagaga ttactttgta 2100
   aatgtactag gtttaccatc tagactgaga gatgttggaa ttgaagaaga aaaattggac 2160
   ataatggcaa aggaatcagt aaagcttaca ggaggaacca taggaaacct aagaccagta 2220
   aacgcctccg aagtcctaca aatattcaaa aaatctgtgt aaggatccca tggtacgcgt 2280
   gctagaggca tcaaataaaa cgaaaggctc agtcgaaaga ctgggccttt cgttttatct 2340
   gttgtttgtc ggtgaacgct ctcctgagta ggacaaatcc gccgccctag acctaggcgt 2400
   tcggctgcgg cgagcggtat cagctcactc aaaggcggta atacggttat ccacagaatc 2460
   aggggataac gcaggaaaga acatgtgagc aaaaggccag caaaaggcca ggaaccgtaa 2520
   aaaggccgcg ttgctggcgt ttttccatag gctccgcccc cctgacgagc atcacaaaaa 2580
   tcgacgctca agtcagaggt ggcgaaaccc gacaggacta taaagatacc aggcgtttcc 2640
   ccctggaagc tccctcgtgc gctctcctgt tccgaccctg ccgcttaccg gatacctgtc 2700
   cgcctttctc ccttcgggaa gcgtggcgct ttctcaatgc tcacgctgta ggtatctcag 2760
   ttcggtgtag gtcgttcgct ccaagctggg ctgtgtgcac gaaccccccg ttcagcccga 2820
   ccgctgcgcc ttatccggta actatcgtct tgagtccaac ccggtaagac acgacttatc 2880
   gccactggca gcagccactg gtaacaggat tagcagagcg aggtatgtag gcggtgctac 2940
   agagttcttg aagtggtggc ctaactacgg ctacactaga aggacagtat ttggtatctg 3000
   cgctctgctg aagccagtta ccttcggaaa aagagttggt agctcttgat ccggcaaaca 3060
   aaccaccgct ggtagcggtg gtttttttgt ttgcaagcag cagattacgc gcagaaaaaa 3120
   aggatctcaa gaagatcctt tgatcttttc tacggggtct gacgctcagt ggaacgaaaa 3180
   ctcacgttaa gggattttgg tcatga 3206
<210> 13
   <211> 2836
   <212> DNA
   <213> pGV1094
<400> 13
   ctagtgcttg gattctcacc aataaaaaac gcccggcggc aaccgagcgt tctgaacaaa     60
   tccagatgga gttctgaggt cattactgga tctatcaaca ggagtccaag cgagctcgat    120
   atcaaattac gccccgccct gccactcatc gcagtactgt tgtaattcat taagcattct    180
   gccgacatgg aagccatcac agacggcatg atgaacctga atcgccagcg gcatcagcac 240
   cttgtcgcct tgcgtataat atttgcccat ggtgaaaacg ggggcgaaga agttgtccat 300
   attggccacg tttaaatcaa aactggtgaa actcacccag ggattggctg agacgaaaaa 360
   catattctca ataaaccctt tagggaaata ggccaggttt tcaccgtaac acgccacatc 420
   ttgcgaatat atgtgtagaa actgccggaa atcgtcgtgg tattcactcc agagcgatga 480
   aaacgtttca gtttgctcat ggaaaacggt gtaacaaggg tgaacactat cccatatcac 540
   cagctcaccg tctttcattg ccatacgaaa ctccggatga gcattcatca ggcgggcaag 600
   aatgtgaata aaggccggat aaaacttgtg cttatttttc tttacggtct ttaaaaaggc 660
   cgtaatatcc agctgaacgg tctggttata ggtacattga gcaactgact gaaatgcctc 720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccggg aattcctatc 1080
   tatttttgaa gccttcaatt tttcttttct ctatgaaagc tgtcattgca tccttttgat 1140
   cctctgttga aaagcattct ccaaatgctt ctgattcaaa tgctaaagca gtatcaatat 1200
   cacactgcat tcctctatta atagcctgtt tgcttaactt aacagctact ggagcattgc 1260
   tcacaatttt gtttgcaatt tcttttgctg tattcattaa ttcactaggt tctactacct 1320
   tatttacaag tccgattctt aatgcttcat ctgcctttat attttgtgca gtaaatataa 1380
   gctgctttgc catgcccatt ccaactaatc ttgaaagtct ttgtgtacca ccaaaaccag 1440
   gtgttattcc gagacctact tctggttgac caaatcttgc gttgcttgaa gctattctta 1500
   tatcacaaga catagctatt tcgcatccgc ctcctaaagc aaaaccatta acagctgcta 1560
   ttacaggctt ttcaagaagt tctaatcttc taaacacttt atttccaagt atcccgaatt 1620
   ttctaccttc aatggtattc atttccttca tctcagaaat atctgctcct gctacaaatg 1680
   atttttctcc tgctccagtt aaaattactg caagtacttc gctatcattt tcaatttcac 1740
   ctataacata atccatttct tttagtgtat cactatttaa cgcatttaat gctttaggtc 1800
   tgttaatggt aactacagca actttacctt ccttttcaag gatgacattg tttagttcca 1860
   tgactaatcc tcctaaaata ttggatccga tccgatccca tggtacgcgt gctagaggca 1920
   tcaaataaaa cgaaaggctc agtcgaaaga ctgggccttt cgttttatct gttgtttgtc 1980
   ggtgaacgct ctcctgagta ggacaaatcc gccgccctag acctaggcgt tcggctgcgg 2040
   cgagcggtat cagctcactc aaaggcggta atacggttat ccacagaatc aggggataac 2100
   gcaggaaaga acatgtgagc aaaaggccag caaaaggcca ggaaccgtaa aaaggccgcg   2160
   ttgctggcgt ttttccatag gctccgcccc cctgacgagc atcacaaaaa tcgacgctca   2220
   agtcagaggt ggcgaaaccc gacaggacta taaagatacc aggcgtttcc ccctggaagc   2280
   tccctcgtgc gctctcctgt tccgaccctg ccgcttaccg gatacctgtc cgcctttctc   2340
   ccttcgggaa gcgtggcgct ttctcaatgc tcacgctgta ggtatctcag ttcggtgtag   2400
   gtcgttcgct ccaagctggg ctgtgtgcac gaaccccccg ttcagcccga ccgctgcgcc   2460
   ttatccggta actatcgtct tgagtccaac ccggtaagac acgacttatc gccactggca   2520
   gcagccactg gtaacaggat tagcagagcg aggtatgtag gcggtgctac agagttcttg   2580
   aagtggtggc ctaactacgg ctacactaga aggacagtat ttggtatctg cgctctgctg   2640
   aagccagtta ccttcggaaa aagagttggt agctcttgat ccggcaaaca aaccaccgct   2700
   ggtagcggtg gtttttttgt ttgcaagcag cagattacgc gcagaaaaaa aggatctcaa   2760
   gaagatcctt tgatcttttc tacggggtct gacgctcagt ggaacgaaaa ctcacgttaa   2820
   gggattttgg tcatga                                                   2836
<210> 14
   <211> 2908
   <212> DNA
   <213> pGV1037
<400> 14
   ctagtgcttg gattctcacc aataaaaaac gcccggcggc aaccgagcgt tctgaacaaa      60
   tccagatgga gttctgaggt cattactgga tctatcaaca ggagtccaag cgagctcgat     120
   atcaaattac gccccgccct gccactcatc gcagtactgt tgtaattcat taagcattct     180
   gccgacatgg aagccatcac agacggcatg atgaacctga atcgccagcg gcatcagcac     240
   cttgtcgcct tgcgtataat atttgcccat ggtgaaaacg ggggcgaaga agttgtccat     300
   attggccacg tttaaatcaa aactggtgaa actcacccag ggattggctg agacgaaaaa     360
   catattctca ataaaccctt tagggaaata ggccaggttt tcaccgtaac acgccacatc     420
   ttgcgaatat atgtgtagaa actgccggaa atcgtcgtgg tattcactcc agagcgatga     480
   aaacgtttca gtttgctcat ggaaaacggt gtaacaaggg tgaacactat cccatatcac     540
   cagctcaccg tctttcattg ccatacgaaa ctccggatga gcattcatca ggcgggcaag     600
   aatgtgaata aaggccggat aaaacttgtg cttatttttc tttacggtct ttaaaaaggc     660
   cgtaatatcc agctgaacgg tctggttata ggtacattga gcaactgact gaaatgcctc     720
   aaaatgttct ttacgatgcc attgggatat atcaacggtg gtatatccag tgattttttt     780
   ctccatttta gcttccttag ctcctgaaaa tctcgataac tcaaaaaata cgcccggtag     840
   tgatcttatt tcattatggt gaaagttgga acctcttacg tgccgatcaa cgtctcattt     900
   tcgccagata tcgacgtcta agaaaccatt attatcatga cattaaccta taaaaatagg     960
   cgtatcacga ggccctttcg tcttcacctc gagaaatgtg agcggataac aattgacatt 1020
   gtgagcggat aacaagatac tgagcacatc agcaggacgc actgaccgga attcattgat 1080
   agtttcttta aatttaggga ggtctgttta atgaaaaagg tatgtgttat aggtgcaggt 1140
   actatgggtt caggaattgc tcaggcattt gcagctaaag gatttgaagt agtattaaga 1200
   gatattaaag atgaatttgt tgatagagga ttagatttta tcaataaaaa tctttctaaa 1260
   ttagttaaaa aaggaaagat agaagaagct actaaagttg aaatcttaac tagaatttcc 1320
   ggaacagttg accttaatat ggcagctgat tgcgatttag ttatagaagc agctgttgaa 1380
   agaatggata ttaaaaagca gatttttgct gacttagaca atatatgcaa gccagaaaca 1440
   attcttgcat caaatacatc atcactttca ataacagaag tggcatcagc aactaaaaga 1500
   cctgataagg ttataggtat gcatttcttt aatccagctc ctgttatgaa gcttgtagag 1560
   gtaataagag gaatagctac atcacaagaa acttttgatg cagttaaaga gacatctata 1620
   gcaataggaa aagatcctgt agaagtagca gaagcaccag gatttgttgt aaatagaata 1680
   ttaataccaa tgattaatga agcagttggt atattagcag aaggaatagc ttcagtagaa 1740
   gacatagata aagctatgaa acttggagct aatcacccaa tgggaccatt agaattaggt 1800
   gattttatag gtcttgatat atgtcttgct ataatggatg ttttatactc agaaactgga 1860
   gattctaagt atagaccaca tacattactt aagaagtatg taagagcagg atggcttgga 1920
   agaaaatcag gaaaaggttt ctacgattat tcaaaataag gatccgatcc catggtacgc 1980
   gtgctagagg catcaaataa aacgaaaggc tcagtcgaaa gactgggcct ttcgttttat 2040
   ctgttgtttg tcggtgaacg ctctcctgag taggacaaat ccgccgccct agacctaggc 2100
   gttcggctgc ggcgagcggt atcagctcac tcaaaggcgg taatacggtt atccacagaa 2160
   tcaggggata acgcaggaaa gaacatgtga gcaaaaggcc agcaaaaggc caggaaccgt 2220
   aaaaaggccg cgttgctggc gtttttccat aggctccgcc cccctgacga gcatcacaaa 2280
   aatcgacgct caagtcagag gtggcgaaac ccgacaggac tataaagata ccaggcgttt 2340
   ccccctggaa gctccctcgt gcgctctcct gttccgaccc tgccgcttac cggatacctg 2400
   tccgcctttc tcccttcggg aagcgtggcg ctttctcaat gctcacgctg taggtatctc 2460
   agttcggtgt aggtcgttcg ctccaagctg ggctgtgtgc acgaaccccc cgttcagccc 2520
   gaccgctgcg ccttatccgg taactatcgt cttgagtcca acccggtaag acacgactta 2580
   tcgccactgg cagcagccac tggtaacagg attagcagag cgaggtatgt aggcggtgct 2640
   acagagttct tgaagtggtg gcctaactac ggctacacta gaaggacagt atttggtatc 2700
   tgcgctctgc tgaagccagt taccttcgga aaaagagttg gtagctcttg atccggcaaa 2760
   caaaccaccg ctggtagcgg tggttttttt gtttgcaagc agcagattac gcgcagaaaa 2820
   aaaggatctc aagaagatcc tttgatcttt tctacggggt ctgacgctca gtggaacgaa 2880
   aactcacgtt aagggatttt ggtcatga 2908
<210> 15
   <211> 6219
   <212> DNA
   <213> pGV1031
<400> 15
   tcgcgcgttt cggtgatgac ggtgaaaacc tctgacacat gcagctcccg gagacggtca 60
   cagcttgtct gtaagcggat gccgggagca gacaagcccg tcagggcgcg tcagcgggtg 120
   ttggcgggtg tcggggctgg cttaactatg cggcatcaga gcagattgta ctgagagtgc 180
   accatatgcg gtgtgaaata ccgcacagat gcgtaaggag aaaataccgc atcaggcgcc 240
   attcgccatt caggctgcgc aactgttggg aagggcgatc ggtgcgggcc tcttcgctat 300
   tacgccagct ggcgaaaggg ggatgtgctg caaggcgatt aagttgggta acgccagggt 360
   tttcccagtc acgacgttgt aaaacgacgg ccagtgaatt cgagctcggt accatatgca 420
   taagtttaat ttttttgtta aaaaatatta aactttgtgt tttttttaac aaaatatatt 480
   gataaaaata ataatagtgg gtataattaa gttgttagag aaaacgtata aattagggat 540
   aaactatgga acttatgaaa tagattgaaa tggtttatct gttaccccgt atcaaaattt 600
   aggaggttag ttagaatgaa agaagttgta atagctagtg cagtaagaac agcgattgga 660
   tcttatggaa agtctcttaa ggatgtacca gcagtagatt taggagctac agctataaag 720
   gaagcagtta aaaaagcagg aataaaacca gaggatgtta atgaagtcat tttaggaaat 780
   gttcttcaag caggtttagg acagaatcca gcaagacagg catcttttaa agcaggatta 840
   ccagttgaaa ttccagctat gactattaat aaggtttgtg gttcaggact tagaacagtt 900
   agcttagcag cacaaattat aaaagcagga gatgctgacg taataatagc aggtggtatg 960
   gaaaatatgt ctagagctcc ttacttagcg aataacgcta gatggggata tagaatggga 1020
   aacgctaaat ttgttgatga aatgatcact gacggattgt gggatgcatt taatgattac 1080
   cacatgggaa taacagcaga aaacatagct gagagatgga acatttcaag agaagaacaa 1140
   gatgagtttg ctcttgcatc acaaaaaaaa gctgaagaag ctataaaatc aggtcaattt 1200
   aaagatgaaa tagttcctgt agtaattaaa ggcagaaagg gagaaactgt agttgataca 1260
   gatgagcacc ctagatttgg atcaactata gaaggacttg caaaattaaa acctgccttc 1320
   aaaaaagatg gaacagttac agctggtaat gcatcaggat taaatgactg tgcagcagta 1380
   cttgtaatca tgagtgcaga aaaagctaaa gagcttggag taaaaccact tgctaagata 1440
   gtttcttatg gttcagcagg agttgaccca gcaataatgg gatatggacc tttctatgca 1500
   acaaaagcag ctattgaaaa agcaggttgg acagttgatg aattagattt aatagaatca 1560
   aatgaagctt ttgcagctca aagtttagca gtagcaaaag atttaaaatt tgatatgaat 1620
   aaagtaaatg taaatggagg agctattgcc cttggtcatc caattggagc atcaggtgca 1680
   agaatactcg ttactcttgt acacgcaatg caaaaaagag atgcaaaaaa aggcttagca 1740
   actttatgta taggtggcgg acaaggaaca gcaatattgc tagaaaagtg ctagaaagga 1800
   tccagaattt aaaaggaggg attaaaatga actctaaaat aattagattt gaaaatttaa 1860
   ggtcattctt taaagatggg atgacaatta tgattggagg ttttttaaac tgtggcactc 1920
   caaccaaatt aattgatttt ttagttaatt taaatataaa gaatttaacg attataagta 1980
   atgatacatg ttatcctaat acaggtattg gtaagttaat atcaaataat caagtaaaaa 2040
   agcttattgc ttcatatata ggcagcaacc cagatactgg caaaaaactt tttaataatg 2100
   aacttgaagt agagctctct ccccaaggaa ctctagtgga aagaatacgt gcaggcggat 2160
   ctggcttagg tggtgtacta actaaaacag gtttaggaac tttgattgaa aaaggaaaga 2220
   aaaaaatatc tataaatgga acggaatatt tgttagagct acctcttaca gccgatgtag 2280
   cattaattaa aggtagtatt gtagatgagg ccggaaacac cttctataaa ggtactacta 2340
   aaaactttaa tccctatatg gcaatggcag ctaaaaccgt aatagttgaa gctgaaaatt 2400
   tagttagctg tgaaaaacta gaaaaggaaa aagcaatgac ccccggagtt cttataaatt 2460
   atatagtaaa ggagcctgca taaaatgatt aatgataaaa acctagcgaa agaaataata 2520
   gccaaaagag ttgcaagaga attaaaaaat ggtcaacttg taaacttagg tgtaggtctt 2580
   cctaccatgg ttgcagatta tataccaaaa aatttcaaaa ttactttcca atcagaaaac 2640
   ggaatagttg gaatgggcgc tagtcctaaa ataaatgagg cagataaaga tgtagtaaat 2700
   gcaggaggag actatacaac agtacttcct gacggcacat ttttcgatag ctcagtttcg 2760
   ttttcactaa tccgtggtgg tcacgtagat gttactgttt taggggctct ccaggtagat 2820
   gaaaagggta atatagccaa ttggattgtt cctggaaaaa tgctctctgg tatgggtgga 2880
   gctatggatt tagtaaatgg agctaagaaa gtaataattg caatgagaca tacaaataaa 2940
   ggtcaaccta aaattttaaa aaaatgtaca cttcccctca cggcaaagtc tcaagcaaat 3000
   ctaattgtaa cagaacttgg agtaattgag gttattaatg atggtttact tctcactgaa 3060
   attaataaaa acacaaccat tgatgaaata aggtctttaa ctgctgcaga tttactcata 3120
   tccaatgaac ttagacccat ggctgtttag aaagaattct tgatatcagg aaggtgactt 3180
   ttatgttaaa ggatgaagta attaaacaaa ttagcacgcc attaacttcg cctgcatttc 3240
   ctagaggacc ctataaattt cataatcgtg agtattttaa cattgtatat cgtacagata 3300
   tggatgctct tcgtaaagtt gtgccagagc ctttagaaat tgatgagccc ttagtcaggt 3360
   ttgaaattat ggcaatgcat gatacgagtg gacttggttg ttatacagaa agcggacagg 3420
   ctattcccgt aagctgtaat ggagttaagg gagattatct tcatatgatg tatttagata 3480
   atgagcctgc aattgcagta ggaagggaat taagtgcata tcctaaaaag ctcgggtatc 3540
   caaagctttt tgtggattca gatactttag taggaacttt agactatgga aaacttagag 3600
   ttgcgacagc tacaatgggg tacaaacata aagccttaga tgctaatgaa gcaaaggatc 3660
   aaatttgtcg ccctaattat atgttgaaaa taatacccaa ttatgatgga agccctagga 3720
   tatgtgagct tataaatgcg aaaatcacag atgttaccgt acatgaagct tggacaggac 3780
   caactcgact gcagttattt gatcacgcta tggcgccact taatgatttg ccagtaaaag 3840
   agattgtttc tagctctcac attcttgcag atataatatt gcctagagct gaagttatat 3900
   atgattatct taagtaataa aaataagagt taccttaaat ggtaactctt atttttttaa 3960
   tgtcgacctg caggcatgca agcttggcgt aatcatggtc atagctgttt cctgtgtgaa 4020
   attgttatcc gctcacaatt ccacacaaca tacgagccgg aagcataaag tgtaaagcct 4080
   ggggtgccta atgagtgagc taactcacat taattgcgtt gcgctcactg cccgctttcc 4140
   agtcgggaaa cctgtcgtgc cagctgcatt aatgaatcgg ccaacgcgcg gggagaggcg 4200
   gtttgcgtat tgggcgctct tccgcttcct cgctcactga ctcgctgcgc tcggtcgttc 4260
   ggctgcggcg agcggtatca gctcactcaa aggcggtaat acggttatcc acagaatcag 4320
   gggataacgc aggaaagaac atgtgagcaa aaggccagca aaaggccagg aaccgtaaaa 4380
   aggccgcgtt gctggcgttt ttccataggc tccgcccccc tgacgagcat cacaaaaatc 4440
   gacgctcaag tcagaggtgg cgaaacccga caggactata aagataccag gcgtttcccc 4500
   ctggaagctc cctcgtgcgc tctcctgttc cgaccctgcc gcttaccgga tacctgtccg 4560
   cctttctccc ttcgggaagc gtggcgcttt ctcatagctc acgctgtagg tatctcagtt 4620
   cggtgtaggt cgttcgctcc aagctgggct gtgtgcacga accccccgtt cagcccgacc 4680
   gctgcgcctt atccggtaac tatcgtcttg agtccaaccc ggtaagacac gacttatcgc 4740
   cactggcagc agccactggt aacaggatta gcagagcgag gtatgtaggc ggtgctacag 4800
   agttcttgaa gtggtggcct aactacggct acactagaag gacagtattt ggtatctgcg 4860
   ctctgctgaa gccagttacc ttcggaaaaa gagttggtag ctcttgatcc ggcaaacaaa 4920
   ccaccgctgg tagcggtggt ttttttgttt gcaagcagca gattacgcgc agaaaaaaag 4980
   gatctcaaga agatcctttg atcttttcta cggggtctga cgctcagtgg aacgaaaact 5040
   cacgttaagg gattttggtc atgagattat caaaaaggat cttcacctag atccttttaa 5100
   attaaaaatg aagttttaaa tcaatctaaa gtatatatga gtaaacttgg tctgacagtt 5160
   accaatgctt aatcagtgag gcacctatct cagcgatctg tctatttcgt tcatccatag 5220
   ttgcctgact ccccgtcgtg tagataacta cgatacggga gggcttacca tctggcccca 5280
   gtgctgcaat gataccgcga gacccacgct caccggctcc agatttatca gcaataaacc 5340
   agccagccgg aagggccgag cgcagaagtg gtcctgcaac tttatccgcc tccatccagt 5400
   ctattaattg ttgccgggaa gctagagtaa gtagttcgcc agttaatagt ttgcgcaacg 5460
   ttgttgccat tgctacaggc atcgtggtgt cacgctcgtc gtttggtatg gcttcattca 5520
   gctccggttc ccaacgatca aggcgagtta catgatcccc catgttgtgc aaaaaagcgg 5580
   ttagctcctt cggtcctccg atcgttgtca gaagtaagtt ggccgcagtg ttatcactca 5640
   tggttatggc agcactgcat aattctctta ctgtcatgcc atccgtaaga tgcttttctg   5700
   tgactggtga gtactcaacc aagtcattct gagaatagtg tatgcggcga ccgagttgct   5760
   cttgcccggc gtcaatacgg gataataccg cgccacatag cagaacttta aaagtgctca   5820
   tcattggaaa acgttcttcg gggcgaaaac tctcaaggat cttaccgctg ttgagatcca   5880
   gttcgatgta acccactcgt gcacccaact gatcttcagc atcttttact ttcaccagcg   5940
   tttctgggtg agcaaaaaca ggaaggcaaa atgccgcaaa aaagggaata agggcgacac   6000
   ggaaatgttg aatactcata ctcttccttt ttcaatatta ttgaagcatt tatcagggtt   6060
   attgtctcat gagcggatac atatttgaat gtatttagaa aaataaacaa ataggggttc   6120
   cgcgcacatt tccccgaaaa gtgccacctg acgtctaaga aaccattatt atcatgacat   6180
   taacctataa aaataggcgt atcacgaggc cctttcgtc                          6219
<210> 16
   <211> 2855
   <212> DNA
   <213> pGV1049
<400> 16
   ctagtgcttg gattctcacc aataaaaaac gcccggcggc aaccgagcgt tctgaacaaa 60
   tccagatgga gttctgaggt cattactgga tctatcaaca ggagtccaag cgagctcgat 120
   atcaaattac gccccgccct gccactcatc gcagtactgt tgtaattcat taagcattct 180
   gccgacatgg aagccatcac agacggcatg atgaacctga atcgccagcg gcatcagcac 240
   cttgtcgcct tgcgtataat atttgcccat ggtgaaaacg ggggcgaaga agttgtccat 300
   attggccacg tttaaatcaa aactggtgaa actcacccag ggattggctg agacgaaaaa 360
   catattctca ataaaccctt tagggaaata ggccaggttt tcaccgtaac acgccacatc 420
   ttgcgaatat atgtgtagaa actgccggaa atcgtcgtgg tattcactcc agagcgatga 480
   aaacgtttca gtttgctcat ggaaaacggt gtaacaaggg tgaacactat cccatatcac 540
   cagctcaccg tctttcattg ccatacgaaa ctccggatga gcattcatca ggcgggcaag 600
   aatgtgaata aaggccggat aaaacttgtg cttatttttc tttacggtct ttaaaaaggc 660
   cgtaatatcc agctgaacgg tctggttata ggtacattga gcaactgact gaaatgcctc 720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccgaa ttcattaaag 1080
   aggagaaagg taccaaaata agcaagtttg aaggaggtcc ttagaatgga attaaaaaat 1140
   gttattcttg aaaaagaagg gcatttagct attgttacaa tcaatagacc aaaggcatta 1200
   aatgcattga attcagaaac actaaaagat ttaaatgttg ttttagatga tttagaagca 1260
   gacaacaatg tgtatgcagt tatagttaca ggtgctggtg agaaatcttt tgttgctgga 1320
   gcagatattt cagaaatgaa agatcttaat gaagaacaag gtaaagaatt tggtatttta 1380
   ggaaacaatg tcttcagaag attagaaaaa ttggataagc cagttatcgc agctatatca 1440
   ggatttgctc ttggtggtgg atgtgaactt gctatgtcat gtgacataag aatagcttca 1500
   gttaaagcta aatttggtca accagaagca ggacttggaa taactccagg atttggtgga 1560
   actcaaagat tagctagaat tgtagggcca ggaaaagcta aagaattaat ttatacttgt 1620
   gaccttataa atgcagaaga agcttataga ataggtttag ttaataaagt agttgaatta 1680
   gaaaaattga tggaagaagc aaaagcaatg gctaacaaga ttgcagctaa tgctccaaaa 1740
   gcagttgcat attgtaaaga tgctatagac agaggaatgc aagttgatat agatgcagct 1800
   atattaatag aagcagaaga ctttggaaag tgctttgcaa cagaagatca aacagaagga 1860
   atgactgcgt tcttagaaag aagagcagaa aagaattttc aaaataaata aggatcccat 1920
   ggtacgcgtg ctagaggcat caaataaaac gaaaggctca gtcgaaagac tgggcctttc 1980
   gttttatctg ttgtttgtcg gtgaacgctc tcctgagtag gacaaatccg ccgccctaga 2040
   cctaggcgtt cggctgcggc gagcggtatc agctcactca aaggcggtaa tacggttatc 2100
   cacagaatca ggggataacg caggaaagaa catgtgagca aaaggccagc aaaaggccag 2160
   gaaccgtaaa aaggccgcgt tgctggcgtt tttccatagg ctccgccccc ctgacgagca 2220
   tcacaaaaat cgacgctcaa gtcagaggtg gcgaaacccg acaggactat aaagatacca 2280
   ggcgtttccc cctggaagct ccctcgtgcg ctctcctgtt ccgaccctgc cgcttaccgg 2340
   atacctgtcc gcctttctcc cttcgggaag cgtggcgctt tctcaatgct cacgctgtag 2400
   gtatctcagt tcggtgtagg tcgttcgctc caagctgggc tgtgtgcacg aaccccccgt 2460
   tcagcccgac cgctgcgcct tatccggtaa ctatcgtctt gagtccaacc cggtaagaca 2520
   cgacttatcg ccactggcag cagccactgg taacaggatt agcagagcga ggtatgtagg 2580
   cggtgctaca gagttcttga agtggtggcc taactacggc tacactagaa ggacagtatt 2640
   tggtatctgc gctctgctga agccagttac cttcggaaaa agagttggta gctcttgatc 2700
   cggcaaacaa accaccgctg gtagcggtgg tttttttgtt tgcaagcagc agattacgcg 2760
   cagaaaaaaa ggatctcaag aagatccttt gatcttttct acggggtctg acgctcagtg 2820
   gaacgaaaac tcacgttaag ggattttggt catga 2855
<210> 17
   <211> 2891
   <212> DNA
   <213> pGV1050
<400> 17
   ctagtgcttg gattctcacc aataaaaaac gcccggcggc aaccgagcgt tctgaacaaa      60
   tccagatgga gttctgaggt cattactgga tctatcaaca ggagtccaag cgagctcgat     120
   atcaaattac gccccgccct gccactcatc gcagtactgt tgtaattcat taagcattct     180
   gccgacatgg aagccatcac agacggcatg atgaacctga atcgccagcg gcatcagcac     240
   cttgtcgcct tgcgtataat atttgcccat ggtgaaaacg ggggcgaaga agttgtccat     300
   attggccacg tttaaatcaa aactggtgaa actcacccag ggattggctg agacgaaaaa     360
   catattctca ataaaccctt tagggaaata ggccaggttt tcaccgtaac acgccacatc     420
   ttgcgaatat atgtgtagaa actgccggaa atcgtcgtgg tattcactcc agagcgatga     480
   aaacgtttca gtttgctcat ggaaaacggt gtaacaaggg tgaacactat cccatatcac     540
   cagctcaccg tctttcattg ccatacgaaa ctccggatga gcattcatca ggcgggcaag     600
   aatgtgaata aaggccggat aaaacttgtg cttatttttc tttacggtct ttaaaaaggc     660
   cgtaatatcc agctgaacgg tctggttata ggtacattga gcaactgact gaaatgcctc     720
   aaaatgttct ttacgatgcc attgggatat atcaacggtg gtatatccag tgattttttt     780
   ctccatttta gcttccttag ctcctgaaaa tctcgataac tcaaaaaata cgcccggtag     840
   tgatcttatt tcattatggt gaaagttgga acctcttacg tgccgatcaa cgtctcattt     900
   tcgccagata tcgacgtcta agaaaccatt attatcatga cattaaccta taaaaatagg     960
   cgtatcacga ggccctttcg tcttcacctc gagaaatgtg agcggataac aattgacatt    1020
   gtgagcggat aacaagatac tgagcacatc agcaggacgc actgaccgaa ttcaaaagat    1080
   ttagaggagg aataattcat gaaaaagatt tttgtacttg gagcaggaac aatgggtgct    1140
   ggtatcgttc aagcattcgc tcaaaaaggt tgtgaagtaa ttgtaagaga cataaaggaa    1200
   gaatttgttg acagaggaat agctggaatc actaaaggat tagaaaagca agttgctaaa    1260
   ggaaaaatgt ctgaagaaga taaagaagct atactttcaa gaatttcagg aacaactgat    1320
   atgaaattag ctgctgactg tgatttagta gttgaagctg caatcgaaaa catgaaaatt    1380
   aagaaggaaa tcttcgctga attagatgga atttgtaagc cagaagcgat tttagcttca    1440
   aacacttcat ctttatcaat tactgaagtt gcttcagcta caaagagacc tgataaagtt    1500
   atcggaatgc atttctttaa tccagctcca gtaatgaagc ttgttgaaat tattaaagga    1560
   atagctactt ctcaagaaac ttttgatgct gttaaggaat tatcagttgc tattggaaaa    1620
   gaaccagtag aagttgcaga agctccagga ttcgttgtaa acagaatatt aatcccaatg    1680
   attaacgaag cttcatttat cctacaagaa ggaatagctt cagttgaaga tattgataca    1740
   gctatgaaat atggtgctaa ccatccaatg ggacctttag ctttaggaga tcttattgga    1800
   ttagacgttt gcttagctat catggatgtt ttattcactg aaacaggtga taacaagtac    1860
   agagctagca gcatattaag aaaatatgtt agagctggat ggcttggaag aaaatcagga    1920
   aaaggattct atgattattc taaataagga tcccatggta cgcgtgctag aggcatcaaa   1980
   taaaacgaaa ggctcagtcg aaagactggg cctttcgttt tatctgttgt ttgtcggtga   2040
   acgctctcct gagtaggaca aatccgccgc cctagaccta ggcgttcggc tgcggcgagc   2100
   ggtatcagct cactcaaagg cggtaatacg gttatccaca gaatcagggg ataacgcagg   2160
   aaagaacatg tgagcaaaag gccagcaaaa ggccaggaac cgtaaaaagg ccgcgttgct   2220
   ggcgtttttc cataggctcc gcccccctga cgagcatcac aaaaatcgac gctcaagtca   2280
   gaggtggcga aacccgacag gactataaag ataccaggcg tttccccctg gaagctccct   2340
   cgtgcgctct cctgttccga ccctgccgct taccggatac ctgtccgcct ttctcccttc   2400
   gggaagcgtg gcgctttctc aatgctcacg ctgtaggtat ctcagttcgg tgtaggtcgt   2460
   tcgctccaag ctgggctgtg tgcacgaacc ccccgttcag cccgaccgct gcgccttatc   2520
   cggtaactat cgtcttgagt ccaacccggt aagacacgac ttatcgccac tggcagcagc   2580
   cactggtaac aggattagca gagcgaggta tgtaggcggt gctacagagt tcttgaagtg   2640
   gtggcctaac tacggctaca ctagaaggac agtatttggt atctgcgctc tgctgaagcc   2700
   agttaccttc ggaaaaagag ttggtagctc ttgatccggc aaacaaacca ccgctggtag   2760
   cggtggtttt tttgtttgca agcagcagat tacgcgcaga aaaaaaggat ctcaagaaga   2820
   tcctttgatc ttttctacgg ggtctgacgc tcagtggaac gaaaactcac gttaagggat   2880
   tttggtcatg a                                                        2891
<210> 18
   <211> 3205
   <212> DNA
   <213> pGV1091
<400> 18
   ctagtgcttg gattctcacc aataaaaaac gcccggcggc aaccgagcgt tctgaacaaa      60
   tccagatgga gttctgaggt cattactgga tctatcaaca ggagtccaag cgagctcgat     120
   atcaaattac gccccgccct gccactcatc gcagtactgt tgtaattcat taagcattct     180
   gccgacatgg aagccatcac agacggcatg atgaacctga atcgccagcg gcatcagcac     240
   cttgtcgcct tgcgtataat atttgcccat ggtgaaaacg ggggcgaaga agttgtccat     300
   attggccacg tttaaatcaa aactggtgaa actcacccag ggattggctg agacgaaaaa     360
   catattctca ataaaccctt tagggaaata ggccaggttt tcaccgtaac acgccacatc     420
   ttgcgaatat atgtgtagaa actgccggaa atcgtcgtgg tattcactcc agagcgatga     480
   aaacgtttca gtttgctcat ggaaaacggt gtaacaaggg tgaacactat cccatatcac     540
   cagctcaccg tctttcattg ccatacgaaa ctccggatga gcattcatca ggcgggcaag     600
   aatgtgaata aaggccggat aaaacttgtg cttatttttc tttacggtct ttaaaaaggc     660
   cgtaatatcc agctgaacgg tctggttata ggtacattga gcaactgact gaaatgcctc     720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccgaa ttcattaaag 1080
   aggagaaagg taccatggca cgttttactt taccaagaga catttatcat ggagaaggag 1140
   cacttgaggc acttaaaact ttaaaaggta agaaagcttt cttagtagtt ggtggcggat 1200
   caatgaaaag atttggattt cttaaacaag ttgaagatta tttaaaagaa gcaggaatgg 1260
   aagtagaatt atttgaaggt gttgaaccag atccatcagt ggaaacagta atgaaaggcg 1320
   cagaagctat gagaaacttt gagcctgatt ggatagttgc aatgggtgga ggatcaccaa 1380
   ttgatgctgc aaaggctatg tggatattct acgaataccc agattttact tttgaacaag 1440
   cagttgttcc atttggatta ccagacctta gacaaaaagc taagtttgta gctattccat 1500
   caacaagcgg tacagctaca gaagttacag cattctcagt tatcacaaat tattcagaaa 1560
   aaattaaata tcctttagct gattttaaca taactccaga tatagcaata gttgatccag 1620
   cacttgctca aactatgcca aaaactttaa cagctcatac tggaatggat gcattaactc 1680
   acgctataga agcatacact gcatcacttc aatcaaattt ctcagatcca ttagcaatta 1740
   aagctgtaga aatggttcaa gaaaatttaa tcaaatcatt tgaaggagat aaagaagcta 1800
   gaaatctaat gcatgaagct caatgtttag ctggaatggc attttctaat gcattacttg 1860
   gaatagttca ctcaatggct cataaggttg gtgctgtatt ccatattcct catggatgtg 1920
   caaatgctat atttttacca tatgtaattg agtataacag aacaaaatgc gaaaatagat 1980
   atggagatat tgcgagagcc ttaaaattaa aaggaaacaa tgatgccgag ttaactgatt 2040
   cattaattga attaattaat ggattaaatg ataagttaga gattcctcac tcaatgaaag 2100
   agtatggagt tactgaagaa gattttaaag ctaatctttc atttatcgct cataacgcag 2160
   tattagatgc atgcacagga tcaaatccta gagaaataga tgatgctaca atggaaaaat 2220
   tatttgaatg cacatactat ggaactaaag ttaatttgta aggatcccat ggtacgcgtg 2280
   ctagaggcat caaataaaac gaaaggctca gtcgaaagac tgggcctttc gttttatctg 2340
   ttgtttgtcg gtgaacgctc tcctgagtag gacaaatccg ccgccctaga cctaggcgtt 2400
   cggctgcggc gagcggtatc agctcactca aaggcggtaa tacggttatc cacagaatca 2460
   ggggataacg caggaaagaa catgtgagca aaaggccagc aaaaggccag gaaccgtaaa 2520
   aaggccgcgt tgctggcgtt tttccatagg ctccgccccc ctgacgagca tcacaaaaat 2580
   cgacgctcaa gtcagaggtg gcgaaacccg acaggactat aaagatacca ggcgtttccc 2640
   cctggaagct ccctcgtgcg ctctcctgtt ccgaccctgc cgcttaccgg atacctgtcc 2700
   gcctttctcc cttcgggaag cgtggcgctt tctcaatgct cacgctgtag gtatctcagt 2760
   tcggtgtagg tcgttcgctc caagctgggc tgtgtgcacg aaccccccgt tcagcccgac 2820
   cgctgcgcct tatccggtaa ctatcgtctt gagtccaacc cggtaagaca cgacttatcg 2880
   ccactggcag cagccactgg taacaggatt agcagagcga ggtatgtagg cggtgctaca 2940
   gagttcttga agtggtggcc taactacggc tacactagaa ggacagtatt tggtatctgc 3000
   gctctgctga agccagttac cttcggaaaa agagttggta gctcttgatc cggcaaacaa 3060
   accaccgctg gtagcggtgg tttttttgtt tgcaagcagc agattacgcg cagaaaaaaa 3120
   ggatctcaag aagatccttt gatcttttct acggggtctg acgctcagtg gaacgaaaac 3180
   tcacgttaag ggattttggt catga 3205
<210> 19
   <211> 3449
   <212> DNA
   <213> pGV1096
<400> 19
   ctagtgcttg gattctcacc aataaaaaac gcccggcggc aaccgagcgt tctgaacaaa 60
   tccagatgga gttctgaggt cattactgga tctatcaaca ggagtccaag cgagctcgat 120
   atcaaattac gccccgccct gccactcatc gcagtactgt tgtaattcat taagcattct 180
   gccgacatgg aagccatcac agacggcatg atgaacctga atcgccagcg gcatcagcac 240
   cttgtcgcct tgcgtataat atttgcccat ggtgaaaacg ggggcgaaga agttgtccat 300
   attggccacg tttaaatcaa aactggtgaa actcacccag ggattggctg agacgaaaaa 360
   catattctca ataaaccctt tagggaaata ggccaggttt tcaccgtaac acgccacatc 420
   ttgcgaatat atgtgtagaa actgccggaa atcgtcgtgg tattcactcc agagcgatga 480
   aaacgtttca gtttgctcat ggaaaacggt gtaacaaggg tgaacactat cccatatcac 540
   cagctcaccg tctttcattg ccatacgaaa ctccggatga gcattcatca ggcgggcaag 600
   aatgtgaata aaggccggat aaaacttgtg cttatttttc tttacggtct ttaaaaaggc 660
   cgtaatatcc agctgaacgg tctggttata ggtacattga gcaactgact gaaatgcctc 720
   aaaatgttct ttacgatgcc attgggatat atcaacggtg gtatatccag tgattttttt 780
   ctccatttta gcttccttag ctcctgaaaa tctcgataac tcaaaaaata cgcccggtag 840
   tgatcttatt tcattatggt gaaagttgga acctcttacg tgccgatcaa cgtctcattt 900
   tcgccagata tcgacgtcta agaaaccatt attatcatga cattaaccta taaaaatagg 960
   cgtatcacga ggccctttcg tcttcacctc gagaaatgtg agcggataac aattgacatt 1020
   gtgagcggat aacaagatac tgagcacatc agcaggacgc actgaccggg aattcggagg 1080
   aatagttcat gaataaagac acactaatac ctacaactaa agatttaaaa gtaaaaacaa 1140
   atggtgaaaa cattaattta aagaactaca aggataattc ttcatgtttc ggagtattcg 1200
   aaaatgttga aaatgctata agcagcgctg tacacgcaca aaagatatta tcccttcatt 1260
   atacaaaaga gcaaagagaa aaaatcataa ctgagataag aaaggccgca ttacaaaata 1320
   aagaggtctt ggctacaatg attctagaag aaacacatat gggaagatat gaggataaaa 1380
   tattaaaaca tgaattggta gctaaatata ctcctggtac agaagattta actactactg 1440
   cttggtcagg tgataatggt cttacagttg tagaaatgtc tccatatggt gttataggtg 1500
   caataactcc ttctacgaat ccaactgaaa ctgtaatatg taatagcata ggcatgatag 1560
   ctgctggaaa tgctgtagta tttaacggac acccatgcgc taaaaaatgt gttgcctttg 1620
   ctgttgaaat gataaataag gcaattattt catgtggcgg tcctgaaaat ctagtaacaa 1680
   ctataaaaaa tccaactatg gagtctctag atgcaattat taagcatcct tcaataaaac 1740
   ttctttgcgg aactgggggt ccaggaatgg taaaaaccct cttaaattct ggtaagaaag 1800
   ctataggtgc tggtgctgga aatccaccag ttattgtaga tgatactgct gatatagaaa 1860
   aggctggtag gagcatcatt gaaggctgtt cttttgataa taatttacct tgtattgcag 1920
   aaaaagaagt atttgttttt gagaatgttg cagatgattt aatatctaac atgctaaaaa 1980
   ataatgctgt aattataaat gaagatcaag tatcaaaatt aatagattta gtattacaaa 2040
   aaaataatga aactcaagaa tactttataa acaaaaaatg ggtaggaaaa gatgcaaaat 2100
   tattcttaga tgaaatagat gttgagtctc cttcaaatgt taaatgcata atctgcgaag 2160
   taaatgcaaa tcatccattt gttatgacag aactcatgat gccaatattg ccaattgtaa 2220
   gagttaaaga tatagatgaa gctattaaat atgcaaagat agcagaacaa aatagaaaac 2280
   atagtgccta tatttattct aaaaatatag acaacctaaa tagatttgaa agagaaatag 2340
   atactactat ttttgtaaag aatgctaaat cttttgctgg tgttggttat gaagcagaag 2400
   gatttacaac tttcactatt gctggatcta ctggtgaggg aataacctct gcaaggaatt 2460
   ttacaagaca aagaagatgt gtacttgccg gctaaggatc cgatccgatc ccatggtacg 2520
   cgtgctagag gcatcaaata aaacgaaagg ctcagtcgaa agactgggcc tttcgtttta 2580
   tctgttgttt gtcggtgaac gctctcctga gtaggacaaa tccgccgccc tagacctagg 2640
   cgttcggctg cggcgagcgg tatcagctca ctcaaaggcg gtaatacggt tatccacaga 2700
   atcaggggat aacgcaggaa agaacatgtg agcaaaaggc cagcaaaagg ccaggaaccg 2760
   taaaaaggcc gcgttgctgg cgtttttcca taggctccgc ccccctgacg agcatcacaa 2820
   aaatcgacgc tcaagtcaga ggtggcgaaa cccgacagga ctataaagat accaggcgtt 2880
   tccccctgga agctccctcg tgcgctctcc tgttccgacc ctgccgctta ccggatacct 2940
   gtccgccttt ctcccttcgg gaagcgtggc gctttctcaa tgctcacgct gtaggtatct 3000
   cagttcggtg taggtcgttc gctccaagct gggctgtgtg cacgaacccc ccgttcagcc 3060
   cgaccgctgc gccttatccg gtaactatcg tcttgagtcc aacccggtaa gacacgactt 3120
   atcgccactg gcagcagcca ctggtaacag gattagcaga gcgaggtatg taggcggtgc   3180
   tacagagttc ttgaagtggt ggcctaacta cggctacact agaaggacag tatttggtat   3240
   ctgcgctctg ctgaagccag ttaccttcgg aaaaagagtt ggtagctctt gatccggcaa   3300
   acaaaccacc gctggtagcg gtggtttttt tgtttgcaag cagcagatta cgcgcagaaa   3360
   aaaaggatct caagaagatc ctttgatctt ttctacgggg tctgacgctc agtggaacga   3420
   aaactcacgt taagggattt tggtcatga                                     3449
<210> 20
   <211> 1425
   <212> DNA
   <213> 1pdA
<400> 20
   atgagtactg aaatcaaaac tcaggtcgtg gtacttgggg caggccccgc aggttactcc      60
   gctgccttcc gttgcgctga tttaggtctg gaaaccgtaa tcgtagaacg ttacaacacc     120
   cttggcggtg tttgcctgaa cgtcggctgt atcccttcta aagcactgct gcacgtagca     180
   aaagttatcg aagaagccaa agcgctggct gaacacggta tcgtcttcgg cgaaccgaaa     240
   accgatatcg acaagattcg tacctggaaa gagaaagtga tcaatcagct gaccggtggt     300
   ctggctggta tggcgaaagg ccgcaaagtc aaagtggtca acggtctggg taaattcacc     360
   ggggctaaca ccctggaagt tgaaggtgag aacggcaaaa ccgtgatcaa cttcgacaac     420
   gcgatcattg cagcgggttc tcgcccgatc caactgccgt ttattccgca tgaagatccg     480
   cgtatctggg actccactga cgcgctggaa ctgaaagaag taccagaacg cctgctggta     540
   atgggtggcg gtatcatcgg tctggaaatg ggcaccgttt accacgcgct gggttcacag     600
   attgacgtgg ttgaaatgtt cgaccaggtt atcccggcag ctgacaaaga catcgttaaa     660
   gtcttcacca agcgtatcag caagaaattc aacctgatgc tggaaaccaa agttaccgcc     720
   gttgaagcga aagaagacgg catttatgtg acgatggaag gcaaaaaagc acccgctgaa     780
   ccgcagcgtt acgacgccgt gctggtagcg attggtcgtg tgccgaacgg taaaaacctc     840
   gacgcaggca aagcaggcgt ggaagttgac gaccgtggtt tcatccgcgt tgacaaacag     900
   ctgcgtacca acgtaccgca catctttgct atcggcgata tcgtcggtca accgatgctg     960
   gcacacaaag gtgttcacga aggtcacgtt gccgctgaag ttatcgccgg taagaaacac    1020
   tacttcgatc cgaaagttat cccgtccatc gcctataccg aaccagaagt tgcatgggtg    1080
   ggtctgactg agaaagaagc gaaagagaaa ggcatcagct atgaaaccgc caccttcccg    1140
   tgggctgctt ctggtcgtgc tatcgcttcc gactgcgcag acggtatgac caagctgatt    1200
   ttcgacaaag aatctcaccg tgtgatcggt ggtgcgattg tcggtactaa cggcggcgag    1260
   ctgctgggtg aaatcggcct ggcaatcgaa atgggttgtg atgctgaaga catcgcactg    1320
   accatccacg cgcacccgac tctgcacgag tctgtgggcc tggcggcaga agtgttcgaa    1380
   ggtagcatta ccgacctgcc gaacccgaaa gcgaagaaga agtaa                    1425
<210> 21
   <211> 2664
   <212> DNA
   <213> *aceE*
<400> 21
   atgtcagaac gtttcccaaa tgacgtggat ccgatcgaaa ctcgcgactg gctccaggcg 60
   atcgaatcgg tcatccgtga agaaggtgtt gagcgtgctc agtatctgat cgaccaactg 120
   cttgctgaag cccgcaaagg cggtgtaaac gtagccgcag gcacaggtat cagcaactac 180
   atcaacacca tccccgttga agaacaaccg gagtatccgg gtaatctgga actggaacgc 240
   cgtattcgtt cagctatccg ctggaacgcc atcatgacgg tgctgcgtgc gtcgaaaaaa 300
   gacctcgaac tgggcggcca tatggcgtcc ttccagtctt ccgcaaccat ttatgatgtg 360
   tgctttaacc acttcttccg tgcacgcaac gagcaggatg gcggcgacct ggtttacttc 420
   cagggccaca tctccccggg cgtgtacgct cgtgctttcc tggaaggtcg tctgactcag 480
   gagcagctgg ataacttccg tcaggaagtt cacggcaatg gcctctcttc ctatccgcac 540
   ccgaaactga tgccggaatt ctggcagttc ccgaccgtat ctatgggtct gggtccgatt 600
   ggtgctattt accaggctaa attcctgaaa tatctggaac accgtggcct gaaagatacc 660
   tctaaacaaa ccgtttacgc gttcctcggt gacggtgaaa tggacgaacc ggaatccaaa 720
   ggtgcgatca ccatcgctac ccgtgaaaaa ctggataacc tggtcttcgt tatcaactgt 780
   aacctgcagc gtcttgacgg cccggtcacc ggtaacggca agatcatcaa cgaactggaa 840
   ggcatcttcg aaggtgctgg ctggaacgtg atcaaagtga tgtggggtag ccgttgggat 900
   gaactgctgc gtaaggatac cagcggtaaa ctgatccagc tgatgaacga aaccgttgac 960
   ggcgactacc agaccttcaa atcgaaagat ggtgcgtacg ttcgtgaaca cttcttcggt 1020
   aaatatcctg aaaccgcagc actggttgca gactggactg acgagcagat ctgggcactg 1080
   aaccgtggtg gtcacgatcc gaagaaaatc tacgctgcat tcaagaaagc gcaggaaacc 1140
   aaaggcaaag cgacagtaat ccttgctcat accattaaag gttacggcat gggcgacgcg 1200
   gctgaaggta aaaacatcgc gcaccaggtt aagaaaatga acatggacgg tgtgcgtcat 1260
   atccgcgacc gtttcaatgt gccggtgtct gatgcagata tcgaaaaact gccgtacatc 1320
   accttcccgg aaggttctga agagcatacc tatctgcacg ctcagcgtca gaaactgcac 1380
   ggttatctgc caagccgtca gccgaacttc accgagaagc ttgagctgcc gagcctgcaa 1440
   gacttcggcg cgctgttgga agagcagagc aaagagatct ctaccactat cgctttcgtt 1500
   cgtgctctga acgtgatgct gaagaacaag tcgatcaaag atcgtctggt accgatcatc 1560
   gccgacgaag cgcgtacttt cggtatggaa ggtctgttcc gtcagattgg tatttacagc 1620
   ccgaacggtc agcagtacac cccgcaggac cgcgagcagg ttgcttacta taaagaagac 1680
   gagaaaggtc agattctgca ggaagggatc aacgagctgg gcgcaggttg ttcctggctg 1740
   gcagcggcga cctcttacag caccaacaat ctgccgatga tcccgttcta catctattac 1800
   tcgatgttcg gcttccagcg tattggcgat ctgtgctggg cggctggcga ccagcaagcg 1860
   cgtggcttcc tgatcggcgg tacttccggt cgtaccaccc tgaacggcga aggtctgcag 1920
   cacgaagatg gtcacagcca cattcagtcg ctgactatcc cgaactgtat ctcttacgac 1980
   ccggcttacg cttacgaagt tgctgtcatc atgcatgacg gtctggagcg tatgtacggt 2040
   gaaaaacaag agaacgttta ctactacatc actacgctga acgaaaacta ccacatgccg 2100
   gcaatgccgg aaggtgctga ggaaggtatc cgtaaaggta tctacaaact cgaaactatt 2160
   gaaggtagca aaggtaaagt tcagctgctc ggctccggtt ctatcctgcg tcacgtccgt 2220
   gaagcagctg agatcctggc gaaagattac ggcgtaggtt ctgacgttta tagcgtgacc 2280
   tccttcaccg agctggcgcg tgatggtcag gattgtgaac gctggaacat gctgcacccg 2340
   ctggaaactc cgcgcgttcc gtatatcgct caggtgatga acgacgctcc ggcagtggca 2400
   tctaccgact atatgaaact gttcgctgag caggtccgta cttacgtacc ggctgacgac 2460
   taccgcgtac tgggtactga tggcttcggt cgttccgaca gccgtgagaa cctgcgtcac 2520
   cacttcgaag ttgatgcttc ttatgtcgtg gttgcggcgc tgggcgaact ggctaaacgt 2580
   ggcgaaatcg ataagaaagt ggttgctgac gcaatcgcca aattcaacat cgatgcagat 2640
   aaagttaacc cgcgtctggc gtaa 2664
<210> 22
   <211> 1893
   <212> DNA
   <213> *aceF*
<400> 22
   atggctatcg aaatcaaagt accggacatc ggggctgatg aagttgaaat caccgagatc      60
   ctggtcaaag tgggcgacaa agttgaagcc gaacagtcgc tgatcaccgt agaaggcgac     120
   aaagcctcta tggaagttcc gtctccgcag gcgggtatcg ttaaagagat caaagtctct     180
   gttggcgata aaacccagac cggcgcactg attatgattt tcgattccgc cgacggtgca     240
   gcagacgctg cacctgctca ggcagaagag aagaaagaag cagctccggc agcagcacca     300
   gcggctgcgg cggcaaaaga cgttaacgtt ccggatatcg gcagcgacga agttgaagtg     360
   accgaaatcc tggtgaaagt tggcgataaa gttgaagctg aacagtcgct gatcaccgta     420
   gaaggcgaca aggcttctat ggaagttccg gctccgtttg ctggcaccgt gaaagagatc     480
   aaagtgaacg tgggtgacaa agtgtctacc ggctcgctga ttatggtctt cgaagtcgcg     540
   ggtgaagcag gcgcggcagc tccggccgct aaacaggaag cagctccggc agcggcccct     600
   gcaccagcgg ctggcgtgaa agaagttaac gttccggata tcggcggtga cgaagttgaa     660
   gtgactgaag tgatggtgaa agtgggcgac aaagttgccg ctgaacagtc actgatcacc     720
   gtagaaggcg acaaagcttc tatggaagtt ccggcgccgt ttgcaggcgt cgtgaaggaa 780
   ctgaaagtca acgttggcga taaagtgaaa actggctcgc tgattatgat cttcgaagtt 840
   gaaggcgcag cgcctgcggc agctcctgcg aaacaggaag cggcagcgcc ggcaccggca 900
   gcaaaagctg aagccccggc agcagcacca gctgcgaaag cggaaggcaa atctgaattt 960
   gctgaaaacg acgcttatgt tcacgcgact ccgctgatcc gccgtctggc acgcgagttt 1020
   ggtgttaacc ttgcgaaagt gaagggcact ggccgtaaag gtcgtatcct gcgcgaagac 1080
   gttcaggctt acgtgaaaga agctatcaaa cgtgcagaag cagctccggc agcgactggc 1140
   ggtggtatcc ctggcatgct gccgtggccg aaggtggact tcagcaagtt tggtgaaatc 1200
   gaagaagtgg aactgggccg catccagaaa atctctggtg cgaacctgag ccgtaactgg 1260
   gtaatgatcc cgcatgttac tcacttcgac aaaaccgata tcaccgagtt ggaagcgttc 1320
   cgtaaacagc agaacgaaga agcggcgaaa cgtaagctgg atgtgaagat caccccggtt 1380
   gtcttcatca tgaaagccgt tgctgcagct cttgagcaga tgcctcgctt caatagttcg 1440
   ctgtcggaag acggtcagcg tctgaccctg aagaaataca tcaacatcgg tgtggcggtg 1500
   gataccccga acggtctggt tgttccggta ttcaaagacg tcaacaagaa aggcatcatc 1560
   gagctgtctc gcgagctgat gactatttct aagaaagcgc gtgacggtaa gctgactgcg 1620
   ggcgaaatgc agggcggttg cttcaccatc tccagcatcg gcggcctggg tactacccac 1680
   ttcgcgccga ttgtgaacgc gccggaagtg gctatcctcg gcgtttccaa gtccgcgatg 1740
   gagccggtgt ggaatggtaa agagttcgtg ccgcgtctga tgctgccgat ttctctctcc 1800
   ttcgaccacc gcgtgatcga cggtgctgat ggtgcccgtt tcattaccat cattaacaac 1860
   acgctgtctg acattcgccg tctggtgatg taa 1893
<210> 23
   <211> 1263
   <212> DNA
   <213> PDA1
<400> 23
   atgcttgctg cttcattcaa acgccaacca tcacaattgg tccgcgggtt aggagctgtt 60
   cttcgcactc ccaccaggat aggtcatgtt cgtaccatgg caactttaaa aacaactgat 120
   aagaaggccc ctgaggacat cgagggctcg gacacagtgc aaattgagtt gcctgaatct 180
   tccttcgagt cgtatatgct agagcctcca gacttgtctt atgagacttc gaaagccacc 240
   ttgttacaga tgtataaaga tatggtcatc atcagaagaa tggagatggc ttgtgacgcc 300
   ttgtacaagg ccaagaaaat cagaggtttt tgccatctat ctgttggtca ggaggccatt 360
   gctgtcggta tcgagaatgc catcacaaaa ttggattcca tcatcacatc ttacagatgt 420
   cacggtttca cttttatgag aggtgcctca gtgaaagccg ttctggctga attgatgggt 480
   agaagagccg gtgtctctta tggtaagggt ggttccatgc acctttacgc tccaggcttc 540
   tatggtggta atggtatcgt gggtgcccag gttcctttag gtgcaggttt agcttttgct 600
   caccaataca agaacgagga cgcctgctct ttcactttgt atggtgatgg tgcctctaat 660
   caaggtcaag tttttgaatc tttcaacatg gccaaattat ggaatttgcc cgtcgtgttt 720
   tgctgtgaga acaacaagta cggtatgggt accgccgctt caagatcctc cgcgatgact 780
   gaatatttca agcgtggtca atatattcca ggtttaaaag ttaacggtat ggatattcta 840
   gctgtctacc aagcatccaa gtttgctaag gactggtgtc tatccggcaa aggtcctctc 900
   gttctagaat atgaaaccta taggtacggt ggccattcta tgtctgatcc cggtactacc 960
   tacagaacta gagacgagat tcagcatatg agatccaaga acgatccaat tgctggtctt 1020
   aagatgcatt tgattgatct aggtattgcc actgaagctg aagtcaaagc ttacgacaag 1080
   tccgctagaa aatacgttga cgaacaagtt gaattagctg atgctgctcc tcctccagaa 1140
   gccaaattat ccatcttgtt tgaagacgtc tacgtgaaag gtacagaaac tccaacccta 1200
   agaggtagga tccctgaaga tacttgggac ttcaaaaagc aaggttttgc ctctagggat 1260
   taa 1263
<210> 24
   <211> 1101
   <212> DNA
   <213> PDB1
<400> 24
   atgttttcca gactgccaac atcattggcc agaaatgttg cacgtcgtgc cccaacttct 60
   tttgtaagac cctctgcagc agcagcagca ttgagattct catcaacaaa gacgatgacc 120
   gtcagagagg ccttgaatag tgccatggcg gaagaattgg accgtgatga tgatgtcttc 180
   cttattggtg aagaagttgc acaatataac ggggcttata aggtgtcaaa gggtttattg 240
   gacaggttcg gtgaacgtcg tgtggttgac acacctatta ccgaatacgg gttcacaggt 300
   ttggccgttg gtgccgcttt gaagggtttg aagccaattg tagagtttat gtcgttcaat 360
   ttctctatgc aagctatcga tcatgttgtc aattccgctg caaagactca ctacatgtct 420
   ggtggtactc aaaaatgtca aatggtcttc agaggtccta atggtgctgc agtgggtgtt 480
   ggtgctcaac attcacagga cttttctcct tggtacggtt ccattccagg gttaaaggtc 540
   cttgtccctt attctgctga agatgctagg ggtttgttaa aggccgccat cagagatcca 600
   aaccctgttg tatttttaga gaacgaattg ttgtacggtg aatcttttga aatctcagaa 660
   gaagctttat cccctgagtt caccttgcca tacaaggcta agatcgaaag agaaggtacc 720
   gatatttcca ttgttacgta cacaagaaac gttcagtttt ctttggaagc cgctgaaatt 780
   ctacaaaaga aatatggtgt ctctgcagaa gttatcaact tgcgttctat tagaccttta 840
   gatactgaag ctatcatcaa aactgtcaag aagacaaacc acttgattac tgttgaatcc 900
   actttcccat catttggtgt tggtgctgaa attgtcgccc aagttatgga gtctgaagcc 960
   tttgattact tggatgctcc aatccaaaga gttactggtg ccgatgttcc aacaccttac 1020
   gctaaagaat tagaagattt cgctttccct gatactccaa ccatcgttaa agctgtcaaa 1080
   gaagtcttgt caattgaata a 1101
<210> 25
   <211> 1233
   <212> DNA
   <213> PDX1
<400> 25
   atgctaagtg caatttccaa agtctccact ttaaaatcat gtacaagata tttaaccaaa 60
   tgcaactatc atgcatcagc taaattactt gctgtaaaga cattttcaat gcctgcaatg 120
   tctcctacta tggagaaagg ggggattgtg tcttggaaat ataaagttgg cgaaccattc 180
   agcgcgggcg atgtgatatt agaagtggaa acagataaat ctcaaattga tgtggaagca 240
   ctggacgatg gtaaactagc taagatcctg aaagatgaag gctctaaaga tgttgatgtt 300
   ggtgaaccta ttgcttatat tgctgatgtt gatgatgatt tagctactat aaagttaccc 360
   caagaggcca acaccgcaaa tgcgaaatct attgaaatta agaagccatc cgcagatagt 420
   actgaagcaa cacaacaaca tttaaaaaaa gccacagtta caccaataaa aaccgttgac 480
   ggcagccaag ccaatcttga acagacgcta ttaccatccg tgtcattact actggctgag 540
   aacaatatat ccaaacaaaa ggctttgaag gaaattgcgc catctggttc caacggtaga 600
   ctattaaagg gtgatgtgct agcataccta gggaaaatac cacaagattc ggttaacaag 660
   gtaacagaat ttatcaagaa gaacgaacgt ctcgatttat cgaacattaa acctatacag 720
   ctcaaaccaa aaatagccga gcaagctcaa acaaaagctg ccgacaagcc aaagattact 780
   cctgtagaat ttgaagagca attagtgttc catgctcccg cctctattcc gtttgacaaa 840
   ctgagtgaat cattgaactc tttcatgaaa gaagcttacc agttctcaca cggaacacca 900
   ctaatggaca caaattcgaa atactttgac cctattttcg aggaccttgt caccttgagc 960
   ccaagagagc caagatttaa attttcctat gacttgatgc aaattcccaa agctaataac 1020
   atgcaagaca cgtacggtca agaagacata tttgacctct taacaggttc agacgcgact 1080
   gcctcatcag taagacccgt tgaaaagaac ttacctgaaa aaaacgaata tatactagcg 1140
   ttgaatgtta gcgtcaacaa caagaagttt aatgacgcgg aggccaaggc aaaaagattc 1200
   cttgattacg taagggagtt agaatcattt tga 1233
<210> 26
   <211> 1449
   <212> DNA
   <213> LAT1
<400> 26
   atgtctgcct ttgtcagggt ggttccaaga atatccagaa gttcagtact caccagatca 60
   ttgagactgc aattgagatg ctacgcatcg tacccagagc acaccattat tggtatgccg 120
   gcactgtctc ctacgatgac gcaaggtaat cttgctgctt ggactaagaa ggaaggtgac    180
   caattgtctc ccggtgaagt tattgccgaa atagaaacag acaaggctca aatggacttt    240
   gagttccaag aagatggtta cttagccaag attctagttc ctgaaggtac aaaggacatt    300
   cctgtcaaca agcctattgc cgtctatgtg gaggacaaag ctgatgtgcc agcttttaag    360
   gactttaagc tggaggattc aggttctgat tcaaagacca gtacgaaggc tcagcctgcc    420
   gaaccacagg cagaaaagaa acaagaagcg ccagctgaag agaccaagac ttctgcacct    480
   gaagctaaga aatctgacgt tgctgctcct caaggtagga tttttgcctc tccacttgcc    540
   aagactatcg ccttggaaaa gggtatttct ttgaaggatg ttcacggcac tggaccccgc    600
   ggtagaatta ccaaggctga cattgagtca tatctagaaa agtcgtctaa gcagtcttct    660
   caaaccagtg gtgctgccgc cgccactcct gccgccgcta cctcaagcac tactgctggc    720
   tctgctccat cgccttcttc tacagcatca tatgaggatg ttccaatttc aaccatgaga    780
   agcatcattg gagaacgttt attgcaatct actcaaggca ttccatcata catcgtttcc    840
   tccaagatat ccatctccaa acttttgaaa ttgagacagt ccttgaacgc tacagcaaac    900
   gacaagtaca aactgtccat taatgaccta ttagtaaaag ccatcactgt tgcggctaag    960
   agggtgccag atgccaatgc ctactggtta cctaatgaga acgttatccg taaattcaag   1020
   aatgtcgatg tctcagtcgc tgttgccaca ccaacaggat tattgacacc aattgtcaag   1080
   aattgtgagg ccaagggctt gtcgcaaatc tctaacgaaa tcaaggaact agtcaagcgt   1140
   gccagaataa acaaattggc accagaggaa ttccaaggtg ggaccatttg catatccaat   1200
   atgggcatga ataatgctgt taacatgttt acttcgatta tcaacccacc acagtctaca   1260
   atcttggcca tcgctactgt tgaaagggtc gctgtggaag acgccgctgc tgagaacgga   1320
   ttctcctttg ataaccaggt taccataaca gggacctttg atcatagaac cattgatggc   1380
   gccaaaggtg cagaattcat gaaggaattg aaaactgtta ttgaaaatcc tttggaaatg   1440
   ctattgtga                                                           1449
<210> 27
   <211> 1500
   <212> DNA
   <213> LPD1
<400> 27
   atgttaagaa tcagatcact cctaaataat aagcgtgcct tttcgtccac agtcaggaca 60
   ttgaccatta acaagtcaca tgatgtagtc atcatcggtg gtggccctgc tggttacgtg 120
   gctgctatca aagctgctca attgggattt aacactgcat gtgtagaaaa aagaggcaaa 180
   ttaggcggta cctgtcttaa cgttggatgt atcccctcca aagcacttct aaataattct 240
   catttattcc accaaatgca tacggaagcg caaaagagag gtattgacgt caacggtgat 300
   atcaaaatta acgtagcaaa cttccaaaag gctaaggatg acgctgttaa gcaattaact 360
   ggaggtattg agcttctgtt caagaaaaat aaggtcacct attataaagg taatggttca 420
   ttcgaagacg aaacgaagat cagagtaact cccgttgatg ggttggaagg cactgtcaag 480
   gaagaccaca tactagatgt taagaacatc atagtcgcca cgggctctga agttacaccc 540
   ttccccggta ttgaaataga tgaggaaaaa attgtctctt caacaggtgc tctttcgtta 600
   aaggaaattc ccaaaagatt aaccatcatt ggtggaggaa tcatcggatt ggaaatgggt 660
   tcagtttact ctagattagg ctccaaggtt actgtagtag aatttcaacc tcaaattggt 720
   gcatctatgg acggcgaggt tgccaaagcc acccaaaagt tcttgaaaaa gcaaggtttg 780
   gacttcaaat taagcaccaa agttatttct gcaaagagaa acgacgacaa gaacgtcgtc 840
   gaaattgttg tagaagatac taaaacgaat aagcaagaaa atttggaagc tgaagttttg 900
   ctggttgctg ttggtagaag accttacatt gctggcttag ggctgaaaaa gattggatta 960
   gaagtagaca aaaggggacg cctagtcatt gatgaccaat ttaattccaa gttcccacac 1020
   attaaagtgg taggagatgt tacatttggt ccaatgctgg ctcacaaagc cgaagaggaa 1080
   ggtattgcag ctgtcgaaat gttgaaaact ggtcacggtc atgtcaacta taacaacatt 1140
   ccttcggtca tgtattctca cccagaagta gcatgggttg gtaaaaccga agagcaattg 1200
   aaagaagccg gcattgacta taaaattggt aagttcccct ttgcggccaa ttcaagagcc 1260
   aagaccaacc aagacactga aggtttcgtg aagattttga tcgattccaa gaccgagcgt 1320
   attttggggg ctcacattat cggtccaaat gccggtgaaa tgattgctga agctggctta 1380
   gccttagaat atggcgcttc cgcagaagat gttgctaggg tctgccatgc tcatcctact 1440
   ttgtccgaag catttaagga agctaacatg gctgcctatg ataaagctat tcattgttga 1500
<210> 28
   <211> 1692
   <212> DNA
   <213> PDC1
<400> 28
   atgtctgaaa ttactttggg taaatatttg ttcgaaagat taaagcaagt caacgttaac 60
   accgttttcg gtttgccagg tgacttcaac ttgtccttgt tggacaagat ctacgaagtt 120
   gaaggtatga gatgggctgg taacgccaac gaattgaacg ctgcttacgc cgctgatggt 180
   tacgctcgta tcaagggtat gtcttgtatc atcaccacct tcggtgtcgg tgaattgtct 240
   gctttgaacg gtattgccgg ttcttacgct gaacacgtcg gtgttttgca cgttgttggt 300
   gtcccatcca tctctgctca agctaagcaa ttgttgttgc accacacctt gggtaacggt 360
   gacttcactg ttttccacag aatgtctgcc aacatttctg aaaccactgc tatgatcact 420
   gacattgcta ccgccccagc tgaaattgac agatgtatca gaaccactta cgtcacccaa 480
   agaccagtct acttaggttt gccagctaac ttggtcgact tgaacgtccc agctaagttg 540
   ttgcaaactc caattgacat gtctttgaag ccaaacgatg ctgaatccga aaaggaagtc 600
   attgacacca tcttggcttt ggtcaaggat gctaagaacc cagttatctt ggctgatgct 660
   tgttgttcca gacacgacgt caaggctgaa actaagaagt tgattgactt gactcaattc 720
   ccagctttcg tcaccccaat gggtaagggt tccattgacg aacaacaccc aagatacggt 780
   ggtgtttacg tcggtacctt gtccaagcca gaagttaagg aagccgttga atctgctgac 840
   ttgattttgt ctgtcggtgc tttgttgtct gatttcaaca ccggttcttt ctcttactct 900
   tacaagacca agaacattgt cgaattccac tccgaccaca tgaagatcag aaacgccact 960
   ttcccaggtg tccaaatgaa attcgttttg caaaagttgt tgaccactat tgctgacgcc 1020
   gctaagggtt acaagccagt tgctgtccca gctagaactc cagctaacgc tgctgtccca 1080
   gcttctaccc cattgaagca agaatggatg tggaaccaat tgggtaactt cttgcaagaa 1140
   ggtgatgttg tcattgctga aaccggtacc tccgctttcg gtatcaacca aaccactttc 1200
   ccaaacaaca cctacggtat ctctcaagtc ttatggggtt ccattggttt caccactggt 1260
   gctaccttgg gtgctgcttt cgctgctgaa gaaattgatc caaagaagag agttatctta 1320
   ttcattggtg acggttcttt gcaattgact gttcaagaaa tctccaccat gatcagatgg 1380
   ggcttgaagc catacttgtt cgtcttgaac aacgatggtt acaccattga aaagttgatt 1440
   cacggtccaa aggctcaata caacgaaatt caaggttggg accacctatc cttgttgcca 1500
   actttcggtg ctaaggacta tgaaacccac agagtcgcta ccaccggtga atgggacaag 1560
   ttgacccaag acaagtcttt caacgacaac tctaagatca gaatgattga aatcatgttg 1620
   ccagtcttcg atgctccaca aaacttggtt gaacaagcta agttgactgc tgctaccaac 1680
   gctaagcaat aa 1692
<210> 29
   <211> 1503
   <212> DNA
   <213> ALD6
<400> 29
   atgactaagc tacactttga cactgctgaa ccagtcaaga tcacacttcc aaatggtttg 60
   acatacgagc aaccaaccgg tctattcatt aacaacaagt ttatgaaagc tcaagacggt 120
   aagacctatc ccgtcgaaga tccttccact gaaaacaccg tttgtgaggt ctcttctgcc 180
   accactgaag atgttgaata tgctatcgaa tgtgccgacc gtgctttcca cgacactgaa 240
   tgggctaccc aagacccaag agaaagaggc cgtctactaa gtaagttggc tgacgaattg 300
   gaaagccaaa ttgacttggt ttcttccatt gaagctttgg acaatggtaa aactttggcc 360
   ttagcccgtg gggatgttac cattgcaatc aactgtctaa gagatgctgc tgcctatgcc 420
   gacaaagtca acggtagaac aatcaacacc ggtgacggct acatgaactt caccacctta 480
   gagccaatcg gtgtctgtgg tcaaattatt ccatggaact ttccaataat gatgttggct 540
   tggaagatcg ccccagcatt ggccatgggt aacgtctgta tcttgaaacc cgctgctgtc 600
   acacctttaa atgccctata ctttgcttct ttatgtaaga aggttggtat tccagctggt 660
   gtcgtcaaca tcgttccagg tcctggtaga actgttggtg ctgctttgac caacgaccca 720
   agaatcagaa agctggcttt taccggttct acagaagtcg gtaagagtgt tgctgtcgac 780
   tcttctgaat ctaacttgaa gaaaatcact ttggaactag gtggtaagtc cgcccatttg 840
   gtctttgacg atgctaacat taagaagact ttaccaaatc tagtaaacgg tattttcaag 900
   aacgctggtc aaatttgttc ctctggttct agaatttacg ttcaagaagg tatttacgac 960
   gaactattgg ctgctttcaa ggcttacttg gaaaccgaaa tcaaagttgg taatccattt 1020
   gacaaggcta acttccaagg tgctatcact aaccgtcaac aattcgacac aattatgaac 1080
   tacatcgata tcggtaagaa agaaggcgcc aagatcttaa ctggtggcga aaaagttggt 1140
   gacaagggtt acttcatcag accaaccgtt ttctacgatg ttaatgaaga catgagaatt 1200
   gttaaggaag aaatttttgg accagttgtc actgtcgcaa agttcaagac tttagaagaa 1260
   ggtgtcgaaa tggctaacag ctctgaattc ggtctaggtt ctggtatcga aacagaatct 1320
   ttgagcacag gtttgaaggt ggccaagatg ttgaaggccg gtaccgtctg gatcaacaca 1380
   tacaacgatt ttgactccag agttccattc ggtggtgtta agcaatctgg ttacggtaga 1440
   gaaatgggtg aagaagtcta ccatgcatac actgaagtaa aagctgtcag aattaagttg 1500
   taa 1503
<210> 30
   <211> 2142
   <212> DNA
   <213> ACS1
<400> 30
   atgtcgccct ctgccgtaca atcatcaaaa ctagaagaac agtcaagtga aattgacaag 60
   ttgaaagcaa aaatgtccca gtctgccgcc actgcgcagc agaagaagga acatgagtat 120
   gaacatttga cttcggtcaa gatcgtgcca caacggccca tctcagatag actgcagccc 180
   gcaattgcta cccactattc tccacacttg gacgggttgc aggactatca gcgcttgcac 240
   aaggagtcta ttgaagaccc tgctaagttc ttcggttcta aagctaccca atttttaaac 300
   tggtctaagc cattcgataa ggtgttcatc ccagacccta aaacgggcag gccctccttc 360
   cagaacaatg catggttcct caacggccaa ttaaacgcct gttacaactg tgttgacaga 420
   catgccttga agactcctaa caagaaagcc attattttcg aaggtgacga gcctggccaa 480
   ggctattcca ttacctacaa ggaactactt gaagaagttt gtcaagtggc acaagtgctg 540
   acttactcta tgggcgttcg caagggcgat actgttgccg tgtacatgcc tatggtccca 600
   gaagcaatca taaccttgtt ggccatttcc cgtatcggtg ccattcactc cgtagtcttt 660
   gccgggtttt cttccaactc cttgagagat cgtatcaacg atggggactc taaagttgtc 720
   atcactacag atgaatccaa cagaggtggt aaagtcattg agactaaaag aattgttgat 780
   gacgcgctaa gagagacccc aggcgtgaga cacgtcttgg tttatagaaa gaccaacaat    840
   ccatctgttg ctttccatgc ccccagagat ttggattggg caacagaaaa gaagaaatac    900
   aagacctact atccatgcac acccgttgat tctgaggatc cattattctt gttgtatacg    960
   tctggttcta ctggtgcccc caagggtgtt caacattcta ccgcaggtta cttgctggga   1020
   gctttgttga ccatgcgcta cacttttgac actcaccaag aagacgtttt cttcacagct   1080
   ggagacattg gctggattac aggccacact tatgtggttt atggtccctt actatatggt   1140
   tgtgccactt tggtctttga agggactcct gcgtacccaa attactcccg ttattgggat   1200
   attattgatg aacacaaagt cacccaattt tatgttgcgc caactgcttt gcgtttgttg   1260
   aaaagagctg gtgattccta catcgaaaat cattccttaa aatctttgcg ttgcttgggt   1320
   tcggtcggtg agccaattgc tgctgaagtt tgggagtggt actctgaaaa aataggtaaa   1380
   aatgaaatcc ccattgtaga cacctactgg caaacagaat ctggttcgca tctggtcacc   1440
   ccgctggctg gtggtgttac accaatgaaa ccgggttctg cctcattccc cttcttcggt   1500
   attgatgcag ttgttcttga ccctaacact ggtgaagaac ttaacaccag ccacgcagag   1560
   ggtgtccttg ccgtcaaagc tgcatggcca tcatttgcaa gaactatttg gaaaaatcat   1620
   gataggtatc tagacactta tttgaaccct taccctggct actatttcac tggtgatggt   1680
   gctgcaaagg ataaggatgg ttatatctgg attttgggtc gtgtagacga tgtggtgaac   1740
   gtctctggtc accgtctgtc taccgctgaa attgaggctg ctattatcga agatccaatt   1800
   gtggccgagt gtgctgttgt cggattcaac gatgacttga ctggtcaagc agttgctgca   1860
   tttgtggtgt tgaaaaacaa atctagttgg tccaccgcaa cagatgatga attacaagat   1920
   atcaagaagc atttggtctt tactgttaga aaagacatcg ggccatttgc cgcaccaaaa   1980
   ttgatcattt tagtggatga cttgcccaag acaagatccg gcaaaattat gagacgtatt   2040
   ttaagaaaaa tcctagcagg agaaagtgac caactaggcg acgtttctac attgtcaaac   2100
   cctggcattg ttagacatct aattgattcg gtcaagttgt aa                      2142
<210> 31
   <211> 2052
   <212> DNA
   <213> ACS2
<400> 31
   atgacaatca aggaacataa agtagtttat gaagctcaca acgtaaaggc tcttaaggct     60
   cctcaacatt tttacaacag ccaacccggc aagggttacg ttactgatat gcaacattat    120
   caagaaatgt atcaacaatc tatcaatgag ccagaaaaat tctttgataa gatggctaag    180
   gaatacttgc attgggatgc tccatacacc aaagttcaat ctggttcatt gaacaatggt    240
   gatgttgcat ggtttttgaa cggtaaattg aatgcatcat acaattgtgt tgacagacat    300
   gcctttgcta atcccgacaa gccagctttg atctatgaag ctgatgacga atccgacaac    360
   aaaatcatca catttggtga attactcaga aaagtttccc aaatcgctgg tgtcttaaaa 420
   agctggggcg ttaagaaagg tgacacagtg gctatctatt tgccaatgat tccagaagcg 480
   gtcattgcta tgttggctgt ggctcgtatt ggtgctattc actctgttgt ctttgctggg 540
   ttctccgctg gttcgttgaa agatcgtgtc gttgacgcta attctaaagt ggtcatcact 600
   tgtgatgaag gtaaaagagg tggtaagacc atcaacacta aaaaaattgt tgacgaaggt 660
   ttgaacggag tcgatttggt ttcccgtatc ttggttttcc aaagaactgg tactgaaggt 720
   attccaatga aggccggtag agattactgg tggcatgagg aggccgctaa gcagagaact 780
   tacctacctc ctgtttcatg tgacgctgaa gatcctctat ttttattata cacttccggt 840
   tccactggtt ctccaaaggg tgtcgttcac actacaggtg gttatttatt aggtgccgct 900
   ttaacaacta gatacgtttt tgatattcac ccagaagatg ttctcttcac tgccggtgac 960
   gtcggctgga tcacgggtca cacctatgct ctatatggtc cattaacctt gggtaccgcc 1020
   tcaataattt tcgaatccac tcctgcctac ccagattatg gtagatattg gagaattatc 1080
   caacgtcaca aggctaccca tttctatgtg gctccaactg ctttaagatt aatcaaacgt 1140
   gtaggtgaag ccgaaattgc caaatatgac acttcctcat tacgtgtctt gggttccgtc 1200
   ggtgaaccaa tctctccaga cttatgggaa tggtatcatg aaaaagtggg taacaaaaac 1260
   tgtgtcattt gtgacactat gtggcaaaca gagtctggtt ctcatttaat tgctcctttg 1320
   gcaggtgctg tcccaacaaa acctggttct gctaccgtgc cattctttgg tattaacgct 1380
   tgtatcattg accctgttac aggtgtggaa ttagaaggta atgatgtcga aggtgtcctt 1440
   gccgttaaat caccatggcc atcaatggct agatctgttt ggaaccacca cgaccgttac 1500
   atggatactt acttgaaacc ttatcctggt cactatttca caggtgatgg tgctggtaga 1560
   gatcatgatg gttactactg gatcaggggt agagttgacg acgttgtaaa tgtttccggt 1620
   catagattat ccacatcaga aattgaagca tctatctcaa atcacgaaaa cgtctcggaa 1680
   gctgctgttg tcggtattcc agatgaattg accggtcaaa ccgtcgttgc atatgtttcc 1740
   ctaaaagatg gttatctaca aaacaacgct actgaaggtg atgcagaaca catcacacca 1800
   gataatttac gtagagaatt gatcttacaa gttaggggtg agattggtcc tttcgcctca 1860
   ccaaaaacca ttattctagt tagagatcta ccaagaacaa ggtcaggaaa gattatgaga 1920
   agagttctaa gaaaggttgc ttctaacgaa gccgaacagc taggtgacct aactactttg 1980
   gccaacccag aagttgtacc tgccatcatt tctgctgtag agaaccaatt tttctctcaa 2040
   aaaaagaaat aa 2052
<210> 32
   <211> 5206
   <212> DNA
   <213> pGV1428
<400> 32
   ccataacaca gtcctttccc gcaattttct ttttctatta ctcttggcct cctctagtac 60
   actctatatt tttttatgcc tcggtaatga ttttcatttt tttttttccc ctagcggatg 120
   actctttttt tttcttagcg attggcatta tcacataatg aattatacat tatataaagt 180
   aatgtgattt cttcgaagaa tatactaaaa aatgagcagg caagataaac gaaggcaaag 240
   atgacagagc agaaagccct agtaaagcgt attacaaatg aaaccaagat tcagattgcg 300
   atctctttaa agggtggtcc cctagcgata gagcactcga tcttcccaga aaaagaggca 360
   gaagcagtag cagaacaggc cacacaatcg caagtgatta acgtccacac aggtataggg 420
   tttctggacc atatgataca tgctctggcc aagcattccg gctggtcgct aatcgttgag 480
   tgcattggtg acttacacat agacgaccat cacaccactg aagactgcgg gattgctctc 540
   ggtcaagctt ttaaagaggc cctaggggcc gtgcgtggag taaaaaggtt tggatcagga 600
   tttgcgcctt tggatgaggc actttccaga gcggtggtag atctttcgaa caggccgtac 660
   gcagttgtcg aacttggttt gcaaagggag aaagtaggag atctctcttg cgagatgatc 720
   ccgcattttc ttgaaagctt tgcagaggct agcagaatta ccctccacgt tgattgtctg 780
   cgaggcaaga atgatcatca ccgtagtgag agtgcgttca aggctcttgc ggttgccata 840
   agagaagcca cctcgcccaa tggtaccaac gatgttccct ccaccaaagg tgttcttatg 900
   tagtgacacc gattatttaa agctgcagca tacgatatat atacatgtgt atatatgtat 960
   acctatgaat gtcagtaagt atgtatacga acagtatgat actgaagatg acaaggtaat 1020
   gcatcattct atacgtgtca ttctgaacga ggcgacgtcg ccggcgatca cagcggacgg 1080
   tggtggcatg atggggcttg cgatgctatg tttgtttgtt ttgtgatgat gtatattatt 1140
   attgaaaaac gatatcagac atttgtctga taatgcttca ttatcagaca aatgtctgat 1200
   atcgtttgga gaaaaagaaa aggaaaacaa actaaatatc tactatatac cactgtattt 1260
   tatactaatg actttctacg cctagtgtca ccctctcgtg tacccattga ccctgtatcg 1320
   gcgcgttgcc tcgcgttcct gtaccatata tttttgttta tttaggtatt aaaatttact 1380
   ttcctcatac aaatattaaa ttcaccaaac ttctcaaaaa ctaattattc gtagttacaa 1440
   actctatttt acaatcacgt ttattcaacc attctacatc caataaccaa aatgcccatg 1500
   tacctctcag cgaagtccaa cggtactgtc caatattctc attaaatagt ctttcatcta 1560
   tatatcagaa ggtaattata attagagatt tcgaatcatt accgtgccga ttcgcacgct 1620
   gcaacggcat gcatcactaa tgaaaagcat acgacgcctg cgtctgacat gcactcattc 1680
   tgaagaagat tctgggcgcg tttcgttctc gttttcctct gtatattgta ctctggtgga 1740
   caatttgaac ataacgtctt tcacctcgcc attctcaata atgggttcca attctatcca 1800
   ggtagcggtt aattgacggt gcttaagccg tatgctcact ctaacgctac cgttgtccaa 1860
   acaacggacc cctttgtgac gggtgtaaga cccatcatga agtaaaacat ctctaacggt 1920
   atggaaaaga gtggtacggt caagtttcct ggcacgagtc aattttccct cttcgtgtag 1980
   atcggtaccg gccgcaaatt aaagccttcg agcgtcccaa aaccttctca agcaaggttt 2040
   tcagtataat gttacatgcg tacacgcgtc tgtacagaaa aaaaagaaaa atttgaaata 2100
   taaataacgt tcttaatact aacataacta taaaaaaata aatagggacc tagacttcag 2160
   gttgtctaac tccttccttt tcggttagag cggatgtggg gggagggcgt gaatgtaagc 2220
   gtgacataac taattacatg actcgagcgg ccgcggatcc cgggaattcg tcgacaccat 2280
   cttcttctga gatgagtttt tgttccatgc tagttctaga atccgtcgaa actaagttct 2340
   ggtgttttaa aactaaaaaa aagactaact ataaaagtag aatttaagaa gtttaagaaa 2400
   tagatttaca gaattacaat caatacctac cgtctttata tacttattag tcaagtaggg 2460
   gaataatttc agggaactgg tttcaacctt ttttttcagc tttttccaaa tcagagagag 2520
   cagaaggtaa tagaaggtgt aagaaaatga gatagataca tgcgtgggtc aattgccttg 2580
   tgtcatcatt tactccaggc aggttgcatc actccattga ggttgtgccc gttttttgcc 2640
   tgtttgtgcc cctgttctct gtagttgcgc taagagaatg gacctatgaa ctgatggttg 2700
   gtgaagaaaa caatattttg gtgctgggat tctttttttt tctggatgcc agcttaaaaa 2760
   gcgggctcca ttatatttag tggatgccag gaataaactg ttcacccaga cacctacgat 2820
   gttatatatt ctgtgtaacc cgccccctat tttgggcatg tacgggttac agcagaatta 2880
   aaaggctaat tttttgacta aataaagtta ggaaaatcac tactattaat tatttacgta 2940
   ttctttgaaa tggcgagtat tgataatgat aaactgagct agatctgggc ccgagctcca 3000
   gcttttgttc cctttagtga gggttaattg cgcgcttggc gtaatcatgg tcatagctgt 3060
   ttcctgtgtg aaattgttat ccgctcacaa ttccacacaa cataggagcc ggaagcataa 3120
   agtgtaaagc ctggggtgcc taatgagtga ggtaactcac attaattgcg ttgcgctcac 3180
   tgcccgcttt ccagtcggga aacctgtcgt gccagctgca ttaatgaatc ggccaacgcg 3240
   cggggagagg cggtttgcgt attgggcgct cttccgcttc ctcgctcact gactcgctgc 3300
   gctcggtcgt tcggctgcgg cgagcggtat cagctcactc aaaggcggta atacggttat 3360
   ccacagaatc aggggataac gcaggaaaga acatgtgagc aaaaggccag caaaaggcca 3420
   ggaaccgtaa aaaggccgcg ttgctggcgt ttttccatag gctccgcccc cctgacgagc 3480
   atcacaaaaa tcgacgctca agtcagaggt ggcgaaaccc gacaggacta taaagatacc 3540
   aggcgtttcc ccctggaagc tccctcgtgc gctctcctgt tccgaccctg ccgcttaccg 3600
   gatacctgtc cgcctttctc ccttcgggaa gcgtggcgct ttctcatagc tcacgctgta 3660
   ggtatctcag ttcggtgtag gtcgttcgct ccaagctggg ctgtgtgcac gaaccccccg 3720
   ttcagcccga ccgctgcgcc ttatccggta actatcgtct tgagtccaac ccggtaagac 3780
   acgacttatc gccactggca gcagccactg gtaacaggat tagcagagcg aggtatgtag 3840
   gcggtgctac agagttcttg aagtggtggc ctaactacgg ctacactaga aggacagtat 3900
   ttggtatctg cgctctgctg aagccagtta ccttcggaaa aagagttggt agctcttgat 3960
   ccggcaaaca aaccaccgct ggtagcggtg gtttttttgt ttgcaagcag cagattacgc 4020
   gcagaaaaaa aggatctcaa gaagatcctt tgatcttttc tacggggtct gacgctcagt 4080
   ggaacgaaaa ctcacgttaa gggattttgg tcatgagatt atcaaaaagg atcttcacct 4140
   agatcctttt aaattaaaaa tgaagtttta aatcaatcta aagtatatat gagtaaactt 4200
   ggtctgacag ttaccaatgc ttaatcagtg aggcacctat ctcagcgatc tgtctatttc 4260
   gttcatccat agttgcctga ctccccgtcg tgtagataac tacgatacgg gagggcttac 4320
   catctggccc cagtgctgca atgataccgc gagacccacg ctcaccggct ccagatttat 4380
   cagcaataaa ccagccagcc ggaagggccg agcgcagaag tggtcctgca actttatccg 4440
   cctccatcca gtctattaat tgttgccggg aagctagagt aagtagttcg ccagttaata 4500
   gtttgcgcaa cgttgttgcc attgctacag gcatcgtggt gtcacgctcg tcgtttggta 4560
   tggcttcatt cagctccggt tcccaacgat caaggcgagt tacatgatcc cccatgttgt 4620
   gcaaaaaagc ggttagctcc ttcggtcctc cgatcgttgt cagaagtaag ttggccgcag 4680
   tgttatcact catggttatg gcagcactgc ataattctct tactgtcatg ccatccgtaa 4740
   gatgcttttc tgtgactggt gagtactcaa ccaagtcatt ctgagaatag tgtatgcggc 4800
   gaccgagttg ctcttgcccg gcgtcaatac gggataatac cgcgccacat agcagaactt 4860
   taaaagtgct catcattgga aaacgttctt cggggcgaaa actctcaagg atcttaccgc 4920
   tgttgagatc cagttcgatg taacccactc gtgcacccaa ctgatcttca gcatctttta 4980
   ctttcaccag cgtttctggg tgagcaaaaa caggaaggca aaatgccgca aaaaagggaa 5040
   taagggcgac acggaaatgt tgaatactca tactcttcct ttttcaatat tattgaagca 5100
   tttatcaggg ttattgtctc atgagcggat acatatttga atgtatttag aaaaataaac 5160
   aaataggggt tccgcgcaca tttccccgaa aagtgccacc tgacgt 5206
<210> 33
   <211> 5157
   <212> DNA
   <213> pGV1429
<400> 33
   caggcaagtg cacaaacaat acttaaataa atactactca gtaataacct atttcttagc    60
   atttttgacg aaatttgcta ttttgttaga gtcttttaca ccatttgtct ccacacctcc   120
   gcttacatca acaccaataa cgccatttaa tctaagcgca tcaccaacat tttctggcgt   180
   cagtccacca gctaacataa aatgtaagct ttcggggctc tcttgccttc caacccagtc   240
   agaaatcgag ttccaatcca aaagttcacc tgtcccacct gcttctgaat caaacaaggg   300
   aataaacgaa tgaggtttct gtgaagctgc actgagtagt atgttgcagt cttttggaaa   360
   tacgagtctt ttaataactg gcaaaccgag gaactcttgg tattcttgcc acgactcatc   420
   tccatgcagt tggacgatat caatgccgta atcattgacc agagccaaaa catcctcctt 480
   aggttgatta cgaaacacgc caaccaagta tttcggagtg cctgaactat ttttatatgc 540
   ttttacaaga cttgaaattt tccttgcaat aaccgggtca attgttctct ttctattggg 600
   cacacatata atacccagca agtcagcatc ggaatctaga gcacattctg cggcctctgt 660
   gctctgcaag ccgcaaactt tcaccaatgg accagaacta cctgtgaaat taataacaga 720
   catactccaa gctgcctttg tgtgcttaat cacgtatact cacgtgctca atagtcacca 780
   atgccctccc tcttggccct ctccttttct tttttcgacc gaattaattc ttaatcggca 840
   aaaaaagaaa agctccggat caagattgta cgtaaggtga caagctattt ttcaataaag 900
   aatatcttcc actactgcca tctggcgtca taactgcaaa gtacacatat attacgatgc 960
   tgtctattaa atgcttccta tattatatat atagtaatgt cgttgacgtc gccggcgatc 1020
   acagcggacg gtggtggcat gatggggctt gcgatgctat gtttgtttgt tttgtgatga 1080
   tgtatattat tattgaaaaa cgatatcaga catttgtctg ataatgcttc attatcagac 1140
   aaatgtctga tatcgtttgg agaaaaagaa aaggaaaaca aactaaatat ctactatata 1200
   ccactgtatt ttatactaat gactttctac gcctagtgtc accctctcgt gtacccattg 1260
   accctgtatc ggcgcgttgc ctcgcgttcc tgtaccatat atttttgttt atttaggtat 1320
   taaaatttac tttcctcata caaatattaa attcaccaaa cttctcaaaa actaattatt 1380
   cgtagttaca aactctattt tacaatcacg tttattcaac cattctacat ccaataacca 1440
   aaatgcccat gtacctctca gcgaagtcca acggtactgt ccaatattct cattaaatag 1500
   tctttcatct atatatcaga aggtaattat aattagagat ttcgaatcat taccgtgccg 1560
   attcgcacgc tgcaacggca tgcatcacta atgaaaagca tacgacgcct gcgtctgaca 1620
   tgcactcatt ctgaagaaga ttctgggcgc gtttcgttct cgttttcctc tgtatattgt 1680
   actctggtgg acaatttgaa cataacgtct ttcacctcgc cattctcaat aatgggttcc 1740
   aattctatcc aggtagcggt taattgacgg tgcttaagcc gtatgctcac tctaacgcta 1800
   ccgttgtcca aacaacggac ccctttgtga cgggtgtaag acccatcatg aagtaaaaca 1860
   tctctaacgg tatggaaaag agtggtacgg tcaagtttcc tggcacgagt caattttccc 1920
   tcttcgtgta gatcggtacc ggccgcaaat taaagccttc gagcgtccca aaaccttctc 1980
   aagcaaggtt ttcagtataa tgttacatgc gtacacgcgt ctgtacagaa aaaaaagaaa 2040
   aatttgaaat ataaataacg ttcttaatac taacataact ataaaaaaat aaatagggac 2100
   ctagacttca ggttgtctaa ctccttcctt ttcggttaga gcggatgtgg ggggagggcg 2160
   tgaatgtaag cgtgacataa ctaattacat gactcgagcg gccgcggatc ccgggaattc 2220
   gtcgacacca tcttcttctg agatgagttt ttgttccatg ctagttctag aatccgtcga 2280
   aactaagttc tggtgtttta aaactaaaaa aaagactaac tataaaagta gaatttaaga 2340
   agtttaagaa atagatttac agaattacaa tcaataccta ccgtctttat atacttatta 2400
   gtcaagtagg ggaataattt cagggaactg gtttcaacct tttttttcag ctttttccaa 2460
   atcagagaga gcagaaggta atagaaggtg taagaaaatg agatagatac atgcgtgggt 2520
   caattgcctt gtgtcatcat ttactccagg caggttgcat cactccattg aggttgtgcc 2580
   cgttttttgc ctgtttgtgc ccctgttctc tgtagttgcg ctaagagaat ggacctatga 2640
   actgatggtt ggtgaagaaa acaatatttt ggtgctggga ttcttttttt ttctggatgc 2700
   cagcttaaaa agcgggctcc attatattta gtggatgcca ggaataaact gttcacccag 2760
   acacctacga tgttatatat tctgtgtaac ccgcccccta ttttgggcat gtacgggtta 2820
   cagcagaatt aaaaggctaa ttttttgact aaataaagtt aggaaaatca ctactattaa 2880
   ttatttacgt attctttgaa atggcgagta ttgataatga taaactgagc tagatctggg 2940
   cccgagctcc agcttttgtt ccctttagtg agggttaatt gcgcgcttgg cgtaatcatg 3000
   gtcatagctg tttcctgtgt gaaattgtta tccgctcaca attccacaca acataggagc 3060
   cggaagcata aagtgtaaag cctggggtgc ctaatgagtg aggtaactca cattaattgc 3120
   gttgcgctca ctgcccgctt tccagtcggg aaacctgtcg tgccagctgc attaatgaat 3180
   cggccaacgc gcggggagag gcggtttgcg tattgggcgc tcttccgctt cctcgctcac 3240
   tgactcgctg cgctcggtcg ttcggctgcg gcgagcggta tcagctcact caaaggcggt 3300
   aatacggtta tccacagaat caggggataa cgcaggaaag aacatgtgag caaaaggcca 3360
   gcaaaaggcc aggaaccgta aaaaggccgc gttgctggcg tttttccata ggctccgccc 3420
   ccctgacgag catcacaaaa atcgacgctc aagtcagagg tggcgaaacc cgacaggact 3480
   ataaagatac caggcgtttc cccctggaag ctccctcgtg cgctctcctg ttccgaccct 3540
   gccgcttacc ggatacctgt ccgcctttct cccttcggga agcgtggcgc tttctcatag 3600
   ctcacgctgt aggtatctca gttcggtgta ggtcgttcgc tccaagctgg gctgtgtgca 3660
   cgaacccccc gttcagcccg accgctgcgc cttatccggt aactatcgtc ttgagtccaa 3720
   cccggtaaga cacgacttat cgccactggc agcagccact ggtaacagga ttagcagagc 3780
   gaggtatgta ggcggtgcta cagagttctt gaagtggtgg cctaactacg gctacactag 3840
   aaggacagta tttggtatct gcgctctgct gaagccagtt accttcggaa aaagagttgg 3900
   tagctcttga tccggcaaac aaaccaccgc tggtagcggt ggtttttttg tttgcaagca 3960
   gcagattacg cgcagaaaaa aaggatctca agaagatcct ttgatctttt ctacggggtc 4020
   tgacgctcag tggaacgaaa actcacgtta agggattttg gtcatgagat tatcaaaaag 4080
   gatcttcacc tagatccttt taaattaaaa atgaagtttt aaatcaatct aaagtatata 4140
   tgagtaaact tggtctgaca gttaccaatg cttaatcagt gaggcaccta tctcagcgat 4200
   ctgtctattt cgttcatcca tagttgcctg actccccgtc gtgtagataa ctacgatacg 4260
   ggagggctta ccatctggcc ccagtgctgc aatgataccg cgagacccac gctcaccggc 4320
   tccagattta tcagcaataa accagccagc cggaagggcc gagcgcagaa gtggtcctgc 4380
   aactttatcc gcctccatcc agtctattaa ttgttgccgg gaagctagag taagtagttc 4440
   gccagttaat agtttgcgca acgttgttgc cattgctaca ggcatcgtgg tgtcacgctc 4500
   gtcgtttggt atggcttcat tcagctccgg ttcccaacga tcaaggcgag ttacatgatc 4560
   ccccatgttg tgcaaaaaag cggttagctc cttcggtcct ccgatcgttg tcagaagtaa 4620
   gttggccgca gtgttatcac tcatggttat ggcagcactg cataattctc ttactgtcat 4680
   gccatccgta agatgctttt ctgtgactgg tgagtactca accaagtcat tctgagaata 4740
   gtgtatgcgg cgaccgagtt gctcttgccc ggcgtcaata cgggataata ccgcgccaca 4800
   tagcagaact ttaaaagtgc tcatcattgg aaaacgttct tcggggcgaa aactctcaag 4860
   gatcttaccg ctgttgagat ccagttcgat gtaacccact cgtgcaccca actgatcttc 4920
   agcatctttt actttcacca gcgtttctgg gtgagcaaaa acaggaaggc aaaatgccgc 4980
   aaaaaaggga ataagggcga cacggaaatg ttgaatactc atactcttcc tttttcaata 5040
   ttattgaagc atttatcagg gttattgtct catgagcgga tacatatttg aatgtattta 5100
   gaaaaataaa caaatagggg ttccgcgcac atttccccga aaagtgccac ctgacgt 5157
<210> 34
   <211> 6041
   <212> DNA
   <213> pGV1430
<400> 34
   ccagttaact gtgggaatac tcaggtatcg taagatgcaa gagttcgaat ctcttagcaa 60
   ccattatttt tttcctcaac ataacgagaa cacacagggg cgctatcgca cagaatcaaa 120
   ttcgatgact ggaaattttt tgttaatttc agaggtcgcc tgacgcatat acctttttca 180
   actgaaaaat tgggagaaaa aggaaaggtg agagcgccgg aaccggcttt tcatatagaa 240
   tagagaagcg ttcatgacta aatgcttgca tcacaatact tgaagttgac aatattattt 300
   aaggacctat tgttttttcc aataggtggt tagcaatcgt cttactttct aacttttctt 360
   accttttaca tttcagcaat atatatatat atatttcaag gatataccat tctaatgtct 420
   gcccctaaga agatcgtcgt tttgccaggt gaccacgttg gtcaagaaat cacagccgaa 480
   gccattaagg ttcttaaagc tatttctgat gttcgttcca atgtcaagtt cgatttcgaa 540
   aatcatttaa ttggtggtgc tgctatcgat gctacaggtg ttccacttcc agatgaggcg 600
   ctggaagcct ccaagaaggc tgatgccgtt ttgttaggtg ctgtgggtgg tcctaaatgg 660
   ggtaccggta gtgttagacc tgaacaaggt ttactaaaaa tccgtaaaga acttcaattg 720
   tacgccaact taagaccatg taactttgca tccgactctc ttttagactt atctccaatc 780
   aagccacaat ttgctaaagg tactgacttc gttgttgtca gagaattagt gggaggtatt 840
   tactttggta agagaaagga agacgatggt gatggtgtcg cttgggatag tgaacaatac 900
   accgttccag aagtgcaaag aatcacaaga atggccgctt tcatggccct acaacatgag 960
   ccaccattgc ctatttggtc cttggataaa gctaatgttt tggcctcttc aagattatgg 1020
   agaaaaactg tggaggaaac catcaagaac gaattcccta cattgaaggt tcaacatcaa 1080
   ttgattgatt ctgccgccat gatcctagtt aagaacccaa cccacctaaa tggtattata 1140
   atcaccagca acatgtttgg tgatatcatc tccgatgaag cctccgttat cccaggttcc 1200
   ttgggtttgt tgccatctgc gtccttggcc tctttgccag acaagaacac cgcatttggt 1260
   ttgtacgaac catgccacgg ttctgctcca gatttgccaa agaataaggt caaccctatc 1320
   gccactatct tgtctgctgc aatgatgttg aaattgtcat tgaacttgcc tgaagaaggt 1380
   aaggccattg aagatgcagt taaaaaggtt ttggatgcag gtatcagaac tggtgattta 1440
   ggtggttcca acagtaccac cgaagtcggt gatgctgtcg ccgaagaagt taagaaaatc 1500
   cttgcttaaa aagattctct ttttttatga tatttgtaca taaactttat aaatgaaatt 1560
   cataatagaa acgacacgaa attacaaaat ggaatatgtt catagggtag acgaaactat 1620
   atacgcaatc tacatacatt tatcaagaag gagaaaaagg aggatgtaaa ggaatacagg 1680
   taagcaaatt gatactaatg gctcaacgtg ataaggaaaa agaattgcac tttaacatta 1740
   atattgacaa ggaggagggc accacacaaa aagttaggtg taacagaaaa tcatgaaact 1800
   atgattccta atttatatat tggaggattt tctctaaaaa aaaaaaaata caacaaataa 1860
   aaaacactca atgacctgac catttgatgg agttgccggc gatcacagcg gacggtggtg 1920
   gcatgatggg gcttgcgatg ctatgtttgt ttgttttgtg atgatgtata ttattattga 1980
   aaaacgatat cagacatttg tctgataatg cttcattatc agacaaatgt ctgatatcgt 2040
   ttggagaaaa agaaaaggaa aacaaactaa atatctacta tataccactg tattttatac 2100
   taatgacttt ctacgcctag tgtcaccctc tcgtgtaccc attgaccctg tatcggcgcg 2160
   ttgcctcgcg ttcctgtacc atatattttt gtttatttag gtattaaaat ttactttcct 2220
   catacaaata ttaaattcac caaacttctc aaaaactaat tattcgtagt tacaaactct 2280
   attttacaat cacgtttatt caaccattct acatccaata accaaaatgc ccatgtacct 2340
   ctcagcgaag tccaacggta ctgtccaata ttctcattaa atagtctttc atctatatat 2400
   cagaaggtaa ttataattag agatttcgaa tcattaccgt gccgattcgc acgctgcaac 2460
   ggcatgcatc actaatgaaa agcatacgac gcctgcgtct gacatgcact cattctgaag 2520
   aagattctgg gcgcgtttcg ttctcgtttt cctctgtata ttgtactctg gtggacaatt 2580
   tgaacataac gtctttcacc tcgccattct caataatggg ttccaattct atccaggtag 2640
   cggttaattg acggtgctta agccgtatgc tcactctaac gctaccgttg tccaaacaac 2700
   ggaccccttt gtgacgggtg taagacccat catgaagtaa aacatctcta acggtatgga 2760
   aaagagtggt acggtcaagt ttcctggcac gagtcaattt tccctcttcg tgtagatcgg 2820
   taccggccgc aaattaaagc cttcgagcgt cccaaaacct tctcaagcaa ggttttcagt 2880
   ataatgttac atgcgtacac gcgtctgtac agaaaaaaaa gaaaaatttg aaatataaat 2940
   aacgttctta atactaacat aactataaaa aaataaatag ggacctagac ttcaggttgt 3000
   ctaactcctt ccttttcggt tagagcggat gtggggggag ggcgtgaatg taagcgtgac 3060
   ataactaatt acatgactcg agcggccgcg gatcccggga attcgtcgac accatcttct 3120
   tctgagatga gtttttgttc catgctagtt ctagaatccg tcgaaactaa gttctggtgt 3180
   tttaaaacta aaaaaaagac taactataaa agtagaattt aagaagttta agaaatagat 3240
   ttacagaatt acaatcaata cctaccgtct ttatatactt attagtcaag taggggaata 3300
   atttcaggga actggtttca accttttttt tcagcttttt ccaaatcaga gagagcagaa 3360
   ggtaatagaa ggtgtaagaa aatgagatag atacatgcgt gggtcaattg ccttgtgtca 3420
   tcatttactc caggcaggtt gcatcactcc attgaggttg tgcccgtttt ttgcctgttt 3480
   gtgcccctgt tctctgtagt tgcgctaaga gaatggacct atgaactgat ggttggtgaa 3540
   gaaaacaata ttttggtgct gggattcttt ttttttctgg atgccagctt aaaaagcggg 3600
   ctccattata tttagtggat gccaggaata aactgttcac ccagacacct acgatgttat 3660
   atattctgtg taacccgccc cctattttgg gcatgtacgg gttacagcag aattaaaagg 3720
   ctaatttttt gactaaataa agttaggaaa atcactacta ttaattattt acgtattctt 3780
   tgaaatggcg agtattgata atgataaact gagctagatc tgggcccgag ctccagcttt 3840
   tgttcccttt agtgagggtt aattgcgcgc ttggcgtaat catggtcata gctgtttcct 3900
   gtgtgaaatt gttatccgct cacaattcca cacaacatag gagccggaag cataaagtgt 3960
   aaagcctggg gtgcctaatg agtgaggtaa ctcacattaa ttgcgttgcg ctcactgccc 4020
   gctttccagt cgggaaacct gtcgtgccag ctgcattaat gaatcggcca acgcgcgggg 4080
   agaggcggtt tgcgtattgg gcgctcttcc gcttcctcgc tcactgactc gctgcgctcg 4140
   gtcgttcggc tgcggcgagc ggtatcagct cactcaaagg cggtaatacg gttatccaca 4200
   gaatcagggg ataacgcagg aaagaacatg tgagcaaaag gccagcaaaa ggccaggaac 4260
   cgtaaaaagg ccgcgttgct ggcgtttttc cataggctcc gcccccctga cgagcatcac 4320
   aaaaatcgac gctcaagtca gaggtggcga aacccgacag gactataaag ataccaggcg 4380
   tttccccctg gaagctccct cgtgcgctct cctgttccga ccctgccgct taccggatac 4440
   ctgtccgcct ttctcccttc gggaagcgtg gcgctttctc atagctcacg ctgtaggtat 4500
   ctcagttcgg tgtaggtcgt tcgctccaag ctgggctgtg tgcacgaacc ccccgttcag 4560
   cccgaccgct gcgccttatc cggtaactat cgtcttgagt ccaacccggt aagacacgac 4620
   ttatcgccac tggcagcagc cactggtaac aggattagca gagcgaggta tgtaggcggt 4680
   gctacagagt tcttgaagtg gtggcctaac tacggctaca ctagaaggac agtatttggt 4740
   atctgcgctc tgctgaagcc agttaccttc ggaaaaagag ttggtagctc ttgatccggc 4800
   aaacaaacca ccgctggtag cggtggtttt tttgtttgca agcagcagat tacgcgcaga 4860
   aaaaaaggat ctcaagaaga tcctttgatc ttttctacgg ggtctgacgc tcagtggaac 4920
   gaaaactcac gttaagggat tttggtcatg agattatcaa aaaggatctt cacctagatc 4980
   cttttaaatt aaaaatgaag ttttaaatca atctaaagta tatatgagta aacttggtct 5040
   gacagttacc aatgcttaat cagtgaggca cctatctcag cgatctgtct atttcgttca 5100
   tccatagttg cctgactccc cgtcgtgtag ataactacga tacgggaggg cttaccatct 5160
   ggccccagtg ctgcaatgat accgcgagac ccacgctcac cggctccaga tttatcagca 5220
   ataaaccagc cagccggaag ggccgagcgc agaagtggtc ctgcaacttt atccgcctcc 5280
   atccagtcta ttaattgttg ccgggaagct agagtaagta gttcgccagt taatagtttg 5340
   cgcaacgttg ttgccattgc tacaggcatc gtggtgtcac gctcgtcgtt tggtatggct 5400
   tcattcagct ccggttccca acgatcaagg cgagttacat gatcccccat gttgtgcaaa 5460
   aaagcggtta gctccttcgg tcctccgatc gttgtcagaa gtaagttggc cgcagtgtta 5520
   tcactcatgg ttatggcagc actgcataat tctcttactg tcatgccatc cgtaagatgc 5580
   ttttctgtga ctggtgagta ctcaaccaag tcattctgag aatagtgtat gcggcgaccg 5640
   agttgctctt gcccggcgtc aatacgggat aataccgcgc cacatagcag aactttaaaa 5700
   gtgctcatca ttggaaaacg ttcttcgggg cgaaaactct caaggatctt accgctgttg 5760
   agatccagtt cgatgtaacc cactcgtgca cccaactgat cttcagcatc ttttactttc 5820
   accagcgttt ctgggtgagc aaaaacagga aggcaaaatg ccgcaaaaaa gggaataagg 5880
   gcgacacgga aatgttgaat actcatactc ttcctttttc aatattattg aagcatttat 5940
   cagggttatt gtctcatgag cggatacata tttgaatgta tttagaaaaa taaacaaata 6000
   ggggttccgc gcacatttcc ccgaaaagtg ccacctgacg t 6041
<210> 35
   <211> 5639
   <212> DNA
   <213> pGV1431
<400> 35
   ctgattggaa agaccattct gctttacttt tagagcatct tggtcttctg agctcattat      60
   acctcaatca aaactgaaat taggtgcctg tcacggctct ttttttactg tacctgtgac     120
   ttcctttctt atttccaagg atgctcatca caatacgctt ctagatctat tatgcattat     180
   aattaatagt tgtagctaca aaaggtaaaa gaaagtccgg ggcaggcaac aatagaaatc     240
   ggcaaaaaaa actacagaaa tactaagagc ttcttcccca ttcagtcatc gcatttcgaa     300
   acaagagggg aatggctctg gctagggaac taaccaccat cgcctgactc tatgcactaa     360
   ccacgtgact acatatatgt gatcgttttt aacatttttc aaaggctgtg tgtctggctg      420
   tttccattaa ttttcactga ttaagcagtc atattgaatc tgagctcatc accaacaaga     480
   aatactaccg taaaagtgta aaagttcgtt taaatcattt gtaaactgga acagcaagag     540
   gaagtatcat cagctagccc cataaactaa tcaaaggagg atgtctacta agagttactc     600
   ggaaagagca gctgctcata gaagtccagt tgctgccaag cttttaaact tgatggaaga     660
   gaagaagtca aacttatgtg cttctcttga tgttcgtaaa acagcagagt tgttaagatt     720
   agttgaggtt ttgggtccat atatctgtct attgaagaca catgtagata tcttggagga     780
   tttcagcttt gagaatacca ttgtgccgtt gaagcaatta gcagagaaac acaagttttt     840
   gatatttgaa gacaggaagt ttgccgacat tgggaacact gttaaattac aatacacgtc     900
   tggtgtatac cgtatcgccg aatggtctga tatcaccaat gcacacggtg tgactggtgc     960
   gggcattgtt gctggtttga agcaaggtgc cgaggaagtt acgaaagaac ctagagggtt    1020
   gttaatgctt gccgagttat cgtccaaggg gtctctagcg cacggtgaat acactcgtgg    1080
   gaccgtggaa attgccaaga gtgataagga ctttgttatt ggatttattg ctcaaaacga    1140
   tatgggtgga agagaagagg gctacgattg gttgatcatg acgccaggtg ttggtcttga    1200
   tgacaaaggt gatgctttgg gacaacaata cagaactgtg gatgaagttg ttgccggtgg    1260
   atcagacatc attattgttg gtagaggtct tttcgcaaag ggaagagatc ctgtagtgga    1320
   aggtgagaga tacagaaagg cgggatggga cgcttacttg aagagagtag gcagatccgc    1380
   ttaagagttc tccgagaaca agcagaggtt cgagtgtact cggatcagaa gttacaagtt    1440
   gatcgtttat atataaacta tacagagatg ttagagtgta atggcattgc gtgccggcga    1500
   tcacagcgga cggtggtggc atgatggggc ttgcgatgct atgtttgttt gttttgtgat    1560
   gatgtatatt attattgaaa aacgatatca gacatttgtc tgataatgct tcattatcag    1620
   acaaatgtct gatatcgttt ggagaaaaag aaaaggaaaa caaactaaat atctactata    1680
   taccactgta ttttatacta atgactttct acgcctagtg tcaccctctc gtgtacccat    1740
   tgaccctgta tcggcgcgtt gcctcgcgtt cctgtaccat atatttttgt ttatttaggt    1800
   attaaaattt actttcctca tacaaatatt aaattcacca aacttctcaa aaactaatta    1860
   ttcgtagtta caaactctat tttacaatca cgtttattca accattctac atccaataac    1920
   caaaatgccc atgtacctct cagcgaagtc caacggtact gtccaatatt ctcattaaat    1980
   agtctttcat ctatatatca gaaggtaatt ataattagag atttcgaatc attaccgtgc    2040
   cgattcgcac gctgcaacgg catgcatcac taatgaaaag catacgacgc ctgcgtctga    2100
   catgcactca ttctgaagaa gattctgggc gcgtttcgtt ctcgttttcc tctgtatatt    2160
   gtactctggt ggacaatttg aacataacgt ctttcacctc gccattctca ataatgggtt    2220
   ccaattctat ccaggtagcg gttaattgac ggtgcttaag ccgtatgctc actctaacgc    2280
   taccgttgtc caaacaacgg acccctttgt gacgggtgta agacccatca tgaagtaaaa    2340
   catctctaac ggtatggaaa agagtggtac ggtcaagttt cctggcacga gtcaattttc    2400
   cctcttcgtg tagatcggta ccggccgcaa attaaagcct tcgagcgtcc caaaaccttc 2460
   tcaagcaagg ttttcagtat aatgttacat gcgtacacgc gtctgtacag aaaaaaaaga 2520
   aaaatttgaa atataaataa cgttcttaat actaacataa ctataaaaaa ataaataggg 2580
   acctagactt caggttgtct aactccttcc ttttcggtta gagcggatgt ggggggaggg 2640
   cgtgaatgta agcgtgacat aactaattac atgactcgag cggccgcgga tcccgggaat 2700
   tcgtcgacac catcttcttc tgagatgagt ttttgttcca tgctagttct agaatccgtc 2760
   gaaactaagt tctggtgttt taaaactaaa aaaaagacta actataaaag tagaatttaa 2820
   gaagtttaag aaatagattt acagaattac aatcaatacc taccgtcttt atatacttat 2880
   tagtcaagta ggggaataat ttcagggaac tggtttcaac cttttttttc agctttttcc 2940
   aaatcagaga gagcagaagg taatagaagg tgtaagaaaa tgagatagat acatgcgtgg 3000
   gtcaattgcc ttgtgtcatc atttactcca ggcaggttgc atcactccat tgaggttgtg 3060
   cccgtttttt gcctgtttgt gcccctgttc tctgtagttg cgctaagaga atggacctat 3120
   gaactgatgg ttggtgaaga aaacaatatt ttggtgctgg gattcttttt ttttctggat 3180
   gccagcttaa aaagcgggct ccattatatt tagtggatgc caggaataaa ctgttcaccc 3240
   agacacctac gatgttatat attctgtgta acccgccccc tattttgggc atgtacgggt 3300
   tacagcagaa ttaaaaggct aattttttga ctaaataaag ttaggaaaat cactactatt 3360
   aattatttac gtattctttg aaatggcgag tattgataat gataaactga gctagatctg 3420
   ggcccgagct ccagcttttg ttccctttag tgagggttaa ttgcgcgctt ggcgtaatca 3480
   tggtcatagc tgtttcctgt gtgaaattgt tatccgctca caattccaca caacatagga 3540
   gccggaagca taaagtgtaa agcctggggt gcctaatgag tgaggtaact cacattaatt 3600
   gcgttgcgct cactgcccgc tttccagtcg ggaaacctgt cgtgccagct gcattaatga 3660
   atcggccaac gcgcggggag aggcggtttg cgtattgggc gctcttccgc ttcctcgctc 3720
   actgactcgc tgcgctcggt cgttcggctg cggcgagcgg tatcagctca ctcaaaggcg 3780
   gtaatacggt tatccacaga atcaggggat aacgcaggaa agaacatgtg agcaaaaggc 3840
   cagcaaaagg ccaggaaccg taaaaaggcc gcgttgctgg cgtttttcca taggctccgc 3900
   ccccctgacg agcatcacaa aaatcgacgc tcaagtcaga ggtggcgaaa cccgacagga 3960
   ctataaagat accaggcgtt tccccctgga agctccctcg tgcgctctcc tgttccgacc 4020
   ctgccgctta ccggatacct gtccgccttt ctcccttcgg gaagcgtggc gctttctcat 4080
   agctcacgct gtaggtatct cagttcggtg taggtcgttc gctccaagct gggctgtgtg 4140
   cacgaacccc ccgttcagcc cgaccgctgc gccttatccg gtaactatcg tcttgagtcc 4200
   aacccggtaa gacacgactt atcgccactg gcagcagcca ctggtaacag gattagcaga 4260
   gcgaggtatg taggcggtgc tacagagttc ttgaagtggt ggcctaacta cggctacact 4320
   agaaggacag tatttggtat ctgcgctctg ctgaagccag ttaccttcgg aaaaagagtt 4380
   ggtagctctt gatccggcaa acaaaccacc gctggtagcg gtggtttttt tgtttgcaag 4440
   cagcagatta cgcgcagaaa aaaaggatct caagaagatc ctttgatctt ttctacgggg 4500
   tctgacgctc agtggaacga aaactcacgt taagggattt tggtcatgag attatcaaaa 4560
   aggatcttca cctagatcct tttaaattaa aaatgaagtt ttaaatcaat ctaaagtata 4620
   tatgagtaaa cttggtctga cagttaccaa tgcttaatca gtgaggcacc tatctcagcg 4680
   atctgtctat ttcgttcatc catagttgcc tgactccccg tcgtgtagat aactacgata 4740
   cgggagggct taccatctgg ccccagtgct gcaatgatac cgcgagaccc acgctcaccg 4800
   gctccagatt tatcagcaat aaaccagcca gccggaaggg ccgagcgcag aagtggtcct 4860
   gcaactttat ccgcctccat ccagtctatt aattgttgcc gggaagctag agtaagtagt 4920
   tcgccagtta atagtttgcg caacgttgtt gccattgcta caggcatcgt ggtgtcacgc 4980
   tcgtcgtttg gtatggcttc attcagctcc ggttcccaac gatcaaggcg agttacatga 5040
   tcccccatgt tgtgcaaaaa agcggttagc tccttcggtc ctccgatcgt tgtcagaagt 5100
   aagttggccg cagtgttatc actcatggtt atggcagcac tgcataattc tcttactgtc 5160
   atgccatccg taagatgctt ttctgtgact ggtgagtact caaccaagtc attctgagaa 5220
   tagtgtatgc ggcgaccgag ttgctcttgc ccggcgtcaa tacgggataa taccgcgcca 5280
   catagcagaa ctttaaaagt gctcatcatt ggaaaacgtt cttcggggcg aaaactctca 5340
   aggatcttac cgctgttgag atccagttcg atgtaaccca ctcgtgcacc caactgatct 5400
   tcagcatctt ttactttcac cagcgtttct gggtgagcaa aaacaggaag gcaaaatgcc 5460
   gcaaaaaagg gaataagggc gacacggaaa tgttgaatac tcatactctt cctttttcaa 5520
   tattattgaa gcatttatca gggttattgt ctcatgagcg gatacatatt tgaatgtatt 5580
   tagaaaaata aacaaatagg ggttccgcgc acatttcccc gaaaagtgcc acctgacgt 5639
<210> 36
   <211> 741
   <212> DNA
   <213> pf1A
<400> 36
   atgtcagtta ttggtcgcat tcactccttt gaatcctgtg gaaccgtaga cggcccaggt      60
   attcgcttta tcaccttttt ccagggctgc ctgatgcgct gcctgtattg tcataaccgc     120
   gacacctggg acacgcatgg cggtaaagaa gttaccgttg aagatttgat gaaggaagtg     180
   gtgacctatc gccactttat gaacgcttcc ggcggcggcg ttaccgcatc cggcggtgaa     240
   gcaatcctgc aagctgagtt tgttcgtgac tggttccgcg cctgcaaaaa agaaggcatt     300
   catacctgtc tggacaccaa cggttttgtt cgtcgttacg atccggtgat tgatgaactg     360
   ctggaagtaa ccgacctggt aatgctcgat ctcaaacaga tgaacgacga gatccaccaa     420
   aatctggttg gagtttccaa ccaccgcacg ctggagttcg ctaaatatct ggcgaacaaa     480
   aatgtgaagg tgtggatccg ctacgttgtt gtcccaggct ggtctgacga tgacgattca     540
   gcgcatcgcc tcggtgaatt tacccgtgat atgggcaacg ttgagaaaat cgagcttctc     600
   ccctaccacg agctgggcaa acacaaatgg gtggcaatgg gtgaagagta caaactcgac     660
   ggtgttaaac caccgaagaa agagaccatg gaacgcgtga aaggcattct tgagcagtac     720
   ggtcataagg taatgttcta a                                               741
<210> 37
   <211> 2283
   <212> DNA
   <213> *pflB*
<400> 37
   atgtccgagc ttaatgaaaa gttagccaca gcctgggaag gttttaccaa aggtgactgg     60
   cagaatgaag taaacgtccg tgacttcatt cagaaaaact acactccgta cgagggtgac    120
   gagtccttcc tggctggcgc tactgaagcg accaccaccc tgtgggacaa agtaatggaa    180
   ggcgttaaac tggaaaaccg cactcacgcg ccagttgact ttgacaccgc tgttgcttcc    240
   accatcacct ctcacgacgc tggctacatc aacaagcagc ttgagaaaat cgttggtctg    300
   cagactgaag ctccgctgaa acgtgctctt atcccgttcg gtggtatcaa aatgatcgaa    360
   ggttcctgca aagcgtacaa ccgcgaactg gatccgatga tcaaaaaaat cttcactgaa    420
   taccgtaaaa ctcacaacca gggcgtgttc gacgtttaca ctccggacat cctgcgttgc    480
   cgtaaatctg gtgttctgac cggtctgcca gatgcatatg gccgtggccg tatcatcggt    540
   gactaccgtc gcgttgcgct gtacggtatc gactacctga tgaaagacaa actggcacag    600
   ttcacttctc tgcaggctga tctggaaaac ggcgtaaacc tggaacagac tatccgtctg    660
   cgcgaagaaa tcgctgaaca gcaccgcgct ctgggtcaga tgaaagaaat ggctgcgaaa    720
   tacggctacg acatctctgg tccggctacc aacgctcagg aagctatcca gtggacttac    780
   ttcggctacc tggctgctgt taagtctcag aacggtgctg caatgtcctt cggtcgtacc    840
   tccaccttcc tggatgtgta catcgaacgt gacctgaaag ctggcaagat caccgaacaa    900
   gaagcgcagg aaatggttga ccacctggtc atgaaactgc gtatggttcg cttcctgcgt    960
   actccggaat acgatgaact gttctctggc gacccgatct gggcaaccga atctatcggt   1020
   ggtatgggcc tcgacggtcg taccctggtt accaaaaaca gcttccgttt cctgaacacc   1080
   ctgtacacca tgggtccgtc tccggaaccg aacatgacca ttctgtggtc tgaaaaactg   1140
   ccgctgaact tcaagaaatt cgccgctaaa gtgtccatcg acacctcttc tctgcagtat   1200
   gagaacgatg acctgatgcg tccggacttc aacaacgatg actacgctat tgcttgctgc   1260
   gtaagcccga tgatcgttgg taaacaaatg cagttcttcg gtgcgcgtgc aaacctggcg   1320
   aaaaccatgc tgtacgcaat caacggcggc gttgacgaaa aactgaaaat gcaggttggt   1380
   ccgaagtctg aaccgatcaa aggcgatgtc ctgaactatg atgaagtgat ggagcgcatg   1440
   gatcacttca tggactggct ggctaaacag tacatcactg cactgaacat catccactac 1500
   atgcacgaca agtacagcta cgaagcctct ctgatggcgc tgcacgaccg tgacgttatc 1560
   cgcaccatgg cgtgtggtat cgctggtctg tccgttgctg ctgactccct gtctgcaatc 1620
   aaatatgcga aagttaaacc gattcgtgac gaagacggtc tggctatcga cttcgaaatc 1680
   gaaggcgaat acccgcagtt tggtaacaat gatccgcgtg tagatgacct ggctgttgac 1740
   ctggtagaac gtttcatgaa gaaaattcag aaactgcaca cctaccgtga cgctatcccg 1800
   actcagtctg ttctgaccat cacttctaac gttgtgtatg gtaagaaaac gggtaacacc 1860
   ccagacggtc gtcgtgctgg cgcgccgttc ggaccgggtg ctaacccgat gcacggtcgt 1920
   gaccagaaag gtgcagtagc ctctctgact tccgttgcta aactgccgtt tgcttacgct 1980
   aaagatggta tctcctacac cttctctatc gttccgaacg cactgggtaa agacgacgaa 2040
   gttcgtaaga ccaacctggc tggtctgatg gatggttact tccaccacga agcatccatc 2100
   gaaggtggtc agcacctgaa cgttaacgtg atgaaccgtg aaatgctgct cgacgcgatg 2160
   gaaaacccgg aaaaatatcc gcagctgacc atccgtgtat ctggctacgc agtacgtttc 2220
   aactcgctga ctaaagaaca gcagcaggac gttattactc gtaccttcac tcaatctatg 2280
   taa 2283
<210> 38
   <211> 1095
   <212> DNA
   <213> Cb-FDH1
<400> 38
   atgaagatcg ttttagtctt atatggtgct ggtaaacacg ctgccgatga agaaaaatta 60
   tacggttgta ctgaaaacaa attaggtatt gccaattggt tgaaagatca aggacatgaa 120
   ctaatcacca cgtctgataa agaaggcgga aacagtgtgt tggatcaaca tataccagat 180
   gccgatatta tcattacaac tcctttccat cctgcttata tcactaagga aagaatcgac 240
   aaggctaaaa aattgaaatt agttgttgtc gctggtgtcg gttctgatca tattgatttg 300
   gattatatca accaaacagg taggaaaatc tccgtcttgg aagttaccgg ttctaatgtt 360
   gtctctgttg cagaacacgt tgtcatgacc atgcttgtct tggttagaaa ttttgttcca 420
   gctcacgaac aaaacattaa ccacgattgg gaggttgctg ctatcgctaa ggatgcttac 480
   gatatcgaag gtaaaactat cgccaccatt ggtgccggta gaattggtta cagagtcttg 540
   gaaagattag tcccattcaa tcctaaagaa ttattatact acgattatca agctttacca 600
   aaagatgctg aagaaaaagt tggtgctaga agggttgaaa atattgaaga attggttgcc 660
   caagctgata tagttacagt taatgctcca ttacacgctg gtacaaaagg tttaattaac 720
   aaggaattat tgtctaaatt caagaaaggt gcttggttag tcaatactgc aagaggtgcc 780
   atttgtgttg ccgaagatgt tgctgcagct ttagaatctg gtcaattaag aggttatggt 840
   ggtgatgttt ggttcccaca accagctcca aaagatcacc catggagaga tatgagaaac 900
   aaatatggtg ctggtaacgc cacgactcct cattactctg gtactacttt agatgctcaa 960
   actagatacg ctcaaggtac taaaaatatc ttggagtcat tctttactgg taagtttgat 1020
   tacagaccac aagatatcat cttattaaac ggtgaatacg ttaccaaagc ttacggtaaa 1080
   cacgataaga aataa 1095
<210> 39
   <211> 1524
   <212> DNA
   <213> KlALD6
<400> 39
   atgtcctcta caattgctga gaaattgaac ctcaagatcg tcgaacaaga cgctgttagc      60
   atcactttgc caaacggttt gacttaccaa caaccaactg gtttgttcat caacaatcag     120
   ttcatcaagt ctcaagacgg taagactttg aaggttgaaa acccatctac tgaggaaatc     180
   attgtcgaag tccaatctgc tacttctcaa gacgtcgagt acgccgttga agctgccgat     240
   gctgctttca actccgaatg gtctactatg gacccaaaaa agcgtggttc tttgttgttt     300
   aagttggctg acttgattga agctcaaaag gaattgattg cttctatcga atctgctgac     360
   aacggtaaga ctttggccct agccagaggt gatgttggtt tggtcattga ctacatcaga     420
   tctgctgctg gttatgctga caagttgggt ggtagaacta tcaacactgg tgatggttac     480
   gctaacttca cttacaagga acctctaggt gtctgtggtc aaatcatccc atggaacttc     540
   ccattgatga tgctttcttg gaagatcgcc cctgctttgg ttgctggtaa caccgttatc     600
   ttgaagccag cttccccaac cccattgaac gctttgttct ttgcttcttt gtgtaaggaa     660
   gcaggtatcc cagctggtgt cgttaacatc gttccaggtc caggtagatc cgttggtgac     720
   accatcacca accatccaaa aattagaaag attgccttca ctggttccac tgacattggt     780
   agagacgttg ctatcaaggc tgcccaatct aacttgaaga aggtcacctt ggaattgggt     840
   ggtaaatccg ctcatttggt ctttgaagat gccaacatta agaagactat tccaaacttg     900
   gtcaacggta ttttcaagaa tgctggtcaa atttgttcct ctggttccag aatctatgtc     960
   caagacacca tctacgatca actattgtct gaattcaaga cttacctgga aactgaaatt    1020
   aaggtcggtt ccccattcga tgaatctaac ttccaagctg ctatcaacaa caaggctcaa    1080
   ttcgaaacta tcttgaacta catcgacatc ggtaagaagg aaggtgcttc tatcttgact    1140
   ggtggtgaaa gagtaggcaa caagggttac ttcattaaac caactgtatt ctacaacgtt    1200
   aaggaagata tgagaatcgt caaggaagaa atctttggtc ctgtcgtcac catctccaag    1260
   ttctctactg tcgacgaagc tgtcgctttg gctaacgact ccgaattcgg tttgggtgct    1320
   ggtatcgaaa ctgaaaacat ctccgttgcc ttgaaggtcg caaagagact aaaagctggt    1380
   accgtctgga tcaacactta caacgatttc gacgctgccg ttccattcgg tggttacaag    1440
   caatctggtt acggtagaga aatgggtgaa gaagctttcg aatcttacac tcaaatcaag    1500
   gccgtcagga tcaagttgga ttaa                                           1524
<210> 40
   <211> 2124
   <212> DNA
   <213> KlACS1
<400> 40
   atgtctcctg ctgttgatac cgcttccacc gccaaagatc caatctcagt catgaaatct 60
   aacgcttcag ctgccgctgc agaccaaatt aagacccatg aatacgaaca tttaacttct 120
   gtgcctatag tgcagcctct accaattact gataggttga gcagcgaagc agctcaaaaa 180
   tataaaccta atttgccagg tgggttcgaa gagtacaagt ctttgcacaa ggaatcactt 240
   gaaaatccag ccaagtttta ccatgaacgt gctcagctgt tgaattggtt caaaccatac 300
   gatcaagttt tcatcccaga taccgaaggt aaaccaactt ttgagaacaa cgcttggttt 360
   accaacggtc aattgaacgc ttgttacaat ttggtagaca gacatgcctt cactcaacca 420
   aacaaggttg ccattcttta tgaagctgat gaaccaggtc aaggttatag tctcacttat 480
   gcggaattgt tagaacaagt ctgtaaagtt gctcaaatct tgcaatactc gatgaacgtc 540
   aagaaaggtg acacggtcgc agtttatatg ccaatgatcc cacaggcttt gattaccttg 600
   ttggcaatta ctcgtatcgg tgccattcat tccgttgttt ttgctgggtt ctcttcgaat 660
   tcattgcgtg atcgtattaa cgatgcttac tcaaagacag tcatcaccac cgatgaatct 720
   aagagaggtg gtaagaccat cgaaaccaag cgtatcgtcg atgaagcctt gaaggatacc 780
   cctcaagtaa caaacgtttt ggtcttcaaa cgtactcata acgaaaatat caagtacatt 840
   ccaggtaggg atttggactg ggatgaggaa gtcaagaagt acaaatctta caccccatgc 900
   gaacctgttg actctgaaca tcctttgttc ttattgtata cttcgggttc caccggtgct 960
   ccaaagggtg ttcaacattc tacagcaggt tacttgctcc aagcattatt aagtatgaaa 1020
   tacacctttg acatccaaaa cgatgacatc ttcttcaccg caggtgacat tggttggatc 1080
   actggtcaca catactgtgt ttacggtcca ttgttacaag gttgtactac tttggtgttc 1140
   gaaggtacac ctgcctatcc aaacttttct cgttattggg aaattgttga caagtaccaa 1200
   gtgactcaat tctatgtagc cccaactgca ctacgtctat tgaagagagc tggtgattcc 1260
   tttactgaag gattctctct caagtcattg cgctccttgg gttccgttgg tgaacctatc 1320
   gctgctgaag tttgggaatg gtactctgaa aagattggta agaatgagct accaatcgta 1380
   gacacatact ggcaaactga atctggctcc cacttggtca ctccattggc tggtggtgct 1440
   actccaatga aaccaggtgc agcggcattc ccattctttg gtattgattt ggcagtgttg 1500
   gatccaacca caggtatcga gcaaactggt gaacatgcag aaggtgttct tgccattaaa 1560
   agaccttggc catctttcgc aagaaccatt tggaagaata acgataggtt cttagacacg 1620
   tacttgaaac catacccggg ctattacttc actggtgatg gtgttgcccg tgataaagat   1680
   ggattcttct ggatcttggg tcgtgttgat gatgttgtta acgtctcagg tcacaggttg   1740
   tctactgctg aaattgaagc tgctatcatt gaagatgata tggttgccga atgtgcagtt   1800
   gttgggttta acgacgaatt gactggtcaa gccgttgctg cctttgtagt attgaagaac   1860
   aagtctagtt taactgctgc aagcgagtcc gagttacaag acatcaaaaa gcatttgatc   1920
   atcaccgtta gaaaggatat tggtccattc gctgctccta agttgatcgt cctagttgat   1980
   gatctaccaa agactagatc tggcaagatt atgagacgta ttttgagaaa gatcctagcc   2040
   ggtgaatctg atcaattggg cgacgtctcc acattatcca accctggtat cgttaagcac   2100
   ttgatcgatt ccgtgaaatt ataa                                          2124
<210> 41
   <211> 2055
   <212> DNA
   <213> KlACS2
<400> 41
   atgtcgtcgg ataaattgca taaggttgtg catgaagctc acgatgttga agctcgtcat      60
   gctccagaac atttctacaa ttctcaaccg ggtaaatcgt actgtactga tgaagaacat     120
   taccgtgaga tgtacactca gtccattgag gacccagcag ggtttttcgg tccattggcc     180
   aaggaatatc tagattggga tcgtccattc acccaagtcc aaagcggttc tttggaacac     240
   ggtgacattg cctggttctt aaatggtgaa ctgaatgctt cttataactg tgttgacaga     300
   cacgcttttg ccaacccaga caagccagct ttaatctacg aagccgacga tgaatctgaa     360
   aacaaggtga tcacttttgg cgaattgttg agacaggtct ctgaagtggc tggtgtcttg     420
   caatcttggg gtgtcaagaa aggagacacc gtcgccgttt acttgccaat gattcctgct     480
   gcagttgttg ctatgttggc cgttgcaaga ttaggtgcca ttcattcggt tatctttgcc     540
   ggtttctctg ccggttcctt gaaggaaaga gttgtcgatg caggctgtaa agtggtcatc     600
   acttgcgatg aaggtaagag aggtggtaag accgttcata ccaagaagat tgtcgacgaa     660
   ggtttggccg gtgtcgattc cgtttccaag atcttggttt tccaaagaac tggtactcaa     720
   ggtatcccaa tgaagccagc tagagatttc tggtggcacg aagagtgtgt caagcaaaga     780
   ggttacttgc cacctgtccc agtcaactcc gaagatccat tgttcttgtt gtacacctct     840
   ggttccaccg gttctccaaa aggtgtcgtg cactctactg ctggttactt gttaggttct     900
   gctttgacca ccagattcgt tttcgatatt catccagaag atgttttgtt cactgctggt     960
   gacgtcggtt ggattaccgg ccacacttac gccttgtacg gtccattgac cttaggtacc    1020
   gctaccatta ttttcgaatc tactccagct tacccagatt atggtagata ctggagaatc    1080
   attgaacgtc atagagctac ccacttctac gtcgccccaa ctgccctaag attgatcaaa    1140
   cgtgtcggtg aagaagaaat tgccaagtat gatacctcct ccttaagagt cttgggttct    1200
   gtcggtgaac caatctctcc agatctatgg gaatggtacc acgaaaaggt tggtaagaat 1260
   aactgtgtta tctgtgacac catgtggcaa accgaatccg gttcacactt gattgcccca 1320
   ttggctggtg ctgtcccaac caaaccaggt tccgctaccg tcccattctt cggtattaac 1380
   gcctgtatca tcgacccagt ttctggtgaa gaattgaagg gtaacgatgt tgaaggtgtc 1440
   ttggcagtga agtccccatg gccttctatg gccagatctg tctggaacaa ccatgctcgt 1500
   tacttcgaaa cttatttgaa gccataccca ggatactact tcacaggtga tggtgctggt 1560
   agagatcacg acggttacta ctggatcaga ggtagagttg acgatgtcgt taacgtttcc 1620
   ggtcacagac tttctactgc tgaaatcgaa gctgctctag ctgaacacga aggtgtttct 1680
   gaagctgccg ttgttggtat cactgatgaa ctaacaggtc aagctgtcat tgcattcgtt 1740
   tccttgaagg acggctatct gtctgaaaat gcggtagagg gtgacagtac ccacatctct 1800
   ccagacaact tacgtcgtga gttgattcta caagtcagag gtgaaattgg tccattcgct 1860
   gcaccaaaga ccgttgttgt tgtcaacgat ttgccaaaaa ctagatccgg taaaattatg 1920
   agaagagtct tgagaaaggt tgcatccaag gaagctgatc aattgggtga tctaagtacc 1980
   ttagccaatg cagacgttgt accatctatc atttctgcag tagaaaatca atttttcagt 2040
   cagcagaaga aataa 2055
<210> 42
   <211> 37
   <212> DNA
   <213> Gevo-311
<400> 42
   gaggttgtcg acatgaaaaa gatttttgta cttggag 37
<210> 43
   <211> 35
   <212> DNA
   <213> Gevo-175
<400> 43
   aattggatcc ttatttagaa taatcataga atcct 35
<210> 44
   <211> 37
   <212> DNA
   <213> Gevo-312
<400> 44
   gttcttgtcg acatggaatt aaaaaatgtt attcttg 37
<210> 45
   <211> 37
   <212> DNA
   <213> Gevo-171
<400> 45
   aattggatcc ttatttattt tgaaaattct tttctgc 37
<210> 46
   <211> 37
   <212> DNA
   <213> Gevo-313
<400> 46
   caagaggtcg acatgaattt ccaattaact agagaac 37
<210> 47
   <211> 34
   <212> DNA
   <213> Gevo-314
<400> 47
   gcgtccggat ccctatctta aaatgcttcc tgcg 34
<210> 48
   <211> 36
   <212> DNA
   <213> Gevo-315
<400> 48
   cggaaagtcg acatgaatat agcagattac aaaggc 36
<210> 49
   <211> 35
   <212> DNA
   <213> Gevo-173
<400> 49
   aattggatcc ttattcagcg ctctttattt cttta 35
<210> 50
   <211> 37
   <212> DNA
   <213> Gevo-316
<400> 50
   caaaatgtcg acatgaatat agtagtttgt gtaaaac 37
<210> 51
   <211> 37
   <212> DNA
   <213> Gevo-317
<400> 51
   taatttggat ccttagatgt agtgtttttc ttttaat 37
<210> 52
   <211> 35
   <212> DNA
   <213> Gevo-319
<400> 52
   gaaccagtcg acatggcacg ttttacttta ccaag 35
<210> 53
   <211> 35
   <212> DNA
   <213> Gevo-177
<400> 53
   aattggatcc ttacaaatta actttagttc catag 35
<210> 54
   <211> 36
   <212> DNA
   <213> Gevo-318
<400> 54
   tccatagtcg acatgaataa agacacacta atacct 36
<210> 55
   <211> 40
   <212> DNA
   <213> Gevo-249
<400> 55
   aattggatcc ttagccggca agtacacatc ttctttgtct 40
<210> 56
   <211> 35
   <212> DNA
   <213> Gevo-308
<400> 56
   gatcgagtcg acatgaaaga agttgtaata gctag 35
<210> 57
   <211> 35
   <212> DNA
   <213> Gevo-309
<400> 57
   gttataggat ccctagcact tttctagcaa tattg 35
<210> 58
   <211> 37
   <212> DNA
   <213> Gevo-281
<400> 58
   gtggatgtcg acatgaaaaa ggtatgtgtt ataggtg 37
<210> 59
   <211> 35
   <212> DNA
   <213> Gevo-161
<400> 59
   aattggatcc ttattttgaa taatcgtaga aacct 35
<210> 60
   <211> 35
   <212> DNA
   <213> Gevo-282
<400> 60
   tcctacgtcg acatggaact aaacaatgtc atcct 35
<210> 61
   <211> 36
   <212> DNA
   <213> Gevo-283
<400> 61
   taacttggat ccctatctat ttttgaagcc ttcaat 36
<210> 62
   <211> 37
   <212> DNA
   <213> Gevo-284
<400> 62
   caagaggtcg acatggattt taatttaaca agagaac 37
<210> 63
   <211> 39
   <212> DNA
   <213> Gevo-285
<400> 63
   caataaggat ccttatctaa aaatttttcc tgaaataac 39
<210> 64
   <211> 36
   <212> DNA
   <213> Gevo-286
<400> 64
   cgggaagtcg acatgaataa agcagattac aagggc 36
<210> 65
   <211> 36
   <212> DNA
   <213> Gevo-287
<400> 65
   gttcaaggat ccttaattat tagcagcttt aacttg 36
<210> 66
   <211> 38
   <212> DNA
   <213> Gevo-288
<400> 66
   caaaattgtc gacatgaata tagttgtttg tttaaaac 38
<210> 67
   <211> 41
   <212> DNA
   <213> Gevo-289
<400> 67
   gttttaggat ccttaaatat agtgttcttc ttttaatttt g 41
<210> 68
   <211> 37
   <212> DNA
   <213> Gevo-292
<400> 68
   caagaagtcg acatgaaagt tacaaatcaa aaagaac 37
<210> 69
   <211> 40
   <212> DNA
   <213> Gevo-293
<400> 69
   tcctatgcgg ccgcttaaaa tgattttata tagatatcct 40
<210> 70
   <211> 35
   <212> DNA
   <213> Gevo-290
<400> 70
   aggaaagtcg acatgaaagt cacaacagta aagga 35
<210> 71
   <211> 40
   <212> DNA
   <213> Gevo-291
<400> 71
   atttaagcgg ccgcttaagg ttgtttttta aaacaattta 40
<210> 72
   <211> 37
   <212> DNA
   <213> Gevo-294
<400> 72
   cataacgtcg acatgctaag ttttgattat tcaatac 37
<210> 73
   <211> 36
   <212> DNA
   <213> Gevo-247
<400> 73
   aattggatcc ttaataagat tttttaaata tctcaa 36
<210> 74
   <211> 37
   <212> DNA
   <213> Gevo-295
<400> 74
   cataacgtcg acatggttga tttcgaatat tcaatac 37
<210> 75
   <211> 36
   <212> DNA
   <213> Gevo-159
<400> 75
   aattggatcc ttacacagat tttttgaata tttgta 36
<210> 76
   <211> 35
   <212> DNA
   <213> Gevo-310
<400> 76
   gatcgagaat tcatgaaaga agttgtaata gctag 35
<210> 77
   <211> 35
   <212> DNA
   <213> Gevo-309
<400> 77
   gttataggat ccctagcact tttctagcaa tattg 35
<210> 78
   <211> 36
   <212> DNA
   <213> Gevo-296
<400> 78
   cggatagtcg acatgaaaaa ggtatgtgtt ataggc 36
<210> 79
   <211> 38
   <212> DNA
   <213> Gevo-297
<400> 79
   tcccaaggat ccttattttg aataatcgta gaaaccct 38
<210> 80
   <211> 35
   <212> DNA
   <213> Gevo-282
<400> 80
   tcctacgtcg acatggaact aaacaatgtc atcct 35
<210> 81
   <211> 36
   <212> DNA
   <213> Gevo-283
<400> 81
   taacttggat ccctatctat ttttgaagcc ttcaat 36
<210> 82
   <211> 37
   <212> DNA
   <213> Gevo-284
<400> 82
   caagaggtcg acatggattt taatttaaca agagaac 37
<210> 83
   <211> 37
   <212> DNA
   <213> Gevo-298
<400> 83
   gtaaagggat ccttaactaa aaatttttcc tgaaatg 37
<210> 84
   <211> 36
   <212> DNA
   <213> Gevo-286
<400> 84
   cgggaagtcg acatgaataa agcagattac aagggc 36
<210> 85
   <211> 36
   <212> DNA
   <213> Gevo-299
<400> 85
   gttcaaggat ccttaattat tagcagcttt aacctg 36
<210> 86
   <211> 38
   <212> DNA
   <213> Gevo-288
<400> 86
   caaaattgtc gacatgaata tagttgtttg tttaaaac 38
<210> 87
   <211> 36
   <212> DNA
   <213> Gevo-300
<400> 87
   gactttggat ccttaaatat agtgttcttc tttcag 36
<210> 88
   <211> 37
   <212> DNA
   <213> Gevo-292
<400> 88
   caagaagtcg acatgaaagt tacaaatcaa aaagaac 37
<210> 89
   <211> 41
   <212> DNA
   <213> Gevo-301
<400> 89
   attttcggat ccttaaaatg attttatata gatatctttt a 41
<210> 90
   <211> 37
   <212> DNA
   <213> Gevo-302
<400> 90
   cttatagtcg acatggattt taacttaaca gatattc 37
<210> 91
   <211> 36
   <212> DNA
   <213> Gevo-303
<400> 91
   ccgccaggat ccttaacgta acagagcacc gccggt 36
<210> 92
   <211> 36
   <212> DNA
   <213> Gevo-304
<400> 92
   cggaaagtcg acatggattt agcagaatac aaaggc 36
<210> 93
   <211> 34
   <212> DNA
   <213> Gevo-305
<400> 93
   ctttgtggat ccttatgcaa tgcctttctg tttc 34
<210> 94
   <211> 37
   <212> DNA
   <213> Gevo-306
<400> 94
   caaactgaat tcatggaaat attggtatgt gtcaaac 37
<210> 95
   <211> 35
   <212> DNA
   <213> Gevo-307
<400> 95
   accaacggat ccttaaatga ttttctgggc aacca 35
<210> 96
   <211> 34
   <212> DNA
   <213> Gevo-273
<400> 96
   gttacagtcg acatgtctca gaacgtttac attg 34
<210> 97
   <211> 35
   <212> DNA
   <213> Gevo-274
<400> 97
   gataacggat cctcatatct tttcaatgac aatag 35
<210> 98
   <211> 34
   <212> DNA
   <213> PflA_forw
<400> 98
   cattgaattc atgtcagtta ttggtcgcat tcac 34
<210> 99
   <211> 37
   <212> DNA
   <213> PflA_Rev
<400> 99
   cattgtcgac ttagaacatt accttatgac cgtactg 37
<210> 100
   <211> 37
   <212> DNA
   <213> PflB_forw
<400> 100
   cattgaattc atgtccgagc ttaatgaaaa gttagcc 37
<210> 101
   <211> 36
   <212> DNA
   <213> PflB_Rev
<400> 101
   cattgtcgac ttacatagat tgagtgaagg tacgag 36
<210> 102
   <211> 39
   <212> DNA
   <213> fdh1_forw
<400> 102
   cattgaattc atgaagatcg ttttagtctt atatggtgc 39
<210> 103
   <211> 37
   <212> DNA
   <213> fdh1_rev
<400> 103
   cattgtcgac ttatttctta tcgtgtttac cgtaagc 37
<210> 104
   <211> 38
   <212> DNA
   <213> KlALD6_right3
<400> 104
   gttaggatcc ttaatccaac ttgatcctga cggccttg 38
<210> 105
   <211> 43
   <212> DNA
   <213> KlALDD6_Left5
<400> 105
   ccaagtcgac atgtcctcta caattgctga gaaattgaac ctc 43
<210> 106
   <211> 40
   <212> DNA
   <213> KlACS1_Right3
<400> 106
   gttagcggcc gcttataatt tcacggaatc gatcaagtgc 40
<210> 107
   <211> 37
   <212> DNA
   <213> KlACS1_Left5
<400> 107
   ccaagctagc atgtctcctg ctgttgatac cgcttcc 37
<210> 108
   <211> 49
   <212> DNA
   <213> KlACS2_right3
<400> 108
   ggttggatcc ttatttcttc tgctgactga aaaattgatt ttctactgc 49
<210> 109
   <211> 35
   <212> DNA
   <213> KlACS2_Left5
<400> 109
   ccaagaattc atgtcgtcgg ataaattgca taagg 35
<210> 110
   <211> 26
   <212> DNA
   <213> Gevo-350
<400> 110
   cttaaattct acttttatag ttagtc 26
<210> 111
   <211> 20
   <212> DNA
   <213> Gevo-352
<400> 111
   ccttcctttt cggttagagc 20
<210> 112
   <211> 31
   <212> DNA
   <213> Gevo-479
<400> 112
   catgccgtcg acatgtcgcc ctctgccgta c 31
<210> 113
   <211> 38
   <212> DNA
   <213> Gevo-480
<400> 113
   gattaagcgg ccgcttacaa cttgaccgaa tcaattag 38
<210> 114
   <211> 37
   <212> DNA
   <213> Gevo-483
<400> 114
   gatgaagtcg acatgacaat caaggaacat aaagtag 37
<210> 115
   <211> 39
   <212> DNA
   <213> Gevo-484
<400> 115
   gttaaaggat ccttatttct ttttttgaga gaaaaattg 39
<210> 116
   <211> 44
   <212> DNA
   <213> Gevo-606
<400> 116
   ttttgtcgac actagtatgt cagaacgttt cccaaatgac gtgg 44
<210> 117
   <211> 39
   <212> DNA
   <213> Gevo-607
<400> 117
   ttttctcgag ttacgccaga cgcgggttaa ctttatctg 39
<210> 118
   <211> 40
   <212> DNA
   <213> Gevo-609
<400> 118
   ttttctcgag ttacatcacc agacggcgaa tgtcagacag 40
<210> 119
   <211> 46
   <212> DNA
   <213> Gevo-610
<400> 119
   ttttgtcgac actagtatga gtactgaaat caaaactcag gtcgtg 46
<210> 120
   <211> 37
   <212> DNA
   <213> Gevo-611
<400> 120
   ttttctcgag ttacttcttc ttcgctttcg ggttcgg 37
<210> 121
   <211> 21
   <212> DNA
   <213> Gevo-616
<400> 121
   ctatttacca ggctaaattc c 21
<210> 122
   <211> 22
   <212> DNA
   <213> Gevo-617
<400> 122
   tgaaggtaaa aacatcgcgc ac 22
<210> 123
   <211> 23
   <212> DNA
   <213> Gevo-618
<400> 123
   cgtggcttcc tgatcggcgg tac 23
<210> 124
   <211> 24
   <212> DNA
   <213> Gevo-619
<400> 124
   caccagcggc tggcgtgaaa gaag 24
<210> 125
   <211> 24
   <212> DNA
   <213> Gevo-620
<400> 125
   gaagtggaac tgggccgcat ccag 24
<210> 126
   <211> 22
   <212> DNA
   <213> Gevo-621
<400> 126
   gacgtggttg aaatgttcga cc 22
<210> 127
   <211> 35
   <212> DNA
   <213> Gevo-637
<400> 127
   ttttgagctc gccgatccca ttaccgacat ttggg 35
<210> 128
   <211> 96
   <212> DNA
   <213> Gevo-638
<400> 128
   aaagtcgaca ccgatatacc tgtatgtgtc accaccaatg tatctataag tatccatgct 60
   agccctaggt ttatgtgatg attgattgat tgattg 96
<210> 129
   <211> 36
   <212> DNA
   <213> Gevo-639
<400> 129
   ttttctcgag actagtatgt ctgaaattac tttggg 36
<210> 130
   <211> 36
   <212> DNA
   <213> Gevo-640
<400> 130
   ttttggatcc ttattgctta gcgttggtag cagcag 36
<210> 131
   <211> 27
   <212> DNA
   <213> Gevo-641
<400> 131
   gttatcttgg ctgatgcttg ttgttcc 27
<210> 132
   <211> 41
   <212> DNA
   <213> Gevo-642
<400> 132
   ttttgtcgac actagtatga gtactgaaat caaaactcag g 41
<210> 133
   <211> 33
   <212> DNA
   <213> Gevo-643
<400> 133
   ccaagtcgac atgactaagc tacactttga cac 33
<210> 134
   <211> 27
   <212> DNA
   <213> Gevo-644
<400> 134
   gtcggtaaga gtgttgctgt ggactcg 27
<210> 135
   <211> 42
   <212> DNA
   <213> Gevo-646
<400> 135
   ccaaggatcc ttacaactta attctgacag cttttacttc ag 42
<210> 136
   <211> 49
   <212> DNA
   <213> Gevo-653
<400> 136
   ttttgtcgac actagtatgg ctatcgaaat caaagtaccg gacatcggg 49
<210> 137
   <211> 25
   <212> DNA
   <213> Gevo-654
<400> 137
   cttccataga agctttgtcg ccttc 25
<210> 138
   <211> 25
   <212> DNA
   <213> Gevo-655
<400> 138
   gtgcaatatc atatagaagt catcg 25
<210> 139
   <211> 48
   <212> DNA
   <213> Gevo-656
<400> 139
   ttttctcgag gctagcatgg catcgtaccc agagcacacc attattgg 48
<210> 140
   <211> 40
   <212> DNA
   <213> Gevo-657
<400> 140
   ttttggatcc tcacaatagc atttccaaag gattttcaat 40
<210> 141
   <211> 45
   <212> DNA
   <213> Gevo-658
<400> 141
   ttttctcgag actagtatgg tcatcatcgg tggtggccct gctgg 45
<210> 142
   <211> 36
   <212> DNA
   <213> Gevo-659
<400> 142
   ttttggatcc tcaacaatga atagctttat catagg 36
<210> 143
   <211> 47
   <212> DNA
   <213> Gevo-660
<400> 143
   ttttctcgag actagtatgg caactttaaa aacaactgat aagaagg 47
<210> 144
   <211> 35
   <212> DNA
   <213> Gevo-661
<400> 144
   ttttagatct ttaatcccta gaggcaaaac cttgc 35
<210> 145
   <211> 44
   <212> DNA
   <213> Gevo-662
<400> 145
   ttttctcgag actagtatgg cggaagaatt ggaccgtgat gatg 44
<210> 146
   <211> 38
   <212> DNA
   <213> Gevo-663
<400> 146
   tttggatcct tattcaattg acaagacttc tttgacag 38
<210> 147
   <211> 48
   <212> DNA
   <213> Gevo-664
<400> 147
   ttttctcgag actagtatgt tacttgctgt aaagacattt tcaatgcc 48
<210> 148
   <211> 40
   <212> DNA
   <213> Gevo-665
<400> 148
   ttttggatcc tcaaaatgat tctaactccc ttacgtaatc 40
<210> 149
   <211> 27
   <212> DNA
   <213> Gevo-666
<400> 149
   ggtagaatta ccaaggctga cattgag 27
<210> 150
   <211> 21
   <212> DNA
   <213> Gevo-667
<400> 150
   cattggtgga ggaatcatcg g 21
<210> 151
   <211> 24
   <212> DNA
   <213> Gevo-668
<400> 151
   caccaataca agaacgagga cgcc 24
<210> 152
   <211> 28
   <212> DNA
   <213> Gevo-669
<400> 152
   tggtacggtt ccattccagg gttaaagg 28
<210> 153
   <211> 25
   <212> DNA
   <213> Gevo-670
<400> 153
   gggtgatgtg ctagcatacc taggg 25
<210> 154
   <211> 1197
   <212> DNA
   <213> ERG10
<400> 154
   atgtctcaga acgtttacat tgtatcgact gccagaaccc caattggttc attccagggt 60
   tctctatcct ccaagacagc agtggaattg ggtgctgttg ctttaaaagg cgccttggct 120
   aaggttccag aattggatgc atccaaggat tttgacgaaa ttatttttgg taacgttctt 180
   tctgccaatt tgggccaagc tccggccaga caagttgctt tggctgccgg tttgagtaat 240
   catatcgttg caagcacagt taacaaggtc tgtgcatccg ctatgaaggc aatcattttg 300
   ggtgctcaat ccatcaaatg tggtaatgct gatgttgtcg tagctggtgg ttgtgaatct 360
   atgactaacg caccatacta catgccagca gcccgtgcgg gtgccaaatt tggccaaact 420
   gttcttgttg atggtgtcga aagagatggg ttgaacgatg cgtacgatgg tctagccatg 480
   ggtgtacacg cagaaaagtg tgcccgtgat tgggatatta ctagagaaca acaagacaat 540
   tttgccatcg aatcctacca aaaatctcaa aaatctcaaa aggaaggtaa attcgacaat 600
   gaaattgtac ctgttaccat taagggattt agaggtaagc ctgatactca agtcacgaag 660
   gacgaggaac ctgctagatt acacgttgaa aaattgagat ctgcaaggac tgttttccaa 720
   aaagaaaacg gtactgttac tgccgctaac gcttctccaa tcaacgatgg tgctgcagcc 780
   gtcatcttgg tttccgaaaa agttttgaag gaaaagaatt tgaagccttt ggctattatc 840
   aaaggttggg gtgaggccgc tcatcaacca gctgatttta catgggctcc atctcttgca 900
   gttccaaagg ctttgaaaca tgctggcatc gaagacatca attctgttga ttactttgaa 960
   ttcaatgaag ccttttcggt tgtcggtttg gtgaacacta agattttgaa gctagaccca 1020
   tctaaggtta atgtatatgg tggtgctgtt gctctaggtc acccattggg ttgttctggt 1080
   gctagagtgg ttgttacact gctatccatc ttacagcaag aaggaggtaa gatcggtgtt 1140
   gccgccattt gtaatggtgg tggtggtgct tcctctattg tcattgaaaa gatatga 1197
<210> 155
   <211> 849
   <212> DNA
   <213> Cb-hbd
<400> 155
   atgaaaaaga tttttgtact tggagcagga acaatgggtg ctggtatcgt tcaagcattc 60
   gctcaaaaag gttgtgaagt aattgtaaga gacataaagg aagaatttgt tgacagagga 120
   atagctggaa tcactaaagg attagaaaag caagttgcta aggaaaaaat gtctgaagaa 180
   gataaagaag ctatactttc aagaatttca ggaacaactg atatgaaatt agctgctgac 240
   tgtgatttag tagttgaagc tgcaatcgaa aacatgaaaa ttaagaagga aatcttcgct 300
   gaattagatg gaatttgtaa gccagaagcg attttagctt caaacacttc atctttatca 360
   attactgaag ttgcttcagc tacaaagaga cctgataaag ttatcggaat gcatttcttt 420
   aatccagctc cagtaatgaa gcttgttgaa attattaaag gaatagctac ttctcaagaa 480
   acttttgatg ctgttaagga attatcagtt gctattggaa aagaaccagt agaagttgca 540
   gaagctccag gattcgttgt aaacagaata ttaatcccaa tgattaacga agcttcattt 600
   atcctacaag aaggaatagc ttcagttgaa gatattgata cagctatgaa atatggtgct 660
   aaccatccaa tgggaccttt agctttagga gatcttattg gattagacgt ttgcttagct 720
   atcatggatg ttttattcac tgaaacaggt gataacaagt acagagctag cagcatatta 780
   agaaaatatg ttagagctgg atggcttgga agaaaatcag gaaaaggatt ctatgattat 840
   tctaaataa 849
<210> 156
   <211> 786
   <212> DNA
   <213> Cb-crt
<400> 156
   atggaattaa aaaatgttat tcttgaaaaa gaagggcatt tagctattgt tacaatcaat 60
   agaccaaagg cattaaatgc attgaattca gaaacactaa aagatttaaa tgttgtttta 120
   gatgatttag aagcagacaa caatgtgtat gcagttatag ttacaggtgc tggtgagaaa 180
   tcttttgttg ctggagcaga tatttcagaa atgaaagatc ttaatgaaga acaaggtaaa 240
   gaatttggta ttttaggaaa caatgtcttc agaagattag aaaaattgga taagccagtt 300
   atcgcagcta tatcaggatt tgctcttggt ggtggatgtg aacttgctat gtcatgtgac 360
   ataagaatag cttcagttaa agctaaattt ggtcaaccag aagcaggact tggaataact 420
   ccaggatttg gtggaactca aagattagct agaattgtag ggccaggaaa agctaaagaa 480
   ttaatttata cttgtgacct tataaatgca gaagaagctt atagaatagg tttagttaat 540
   aaagtagttg aattagaaaa attgatggaa gaagcaaaag caatggctaa caagattgca 600
   gctaatgctc caaaagcagt tgcatattgt aaagatgcta tagacagagg aatgcaagtt 660
   gatatagatg cagctatatt aatagaagca gaagactttg gaaagtgctt tgcaacagaa 720
   gatcaaacag aaggaatgac tgcgttctta gaaagaagag cagaaaagaa ttttcaaaat 780
   aaataa 786
<210> 157
   <211> 1140
   <212> DNA
   <213> Cb-bcd
<400> 157
   atgaatttcc aattaactag agaacaacaa ttagtacaac aaatggttag agaattcgca      60
   gtaaatgaag ttaagccaat agctgctgaa atcgacgaat cagaaagatt ccctatggaa     120
   aacgttgaaa aaatggctaa gcttaaaatg atgggtatcc cattttctaa agaatttggt     180
   ggagcaggcg gagatgttct ttcatatata atatctgtgg aagaattatc aaaagtttgt     240
   ggtactacag gagttattct ttcagcgcat acatcattat gtgcatcagt aattaatgaa     300
   aatggaacta acgaacaaag agcaaaatat ttgccagatc tttgtagtgg taagaaaatc     360
   ggtgctttcg gattaacaga accaggcgct ggtacagatg ctgcaggaca acaaacaact     420
   gctgtattag aaggagacca ttatgtatta aatggttcaa aaatcttcat aacaaatggt     480
   ggagttgctg aaactttcat aatatttgct atgacagata agagtcaagg aacaaaagga     540
   atttctgcat tcatagtaga aaagtcattc ccaggattct caataggaaa attagaaaac     600
   aagatgggga tcagagcatc ttcaactact gagttagtta tggaaaactg tatagtacca     660
   aaagaaaacc tacttagcaa agaaggtaag ggatttggta tagcaatgaa aactcttgat     720
   ggaggaagaa ttggtatagc tgctcaagct ttaggtattg cagaaggagc ttttgaagaa     780
   gctgttaact atatgaaaga aagaaaacaa tttggtaaac cattatcagc attccaagga     840
   ttacaatggt atatagctga aatggatgtt aaaatccaag ctgctaaata cttagtatac     900
   ctagctgcaa caaagaagca agctggtgag ccttactcag tggatgctgc aagagctaaa     960
   ttatttgcgg cagatgttgc aatggaagtt acaactaaag cagttcaaat ctttggtgga    1020
   tatggttaca ctaaggaata cccagtagaa agaatgatga gagatgctaa aatatgcgaa    1080
   atctacgaag gaacttcaga agttcaaaag atggttatcg caggaagcat tttaagatag    1140
<210> 158
   <211> 1140
   <212> DNA
   <213> Cb-etfA
<400> 158
   atgaatttcc aattaactag agaacaacaa ttagtacaac aaatggttag agaattcgca 60
   gtaaatgaag ttaagccaat agctgctgaa atcgacgaat cagaaagatt ccctatggaa 120
   aacgttgaaa aaatggctaa gcttaaaatg atgggtatcc cattttctaa agaatttggt 180
   ggagcaggcg gagatgttct ttcatatata atatctgtgg aagaattatc aaaagtttgt 240
   ggtactacag gagttattct ttcagcgcat acatcattat gtgcatcagt aattaatgaa 300
   aatggaacta acgaacaaag agcaaaatat ttgccagatc tttgtagtgg taagaaaatc 360
   ggtgctttcg gattaacaga accaggcgct ggtacagatg ctgcaggaca acaaacaact 420
   gctgtattag aaggagacca ttatgtatta aatggttcaa aaatcttcat aacaaatggt 480
   ggagttgctg aaactttcat aatatttgct atgacagata agagtcaagg aacaaaagga 540
   atttctgcat tcatagtaga aaagtcattc ccaggattct caataggaaa attagaaaac 600
   aagatgggga tcagagcatc ttcaactact gagttagtta tggaaaactg tatagtacca 660
   aaagaaaacc tacttagcaa agaaggtaag ggatttggta tagcaatgaa aactcttgat 720
   ggaggaagaa ttggtatagc tgctcaagct ttaggtattg cagaaggagc ttttgaagaa 780
   gctgttaact atatgaaaga aagaaaacaa tttggtaaac cattatcagc attccaagga 840
   ttacaatggt atatagctga aatggatgtt aaaatccaag ctgctaaata cttagtatac 900
   ctagctgcaa caaagaagca agctggtgag ccttactcag tggatgctgc aagagctaaa 960
   ttatttgcgg cagatgttgc aatggaagtt acaactaaag cagttcaaat ctttggtgga 1020
   tatggttaca ctaaggaata cccagtagaa agaatgatga gagatgctaa aatatgcgaa 1080
   atctacgaag gaacttcaga agttcaaaag atggttatcg caggaagcat tttaagatag 1140
<210> 159
   <211> 780
   <212> DNA
   <213> Cb-etfB
<400> 159
   atgaatatag tagtttgtgt aaaacaagtt ccagatacta cagcagtaaa aattgatcct      60
   aaaactggta cattaataag agatggtgtt ccatcaataa tgaatccaga ggataaacac     120
   gctttagaag gtgcattaca attaaaagaa aaagttggag gaaaagttac tgtaataagt     180
   atgggacttc caatggctaa agcagtatta agagaagcat tatgtatggg agctgatgaa     240
   gctgtcctat taacagatag agcacttgga ggagcagata ctttagcaac ttcaaaggca     300
   cttgcaggag taatagctaa gttagattat gatttggtat ttgctggaag acaagcaatt     360
   gatggagata ctgcacaagt aggaccagaa atagcagaac atttaaacat tccgcaagta     420
   acttacgttc aagacgttaa agttgaagga aatacattaa tagtaaatag agcactagaa     480
   gatggacatc aagtagtaga agttaaaact ccatgtctat taactgcaat cgaagaatta     540
   aatgaaacta gatatatgaa tgttgtagat atattcgaaa cttcagatga tgaaatcaaa     600
   gttatgagcg cagctgatat agatgtagat gtagctgaat tagggcttaa aggctcacct     660
   acaaaggtta agaagtcaat gactaaggaa gttaaaggtg caggagaaat cgtaagagaa     720
   gcacctaaaa atgcagcata ctatgttgta ggaaaattaa aagaaaaaca ctacatctaa     780
<210> 160
   <211> 1167
   <212> DNA
   <213> Cb-adhA
<400> 160
   atggcacgtt ttactttacc aagagacatt tatcatggag aaggagcact tgaggcactt      60
   aaaactttaa aaggtaagaa agctttctta gtagttggtg gcggatcaat gaaaagattt     120
   ggatttctta aacaagttga agattattta aaagaagcag gaatggaagt agaattattt     180
   gaaggtgttg aaccagatcc atcagtggaa acagtaatga aaggcgcaga agctatgaga     240
   aactttgagc ctgattggat agttgcaatg ggtggaggat caccaattga tgctgcaaag     300
   gctatgtgga tattctacga atacccagat tttacttttg aacaagcagt tgttccattt    360
   ggattaccag accttagaca aaaagctaag tttgtagcta ttccatcaac aagcggtaca    420
   gctacagaag ttacagcatt ctcagttatc acaaattatt cagaaaaaat taaatatcct    480
   ttagctgatt ttaacataac tccagatata gcaatagttg atccagcact tgctcaaact    540
   atgccaaaaa ctttaacagc tcatactgga atggatgcat taactcacgc tatagaagca    600
   tacactgcat cacttcaatc aaatttctca gatccattag caattaaagc tgtagaaatg    660
   gttcaagaaa atttaatcaa atcatttgaa ggagataaag aagctagaaa tctaatgcat    720
   gaagctcaat gtttagctgg aatggcattt tctaatgcat tacttggaat agttcactca    780
   atggctcata aggttggtgc tgtattccat attcctcatg gatgtgcaaa tgctatattt    840
   ttaccatatg taattgagta taacagaaca aaatgcgaaa atagatatgg agatattgcg    900
   agagccttaa aattaaaagg aaacaatgat gccgagttaa ctgattcatt aattgaatta    960
   attaatggat taaatgataa gttagagatt cctcactcaa tgaaagagta tggagttact   1020
   gaagaagatt ttaaagctaa tctttcattt atcgctcata acgcagtatt agatgcatgc   1080
   acaggatcaa atcctagaga aatagatgat gctacaatgg aaaaattatt tgaatgcaca   1140
   tactatggaa ctaaagttaa tttgtaa                                       1167
<210> 161
   <211> 1407
   <212> DNA
   <213> Cb-aldh
<400> 161
   atgaataaag acacactaat acctacaact aaagatttaa aagtaaaaac aaatggtgaa 60
   aacattaatt taaagaacta caaggataat tcttcatgtt tcggagtatt cgaaaatgtt 120
   gaaaatgcta taagcagcgc tgtacacgca caaaagatat tatcccttca ttatacaaaa 180
   gagcaaagag aaaaaatcat aactgagata agaaaggccg cattacaaaa taaagaggtc 240
   ttggctacaa tgattctaga agaaacacat atgggaagat atgaggataa aatattaaaa 300
   catgaattgg tagctaaata tactcctggt acagaagatt taactactac tgcttggtca 360
   ggtgataatg gtcttacagt tgtagaaatg tctccatatg gtgttatagg tgcaataact 420
   ccttctacga atccaactga aactgtaata tgtaatagca taggcatgat agctgctgga 480
   aatgctgtag tatttaacgg acacccatgc gctaaaaaat gtgttgcctt tgctgttgaa 540
   atgataaata aggcaattat ttcatgtggc ggtcctgaaa atctagtaac aactataaaa 600
   aatccaacta tggagtctct agatgcaatt attaagcatc cttcaataaa acttctttgc 660
   ggaactgggg gtccaggaat ggtaaaaacc ctcttaaatt ctggtaagaa agctataggt 720
   gctggtgctg gaaatccacc agttattgta gatgatactg ctgatataga aaaggctggt 780
   aggagcatca ttgaaggctg ttcttttgat aataatttac cttgtattgc agaaaaagaa 840
   gtatttgttt ttgagaatgt tgcagatgat ttaatatcta acatgctaaa aaataatgct 900
   gtaattataa atgaagatca agtatcaaaa ttaatagatt tagtattaca aaaaaataat 960
   gaaactcaag aatactttat aaacaaaaaa tgggtaggaa aagatgcaaa attattctta 1020
   gatgaaatag atgttgagtc tccttcaaat gttaaatgca taatctgcga agtaaatgca 1080
   aatcatccat ttgttatgac agaactcatg atgccaatat tgccaattgt aagagttaaa 1140
   gatatagatg aagctattaa atatgcaaag atagcagaac aaaatagaaa acatagtgcc 1200
   tatatttatt ctaaaaatat agacaaccta aatagatttg aaagagaaat agatactact 1260
   atttttgtaa agaatgctaa atcttttgct ggtgttggtt atgaagcaga aggatttaca 1320
   actttcacta ttgctggatc tactggtgag ggaataacct ctgcaaggaa ttttacaaga 1380
   caaagaagat gtgtacttgc cggctaa 1407
<210> 162
   <211> 1179
   <212> DNA
   <213> Ca-thl
<400> 162
   atgaaagaag ttgtaatagc tagtgcagta agaacagcga ttggatctta tggaaagtct 60
   cttaaggatg taccagcagt agatttagga gctacagcta taaaggaagc agttaaaaaa 120
   gcaggaataa aaccagagga tgttaatgaa gtcattttag gaaatgttct tcaagcaggt 180
   ttaggacaga atccagcaag acaggcatct tttaaagcag gattaccagt tgaaattcca 240
   gctatgacta ttaataaggt ttgtggttca ggacttagaa cagttagctt agcagcacaa 300
   attataaaag caggagatgc tgacgtaata atagcaggtg gtatggaaaa tatgtctaga 360
   gctccttact tagcgaataa cgctagatgg ggatatagaa tgggaaacgc taaatttgtt 420
   gatgaaatga tcactgacgg attgtgggat gcatttaatg attaccacat gggaataaca 480
   gcagaaaaca tagctgagag atggaacatt tcaagagaag aacaagatga gtttgctctt 540
   gcatcacaaa aaaaagctga agaagctata aaatcaggtc aatttaaaga tgaaatagtt 600
   cctgtagtaa ttaaaggcag aaagggagaa actgtagttg atacagatga gcaccctaga 660
   tttggatcaa ctatagaagg acttgcaaaa ttaaaacctg ccttcaaaaa agatggaaca 720
   gttacagctg gtaatgcatc aggattaaat gactgtgcag cagtacttgt aatcatgagt 780
   gcagaaaaag ctaaagagct tggagtaaaa ccacttgcta agatagtttc ttatggttca 840
   gcaggagttg acccagcaat aatgggatat ggacctttct atgcaacaaa agcagctatt 900
   gaaaaagcag gttggacagt tgatgaatta gatttaatag aatcaaatga agcttttgca 960
   gctcaaagtt tagcagtagc aaaagattta aaatttgata tgaataaagt aaatgtaaat 1020
   ggaggagcta ttgcccttgg tcatccaatt ggagcatcag gtgcaagaat actcgttact 1080
   cttgtacacg caatgcaaaa aagagatgca aaaaaaggct tagcaacttt atgtataggt 1140
   ggcggacaag gaacagcaat attgctagaa aagtgctag 1179
<210> 163
   <211> 849
   <212> DNA
   <213> Ca-hbd
<400> 163
   atgaaaaagg tatgtgttat aggtgcaggt actatgggtt caggaattgc tcaggcattt      60
   gcagctaaag gatttgaagt agtattaaga gatattaaag atgaatttgt tgatagagga     120
   ttagatttta tcaataaaaa tctttctaaa ttagttaaaa aaggaaagat agaagaagct     180
   actaaagttg aaatcttaac tagaatttcc ggaacagttg accttaatat ggcagctgat     240
   tgcgatttag ttatagaagc agctgttgaa agaatggata ttaaaaagca gatttttgct     300
   gacttagaca atatatgcaa gccagaaaca attcttgcat caaatacatc atcactttca     360
   ataacagaag tggcatcagc aactaaaaga cctgataagg ttataggtat gcatttcttt     420
   aatccagctc ctgttatgaa gcttgtagag gtaataagag gaatagctac atcacaagaa     480
   acttttgatg cagttaaaga gacatctata gcaataggaa aagatcctgt agaagtagca     540
   gaagcaccag gatttgttgt aaatagaata ttaataccaa tgattaatga agcagttggt     600
   atattagcag aaggaatagc ttcagtagaa gacatagata aagctatgaa acttggagct     660
   aatcacccaa tgggaccatt agaattaggt gattttatag gtcttgatat atgtcttgct     720
   ataatggatg ttttatactc agaaactgga gattctaagt atagaccaca tacattactt     780
   aagaagtatg taagagcagg atggcttgga agaaaatcag gaaaaggttt ctacgattat     840
   tcaaaataa                                                             849
<210> 164
   <211> 786
   <212> DNA
   <213> Ca-crt
<400> 164
   atggaactaa acaatgtcat ccttgaaaag gaaggtaaag ttgctgtagt taccattaac      60
   agacctaaag cattaaatgc gttaaatagt gatacactaa aagaaatgga ttatgttata     120
   ggtgaaattg aaaatgatag cgaagtactt gcagtaattt taactggagc aggagaaaaa     180
   tcatttgtag caggagcaga tatttctgag atgaaggaaa tgaataccat tgaaggtaga     240
   aaattcggga tacttggaaa taaagtgttt agaagattag aacttcttga aaagcctgta     300
   atagcagctg ttaatggttt tgctttagga ggcggatgcg aaatagctat gtcttgtgat     360
   ataagaatag cttcaagcaa cgcaagattt ggtcaaccag aagtaggtct cggaataaca     420
   cctggttttg gtggtacaca aagactttca agattagttg gaatgggcat ggcaaagcag     480
   cttatattta ctgcacaaaa tataaaggca gatgaagcat taagaatcgg acttgtaaat     540
   aaggtagtag aacctagtga attaatgaat acagcaaaag aaattgcaaa caaaattgtg     600
   agcaatgctc cagtagctgt taagttaagc aaacaggcta ttaatagagg aatgcagtgt 660
   gatattgata ctgctttagc atttgaatca gaagcatttg gagaatgctt ttcaacagag 720
   gatcaaaagg atgcaatgac agctttcata gagaaaagaa aaattgaagg cttcaaaaat 780
   agatag 786
<210> 165
   <211> 1140
   <212> DNA
   <213> Ca-bcd
<400> 165
   atggatttta atttaacaag agaacaagaa ttagtaagac agatggttag agaatttgct 60
   gaaaatgaag ttaaacctat agcagcagaa attgatgaaa cagaaagatt tccaatggaa 120
   aatgtaaaga aaatgggtca gtatggtatg atgggaattc cattttcaaa agagtatggt 180
   ggcgcaggtg gagatgtatt atcttatata atcgccgttg aggaattatc aaaggtttgc 240
   ggtactacag gagttattct ttcagcacat acatcacttt gtgcttcatt aataaatgaa 300
   catggtacag aagaacaaaa acaaaaatat ttagtacctt tagctaaagg tgaaaaaata 360
   ggtgcttatg gattgactga gccaaatgca ggaacagatt ctggagcaca acaaacagta 420
   gctgtacttg aaggagatca ttatgtaatt aatggttcaa aaatattcat aactaatgga 480
   ggagttgcag atacttttgt tatatttgca atgactgaca gaactaaagg aacaaaaggt 540
   atatcagcat ttataataga aaaaggcttc aaaggtttct ctattggtaa agttgaacaa 600
   aagcttggaa taagagcttc atcaacaact gaacttgtat ttgaagatat gatagtacca 660
   gtagaaaaca tgattggtaa agaaggaaaa ggcttcccta tagcaatgaa aactcttgat 720
   ggaggaagaa ttggtatagc agctcaagct ttaggtatag ctgaaggtgc tttcaacgaa 780
   gcaagagctt acatgaagga gagaaaacaa tttggaagaa gccttgacaa attccaaggt 840
   cttgcatgga tgatggcaga tatggatgta gctatagaat cagctagata tttagtatat 900
   aaagcagcat atcttaaaca agcaggactt ccatacacag ttgatgctgc aagagctaag 960
   cttcatgctg caaatgtagc aatggatgta acaactaagg cagtacaatt atttggtgga 1020
   tacggatata caaaagatta tccagttgaa agaatgatga gagatgctaa gataactgaa 1080
   atatatgaag gaacttcaga agttcagaaa ttagttattt caggaaaaat ttttagataa 1140
<210> 166
   <211> 1011
   <212> DNA
   <213> Ca-etfA
<400> 166
   atgaataaag cagattacaa gggcgtatgg gtgtttgctg aacaaagaga cggagaatta      60
   caaaaggtat cattggaatt attaggtaaa ggtaaggaaa tggctgagaa attaggcgtt     120
   gaattaacag ctgttttact tggacataat actgaaaaaa tgtcaaagga tttattatct     180
   catggagcag ataaggtttt agcagcagat aatgaacttt tagcacattt ttcaacagat     240
   ggatatgcta aagttatatg tgatttagtt aatgaaagaa agccagaaat attattcata     300
   ggagctactt tcataggaag agatttagga ccaagaatag cagcaagact ttctactggt     360
   ttaactgctg attgtacatc acttgacata gatgtagaaa atagagattt attggctaca     420
   agaccagcgt ttggtggaaa tttgatagct acaatagttt gttcagacca cagaccacaa     480
   atggctacag taagacctgg tgtgtttgaa aaattacctg ttaatgatgc aaatgtttct     540
   gatgataaaa tagaaaaagt tgcaattaaa ttaacagcat cagacataag aacaaaagtt     600
   tcaaaagttg ttaagcttgc taaagatatt gcagatatcg gagaagctaa ggtattagtt     660
   gctggtggta gaggagttgg aagcaaagaa aactttgaaa aacttgaaga gttagcaagt     720
   ttacttggtg gaacaatagc cgcttcaaga gcagcaatag aaaaagaatg ggttgataag     780
   gaccttcaag taggtcaaac tggtaaaact gtaagaccaa ctctttatat tgcatgtggt     840
   atatcaggag ctatccagca tttagcaggt atgcaagatt cagattacat aattgctata     900
   aataaagatg tagaagcccc aataatgaag gtagcagatt tggctatagt tggtgatgta     960
   aataaagttg taccagaatt aatagctcaa gttaaagctg ctaataatta a             1011
<210> 167
   <211> 780
   <212> DNA
   <213> Ca-etfB
<400> 167
   atgaatatag ttgtttgttt aaaacaagtt ccagatacag cggaagttag aatagatcca     60
   gttaagggaa cacttataag agaaggagtt ccatcaataa taaatccaga tgataaaaac    120
   gcacttgagg aagctttagt attaaaagat aattatggtg cacatgtaac agttataagt    180
   atgggacctc cacaagctaa aaatgcttta gtagaagctt tggctatggg tgctgatgaa    240
   gctgtacttt taacagatag agcatttgga ggagcagata cacttgcgac ttcacataca    300
   attgcagcag gaattaagaa gctaaaatat gatatagttt ttgctggaag gcaggctata    360
   gatggagata cagctcaggt tggaccagaa atagctgagc atcttggaat acctcaagta    420
   acttatgttg agaaagttga agttgatgga gatactttaa agattagaaa agcttgggaa    480
   gatggatatg aagttgttga agttaagaca ccagttcttt taacagcaat taaagaatta    540
   aatgttccaa gatatatgag tgtagaaaaa atattcggag catttgataa agaagtaaaa    600
   atgtggactg ccgatgatat agatgtagat aaggctaatt taggtcttaa aggttcacca    660
   actaaagtta agaagtcatc aactaaagaa gttaaaggac agggagaagt tattgataag    720
   cctgttaagg aagcagctgc atatgttgtc tcaaaattaa aagaagaaca ctatatttaa    780
<210> 168
   <211> 2577
   <212> DNA
   <213> Ca-adhE2
<400> 168
   atgaaagtta caaatcaaaa agaactaaaa caaaagctaa atgaattgag agaagcgcaa 60
   aagaagtttg caacctatac tcaagagcaa gttgataaaa tttttaaaca atgtgccata 120
   gccgcagcta aagaaagaat aaacttagct aaattagcag tagaagaaac aggaataggt 180
   cttgtagaag ataaaattat aaaaaatcat tttgcagcag aatatatata caataaatat 240
   aaaaatgaaa aaacttgtgg cataatagac catgacgatt ctttaggcat aacaaaggtt 300
   gctgaaccaa ttggaattgt tgcagccata gttcctacta ctaatccaac ttccacagca 360
   attttcaaat cattaatttc tttaaaaaca agaaacgcaa tattcttttc accacatcca 420
   cgtgcaaaaa aatctacaat tgctgcagca aaattaattt tagatgcagc tgttaaagca 480
   ggagcaccta aaaatataat aggctggata gatgagccat caatagaact ttctcaagat 540
   ttgatgagtg aagctgatat aatattagca acaggaggtc cttcaatggt taaagcggcc 600
   tattcatctg gaaaacctgc aattggtgtt ggagcaggaa atacaccagc aataatagat 660
   gagagtgcag atatagatat ggcagtaagc tccataattt tatcaaagac ttatgacaat 720
   ggagtaatat gcgcttctga acaatcaata ttagttatga attcaatata cgaaaaagtt 780
   aaagaggaat ttgtaaaacg aggatcatat atactcaatc aaaatgaaat agctaaaata 840
   aaagaaacta tgtttaaaaa tggagctatt aatgctgaca tagttggaaa atctgcttat 900
   ataattgcta aaatggcagg aattgaagtt cctcaaacta caaagatact tataggcgaa 960
   gtacaatctg ttgaaaaaag cgagctgttc tcacatgaaa aactatcacc agtacttgca 1020
   atgtataaag ttaaggattt tgatgaagct ctaaaaaagg cacaaaggct aatagaatta 1080
   ggtggaagtg gacacacgtc atctttatat atagattcac aaaacaataa ggataaagtt 1140
   aaagaatttg gattagcaat gaaaacttca aggacattta ttaacatgcc ttcttcacag 1200
   ggagcaagcg gagatttata caattttgcg atagcaccat catttactct tggatgcggc 1260
   acttggggag gaaactctgt atcgcaaaat gtagagccta aacatttatt aaatattaaa 1320
   agtgttgctg aaagaaggga aaatatgctt tggtttaaag tgccacaaaa aatatatttt 1380
   aaatatggat gtcttagatt tgcattaaaa gaattaaaag atatgaataa gaaaagagcc 1440
   tttatagtaa cagataaaga tctttttaaa cttggatatg ttaataaaat aacaaaggta 1500
   ctagatgaga tagatattaa atacagtata tttacagata ttaaatctga tccaactatt 1560
   gattcagtaa aaaaaggtgc taaagaaatg cttaactttg aacctgatac tataatctct 1620
   attggtggtg gatcgccaat ggatgcagca aaggttatgc acttgttata tgaatatcca 1680
   gaagcagaaa ttgaaaatct agctataaac tttatggata taagaaagag aatatgcaat 1740
   ttccctaaat taggtacaaa ggcgatttca gtagctattc ctacaactgc tggtaccggt   1800
   tcagaggcaa caccttttgc agttataact aatgatgaaa caggaatgaa atacccttta   1860
   acttcttatg aattgacccc aaacatggca ataatagata ctgaattaat gttaaatatg 1920
   cctagaaaat taacagcagc aactggaata gatgcattag ttcatgctat agaagcatat 1980
   gtttcggtta tggctacgga ttatactgat gaattagcct taagagcaat aaaaatgata 2040
   tttaaatatt tgcctagagc ctataaaaat gggactaacg acattgaagc aagagaaaaa 2100
   atggcacatg cctctaatat tgcggggatg gcatttgcaa atgctttctt aggtgtatgc 2160
   cattcaatgg ctcataaact tggggcaatg catcacgttc cacatggaat tgcttgtgct 2220
   gtattaatag aagaagttat taaatataac gctacagact gtccaacaaa gcaaacagca 2280
   ttccctcaat ataaatctcc taatgctaag agaaaatatg ctgaaattgc agagtatttg 2340
   aatttaaagg gtactagcga taccgaaaag gtaacagcct taatagaagc tatttcaaag 2400
   ttaaagatag atttgagtat tccacaaaat ataagtgccg ctggaataaa taaaaaagat 2460
   ttttataata cgctagataa aatgtcagag cttgcttttg atgaccaatg tacaacagct 2520
   aatcctaggt atccacttat aagtgaactt aaggatatct atataaaatc attttaa 2577
<210> 169
   <211> 2589
   <212> DNA
   <213> Ca-aad
<400> 169
   atgaaagtca caacagtaaa ggaattagat gaaaaactca aggtaattaa agaagctcaa      60
   aaaaaattct cttgttactc gcaagaaatg gttgatgaaa tctttagaaa tgcagcaatg     120
   gcagcaatcg acgcaaggat agagctagca aaagcagctg ttttggaaac cggtatgggc     180
   ttagttgaag acaaggttat aaaaaatcat tttgcaggcg aatacatcta taacaaatat     240
   aaggatgaaa aaacctgcgg tataattgaa cgaaatgaac cctacggaat tacaaaaata     300
   gcagaaccta taggagttgt agctgctata atccctgtaa caaaccccac atcaacaaca     360
   atatttaaat ccttaatatc ccttaaaact agaaatggaa ttttcttttc gcctcaccca     420
   agggcaaaaa aatccacaat actagcagct aaaacaatac ttgatgcagc cgttaagagt     480
   ggtgccccgg aaaatataat aggttggata gatgaacctt caattgaact aactcaatat     540
   ttaatgcaaa aagcagatat aacccttgca actggtggtc cctcactagt taaatctgct     600
   tattcttccg gaaaaccagc aataggtgtt ggtccgggta acaccccagt aataattgat     660
   gaatctgctc atataaaaat ggcagtaagt tcaattatat tatccaaaac ctatgataat     720
   ggtgttatat gtgcttctga acaatctgta atagtcttaa aatccatata taacaaggta     780
   aaagatgagt tccaagaaag aggagcttat ataataaaga aaaacgaatt ggataaagtc     840
   cgtgaagtga tttttaaaga tggatccgta aaccctaaaa tagtcggaca gtcagcttat     900
   actatagcag ctatggctgg cataaaagta cctaaaacca caagaatatt aataggagaa     960
   gttacctcct taggtgaaga agaacctttt gcccacgaaa aactatctcc tgttttggct 1020
   atgtatgagg ctgacaattt tgatgatgct ttaaaaaaag cagtaactct aataaactta 1080
   ggaggcctcg gccatacctc aggaatatat gcagatgaaa taaaagcacg agataaaata 1140
   gatagattta gtagtgccat gaaaaccgta agaacctttg taaatatccc aacctcacaa 1200
   ggtgcaagtg gagatctata taattttaga ataccacctt ctttcacgct tggctgcgga 1260
   ttttggggag gaaattctgt ttccgagaat gttggtccaa aacatctttt gaatattaaa 1320
   accgtagctg aaaggagaga aaacatgctt tggtttagag ttccacataa agtatatttt 1380
   aagttcggtt gtcttcaatt tgctttaaaa gatttaaaag atctaaagaa aaaaagagcc 1440
   tttatagtta ctgatagtga cccctataat ttaaactatg ttgattcaat aataaaaata 1500
   cttgagcacc tagatattga ttttaaagta tttaataagg ttggaagaga agctgatctt 1560
   aaaaccataa aaaaagcaac tgaagaaatg tcctccttta tgccagacac tataatagct 1620
   ttaggtggta cccctgaaat gagctctgca aagctaatgt gggtactata tgaacatcca 1680
   gaagtaaaat ttgaagatct tgcaataaaa tttatggaca taagaaagag aatatatact 1740
   ttcccaaaac tcggtaaaaa ggctatgtta gttgcaatta caacttctgc tggttccggt 1800
   tctgaggtta ctccttttgc tttagtaact gacaataaca ctggaaataa gtacatgtta 1860
   gcagattatg aaatgacacc aaatatggca attgtagatg cagaacttat gatgaaaatg 1920
   ccaaagggat taaccgctta ttcaggtata gatgcactag taaatagtat agaagcatac 1980
   acatccgtat atgcttcaga atacacaaac ggactagcac tagaggcaat acgattaata 2040
   tttaaatatt tgcctgaggc ttacaaaaac ggaagaacca atgaaaaagc aagagagaaa 2100
   atggctcacg cttcaactat ggcaggtatg gcatccgcta atgcatttct aggtctatgt 2160
   cattccatgg caataaaatt aagttcagaa cacaatattc ctagtggcat tgccaatgca 2220
   ttactaatag aagaagtaat aaaatttaac gcagttgata atcctgtaaa acaagcccct 2280
   tgcccacaat ataagtatcc aaacaccata tttagatatg ctcgaattgc agattatata 2340
   aagcttggag gaaatactga tgaggaaaag gtagatctct taattaacaa aatacatgaa 2400
   ctaaaaaaag ctttaaatat accaacttca ataaaggatg caggtgtttt ggaggaaaac 2460
   ttctattcct cccttgatag aatatctgaa cttgcactag atgatcaatg cacaggcgct 2520
   aatcctagat ttcctcttac aagtgagata aaagaaatgt atataaattg ttttaaaaaa 2580
   caaccttaa 2589
<210> 170
   <211> 1167
   <212> DNA
   <213> Ca-bdhA
<400> 170
   atgctaagtt ttgattattc aataccaact aaagtttttt ttggaaaagg aaaaatagac 60
   gtaattggag aagaaattaa gaaatatggc tcaagagtgc ttatagttta tggcggagga 120
   agtataaaaa ggaacggtat atatgataga gcaacagcta tattaaaaga aaacaatata 180
   gctttctatg aactttcagg agtagagcca aatcctagga taacaacagt aaaaaaaggc 240
   atagaaatat gtagagaaaa taatgtggat ttagtattag caataggggg aggaagtgca 300
   atagactgtt ctaaggtaat tgcagctgga gtttattatg atggcgatac atgggacatg 360
   gttaaagatc catctaaaat aactaaagtt cttccaattg caagtatact tactctttca 420
   gcaacagggt ctgaaatgga tcaaattgca gtaatttcaa atatggagac taatgaaaag 480
   cttggagtag gacatgatga tatgagacct aaattttcag tgttagatcc tacatatact 540
   tttacagtac ctaaaaatca aacagcagcg ggaacagctg acattatgag tcacaccttt 600
   gaatcttact ttagtggtgt tgaaggtgct tatgtgcagg acggtatagc agaagcaatc 660
   ttaagaacat gtataaagta tggaaaaata gcaatggaga agactgatga ttacgaggct 720
   agagctaatt tgatgtgggc ttcaagttta gctataaatg gtctattatc acttggtaag 780
   gatagaaaat ggagttgtca tcctatggaa cacgagttaa gtgcatatta tgatataaca 840
   catggtgtag gacttgcaat tttaacacct aattggatgg aatatattct aaatgacgat 900
   acacttcata aatttgtttc ttatggaata aatgtttggg gaatagacaa gaacaaagat 960
   aactatgaaa tagcacgaga ggctattaaa aatacgagag aatactttaa ttcattgggt 1020
   attccttcaa agcttagaga agttggaata ggaaaagata aactagaact aatggcaaag 1080
   caagctgtta gaaattctgg aggaacaata ggaagtttaa gaccaataaa tgcagaggat 1140
   gttcttgaga tatttaaaaa atcttat 1167
<210> 171
   <211> 1173
   <212> DNA
   <213> Ca-bdhB
<400> 171
   atggttgatt tcgaatattc aataccaact agaatttttt tcggtaaaga taagataaat 60
   gtacttggaa gagagcttaa aaaatatggt tctaaagtgc ttatagttta tggtggagga 120
   agtataaaga gaaatggaat atatgataaa gctgtaagta tacttgaaaa aaacagtatt 180
   aaattttatg aacttgcagg agtagagcca aatccaagag taactacagt tgaaaaagga 240
   gttaaaatat gtagagaaaa tggagttgaa gtagtactag ctataggtgg aggaagtgca 300
   atagattgcg caaaggttat agcagcagca tgtgaatatg atggaaatcc atgggatatt 360
   gtgttagatg gctcaaaaat aaaaagggtg cttcctatag ctagtatatt aaccattgct 420
   gcaacaggat cagaaatgga tacgtgggca gtaataaata atatggatac aaacgaaaaa 480
   ctaattgcgg cacatccaga tatggctcct aagttttcta tattagatcc aacgtatacg 540
   tataccgtac ctaccaatca aacagcagca ggaacagctg atattatgag tcatatattt 600
   gaggtgtatt ttagtaatac aaaaacagca tatttgcagg atagaatggc agaagcgtta 660
   ttaagaactt gtattaaata tggaggaata gctcttgaga agccggatga ttatgaggca 720
   agagccaatc taatgtgggc ttcaagtctt gcgataaatg gacttttaac atatggtaaa 780
   gacactaatt ggagtgtaca cttaatggaa catgaattaa gtgcttatta cgacataaca 840
   cacggcgtag ggcttgcaat tttaacacct aattggatgg agtatatttt aaataatgat 900
   acagtgtaca agtttgttga atatggtgta aatgtttggg gaatagacaa agaaaaaaat 960
   cactatgaca tagcacatca agcaatacaa aaaacaagag attactttgt aaatgtacta 1020
   ggtttaccat ctagactgag agatgttgga attgaagaag aaaaattgga cataatggca 1080
   aaggaatcag taaagcttac aggaggaacc ataggaaacc taagaccagt aaacgcctcc 1140
   gaagtcctac aaatattcaa aaaatctgtg taa 1173
<210> 172
   <211> 48
   <212> DNA
   <213> AU1 tag
<400> 172
   atggatactt atagatacat tggtggtgac acatacaggt atatcggt 48
<210> 173
   <211> 33
   <212> DNA
   <213> HA tag
<400> 173
   atgtacccat acgatgttcc tgactatgcg ggt 33
<210> 174
   <211> 33
   <212> DNA
   <213> myc tag
<400> 174
   atggaacaaa aactcatctc agaagaagat ggt 33
<210> 175
   <211> 403
   <212> DNA
   <213> TEF1 promoter
<400> 175
   catagcttca aaatgtttct actccttttt tactcttcca gattttctcg gactccgcgc 60
   atcgccgtac cacttcaaaa cacccaagca cagcatacta aatttcccct ctttcttcct 120
   ctagggtgtc gttaattacc cgtactaaag gtttggaaaa gaaaaaagag accgcctcgt 180
   ttctttttct tcgtcgaaaa aggcaataaa aatttttatc acgtttcttt ttcttgaaaa 240
   tttttttttt gatttttttc tctttcgatg acctcccatt gatatttaag ttaataaacg 300
   gtcttcaatt tctcaagttt cagtttcatt tttcttgttc tattacaact ttttttactt 360
   cttgctcatt agaaagaaag catagcaatc taatctaagt ttt 403
<210> 176
   <211> 650
   <212> DNA
   <213> TDH3 promoter
<400> 176
   agtttatcat tatcaatact cgccatttca aagaatacgt aaataattaa tagtagtgat 60
   tttcctaact ttatttagtc aaaaaattag ccttttaatt ctgctgtaac ccgtacatgc 120
   ccaaaatagg gggcgggtta cacagaatat ataacatcgt aggtgtctgg gtgaacagtt 180
   tattcctggc atccactaaa tataatggag cccgcttttt aagctggcat ccagaaaaaa 240
   aaagaatccc agcaccaaaa tattgttttc ttcaccaacc atcagttcat aggtccattc 300
   tcttagcgca actacagaga acaggggcac aaacaggcaa aaaacgggca caacctcaat 360
   ggagtgatgc aacctgcctg gagtaaatga tgacacaagg caattgaccc acgcatgtat 420
   ctatctcatt ttcttacacc ttctattacc ttctgctctc tctgatttgg aaaaagctga 480
   aaaaaaaggt tgaaaccagt tccctgaaat tattccccta cttgactaat aagtatataa 540
   agacggtagg tattgattgt aattctgtaa atctatttct taaacttctt aaattctact 600
   tttatagtta gtcttttttt tagttttaaa acaccagaac ttagtttcga 650
<210> 177
   <211> 493
   <212> DNA
   <213> MET3 promoter
<400> 177
   tttagtacta acagagactt ttgtcacaac tacatataag tgtacaaata tagtacagat      60
   atgacacact tgtagcgcca acgcgcatcc tacggattgc tgacagaaaa aaaggtcacg     120
   tgaccagaaa agtcacgtgt aattttgtaa ctcaccgcat tctagcggtc cctgtcgtgc     180
   acactgcact caacaccata aaccttagca acctccaaag gaaatcaccg tataacaaag     240
   ccacagtttt acaacttagt ctcttatgaa gttacttacc aatgagaaat agaggctctt     300
   tctcgagaaa tatgaatatg gatatatata tatatatata tatatatata tatatatatg     360
   taaacttggt tcttttttag cttgtgatct ctagcttggg tctctctctg tcgtaacagt     420
   tgtgatatcg tttcttaaca attgaaaagg aactaagaaa gtataataat aacaagaata     480
   aagtataatt aac                                                        493
<210> 178
   <211> 461
   <212> DNA
   <213> CUP1 promoter
<400> 178
   gccgatccca ttaccgacat ttgggcgcta tacgtgcata tgttcatgta tgtatctgta      60
   tttaaaacac ttttgtatta tttttcctca tatatgtgta taggtttata cggatgattt     120
   aattattact tcaccaccct ttatttcagg ctgatatctt agccttgtta ctagttagaa     180
   aaagacattt ttgctgtcag tcactgtcaa gagattcttt tgctggcatt tcttctagaa     240
   gcaaaaagag cgatgcgtct tttccgctga accgttccag caaaaaagac taccaacgca     300
   atatggattg tcagaatcat ataaaagaga agcaaataac tccttgtctt gtatcaattg     360
   cattataata tcttcttgtt agtgcaatat catatagaag tcatcgaaat agatattaag     420
   aaaaacaaac tgtacaatca atcaatcaat catcacataa a                         461
<210> 179
   <211> 1197
   <212> DNA
   <213> Ca-ter
<400> 179
   atgatagtaa aagcaaagtt tgtaaaagga tttatcagag atgtacatcc ttatggttgc 60
   agaagggaag tactaaatca aatagattat tgtaagaagg ctattgggtt taggggacca 120
   aagaaggttt taattgttgg agcctcatct gggtttggtc ttgctactag aatttcagtt 180
   gcatttggag gtccagaagc tcacacaatt ggagtatcct atgaaacagg agctacagat 240
   agaagaatag gaacagcggg atggtataat aacatatttt ttaaagaatt tgctaaaaaa 300
   aaaggattag ttgcaaaaaa cttcattgag gatgcctttt ctaatgaaac caaagataaa 360
   gttattaagt atataaagga tgaatttggt aaaatagatt tatttgttta tagtttagct 420
   gcgcctagga gaaaggacta taaaactgga aatgtttata cttcaagaat aaaaacaatt 480
   ttaggagatt ttgagggacc gactattgat gttgaaagag acgagattac tttaaaaaag 540
   gttagtagtg ctagcattga agaaattgaa gaaactagaa aggtaatggg tggagaggat 600
   tggcaagagt ggtgtgaaga gctgctttat gaagattgtt tttcggataa agcaactacc 660
   atagcatact cgtatatagg atccccaaga acctacaaga tatatagaga aggtactata 720
   ggaatagcta aaaaggatct tgaagataag gctaagctta taaatgaaaa acttaacaga 780
   gttataggtg gtagagcctt tgtgtctgtg aataaagcat tagttacaaa agcaagtgca 840
   tatattccaa cttttcctct ttatgcagct attttatata aggtcatgaa agaaaaaaat 900
   attcatgaaa attgtattat gcaaattgag agaatgtttt ctgaaaaaat atattcaaat 960
   gaaaaaatac aatttgatga caagggaaga ttaaggatgg acgatttaga gcttagaaaa 1020
   gacgttcaag acgaagttga tagaatatgg agtaatatta ctcctgaaaa ttttaaggaa 1080
   ttatctgatt ataagggata caaaaaagaa ttcatgaact taaacggttt tgatctagat 1140
   ggggttgatt atagtaaaga cctggatata gaattattaa gaaaattaga accttaa 1197
<210> 180
   <211> 1194
   <212> DNA
   <213> Ah-ter
<400> 180
   atgatcatta aaccgaaagt tcgtggcttc atttgtacca ccactcatcc ggttggctgt 60
   gaagctaatg tacgccgcca gatcgcgtat accaaagcaa aaggcactat cgaaaacggc 120
   cctaagaaag tgctggtgat tggtgcgagc accggttacg gtctggcgtc ccgcattgca 180
   gcggcgttcg gtagcggcgc cgcgaccctg ggtgttttct tcgaaaaagc gggctccgaa 240
   actaaaaccg cgaccgcagg ttggtacaac tctgccgcgt ttgacaaagc cgccaaagag 300
   gctggcctgt atgcgaaatc tattaacggt gacgcgttca gcaacgaatg ccgtgctaaa 360
   gtgatcgaac tgatcaaaca ggatctgggc caaattgatc tggttgttta ttctctggcc 420
   tccccggttc gtaaactgcc ggataccggc gaagttgtgc gcagcgctct gaaacctatt 480
   ggtgaagtgt acaccacgac cgcaattgat actaataagg accagattat caccgcaacc 540
   gtcgagccgg ccaacgagga agagatccag aataccatca ctgtgatggg cggtcaagac 600
   tgggaactgt ggatggcagc actgcgcgac gcaggtgttc tggcagacgg tgcaaagagc 660
   gtcgcttact cttacatcgg cactgacctg acttggccga tctactggca tggcaccctg 720
   ggtcgcgcga aagaggatct ggatcgcgca gcggcagcga tccgcggtga tctggccggt 780
   aagggcggta ctgcgcacgt tgccgttctg aaatccgtgg tcacccaggc atcttctgca 840
   atcccggtga tgccgctgta tatttctatg gcctttaaaa tcatgaaaga gaagggtatc 900
   cacgaaggct gtatggagca agtggaccgc atgatgcgta ctcgcctgta cgcggcggac 960
   atggcactgg atgaccaggc gcgtatccgt atggacgatt gggaactgcg tgaagatgtt 1020
   cagcagactt gccgtgatct gtggccgtcc attacctccg aaaacctgtg cgagctgacc 1080
   gattacactg gttacaaaca ggaatttctg cgtctgttcg gtttcggtct ggaagaagta 1140
   gactacgatg cagacgttaa cccggacgtt aaatttgatg ttgtcgaact gtga 1194
<210> 181
   <211> 1218
   <212> DNA
   <213> Eg-ter
<400> 181
   atggccatgt tcaccactac cgccaaggtt attcagccga aaatccgtgg ttttatctgt 60
   acgaccaccc acccgattgg ctgtgaaaaa cgcgtgcagg aagaaattgc ttacgcacgt 120
   gcacatccac cgaccagccc gggtccgaaa cgtgtcctgg tcatcggctg ttccactggc 180
   tacggcctgt ctactcgtat caccgcagct ttcggctatc aggcggctac tctgggcgtg 240
   ttcctggctg gtccgccgac taaaggtcgc ccggctgcgg ccggttggta taacaccgta 300
   gctttcgaaa aagcggccct ggaagccggt ctgtatgccc gctccctgaa cggtgacgct 360
   tttgactcta ctaccaaagc acgcaccgtg gaagctatca aacgtgacct gggcaccgtt 420
   gacctggtgg tttatagcat tgcagctccg aaacgtaccg atccggctac cggcgtgctg 480
   cacaaagcgt gtctgaaacc gatcggtgcg acctacacca accgtacggt aaatactgac 540
   aaagctgaag ttacggacgt gtccatcgaa ccggcgagcc cagaagaaat tgcagacact 600
   gtgaaagtaa tgggtggcga agactgggaa ctgtggattc aggctctgtc tgaagccggc 660
   gttctggcag aaggcgcgaa aaccgtcgca tactcttata tcggtccgga gatgacctgg 720
   ccggtgtact ggtccggcac cattggtgaa gccaaaaagg atgttgaaaa agccgctaaa 780
   cgtattaccc agcagtacgg ctgtccggca tacccggttg tggcaaaagc actggtgacg 840
   caggcatcct ccgcgatccc ggtcgtcccg ctgtatattt gtctgctgta ccgtgtaatg 900
   aaagaaaaag gcactcacga aggttgcatc gaacaaatgg tgcgtctgct gaccacgaaa 960
   ctgtacccgg aaaacggtgc cccgatcgtt gatgaagcgg gccgtgttcg tgtggacgat 1020
   tgggaaatgg cagaagacgt tcagcaagcc gttaaagacc tgtggagcca ggtgagcacg 1080
   gcaaacctga aagatatttc cgacttcgcc ggttaccaaa ccgagttcct gcgcctgttt 1140
   ggttttggta tcgatggcgt ggactatgac cagccggttg acgtagaggc agacctgccg 1200
   agcgcagctc agcagtaa 1218
<210> 182
   <211> 1344
   <212> DNA
   <213> Sc-ccr
<400> 182
   atgaccgtga aagacattct ggacgctatt caatctaaag acgccacttc cgcggatttc 60
   gcagctctgc aactgccgga gtcctaccgt gccatcaccg ttcacaaaga tgaaactgaa 120
   atgttcgcgg gtctggaaac tcgtgacaaa gatccacgta aatccattca cctggacgaa 180
   gttccagtgc cggaactggg tccgggcgaa gccctggtgg cagttatggc aagctccgtt 240
   aactacaact ctgtatggac gtctatcttt gaaccggtaa gcaccttcgc cttcctggaa 300
   cgctacggca aactgtctcc gctgaccaaa cgtcatgatc tgccatacca catcatcggt 360
   tctgacctgg caggcgtcgt cctgcgtacc ggccctggtg ttaacgcctg gcagccgggt 420
   gacgaagtcg ttgcccattg cctgtctgtt gaactggaat cccctgatgg ccatgatgac 480
   accatgctgg acccggagca gcgtatttgg ggcttcgaaa ctaactttgg tggtctggct 540
   gagattgctc tggtgaagac taaccagctg atgccgaaac caaaacacct gacttgggaa 600
   gaagccgcgg ctccgggcct ggtcaacagc actgcgtatc gtcagctggt ttctcgtaac 660
   ggtgctgcta tgaaacaggg tgataacgtt ctgatctggg gcgcgtccgg tggtctgggc 720
   tcttacgcga cccagttcgc actggccggt ggcgcgaatc cgatctgcgt tgttagctct 780
   ccgcagaaag ctgaaatttg tcgttctatg ggcgcagaag cgatcattga tcgcaacgca 840
   gagggctaca aattttggaa agacgaacat acccaggacc ctaaggaatg gaagcgtttc 900
   ggcaaacgta tccgcgaact gactggtggt gaagacattg atatcgtttt tgaacaccct 960
   ggtcgtgaga cttttggtgc gtctgtatac gttacccgca agggcggtac gatcaccacc 1020
   tgtgcatcta cctctggcta catgcatgag tatgataacc gttacctgtg gatgtccctg 1080
   aaacgtatca tcggctctca ctttgctaac tatcgcgaag cctatgaggc aaaccgtctg 1140
   atcgctaaag gcaaaattca tccgactctg tctaaaacct attccctgga ggaaactggc 1200
   caggcggcct acgacgtaca ccgtaacctg caccagggca aagttggcgt tctgtgcctg 1260
   gctccggaag aaggtctggg tgttcgtgac gctgaaatgc gtgctcagca cattgacgcg 1320
   attaaccgtt tccgtaatgt gtga 1344
<210> 183
   <211> 36
   <212> DNA
   <213> Gevo-345
<400> 183
   atgtttgtcg acatgatagt aaaagcaaag tttgta 36
<210> 184
   <211> 41
   <212> DNA
   <213> Gevo-346
<400> 184
   cttaatgcgg ccgcttaagg ttctaatttt cttaataatt c 41
<210> 185
   <211> 35
   <212> DNA
   <213> Gevo-343
<400> 185
   gcttgagtcg acatgatcat taaaccgaaa gttcg 35
<210> 186
   <211> 37
   <212> DNA
   <213> Gevo-344
<400> 186
   atttaaggat cctcacagtt cgacaacatc aaattta 37
<210> 187
   <211> 32
   <212> DNA
   <213> Gevo-347
<400> 187
   catcacgtcg acatggccat gttcaccact ac 32
<210> 188
   <211> 31
   <212> DNA
   <213> Gevo-348
<400> 188
   ctcgcgggat ccttactgct gagctgcgct c 31
<210> 189
   <211> 33
   <212> DNA
   <213> Gevo-341
<400> 189
   gtcttagtcg acatgaccgt gaaagacatt ctg 33
<210> 190
   <211> 34
   <212> DNA
   <213> Gevo-342
<400> 190
   attggcggat cctcacacat tacggaaacg gtta 34

## Description

### Field of the Invention

The present invention relates to metabolically engineering yeast cells for the production of n-butanol at high yield as an alternative and renewable transportation fuel, and for other applications. The yeasts of the invention are engineered to comprise a metabolic pathway that converts a carbon source such as glucose and/or other metabolizable carbohydrates, as well as biomass and the like, to n-butanol.

### Background

Currently, approximately 140 billion gallons of gasoline are consumed in the United States and approximately 340 billion gallons are consumed worldwide per year. These quantities of consumption are only growing. The Energy Policy Act of 2005 stipulates that 7.5 billion gallons of renewable fuels be used in gasoline by 2012. In his 2007 State of the Union address, the President called for increasing the size and expanding the scope of renewable fuel standard (RFS) to require 35 billion gallons of renewable and alternative fuels in 2017. The Department of Energy has set a goal of replacing 30 percent of the United States' current gasoline consumption with biofuels by 2030 (the "30X30" initiative). In March 2007, Brazil and the United States signed "the Ethanol Agreement," to promote the development of biofuels in the Americas, uniting the largest biofuel producers in the world—currently accounting for 70 percent of the world's ethanol production.

Biofuels have the potential to not only reduce the United States' dependency on foreign oil imports, which is vital to homeland security, but to also dramatically decrease greenhouse gas emissions associated with global warming. Biofuels can be obtained from the conversion of carbon based feedstock. Agricultural feedstocks are considered renewable because, although they release carbon dioxide when burned, they capture nearly an equivalent amount of carbon dioxide through photosynthesis.

In the United States, ethanol is increasingly being used as an oxygenate additive for standard gasoline, as a replacement for methyl t-butyl ether (MTBE), the latter chemical being difficult to retrieve from groundwater and soil contamination. At a 10% mixture, ethanol reduces the likelihood of engine knock, by raising the octane rating. The use of 10% ethanol gasoline is mandated in some cities where the possibility of harmful levels of auto emissions are possible, especially during the winter months. North American vehicles from approximately 1980 onward can run on 10% ethanol/90% gasoline (i.e., E10) with no modifications.

In order for ethanol to be used at higher concentrations, however, a vehicle must have its engine and fuel system specially engineered or modified. Flexible fuel vehicles (FFVs), are designed to run on gasoline or a blend of up to 85% ethanol (E85). However, since a gallon of ethanol contains less energy than a gallon of gasoline, FFVs typically get about 20-30% fewer miles per gallon when fueled with E85. Conversion packages are available to convert a conventional vehicle to a FFV that typically include an electronic device to increase injected fuel volume per cycle (because of the lower energy content of ethanol) and, in some cases, a chemical treatment to protect the engine from corrosion. Over 4 million flexible-fuel vehicles are currently operated on the road in the United States, although a 2002 study found that less than 1% of fuel consumed by these vehicles is E85.

Butanol has several advantages over ethanol for fuel. While it can be made from the same feedstocks as ethanol, unlike ethanol, it is compatible with gasoline and petrodiesel at any ratio. Butanol can also be used as a pure fuel in existing cars without modifications and has been proposed as a jet fuel by the Sir Richard Branson Group at Virgin Airlines. Unlike ethanol, butanol does not absorb water and can thus be stored and distributed in the existing petrochemical infrastructure. Due to its higher energy content, the fuel economy (miles per gallon) is better than that of ethanol. Also, butanol-gasoline blends have lower vapor pressure than ethanol-gasoline blends, which is important in reducing evaporative hydrocarbon emissions. These properties provide the potential for butanol to be used in precisely the same manner as gasoline, without vehicle modification and without the burden on consumers of having to refuel more often.

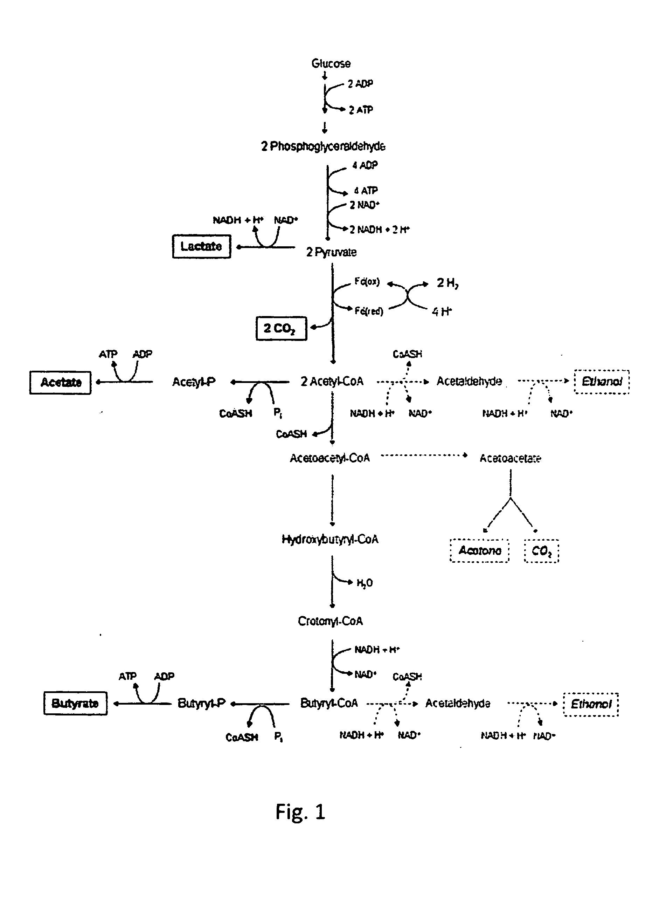

n-Butanol can be produced using *Clostridium* strains that naturally produce n-butanol via a pathway that leads from butyryl-CoA to n-butanol. One disadvantage of *Clostridium* strains is that n-butanol production occurs in a two-step process that involves an acid-producing growth phase followed by a solvent production phase. Also, large quantities of byproducts, such as hydrogen, ethanol, and acetone are produced in this process, thus limiting the stoichiometric yield of n-butanol to about 0.6 mol of n-butanol per mol of glucose consumed. Further, *Clostridium* strains lose their ability to produce solvents under continuous culture conditions (Cornillot et al., J. Bacteriol. 179: 5442-5447, 1997). The *Clostridium* pathway showing the conversion of glucose to acids and solvents in C. *acetobutylicum,* including the path to produce n-butanol from acetyl-CoA, is shown in Fig. 1.

### Summary of the Invention

The present invention is defined in the claims. In an embodiment, there is provided a metabolically-engineered yeast capable of metabolizing a carbon source to produce n-butanol, at least one pathway configured for producing an increased amount of cytosolic acetyl-CoA relative to another amount of cytosolic acetyl-CoA produced by a wild-type yeast, and at least one heterologous gene to encode and express at least one enzyme for a metabolic pathway capable of utilizing NADH to convert acetyl-CoA to the n-butanol, as defined in the claims.

In another embodiment, there is provided a method of producing n-butanol, the method comprising (a) providing metabolically-engineered yeast as defined in the claims capable of metabolizing a carbon source to produce n-butanol, at least one pathway configured for producing an increased amount of cytosolic acetyl-CoA relative to another amount of cytosolic acetyl-CoA produced by a wild-type yeast, and at least one heterologous gene to encode and express at least one enzyme for a metabolic pathway capable of utilizing NADH to convert acetyl-CoA to the n-butanol; and (b) culturing the metabolically-engineered yeast for a period of time and under conditions to produce the n-butanol.

The present disclosure also relates to a method of producing n butanol, using yeast, the method comprising (a) metabolically engineering the yeast to increase cytosolic acetyl-CoA production; (b) metabolically engineering the yeast to express a metabolic pathway that converts a carbon source to n butanol, wherein the pathway requires at least one non-native enzyme of the yeast, wherein steps (a) and (b) can be performed in either order; and (c) culturing the yeast for a period of time and under conditions to produce a recoverable amount of n butanol.

The present disclosure also relates to a method of producing n butanol, using yeast, the method comprising (a) culturing a metabolically-engineered yeast for a period of time and under conditions to produce a yeast-cell biomass without activating n butanol production; and (b) altering the culture conditions for another period of time and under conditions to produce a recoverable amount of n butanol

In another embodiment, there is provided a metabolically-engineered yeast capable of metabolizing a carbon source and producing an increased amount of acetyl-CoA relative to the amount of cytosolic acetyl-CoA produced by a wild-type yeast.

The present disclosure also relates to a method of increasing metabolic activity of yeast, the method comprising producing an increased amount of cytosolic acetyl-CoA of the yeast relative to another amount of cytosolic acetyl-CoA produced by a wild-type yeast.

In still another embodiment, there is provided a metabolically-engineered yeast having at least one pathway configured for producing an increased amount of cytosolic acetyl-CoA relative to another amount of cytosolic acetyl-CoA produced by a wild-type yeast.

The present disclosure also relates to other embodiments, which are also disclosed.

### Brief Description of the Drawing

Illustrative embodiments of the invention are illustrated in the drawings, in which:

Fig. 1 illustrates the metabolic pathways involved in the conversion of glucose, pentose, and granulose to acids and solvents in *Clostridium acetobutylicum.* Hexoses (e.g., glucose) and pentoses are converted to pyruvate, ATP and NADH. Subsequently, pyruvate is oxidatively decarboxylated to acetyl-CoA by a pyruvate-ferredoxin oxidoreductase. The reducing equivalents generated in this step are converted to hydrogen by an iron-only hydrogenase. Acetyl-CoA is the branch-point intermediate, leading to the production of organic acids (acetate and butyrate) and solvents (acetone, butanol and ethanol).

Fig.2 illustrates a chemical pathway to produce butanol in yeasts.

Fig.3 illustrates pathways used by *Sacchromyces cerevisiae* to generate acetyl-CoA.

Figs. 4 and 5 illustrate various exemplary plasmids that may be used to express various enzymes in accordance with the present disclosure.

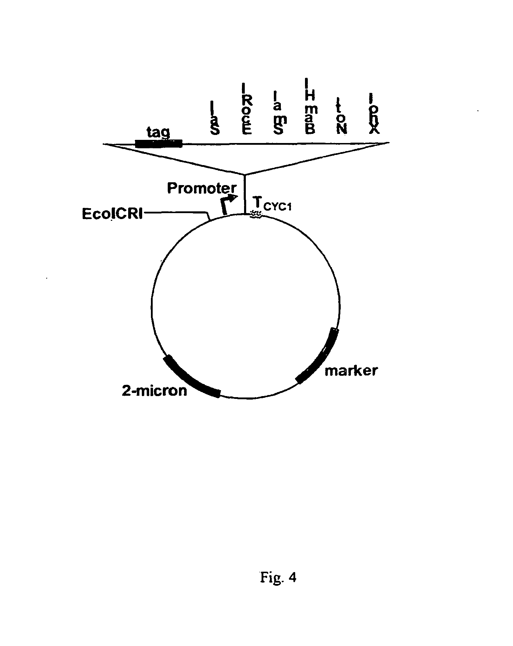

Fig.4 illustrates an exemplary plasmid that may be used to express various enzymes in accordance with the present disclosure as described in Table 1.

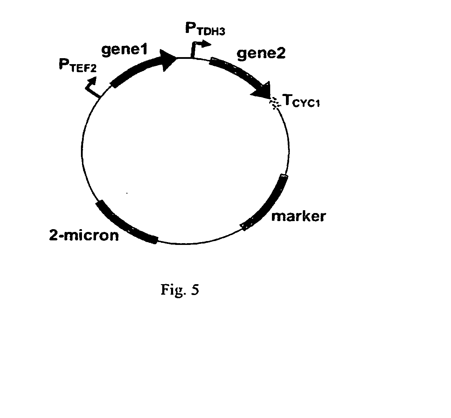

Fig.5 an exemplary plasmid that may be used to express various enzymes in accordance with the present disclosure as described in Table 2.

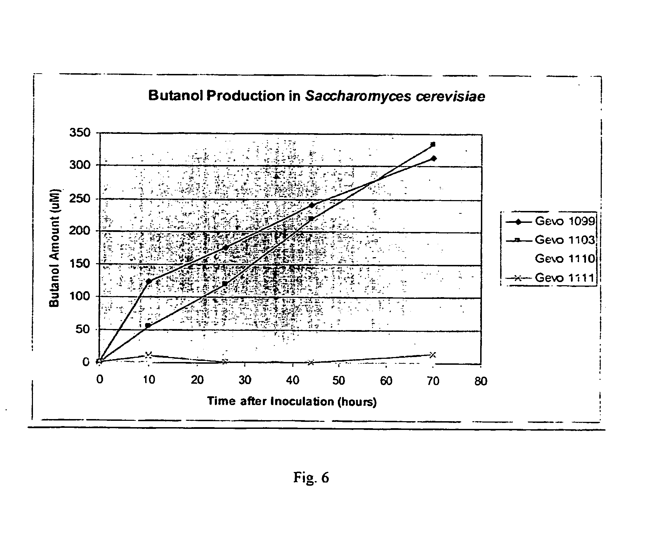

Fig.6 graphically illustrates n-butanol production over time by Gevo 1099 and Gevo 1103 as compared to the Vector only control isolates, Gevo 1110 and Gevo 1111, as follows:
( 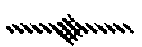 ) Gevo 1099;
( 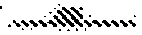 ) Gevo 1103;
( 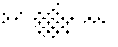 ) Gevo 1110; and
( 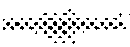 ) Gevo 1111.

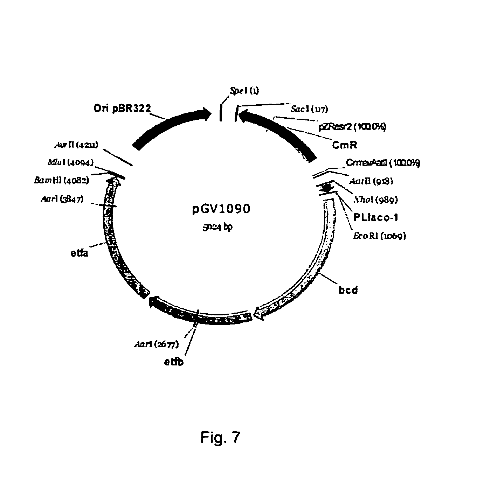

Fig.7 illustrates the pGV1090 plasmid containing *bcd*.. *etfb*, and etfa genes from *C*. *acetabuty*/*icum* inserted at the *Eco*RI and *Bam*HI sites and downstream from a modified phage lambda LacO-1 promoter (P_{L-lac}). The plasmid also carries a replication origin gene of pBR322 and a chloramphenicol resistance gene.

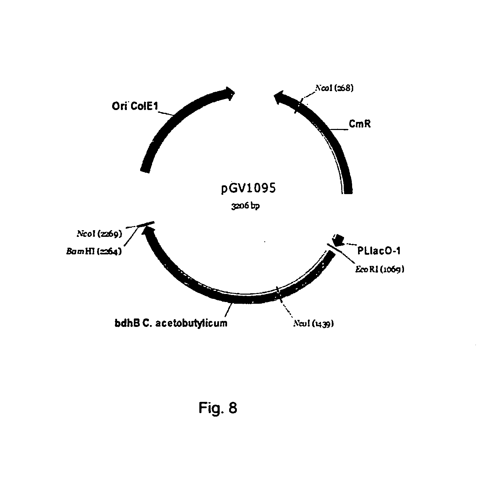

Fig.8 illustrates the pGV1095 plasmid for expression of butyraldehyde dehydrogenase (*bdhB*) from *C. acetobutylicum* inserted at the *Eco*RI and *Bam*HI sites and downstream from a modified phage lambda LacO-1 promoter (P_{L-lac}). The plasmid also carries a replication origin gene of ColE1 and a chloramphenicol resistance gene.

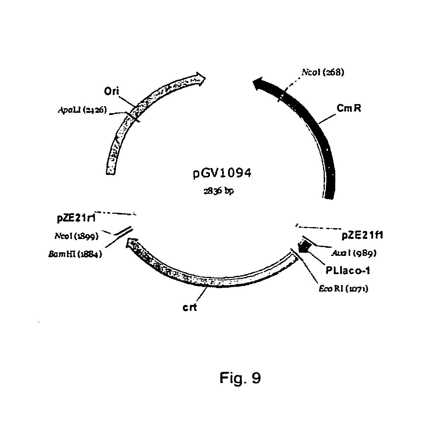

Fig.9 illustrates the pGV1094 plasmid for expression of crotonase (*crt*) from *C*. *acetobutyicum* inserted at the *Eco*RI and *Bam*HI sites and downstream from a modified phage lambda LacO-1 promoter (P_{L-lac}). The plasmid also carries an ori gene and a chloramphenicol resistance gene.

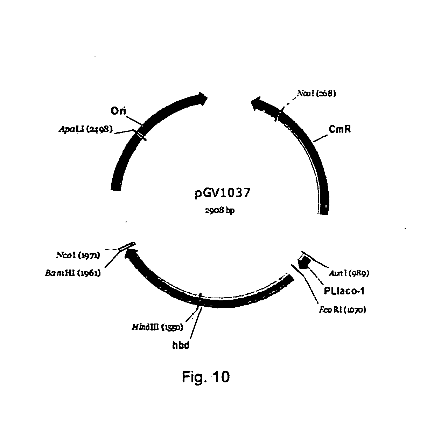

Fig.10 illustrates the pGV1037 plasmid for expression of hydroxybutyl-yl-CoA dehydrogenase (*hbd*) from *C*. *acetobutylicum* inserted at the *Eco*RI and *Bam*HI sites and downstream from a modified phage lambda LacO-1 promoter (P_{L-lac}). The plasmid also carries an on gene and a chloramphenicol resistance gene.

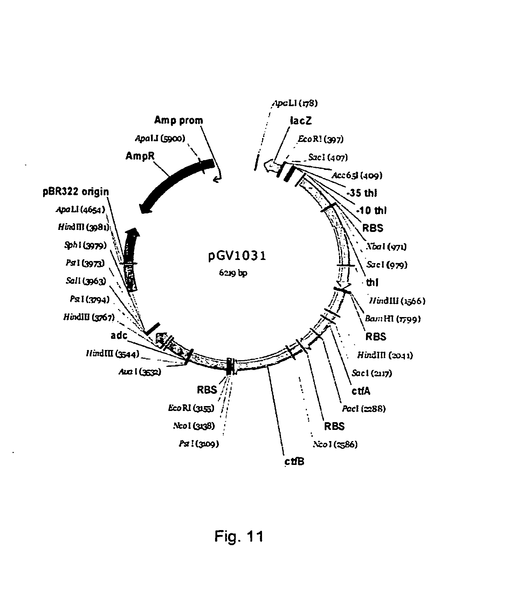

Fig. 11 illustrates the pGV1031 plasmid for expression of thiolase (*thl*) from *C*. *acetobutylicum* inserted at the *Eco*RI and *Bam*HI sites and downstream from a LacZ gene. The plasmid also carries a replication origin gene of pBR322 and an a ampicillin resistance gene.

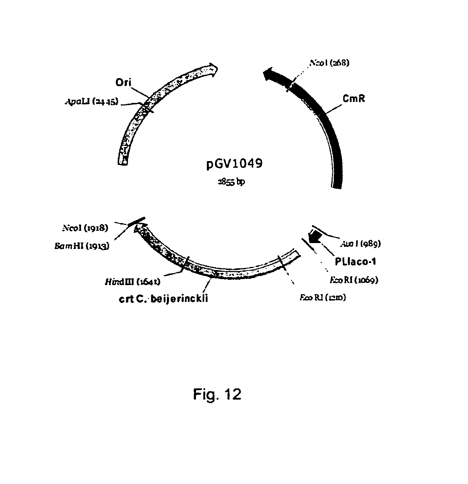

Fig.12 illustrates the pGV1049 plasmid for expression of crotonase (*crt*) from *Clostridium beijerinckii* inserted at the *Eco*RI and *Bam*HI sites and downstream from a modified phage lambda LacO-1 promoter (P_{L-lac}). The plasmid also carries an ori gene and a chloramphenicol resistance gene.

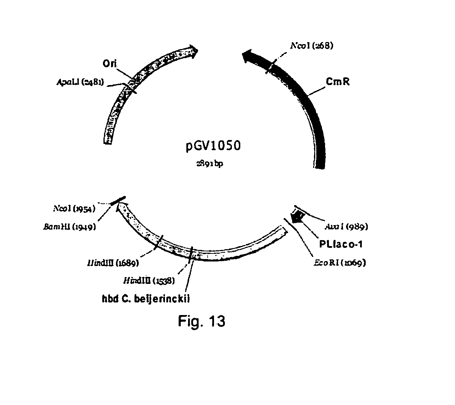

Fig.13 illustrates the pGV1050 plasmid for expression of hydroxybutyryl-CoA dehydrogenase (*hbd*) from *C*. *beijerinckii* inserted at the *Eco*RI and *Bam*HI sites and downstream from a modified phage lambda LacO-1 promoter (P_{L-lac}). The plasmid also carries an ori gene and a chloramphenicol resistance gene.

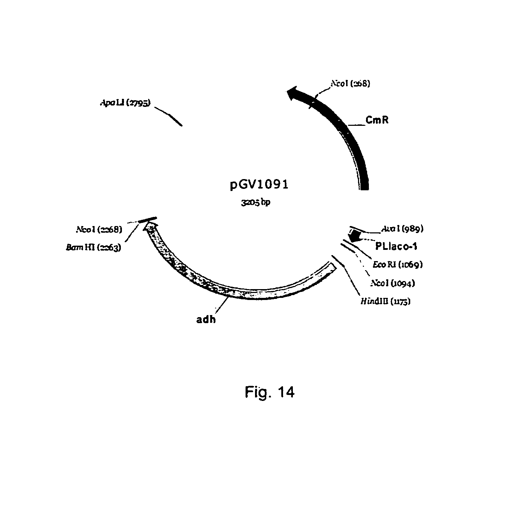

Fig.14 illustrates the pGV1091 plasmid for expression of alcohol dehydrogenase (*adhA*) from C. *beijerinckii* inserted at the *Hind*III and *Bam*HI sites and downstream from a modified phage lambda LacO-1 promoter (P_{L-lac}). The plasmid also carries a chloramphenicol resistance gene.

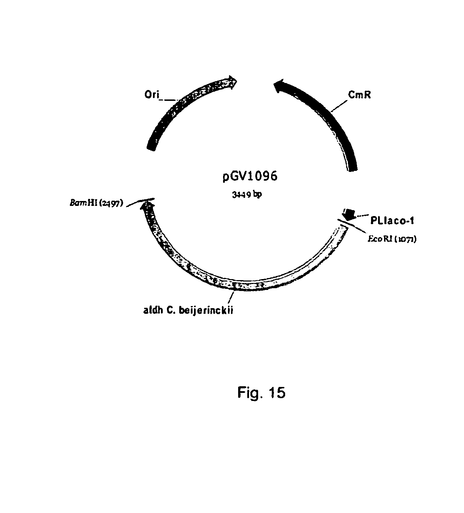

Fig.15 illustrates the pGV1096 plasmid for expression of alcohol dehydrogenase (*aldh*) from C. *beijerinckii* inserted at the *Eco*RI and *Bam*HI sites and downstream from a modified phage lambda LacO-1 promoter (P_{L-lac}). The plasmid also carries an ori gene and a chloramphenicol resistance gene.

### Detailed Description

Recombinant yeast microorganisms are described that are engineered to convert a carbon source into n-butanol at high yield. In particular, recombinant yeast microorganisms are described that are capable of metabolizing a carbon source for producing n-butanol at a yield of at least 5% of theoretical, and, in some cases, a yield of over 50% of theoretical. As used herein, the term "yield" refers to the molar yield. For example, the yield equals 100% when one mole of glucose is converted to one mole of n-butanol. In particular, the term "yield" is defined as the mole of product obtained per mole of carbon source monomer and may be expressed as percent. Unless otherwise noted, yield is expressed as a percentage of the theoretical yield. "Theoretical yield" is defined as the maximum moles of product that can be generated per a given mole of substrate as dictated by the stoichiometry of the metabolic pathway used to make the product. For example, the theoretical yield for one typical conversion of glucose to n-butanol is 100%. As such, a yield of n-butanol from glucose of 95% would be expressed as 95% of theoretical or 95% theoretical yield.

The microorganisms herein disclosed are engineered, using genetic engineering techniques, to provide microorganisms which utilize heterologously expressed enzymes to produce n-butanol at high yield. Butanol yield is dependent on the high-yield conversion of a carbon source to acetyl-CoA, and the subsequent high-yield conversion of acetyl-CoA to butanol. The invention relates to the combination of these two aspects resulting in a microorganism that produces n-butanol at a high yield.

As used herein, the term "microorganism" includes prokaryotic and eukaryotic microbial species from the Domains *Bacteria* and *Eukaryote,* the latter including yeast and filamentous fungi, protozoa, algae, or higher Protista. The terms "cell," "microbial cells," and "microbes" are used interchangeably with the term microorganism. In a preferred embodiment, the microorganism is a *yeast,* for example, *Saccharomyces cerevisiae or Kluyveromyce lactis)* or *E*. *coli.*

"*Yeast*", refers to a domain of eukaryotic organisms, phylogenetically placed in the kingdom fungi, under the phyla Ascomycota and Basidiomycota. Approximately 1500 yeast species are described to date. Yeasts are primarily unicellular microorganisms that reproduce primarily by asexual budding even though some multicellular yeasts and those that reproduce by binary fission are described. Most species are classified as aerobes but facultative and anaerobic yeasts are also well known. Related to yeast fermentative physiology, yeasts are categorized into two groups - Crabtree-positive and Crabtree-negative.

Briefly, the Crabtree effect is defined as the inhibition of oxygen consumption by a microorganism when cultured under aerobic conditions due to the presence of a high glucose concentration (e.g., 50 grams of glucose/L). Thus, a yeast cell having a Crabtree-positive phenotype continues to ferment irrespective of oxygen availability due to the presence of glucose, while a yeast cell having a Crabtree-negative phenotype does not exhibit glucose mediated inhibition of oxygen consumption. Examples of yeast cells typically having a Crabtree-positive phenotype include, without limitation, yeast cells of the genera Saccharomyces, Zygosaccharomyces, Torulaspora and Dekkera. Examples of yeast cells typically having a Crabtree-negative phenotype include, without limitation, yeast cells of the genera Kluyveromyces, Pichis, Hansenula and Candida.

Certain detailed aspects and embodiments of the invention are illustrated below, following a definition of certain terms used in the application. The term "carbon source" generally refers to a substrate or compound suitable to be used as a source of carbon for yeast cell growth. Carbon sources may be in various forms, including, but not limited to polymers such as xylan and pectin, carbohydrates, acids, alcohols, aldehydes, ketones, amino acids, peptides, etc. Such carbons sources more specifically include, for example, various monosaccharides such as glucose and fructose, oligosaccharides such as lactose or sucrose, polysaccharides, cellulosic material, saturated or unsaturated fatty acids, succinate, lactate, acetate, ethanol, or mixtures thereof and unpurified mixtures from renewable feedstocks, such as cheese whey permeate, cornsteep liquor, sugar beet molasses, and barley malt.

Carbon sources which serve as suitable starting materials for the production of n-butanol products include, but are not limited to, biomass hydrolysates, glucose, starch, cellulose, hemicellulose, xylose, lignin, dextrose, fructose, galactose, com, liquefied corn meal, corn steep liquor (a byproduct of corn wet milling process that contains nutrients leached out of corn during soaking), molasses, lignocellulose, and maltose. Photosynthetic organisms can additionally produce a carbon source as a product of photosynthesis. In a preferred embodiment, carbon sources may be selected from biomass hydrolysates and glucose. Glucose, dextrose and starch can be from an endogenous or exogenous source.

It should be noted that other, more accessible and/or inexpensive carbon sources, can be substituted for glucose with relatively minor modifications to the host microorganisms. For example, in certain embodiments, use of other renewable and economically feasible substrates may be preferred. These include: agricultural waste, starch-based packaging materials, corn fiber hydrolysate, soy molasses, fruit processing industry waste, and whey permeate, etc.

Five carbon sugars are only used as carbon sources with microorganism strains that are capable of processing these sugars, for example *E*. *coli* B. In some embodiments, glycerol, a three carbon carbohydrate, may be used as a carbon source for the biotransformations. In other embodiments, glycerin, or impure glycerol obtained by the hydrolysis of triglycerides from plant and animal fats and oils, may be used as a carbon source, as long as any impurities do not adversely affect the host microorganisms.

The term "enzyme" as used herein refers to any substance that catalyzes or promotes one or more chemical or biochemical reactions, which usually includes enzymes totally or partially composed of a polypeptide, but can include enzymes composed of a different molecule including polynucleotides.

The term "polynucleotide" is used herein interchangeably with the term "nucleic acid" and refers to an organic polymer composed of two or more monomers including nucleotides, nucleosides or analogs thereof, including but not limited to single stranded or double stranded, sense or antisense deoxyribonucleic acid (DNA) of any length and, where appropriate, single stranded or double stranded, sense or antisense ribonucleic acid (RNA) of any length, including siRNA. The term "nucleotide refers to any of several compounds that consist of a ribose or deoxyribose sugar joined to a purine or a pyrimidine base and to a phosphate group, and that are the basic structural units of nucleic acids. The term "nucleoside" refers to a compound (as guanosine or adenosine) that consists of a purine or pyrimidine base combined with deoxyribose or ribose and is found especially in nucleic acids. The term "nucleotide analog" or "nucleoside analog" refers, respectively, to a nucleotide or nucleoside in which one or more individual atoms have been replaced with a different atom or with a different functional group. Accordingly, the term polynucleotide includes nucleic acids of any length, DNA, RNA, analogs and fragments thereof. A polynucleotide of three or more nucleotides is also called nucleotidic oligomer or oligonucleotide.

The term "protein" or "polypeptide" as used herein indicates an organic polymer composed of two or more amino acidic monomers and/or analogs thereof. As used herein, the term "amino acid" or "amino acidic monomer" refers to any natural and/or synthetic amino acids including glycine and both D or L optical isomers. The term "amino acid analog" refers to an amino acid in which one or more individual atoms have been replaced, either with a different atom, or with a different functional group. Accordingly, the term polypeptide includes amino acidic polymer of any length including full length proteins, and peptides as well as analogs and fragments thereof. A polypeptide of three or more amino acids is also called a protein oligomer or oligopeptide.

The term "heterologous" or "exogenous" as used herein with reference to molecules and in particular enzymes and polynucleotides, indicates molecules that are expressed in an organism, other than the organism from which they originated or are found in nature, independently on the level of expression that can be lower, equal or higher than the level of expression of the molecule in the native microorganism.

On the other hand, the term "native" or "endogenous" as used herein with reference to molecules, and in particular enzymes and polynucleotides, indicates molecules that are expressed in the organism in which they originated or are found in nature, independently on the level of expression that can be lower, equal or higher than the level of expression of the molecule in the native microorganism.

In certain embodiments, the native, unengineered microorganism is incapable of converting a carbon source to n-butanol, or one or more of the metabolic intermediate(s) thereof, because, for example, such wild-type host lacks one or more required enzymes in a n-butanol-producing pathway.

In certain embodiments, the native, unengineered microorganism is capable of only converting minute amounts of a carbon source to n-butanol, at a yield of smaller than 0.1 % of theoretical.

For instance, microorganisms such as *E*. *coli* or *Saccharomyces* sp. generally do not have a metabolic pathway to convert sugars such as glucose into n-butanol but it is possible to transfer a n-butanol producing pathway from a n-butanol producing strain, (e.g., *Clostridium)* into a bacterial or eukaryotic heterologous host, such as *E*. *coli* or *Saccharomyces sp.,* and use the resulting recombinant microorganism to produce n-butanol.

Microorganisms, in general, are suitable as hosts if they possess inherent properties such as solvent resistance which will allow them to metabolize a carbon source in solvent containing environments.

The terms "host", "host cells" and "recombinant host cells" are used interchangeably herein and refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

Useful hosts for producing n-butanol may be either eukaryotic or prokaryotic microorganisms. A yeast cell is the preferred host such as, but not limited to, *Saccharomyces cerevisiae* or *Kluyveromyces lactis.* In certain embodiments, other suitable yeast host microorganisms include, but are not limited to, *Pichia, Yarrowia, Aspergillus, Kluyveromyces, Pachysolen, Rhodotorula, Zygosaccharomyces, Galactomyces, Schizosaccharomyces, Penicillium, Torulaspora, Debaryomyces, Williopsis, Dekkera, Kloeckera, Metschnikowia* and *Candida* species.

In particular, the recombinant microorganisms herein disclosed are engineered to activate, and in particular express heterologous enzymes that can be used in the production of n-butanol. In particular, in certain embodiments, the recombinant microorganisms are engineered to activate heterologous enzymes that catalyze the conversion of acetyl-CoA to n-butanol.

The terms "activate" or "activation" as used herein with reference to a biologically active molecule, such as an enzyme, indicates any modification in the genome and/or proteome of a microorganism that increases the biological activity of the biologically active molecule in the microorganism. Exemplary activations include but, are not limited, to modifications that result in the conversion of the molecule from a biologically inactive form to a biologically active form and from a biologically active form to a biologically more active form, and modifications that result in the expression of the biologically active molecule in a microorganism wherein the biologically active molecule was previously not expressed. For example, activation of a biologically active molecule can be performed by expressing a native or heterologous polynucleotide encoding for the biologically active molecule in the microorganism, by expressing a native or heterologous polynucleotide encoding for an enzyme involved in the pathway for the synthesis of the biological active molecule in the microorganism, by expressing a native or heterologous molecule that enhances the expression of the biologically active molecule in the microorganism.

A gene or DNA sequence is "heterologous" to a microorganism if it is not part of the genome of that microorganism as it normally exists, i.e., it is not naturally part of the genome of the wild-type version microorganism. By way of example, and without limitation, for *S*. *cerevisiae,* a DNA encoding any one of the following is considered to be heterologous. *Escherichia coli* protein or enzyme, proteins or enzymes from any other microorganisms other than *S*. *cerevisiae,* non-transcriptional and translational control sequences, and a mutant or otherwise modified *S*. *cerevisiae* protein or RNA, whether the mutant arises by selection or is engineered into *S*. *cerevisiae.* Furthermore, constructs that have a wild-type *S*. *cerevisiae* protein under the transcriptional and/or translational control of a heterologous regulatory element (inducible promoter, enhancer, etc.) is also considered to be heterologous DNA.

Metabolization of a carbon source is said to be "balanced" when the NADH produced during the oxidation reactions of the carbon source equal the NADH utilized to convert acetyl-CoA to metabolization end products. Only under these conditions is all the NADH recycled. Without recycling, the NADH/NAD+ ratio becomes imbalanced (i.e. increases) which can lead the organism to ultimately die unless alternate metabolic pathways are available to maintain a balanced NADH/NAD+ ratio.

In certain embodiments, the n-butanol yield is highest if the microorganism does not use aerobic or anaerobic respiration since carbon is lost in the form of carbon dioxide in these cases.

In certain embodiments, the microorganism produces n-butanol fermentatively under anaerobic conditions so that carbon is not lost in form of carbon dioxide.

The term "aerobic respiration" refers to a respiratory pathway in which oxygen is the final electron acceptor and the energy is typically produced in the form of an ATP molecule. The term "aerobic respiratory pathway" is used herein interchangeably with the wording "aerobic metabolism", "oxidative metabolism" or "cell respiration".

On the other hand, the term "anaerobic respiration" refers to a respiratory pathway in which oxygen is not the final electron acceptor and the energy is typically produced in the form of an ATP molecule. This includes a respiratory pathway in which an organic or inorganic molecule other than oxygen (e.g. nitrate, fumarate, dimethylsulfoxide, sulfur compounds such as sulfate, and metal oxides) is the final electron acceptor. The wording "anaerobic respiratory pathway" is used herein interchangeably with the wording "anaerobic metabolism" and "anaerobic respiration".

"Anaerobic respiration" has to be distinguished by "fermentation." In "fermentation", NADH donates its electrons to a molecule produced by the same metabolic pathway that produced the electrons carried in NADH. For example, in one of the fermentative pathways of *E*. *coli,* NADH generated through glycolysis transfers its electrons to pyruvate, yielding lactate.

A microorganism operating under fermentative conditions can only metabolize a carbon source if the fermentation is "balanced." A fermentation is said to be "balanced" when the NADH produced during the oxidation reactions of the carbon source equal the NADH utilized to convert acetyl-CoA to fermentation end products. Only under these conditions is all the NADH recycled. Without recycling, the NADH/NAD⁺ ratio becomes imbalanced which leads the organism to ultimately die unless alternate metabolic pathways are available to maintain a balance NADH/NAD⁺ ratio. A written fermentation is said to be 'balanced' when the hydrogens produced during the oxidations equal the hydrogens transferred to the fermentation end products. Only under these conditions is all the NADH and reduced ferredoxin recycled to oxidized forms. It is important to know whether a fermentation is balanced, because if it is not, then the overall written reaction is incorrect.

Anaerobic conditions are preferred for a high yield n-butanol producing microorganisms.

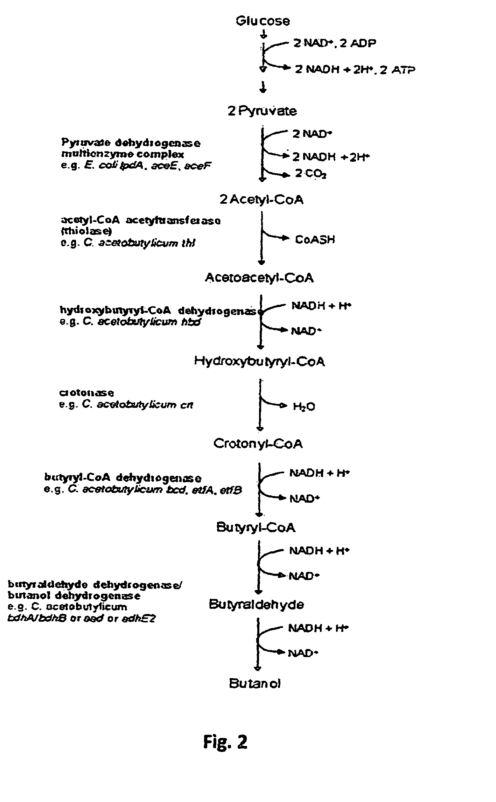

Fig.2 illustrates a pathway in yeast that converts a carbon source to n-butanol according to an embodiment of the present invention. This pathway can be regarded as having two distinct parts, which include(1) conversion of a carbon source to acetyl-CoA, and (2) conversion of acetyl-CoA to n-butanol. Due to the compartmentalization of metabolic reactions in yeasts (and other eukaryotes) and to ensure adequate acetyl-CoA generation from glucose to drive the second part of the pathway, the production of acetyl-CoA in the cytosol is necessary and, therefore, increased in certain engineered variants disclosed herein.

Relevant to part (1) of the conversion of a carbon source to butanol, a yeast microorganism may be engineered to increase the flux of pyruvate to acetyl-CoA in the cytosol.

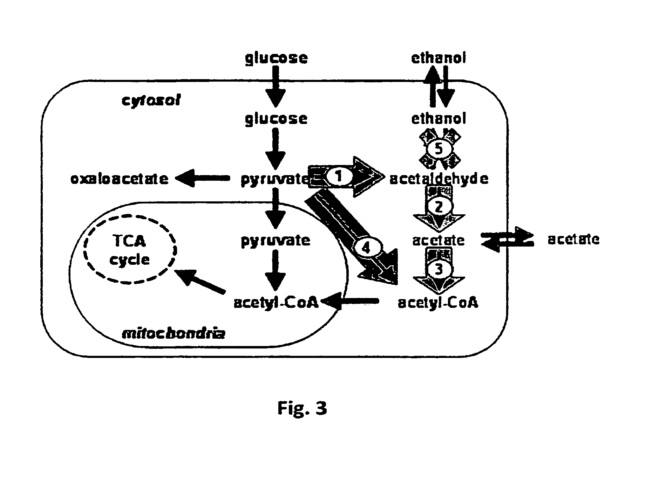

As shown in Fig. 3, *S*. *cerevisiae* generates acetyl-CoA in the mitochondria and in the cytosol. Since the conversion of acetyl-CoA to n-butanol takes part in the cytosol, the generation of acetyl-CoA in the cytosol is increased in the engineered cell. Optionally, the generation of acetyl-CoA in the mitochondrion can be reduced or repressed.

In one embodiment, acetyl-CoA may be generated from pyruvate by increasing the flux through the cytosolic "pyruvate dehydrogenase bypass" (Pronk et al., (1996). Yeast 12(16):1607), as illustrated in Fig. 3, Steps 1-3. To increase the flux through this route, one or more of the enzymes pyruvate decarboxylase (PDC), aldehyde dehydrogenase (ALD), and acetyl-CoA synthase (ACS) may be overexpressed.

This manipulation of increasing the activity or the flux of the "PDH bypass" route, can result in achieving a butanol yield of more than 5% of the theoretical maximum.

Since this route of acetyl-CoA production generates acetaldehyde as an intermediate, it is preferable to minimize diversion of acetaldehye into pathways away from acetyl-CoA synthesis, chiefly the further reduction of acetaldehyde to ethanol by the activity of alcohol dehydrogenase (ADH) enzymes. Therefore, reducing or eliminating ADH activity may further increase acetyl-CoA generation by the pyruvate dehydrogenase bypass pathway.

As an example, the genome of the Crabtree positive yeast *Saccharomyces cerevisiae* contains 7 known ADH genes. Of these, *ADH1* is the predominant source of cytosolic ADH activity, and cells deleted for *ADH1* are unable to grow anaerobically (Drewke et al., (1990). J. Bacteriology 172(7):3909) Thus, *ADH1* may be preferably deleted to minimize conversion of acetaldehyde to ethanol. However, other ADH isoforms may catalyze the reduction of acetaldehyde to ethanol, and we contemplate their reduction or deletion as well.

This manipulation of decreasing the acetaldehyde conversion to ethanol, independently or in combination with the above described "PDH bypass" flux increase can result in achieving a butanol yield of more than 10% of theoretical maximum.

In addition, pyruvate dehydrogenase catalyzes the direct conversion of pyruvate to acetyl-CoA and CO₂, while reducing NAD⁺ to NADH. Thus, in certain embodiments, a pyruvate dehdyrogenase is overexpressed in the yeast cytosol. Alternatively, pyruvate is converted to formate and acetyl-CoA, and the resulting formate is futher metabolized to CO₂ by the activity of formate dehydrogenase, which also reduces NAD⁺ to NADH.

Since the aforementioned routes of acetyl-CoA production utilize pyruvate as a substrate, it is preferable to minimize diversion of pyruvate in to other metabolic pathways. Pyruvate decarboxylase (PDC) activity represents a major cytoplasmic route of pyruvate metabolism. Therefore, reducing or eliminating PDC activity may further increase acetyl-CoA generation by the aforementioned routes.

The manipulation of metabolic pathways to convert pyruvate to acetyl-CoA, in combination with the elimination of the PDC activity (thus eliminating the "PDH bypass" route) may achieve a butanol yield of more than 50% of theoretical maximum. This improvement is the result of three important manipulations of the native metabolic pathways of the yeast cells: (1) eliminating carbon loss via ethanol production; (2) eliminating an energetically costly acetyl-CoA synthetase activity in the cells; and (3) by balancing the generation and consumption of co-factors (e.g. NAD+/NADH) for the entire pathway involved in the conversion of glucose to butanol (4 NADH produced from glucose to acetyl-CoA and 4 NADH consumed by the acetyl-CoA to butanol conversion). The latter two manipulations will mostly contribute to yield increase by increasing the overall metabolic fitness of a host yeast cells, thereby facilitating butanol pathway function by making ATP available for biosynthetic processes and reducing the imbalance of NAD+/NADH ratio in the cell.

Relevant to part (2) of converting a carbon source to butanol, a yeast may be engineered to convert acetyl-CoA to butanol.

In one embodiment illustrated, acetyl-CoA is converted to acetoacetyl-CoA by acetyl-CoA-acetyltransferase, acetoacetyl-CoA is converted to hydroxybutyryl-CoA by hydroxybutyryl-CoA dehydrogenase, hydroxybutyryl-CoA is converted to crotonyl-CoA by crotonase, crotonyl-CoA is converted to butyryl-CoA by butyryl-CoA dehydrogenase (bcd). Bcd requires the presence and activity of electron transfer proteins (etfA and etfB) in order to couple the reduction of crotonyl-CoA to the oxidation of NADH. Butyryl-CoA is then converted to butyraldehyde and butyraldehyde is converted to butanol by butyraldehyde dehydrogenase/butanol dehydrogenase. The enzymes may be from *C*. *acetobutylicum.*

An example of the second part of the pathway for the conversion of acetyl-CoA to n-butanol using a heterologously expressed pathway with the genes from solventogenic bacteria, for example from *Clostridium* species, is described in the U.S. Patent Application Serial No. 11/949,724, filed December 3, 2007.

In some embodiments, the recombinant microorganism may express one or more heterologous genes encoding for enzymes that confer the capability to produce n-butanol. For example, recombinant microorganisms may express heterologous genes encoding one or more of an anaerobically active pyruvate dehydrogenase (Pdh), Pyruvate formate lyase (Pfl), NADH-dependent formate dehydrogenase (Fdh), acetyl-CoA-acetyltransferase (thiolase), hydroxybutyryl-CoA dehydrogenase, crotonase, butyryl-CoA dehydrogenase, butyraldehyde dehydrogenase, n-butanol dehydrogenase, bifunctional butyraldehyde/n-butanol dehydrogenase. Such heterologous DNA sequences are preferably obtained from a heterologous microorganism (such as *Clostridium acetobutylicum* or *Clostridium beijerinckii),* and one or more of these heterologous genes may be introduced into an appropriate host using conventional molecular biology techniques. These heterologous DNA sequences enable the recombinant microorganism to produce n-butanol, at least to produce n-butanol or the metabolic intermediate(s) thereof in an amount greater than that produced by the wild-type counterpart microorganism.

In certain embodiments, the recombinant microorganism herein disclosed expresses a heterologous Thiolase or acetyl-CoA-acetyltransferase, such as one encoded by a *Thl* gene from a *Clostridium.*

Thiolase (E.C. 2.3.1.19) or acetyl-CoA acetyltransferase, is an enzyme that catalyzes the condensation of an acetyl group onto an acetyl-CoA molecule. The enzyme is, in *C*. *acetobutylicum,* encoded by the gene *thl* (GenBank accession U08465, protein ID AAA82724.1), which was overexpressed, amongst other enzymes, in *E*. *coli* under its native promoter for the production of acetone (Bermejo et al., Appl. Environ. Mirobiol. 64:1079-1085, 1998). Homologous enzymes have also been identified, and may be identified by by performing a BLAST search against above protein sequence. These homologs can also serve as suitable thiolases in a heterologously expressed n-butanol pathway. Just to name a few, these homologous enzymes include, but are not limited to, those from *C*. *acetobutylicum sp.* (e.g., protein ID AAC26026.1), *C*. *pasteurianum* (e.g., protein ID ABA18857.1), *C. beijerinckii sp.* (e.g., protein ID EAP59904.1 or EAP59331.1), *Clostridium perfringens sp.* (e.g., protein I D ABG86544.1, ABG83108.1), *Clostridium difficile sp.* (e.g., protein ID CAJ67900.1 or ZP_01231975.1), *Thermoanaerobacterium thermosaccharolyticum* (e.g., protein ID CAB07500.1), *Thermoanaerobacter tengcongensis* (e.g., AAM23825.1), *Carboxydothermus hydrogenoformans* (e.g., protein ID ABB13995.1), *Desulfotomaculum reducens* MI-1 (e.g., protein ID EAR45123.1), *Candida tropicalis* (e.g., protein ID BAA02716.1 or BAA02715.1), *Saccharomyces cerevisiae* (e.g., protein ID AAA62378.1 or CAA30788.1), *Bacillus sp., Megasphaera elsdenii,* and *Butryivibrio fibrisolvens.* In addition, the endogenous *S*. *cerevisiae* thiolase could also be active in a hetorologously expressed n-butanol pathway (ScERG10).

Homologs sharing at least about 55%, 60%, 65%, 70%, 75% or 80% sequence identity, or at least about 65%, 70%, 80% or 90% sequence homology, as calculated by NCBI's BLAST, are suitable thiolase homologs that can be used in recombinant microorganisms of the present invention. Such homologs include, but are not limited to, *Clostridium beijerinckii* NCIMB 8052 (ZP_00909576.1 or ZP_00909989.1), *Clostridium acetobutylicum* ATCC 824 (NP_149242.1), *Clostridium tetani E88* (NP_781017.1), *Clostridium perfringens str.* 13 (NP_563111.1), *Clostridium perfringens SM101* (YP_699470.1), *Clostridium pasteurianum* (ABA18857.1), *Thermoanaerobacterium thermosaccharolyticum* (CAB04793.1), *Clostridium difficile* QCD-32g58 (ZP_01231975.1), and *Clostridium difficile* 630 (CAJ67900.1).

In certain embodiments, recombinant microorganisms of the present invention express a heterologous 3-hydroxybutyryl-CoA dehydrogenase, such as one encoded by an *hbd* gene from a *Clostridium.*

The 3-hydroxybutyryl-CoA dehydrogenase (BHBD) is an enzyme that catalyzes the conversion of acetoacetyl-CoA to 3-hydroxybutyryl-CoA. Different variants of this enzyme exist that produce either the (S) or the (R) isomer of 3-hydroxybutyryl-CoA. Homologous enzymes can easily be identified by one skilled in the art by, for example, performing a BLAST search against aforementioned C. *acetobutylicum* BHBD. All these homologous enzymes could serve as a BHBD in a heterologously expressed n-butanol pathway. These homologous enzymes include, but are not limited to: *Clostridium kluyveri,* which expresses two distinct forms of this enzyme (Miller et al., J. Bacteriol. 138:99-104, 1979), and *Butyrivibrio fibrisolvens,* which contains a *bhbd gene* which is organized within the same locus of the rest of its butyrate pathway (Asanuma et al., Current Microbiology 51:91-94, 2005; Asanuma et al., Current Microbiology 47:203-207, 2003). A gene encoding a short chain acyl-CoA dehydrogenase (SCAD) was cloned from *Megasphaera elsdenii* and expressed in E. *coli. In vitro* activity could be determined (Becker et al., Biochemistry 32:10736-10742, 1993). Other homologues were identified in other *Clostridium* strains such as *C*. *kluyveri* (Hillmer et al., FEBS Lett. 21:351-354, 1972; Madan et al., Eur. J. Biochem. 32:51-56, 1973), *C. beijerinckii, C. thermosaccharolyticum, C. tetani.*

In certain embodiments, wherein a BHBD is expressed it may be beneficial to select an enzyme of the same organism the upstream thiolase or the downstream crotonase originate. This may avoid disrupting potential protein-protein interactions between proteins adjacent in the pathway when enzymes from different organisms are expressed.

In certain embodiments, the recombinant microorganism herein disclosed expresses a heterologous crotonase, such as one encoded by a *crt* gene from a *Clostridium.*

The crotonases or Enoyl-CoA hydratases are enzymes that catalyze the reversible hydration of *cis* and *trans* enoyl-CoA substrates to the corresponding β-hydroxyacyl CoA derivatives. In *C. acetobutylicum,* this step of the butanoate metabolism is catalyzed by EC 4.2.1.55, encoded by the *crt* gene (GenBank protein accession AAA95967, Kanehisa, Novartis Found Symp. 247:91-101, 2002; discussion 01-3,19-28, 244-52). The crotonase (Crt) from C. *acetobutylicum* has been purified to homogeneity and characterized (Waterson et al., J. Biol. Chem. 247:5266-5271, 1972). It behaves as a homogenous protein in both native and denatured states. The enzyme appears to function as a tetramer with a subunit molecular weight of 28.2 kDa and 261 residues (Waterson *et al.* report a molecular mass of 40 kDa and a length of 370 residues). The purified enzyme lost activity when stored in buffer solutions at 4°C or when frozen (Waterson et al., J. Biol. Chem. 247:5266-5271, 1972). The pH optimum for the enzyme is pH 8.4 (Schomburg et al., Nucleic Acids Res. 32:D431-433, 2004). Unlike the mammalian crotonases that have a broad substrate specificity, the bacterial enzyme hydrates only crotonyl-CoA and hexenoyl-CoA. Values of *Vₘₐₓ* and *Kₘ* of 6.5 x 10⁶ moles per min per mole and 3 x 10⁻⁵ M were obtained for crotonyl-CoA. The enzyme is inhibited at crotonyl-CoA concentrations of higher than 7 x 10⁵ M (Waterson et al., J. Biol. Chem. 247:5252-5257, 1972; Waterson et al., J. Biol. Chem. 247:5258-5265, 1972).

The structures of many of the crotonase family of enzymes have been solved (Engel et al., J. Mol. Biol. 275:847-859, 1998). The *crt* gene is highly expressed in *E*. *coli and* exhibits a higher specific activity than seen in *C. acetobutylicum* (187.5 U/mg over 128.6 U/mg) (Boynton et al., J. Bacteriol. 178:3015-3024, 1996). A number of different homologs of crotonase are encoded in eukaryotes and prokaryotes that functions as part of the butanoate metabolism, fatty acid synthesis, β-oxidation and other related pathways (Kanehisa, Novartis Found Symp. 247:91-101, 2002; discussion 01-3, 19-28, 244-52; Schomburg et al., Nucleic Acids Res. 32:D431-433, 2003). A number of these enzymes have been well studied. Enoyl-CoA hydratase from bovine liver is extremely well-studied and thoroughly characterized (Waterson et al., J. Biol. Chem. 247:5252-5257, 1972). A ClustalW alignment of 20 closest orthologs of crotonase from bacteria is generated. The homologs vary in sequence identity from 40-85%.

Homologs sharing at least about 45%, 50%, 55%, 60%, 65% or 70% sequence identity, or at least about 55%, 65%, 75% or 85% sequence homology, as calculated by NCBI's BLAST, are suitable Crt homologs that can be used in recombinant microorganisms of the present invention. Such homologs include, but are not limited to, *Clostridium tetani* E88 (NP_782956.1), *Clostridium perfringens SM101* (YP_699562.1), *Clostridium perfringens str.* 13 (NP_563217.1), *Clostridium beijerinckii* NCIMB 8052 (ZP_00909698.1 or ZP_00910124.1), *Syntrophomonas wolfei subsp. wolfei str. Goettingen* (YP_754604.1), *Desulfotomaculum reducens MI-1* (ZP_01147473.1 or ZP_01149651.1), *Thermoanaerobacterium thermosaccharolyticum* (CAB07495.1), and *Carboxydothermus hydrogenoformans* Z-2901 (YP_360429.1).

Studies in *Clostridia* demonstrate that the *crt* gene that codes for crotonase is encoded as part of the larger BCS operon. However, studies on *B. fibriosolvens,* a butyrate producing bacterium from the rumen, show a slightly different arrangement. While Type I *B. fibriosolvens* have the *thl, crt, hbd, bcd, etfA* and *etfB* genes clustered and arranged as part of an operon, Type II strains have a similar cluster but lack the *crt* gene (Asanuma et al., Curr. Microbiol. 51:91-94, 2005; Asanuma et al., Curr. Microbiol. 47:203-207, 2003). Since the protein is well-expressed in *E. coli* and thoroughly characterized, the *C*. *acetobutylicum* enzyme is the preferred enzyme for the heterologously expressed n-butanol pathway. Other possible targets are homologous genes from *Fusobacterium nucleatum subsp. Vincentii* (Q7P3U9-Q7P3U9_FUSNV), *Clostridium difficile* (P45361-CRT_CLODI), *Clostridium pasteurianum* (P81357-CRT_CLOPA), and *Brucella melitensis* (Q8YDG2-Q8YDG2_BRUME).

In certain embodiments, the recombinant microorganism herein disclosed expresses a heterologous butyryl-CoA dehydrogenase and if necessary the corresponding electron transfer proteins, such as encoded by the *bcd, etfA,* and *etfB* genes from a *Clostridium.*

The *C*. *acetobutylicum* butyryl-CoA dehydrogenase (Bcd) is an enzyme that catalyzes the reduction of the carbon-carbon double bond in crotonyl-CoA to yield butyryl-CoA. This reduction is coupled to the oxidation of NADH. However, the enzyme requires two electron transfer proteins *etfA* and *etfB* (Bennett et al., Fems Microbiology Reviews 17:241-249, 1995).

The *Clostridium acetobutylicum* ATCC 824 genes encoding the enzymes beta-hydroxybutyryl-coenzyme A (CoA) dehydrogenase, crotonase and butyryl-CoA dehydrogenase are clustered on the BCS operon, which GenBank accession number is U17110.

The butyryl-CoA dehydrogenase (Bcd) protein sequence (Genbank accession # AAA95968.1) is given in SEQ ID NO:3.

Homologs sharing at least about 55%, 60%, 65%, 70%, 75% or 80% sequence identity, or at least about 70%, 80%, 85% or 90% sequence homology, as calculated by NCBI's BLAST, are suitable Bcd homologs that can be used in recombinant microorganisms of the present invention. Such homologs include, but are not limited to, *Clostridium tetani* E88 (NP_782955.1 or NP_781376.1), *Clostridium perfringens str.* 13 (NP_563216.1), *Clostridium beijerinckii* (AF494018_2), *Clostridium beijerinckii* NCIMB 8052 (ZP_00910125.1 or ZP_00909697.1), and *Thermoanaerobacterium thermosaccharolyticum* (CAB07496.1), *Thermoanaerobacter tengcongensis MB4* (NP_622217.1).

Homologs sharing at least about 45%, 50%, 55%, 60%, 65% or 70% sequence identity, or at least about 60%, 70%, 80% or 90% sequence homology, as calculated by NCBI's BLAST, are suitable Hbd homologs that can be used in the recombinant microorganism herein described. Such homologs include, but are not limited to, *Clostridium acetobutylicum* ATCC 824 (NP_349314.1), *Clostridium tetani* E88 (NP_782952.1), *Clostridium perfringens SM101* (YP_699558.1), *Clostridium perfringens str.* 13 (NP_563213.1), *Clostridium saccharobutylicum* (AAA23208.1), *Clostridium beijerinckii* NCIMB 8052 (ZP_00910128.1), *lostridium beijerinckii* (AF494018_5), *Thermoanaerobacter tengcongensis MB4* (NP_622220.1), *Thermoanaerobacterium thermosaccharolyticum* (CAB04792.1), and *lkaliphilus metalliredigenes QYMF* (ZP_00802337.1).

The *Kₘ* of Bcd for butyryl-CoA is 5. *C. acetobutylicum bcd* and the genes encoding the respective ETFs have been cloned into an *E. coli - C. acetobutylicum* shuttle vector. Increased Bcd activity was detected in *C. acetobutylicum* ATCC 824 transformed with this plasmid (Boynton et al., Journal of Bacteriology 178:3015-3024, 1996). The *Kₘ* of the *C*. *acetobutylicum* P262 Bcd for butyryl-CoA is approximately 6 µM (DiezGonzalez et al., Current Microbiology 34:162-166, 1997). Homologues of Bcd and the related ETFs have been identified in the butyrate-producing anaerobes *Megasphaera elsdenii* (Williamson et al., Biochemical Journal 218:521-529, 1984), *Peptostreptococcus elsdenii* (Engel et al., Biochemical Journal 125:879, 1971), *Syntrophospora bryanti* (Dong et al., Antonie Van Leeuwenhoek International Journal of General and Molecular Microbiology 67:345-350, 1995), and *Treponema phagedemes* (George et al., Journal of Bacteriology 152:1049-1059, 1982). The structure of the *M*. *elsdenii* Bcd has been solved (Djordjevic et al., Biochemistry 34:2163-2171, 1995). A BLAST search of *C*. *acetobutylicum* ATCC 824 Bcd identified a vast amount of homologous sequences from a wide variety of species, some of the homologs are listed herein above. Any of the genes encoding these homologs may be used for the subject invention. It is noted that expression issues, electron transfer issues, or both issues, may arise when heterologously expressing these genes in one microorganism (such as *E. coli*) but not in another. In addition, one homologous enzyme may have expression and/or electron transfer issues in a given microorganism, but other homologous enzymes may not. The availability of different, largely equivalent genes provides more design choices when engineering the recombinant microorganism.

One promising *bcd* that has already been cloned and expressed in *E*. *coli* is from *Megasphaera elsdenii,* and *in vitro* activity of the expressed enzyme could be determined (Becker et al., Biochemistry 32:10736-10742, 1993). O'Neill *et al.* reported the cloning and heterologous expression in *E*. *coli* of the *etfA* and *eftB* genes and functional characterization of the encoded proteins from *Megasphaera elsdenii* (O'Neill et al., J. Biol. Chem. 273:21015-21024, 1998). Activity was measured with the ETF assay that couples NADH oxidation to the reduction of crotonyl-CoA via Bcd. The activity of recombinant ETF in the ETF assay with Bcd is similar to that of the native enzyme as reported by Whitfield and Mayhew. Therefore, utilizing the *Megasphaera elsdenii* Bcd and its ETF proteins provides a solution to synthesize butyryl-CoA. The *Kₘ* of the *M*. *elsdenii* Bcd was measured as 5 µM when expressed recombinantly, and 14 µM when expressed in the native host (DuPlessis et al., Biochemistry 37:10469-77, 1998). *M*. *elsdenii* Bcd appears to be inhibited by acetoacetate at extremely low concentrations (*Kᵢ* of 0.1 µM) (Vanberkel et al., Eur. J. Biochem. 178:197-207, 1988). A gene cluster containing *thl, crt, hbd, bcd, etfA,* and *etfB* was identified in two butyrate producing strains of *Butyrivibrio fibrisolvens.* The amino acid sequence similarity of these proteins is high, compared to *Clostridium acetobutylicum* (Asanuma et al., Current Microbiology 51:91-94, 2005; Asanuma et al., Current Microbiology 47:203-207, 2003). In mammalian systems, a similar enzyme, involved in short-chain fatty acid oxidation is found in mitochondria.

In certain embodiments, the recombinant microorganism herein disclosed expresses a heterologous "trans-2-enoyl-CoA reductase" or "TER".

Trans-2-enoyl-CoA reductase or TER is a protein that is capable of catalyzing the conversion of crotonyl-CoA to butyryl-CoA. In certain embodiments, the recombinant microorganism expresses a TER which catalyzes the same reaction as Bcd/EtfA/EtfB from *Clostridia* and other bacterial species. Mitochondrial TER from *E*. *gracilis* has been described, and many TER proteins and proteins with TER activity derived from a number of species have been identified forming a TER protein family (U.S. Pat. Appl. 2007/0022497 to Cirpus et al.*;* Hoffmeister et al., J. Biol. Chem., 280:4329-4338, 2005). A truncated cDNA of the E. *gracilis* gene has been functionally expressed in *E*. *coli*. This cDNA or the genes of homologues from other microorganisms can be expressed together with the n-butanol pathway genes *thl, crt, adhE2,* and *hbd* to produce n-butanol in *E*. *coli, S. cerevisiae* or other hosts.

TER proteins can also be identified by generally well known bioinformatics methods, such as BLAST. Examples of TER proteins include, but are not limited to, TERs from species such as: *Euglena* spp. including, but not limited to, *E. gracilis, Aeromonas* spp. including, but not limited, to *A. hydrophila, Psychromonas* spp. including, but not limited to, *P. ingrahamii,* Photobacterium spp. including, but not limited, to *P. profundum,* Vibrio spp. including, but not limited, to *V angusfum, V. cholerae, V alginolyticus, V parahaemolyticus, V vulnificus, V fischeri, V splendidus, Shewanella* spp. including, but not limited to, *S*. *amazonensis, S. woodyi, S. frigidimarina, S. paeleana, S. baltica, S. denitrificans, Oceanospirillum* spp., *Xanthomonas* spp. including, but not limited to, *X oryzae, X campestris, Chromohalobacter* spp. including, but not limited, to *C. salexigens, Idiomarina* spp. including, but not limited, to *I. baltica, Pseudoalteromonas* spp. including, but not limited to, *P. atlantica, Alteromonas* spp., *Saccharophagus* spp. including, but not limited to, S. *degradans, S. marine gamma proteobacterium, S. alpha proteobacterium, Pseudomonas* spp. including, but not limited to, *P. aeruginosa, P. putida, P. fluorescens, Burkholderia* spp. including, but not limited to, *B. phytofirmans, B. cenocepacia, B. cepacia, B. ambifaria, B. vietnamensis, B. multivorans, B. dolosa, Methylbacillus* spp. including, but not limited to, *M. flageliatus, Stenotrophomonas* spp. including, but not limited to, *S*. *maltophilia, Congregibacter* spp. including, but not limited to, *C. litoralis, Serratia* spp. including, but not limited to, *S. proteamaculans, Marinomonas* spp., *Xytella* spp. including, but not limited to, *X fastidiosa, Reinekea* spp., *Colwellia* spp. including, but not limited to, *C. psychrerythraea, Yersinia* spp. including, but not limited to, *Y*. *pestis, Y. pseudotuberculosis, Methylobacillus* spp. including, but not limited to, *M flagellatus, Cytophaga* spp. including, but not limited to, *C. hutchinsonii, Flavobacterium* spp. including, but not limited to, *F. johnsoniae, Microscilla* spp. including, but not limited to, *M marina, Polaribacter* spp. including, but not limited to, *P*. *irgensii, Clostridium* spp. including, but not limited to, C. *acetobutylicum,* C. *beijerenckii,* C. *cellulolyticum, Coxiella* spp. including, but not limited to, *C.burnetii.*

In addition to the foregoing, the terms "trans-2-enoyl-CoA reductase" or "TER" refer to proteins that are capable of catalyzing the conversion of crotonyl-CoA to butyryl-CoA and which share at least about 40%,45%, 50%, 55%, 60%, 65%, 70%, 75%, 80%, 85%, 90%, 95%, 96%, 97%, 98%, 99% or greater sequence identity, or at least about 50%, 60%, 70%, 80%, 90%, 95%,96%,97%,98%,99% or greater sequence similarity, as calculated by NCBI BLAST, using default parameters, to either or both of the truncated *E. gracilis* TER or the full length *A*. *hydrophila* TER.

As used herein, "sequence identity" refers to the occurrence of exactly the same nucleotide or amino acid in the same position in aligned sequences. "Sequence similarity" takes approximate matches into account, and is meaningful only when such substitutions are scored according to some measure of "difference" or "sameness" with conservative or highly probably substitutions assigned more favorable scores than non-conservative or unlikely ones.

Another advantage of using TER instead of Bcd/EtfA/EtfB is that TER is active as a monomer and neither the expression of the protein nor the enzyme itself is sensitive to oxygen.

As used herein, "trans-2-enoyl-CoA reductase (TER) homologue" refers to an enzyme homologous polypeptides from other organisms, e.g., belonging to the phylum *Euglena* or *Aeromonas*, which have the same essential characteristics of TER as defined above, but share less than 40% sequence identity and 50% sequence similarity standards as discussed above. Mutations encompass substitutions, additions, deletions, inversions or insertions of one or more amino acid residues. This allows expression of the enzyme during an aerobic growth and expression phase of the n-butanol process, which could potentially allow for a more efficient biofuel production process.

In certain embodiments, the recombinant microorganism herein disclosed expresses a heterologous butyraldehyde dehydrogenase / n-butanol dehydrogenase, such as encoded by the *bdhA* / *bdhB,* aad, or *adhE2* genes from a *Clostridium.*

The Butyraldehyde dehydrogenase (BYDH) is an enzyme that catalyzes the NADH-dependent reduction of butyryl-CoA to butyraldehyde. Butyraldehyde is further reduced to n-butanol by an n-butanol dehydrogenase (BDH). This reduction is also accompanied by NADH oxidation. *Clostridium acetobutylicum* contains genes for several enzymes that have been shown to convert butyryl-CoA to n-butanol.

One of these enzymes is encoded by aad (Nair et al., J. Bacteriol. 176:871-885, 1994). This gene is referred to as *adhE* in C. *acetobutylicum* strain DSM 792. The enzyme is part of the *sol* operon and it encodes for a bifunctional BYDH/BDH (Fischer et al., Journal of Bacteriology 175:6959-6969, 1993; Nair et al., J. Bacteriol. 176:871-885, 1994).

The gene product of *aad* was functionally expressed in *E. coli.* However, under aerobic conditions, the resulting activity remained very low, indicating oxygen sensitivity. With a greater than 100-fold higher activity for butyraldehyde compared to acetaldehyde, the primary role of Aad is in the formation of n-butanol rather than of ethanol (Nair et al., Journal of Bacteriology 176:5843-5846, 1994).

Homologs sharing at least about 50%, 55%, 60% or 65% sequence identity, or at least about 70%, 75% or 80% sequence homology, as calculated by NCBI's BLAST, are suitable homologs that can be used in the recombinant microorganisms herein disclosed. Such homologs include (without limitation): *Clostridium tetani* E88 (NP_781989.1), *Clostridium perfringens str.* 13 (NP_563447.1), *Clostridium perfringens* ATCC 13124 (YP_697219.1), *Clostridium perfringens* SM101 (YP_699787.1), *Clostridium beijerinckii* NCIMB 8052 (ZP_00910108.1), *Clostridium acetobutylicum* ATCC 824 (NP_149199.1), *Clostridium difficile* 630 (CAJ69859.1), *Clostridium difficile* QCD-32g58 (ZP_01229976.1), and *Clostridium thermocellum* ATCC 27405 (ZP_00504828.1).

Two additional NADH-dependent n-butanol dehydrogenases (BDH I, BDH II) have been purified, and their genes (*bdhA, bdhB*) cloned. The GenBank accession for BDH I is AAA23206.1, and the protein sequence is given in SEQ ID NO:10.

The GenBank accession for BDH II is AAA23207.1, and the protein sequence is given in SEQ ID NO:11.

These genes are adjacent on the chromosome, but are transcribed by their own promoters (Walter et al., Gene 134:107-111, 1993). BDH I utilizes NADPH as the cofactor, while BDH II utilizes NADH. However, it is noted that the relative cofactor preference is pH-dependent. BDH I activity was observed in *E*. *coli* lysates after expressing *bdhA* from a plasmid (Petersen et al., Journal of Bacteriology 173:1831-1834, 1991). BDH II was reported to have a 46-fold higher activity with butyraldehyde than with acetaldehyde and is 50-fold less active in the reverse direction. BDH I is only about two-fold more active with butyraldehyde than with acetaldehyde (Welch et al., Archives of Biochemistry and Biophysics 273:309-318, 1989). Thus in one embodiment, BDH II or a homologue of BDH II is used in a heterologously expressed n-butanol pathway. In addition, these enzymes are most active under a relatively low pH of 5.5, which trait might be taken into consideration when choosing a suitable host and/or process conditions.

While the afore-mentioned genes are transcribed under solventogenic conditions, a different gene, *adhE2* is transcribed under alcohologenic conditions (Fontaine et al., J. Bacteriol. 184:821-830, 2002, GenBank accession # AF321779). These conditions are present at relatively neutral pH. The enzyme has been overexpressed in anaerobic cultures of *E. coli* and with high NADH-dependent BYDH and BDH activities. In certain embodiments, this enzyme is the preferred enzyme. The protein sequence of this enzyme (GenBank accession # AAK09379.1) is listed as SEQ ID NO:1.

Homologs sharing at least about 50%, 55%, 60% or 65% sequence identity, or at least about 70%, 75% or 80% sequence homology, as calculated by NCBI's BLAST, are suitable homologs that can be used in the recombinant microorganisms herein disclosed. Such homologs include, but are not limited to, *Clostridium perfringens SM101* (YP_699787.1), *Clostridium perfringens str.* 13 (NP_563447.1), *Clostridium perfringens* ATCC 13124 (YP_697219.1), *Clostridium tetani* E88 (NP_781989.1), *Clostridium beijerinckii* NCIMB 8052 (ZP_00910108.1), *Clostridium difficile* QCD-32g58 (ZP_01229976.1), *Clostridium difficile* 630 (CAJ69859.1), *Clostridium acetobutylicum* ATCC 824 (NP_149325.1), and *Clostridium thermocellum* ATCC 27405 (ZP_00504828.1).

In certain embodiments, any homologous enzymes that are at least about 70%, 80%, 90%, 95%, 99% identical, or sharing at least about 60%, 70%, 80%, 90%, 95% sequence homology (similar) to any of the above polypeptides may be used in place of these wild-type polypeptides. These enzymes sharing the requisite sequence identity or similarity may be wild-type enzymes from a different organism, or may be artificial, recombinant enzymes.

In certain embodiments, any genes encoding for enzymes with the same activity as any of the above enzymes may be used in place of the genes encoding the above enzymes. These enzymes may be wild-type enzymes from a different organism, or may be artificial, recombinant or engineered enzymes.

Additionally, due to the inherent degeneracy of the genetic code, other nucleic acid sequences which encode substantially the same or a functionally equivalent amino acid sequence can also be used to clone and express the polynucleotides encoding such enzymes. As will be understood by those of skill in the art, it can be advantageous to modify a coding sequence to enhance its expression in a particular host. The codons that are utilized most often in a species are called optimal codons, and those not utilized very often are classified as rare or low-usage codons. Codons can be substituted to reflect the preferred codon usage of the host, a process sometimes called "codon optimization" or "controlling for species codon bias." Methodology for optimizing a nucleotide sequence for expression in a plant is provided, for example, in U.S. Pat. No. 6,015,891, and the references cited therein]

In certain embodiments, the recombinant microorganism herein disclosed has one or more heterologous DNA sequence(s) from a solventogenic *Clostridia,* such as *Clostridium acetobutylicum* or *Clostridium beijerinckii.* An exemplary *Clostridium acetobutylicum* is strain ATCC824, and an exemplary *Clostridium beijerinckii* is strain NCIMB 8052.

Expression of the genes may be accomplished by conventional molecular biology means. For example, the heterologous genes can be under the control of an inducible promoter or a constitutive promoter. The heterologous genes may either be integrated into a chromosome of the host microorganism, or exist as an extra-chromosomal genetic elements that can be stably passed on ("inherited") to daughter cells. Such extra-chromosomal genetic elements (such as plasmids, BAC, YAC, etc.) may additionally contain selection markers that ensure the presence of such genetic elements in daughter cells.

In certain embodiments, the recombinant microorganism herein disclosed may also produce one or more metabolic intermediate(s) of the n-butanol-producing pathway, such as acetoacetyl-CoA, hydroxybutyryl-CoA, crotonyl-CoA, butyryl-CoA, or butyraldehyde, and/or derivatives thereof, such as butyrate.

In some embodiments, the recombinant microorganisms herein described engineered to activate one or more of the above mentioned heterologous enzymes for the production of n-butanol, produce n-butanol via a heterologous pathway.

As used herein, the term "pathway" refers to a biological process including one or more enzymatically controlled chemical reactions by which a substrate is converted into a product. Accordingly, a pathway for the conversion of a carbon source to n-butanol is a biological process including one or more enzymatically controlled reaction by which the carbon source is converted into n-butanol. A "heterologous pathway" refers to a pathway wherein at least one of the at least one or more chemical reactions is catalyzed by at least one heterologous enzyme. On the other hand, a "native pathway" refers to a pathway wherein the one or more chemical reactions is catalyzed by a native enzyme.

In certain embodiments, the recombinant microorganism herein disclosed are engineered to activate an n-butanol producing heterologous pathway (herein also indicated as n-butanol pathway) that comprises: (1) Conversion of 2 Acetyl-CoA to Acetoacetyl-CoA, (2) Conversion of Acetoacetyl CoA to Hydroxybutyryl-CoA, (3) Conversion of Hydroxybutyryl-CoA to Crotonyl-CoA, (4) Conversion of Crotonyl CoA to Butyryl-CoA, (5) Conversion of Butyraldehyde to n-butanol, (see the exemplary illustration of Fig. 2).

The conversion of 2 Acetyl-CoA to Acetoacetyl-CoA can be performed by expressing a native or heterologous gene encoding for an acetyl-CoA-acetyl transferase (thiolase) or Thl in the recombinant microorganism. Exemplary thiolases suitable in the recombinant microorganism herein disclosed are encoded by *thl* from *Clostridium acetobutylicum,* and in particular from strain ATCC824 or a gene encoding a homologous enzyme from *C*. *pasteurianum, C. beijerinckii,* in particular from strain NCIMB 8052 or strain BA101, *Candida tropicalis, Bacillus* spp., *Megasphaera elsdenii,* or *Butyrivibrio fibrisolvens,* or an *E*. *coli* thiolase selected from *fadA* or *atoB.*

The conversion of Acetoacetyl CoA to Hydroxybutyryl-CoA can be performed by expressing a native or heterologous gene encoding for hydroxybutyryl-CoA dehydrogenase Hbd in the recombinant microorganism. Exemplary Hbd suitable in the recombinant microorganism herein disclosed are encoded by *hbd* from *Clostridium acetobutylicum,* and in particular from strain ATCC824, or a gene encoding a homologous enzyme from *Clostridium kluyveri, Clostridium beijerinckii,* and in particular from strain NCIMB 8052 or strain BA101, *Clostridium thermosaccharolyticum, Clostridium tetani, Butyrivibrio fibrisolvens, Megasphaera elsdenii,* or *E*. *coli* (*fadB*).

The conversion of Hydroxybutyryl-CoA to Crotonyl-CoA can be performed by expressing a native or heterologous gene encoding for a crotonase or Crt in the recombinant microorganism. Exemplary *crt* suitable in the recombinant microorganism herein disclosed are encoded by *crt* from *Clostridium acetobutylicum,* and in particular from strain ATCC824, or a gene encoding a homologous enzyme from *B. fibriosolvens, Fusobacterium nucleatum* subsp. *Vincentii, Clostridium difficile, Clostridium pasteurianum,* or *Brucella melitensis.*

The conversion of Crotonyl CoA to Butyryl-CoA can be performed by expressing a native or heterologous gene encoding for a butyryl-CoA dehydrogenase in the recombinant microorganism. Exemplary butyryl-CoA dehydrogenases suitable in the recombinant microorganism herein disclosed are encoded by *bcd*/*etfA*/*etfB* from *Clostridium acetobutylicum,* and in particular from strain ATCC824, or a gene encoding a homologous enzyme from *Megasphaera elsdenii, Peptostreptococcus elsdenii, Syntrophospora bryanti, Treponema phagedemes, Butyrivibrio fibrisolvens,* or a mammalian mitochondria Bcd homolog.

The conversion of Butyraldehyde to n-butanol can be performed by expressing a native or heterologous gene encoding for a butyraldehyde dehydrogenase or a n-butanol dehydrogenase in the recombinant microorganism. Exemplary butyraldehyde dehydrogenase / n-butanol dehydrogenase suitable in the recombinant microorganism herein disclosed are encoded by *bdhA, bdhB, aad,* or *adhE2* from *Clostridium acetobutylicum,* and in particular from strain ATCC824, or a gene encoding ADH-1, ADH-2, or ADH-3 from *Clostridium beijerinckii,* in particular from strain NCIMB 8052 or strain BA101.

In certain embodiments, the enzymes of the metabolic pathway from acetyl-CoA to n-butanol are (i) thiolase (Thl), (ii) hydroxybutyryl-CoA dehydrogenase (Hbd), (iii) crotonase (Crt), (iv) at least one of alcohol dehydrogenase (AdhE2), or n-butanol dehydrogenase (Aad) or butyraldehyde dehydrogenase (Ald) together with a monofunctional n-butanol dehydrogenase (BdhA/BdhB), and (v) trans-2-enoyl-CoA reductase (TER) (Fig. 2). In certain embodiments, the Thl, Hbd, Crt, AdhE2, Aid, BdhA/BdhB and Aad are from *Clostridium.* In certain embodiments, the *Clostridium* is a *C. acetobutylicum.* In certain embodiments, the TER is from *Euglena gracilis* or from *Aeromonas hydrophila.*

In certain embodiments, one or more heterologous genes encodes one or more of acetyl-CoA-acetyltransferase (thiolase), hydroxybutyryl-CoA dehydrogenase *(hbd),* crotonase (*crt*), and alcohol dehydrogenase (*adhE2*), butyryl-CoA dehydrogenase (bcd), butyraldehyde dehydrogenase *(bdhA*/*bdhB)* / butanol dehydrogenase (*aad*), and trans-2-enoyl-CoA reductase (TER).

For example, the acetyl-CoA-acetyltransferase (thiolase) may be thl from *Clostridium acetobutylicum,* or a homologous enzyme from *C*. *pasteurianum, Clostridium beijerinckii, Candida tropicalis, Bacillus sp., Megasphaera elsdenii,* or *Butryivibrio fibrisolvens,* or an *E*. *coli* thiolase selected from fadA or atoB.

The hydroxybutyryl-CoA dehydrogenase may be hbd from *C*. *acetobutylicum,* or a homologous enzyme from *Clostridium kluyveri, Clostridium beijerinckii, Clostridium thermosaccharolyticum, Clostridium tetani, Butyrivibrio fibrisolvens, Megasphaera elsdenii,* or *Escherichia coli* (fadB).

The crotonase may be crt from *Clostridium acetobutylicum,* or a homologous enzyme from B. *fibriosolvens,* Fusobacterium nucleatum subsp. Vincentii, *Clostridium difficile, Clostridium pasteurianum,* or *Brucella melitensis.*

The butyryl-CoA dehydrogenase may be bed / etfA / etfB from *Clostridium acetobutylicum,* or a homologous enzyme from *Megasphaera elsdenii, Peptostreptococcus elsdenii, Syntrophospora bryanti, Treponema phagedemes, Butyrivibrio fibrisolvens,* or a eukaryotic mitochondrial bcd homolog.

The butyraldehyde dehydrogenase / butanol dehydrogenase may be bdhA, bdhB, aad, or adhE2 from *Clostridium acetobutylicum,* or ADH-1, ADH-2, or ADH-3 from *Clostridium beijerinckii.*

The enzyme trans-2-enoyl-CoA reductase (TER), may be from a *Euglena gracilis* or an *Aeromonas hydrophila.*

The one or more heterologous DNA sequence(s) may be from a solventogenic *Clostridium* selected from *Clostridium acetobutylicum* or *Clostridium beijerinckii,* or from *Clostridium difficile, Clostridium pasteurianum, Clostridium kluyveri, Clostridium thermosaccharolyticum, Clostridium tetani, Candida tropicalis, Bacillus sp., Brucella melitensis, Megasphaera elsdenii, Butryivibrio fibrisolvens, Fusobacterium nucleatum subsp. Vincentii, Peptostreptococcus elsdenii, Syntrophospora bryanti, Treponema phagedemes,* or *E. coli.*

In certain embodiments, the *Clostridium acetobutylicum* is strain ATCC824, and the *Clostridium beijerinckii* is strain NCIMB 8052 or strain BA101. In certain embodiments, homologs sharing at least 40%, 45%, 50%, 55%, 60%, 65%, 70%, 75%, 80%, 85%, 90% sequence identity, or at least about 50%, 60%, 70%, 80%, 90% sequence identity (as calculated by NCBI BLAST, using default parameters) are suitable for the subject invention.

### Part (1): Engineering the conversion of pyruvate to acetyl-CoA

As described above, the conversion of pyruvate to acetyl-CoA may occur in an engineered cell by two general routes: (A) the "PDH bypass" route as defined above or (B) the direct conversion of pyruvate to acetyl-CoA in the cytosol by PDH or by PFL.

### (A) Acetyl-CoA generation via the "PDH bypass" route

Relating to the route (A) in generating acetyl CoA from pyruvate, the cytosolic acetyl-CoA generation pathway is catalyzed by three enzymes as shown in Fig. 3, Steps 1, 2 and 3. A more efficient pathway for generation of acetyl-CoA is achieved by increasing the activity of those enzymes that are rate-limiting. For example, in *Saccharomyces cerevisiae,* if ALD activity is limiting in a pathway, overexpression of *ALD6* will thereby increase the overall flux through the pathway. Increased acetyl-CoA formation in the cytosol is achieved via one of the following mechanisms or a combination thereof:

In one embodiment, increased acetyl-CoA may be generated by the overexpression of a pyruvate decarboxylase gene (for example, *S. cerevisiae PDC1, PDC5* and/or *PDC6;* Step 1).

In another embodiment, increased acetyl-CoA may be generated by the overexpression of an acetaldehyde dehydrogenase gene (for example, *S. cerevisiae ALD6;* Step 2).

In yet another embodiment, increased acetyl-CoA may be produced by the overexpression of an acetyl-CoA synthase gene (for example, *S. cerevisiae ACS1* or *ACS2* or both; Step 3).

In a different embodiment, simultaneous overexpression of both ALD and ACS (*S. cerevisiae ALD6;* Step 2) may generate increased acetyl-CoA (Steps 2 and 3).

In another embodiment, simultaneous overexpression of PDC, ALD, and ACS genes may generate increased production of acetyl-CoA (Steps 1-3).

To further increase production of acetyl-CoA, the major cytosolic ethanol production pathway in yeast can be reduced or eliminated. In Crabtree positive, *S*. *cerevisiae,* this is achieved by the deletion of *ADH1* which is the predominant source of cytosolic ADH activity. Cells deleted for *ADH1* are unable to grow anaerobically (Drewke et al., (1990). J. Bacteriology 172(7):3909), and thus may be preferably deleted to minimize conversion of acetaldehyde to ethanol. Eliminating this pathway selectively drives acetaldehyde towards acetate and subsequently to acetyl-CoA production (Fig. 3, Step 5). Therefore, overexpression of the genes described above may be carried out in a cell having reduced or eliminated ADH activity.

Similarly, cytosolic ADH activity may be reduced or eliminated in a Crabtree negative yeast such as *Kluyveromyces lactis* by the deletion of ADHI or ADHII to increase the flux from pyruvate to acetyl-CoA via the "PDH bypass" route. Therefore, in this organism, similar to that proposed to *S. cerevisiae* above, the flux via the "PDH bypass" route could be increased by the over-expression of *KIALD6, KIACS1* or *KIACS2* alone or in combination.

### (B) Direct generation of Acetyl-CoA from Pyruvate

Relating to the route (B) of generating acetyl CoA from pyruvate, acetyl-CoA production may be increased by the overexpression of the genes forming a complete PDH complex. For example, the overexpressed genes may be from *E*. *coli* (*aceE, aceF,* and *IpdA*), *Zymomonas mobilis* (*pdhA*α*, pdhA*β*, pdhB,* and *Ipd*), *S. aureus (pdhA, pdhB, pdhC,* and *Ipd*), *Bacillus subtilis, Corynebacterium glutamicum,* or *Pseudomonas aeruginosa* (Step 4).

Pyruvate dehydrogenase enzyme complex catalyzes the conversion of pyruvate to acetyl-CoA. In *S*. *cerevisiae,* this complex is localized in the mitochondrial inner membrane space. Consequently, another method to obtain higher levels of acetyl-CoA in the cytoplasm of *S*. *cerevisiae* is to engineer a cell to overexpress a eukaryotic or prokaryotic pyruvate dehydrogenase complex which can function in the cytoplasm (Step 4). In certain embodiments, the recombinant microorganism herein disclosed includes an active pyruvate dehydrogenase (Pdh) under anaerobic or microaerobic conditions. The pyruvate dehydrogenase or NADH-dependent formate dehydrogenase may be heterologous to the recombinant microorganism, in that the coding sequence encoding these enzymes is heterologous, or the transcriptional regulatory region is heterologous (including artificial), or the encoded polypeptides comprise sequence changes that renders the enzyme resistant to feedback inhibition by certain metabolic intermediates or substrates.

Until recently, it was widely accepted that Pdh does not function under anaerobic conditions, but several recent reports have demonstrated that this is not the case (de Graef, M. et al, 1999, Journal of Bacteriology, 181, 2351-57; Vernuri, G.N. et al, 2002, Applied and Environmental Microbiology, 68, 1715-27). Moreover, other microorganisms such as *Enterococcus faecalis* exhibit high in vivo activity of the Pdh complex, even under anaerobic conditions, provided that growth conditions were such that the steady-state NADH/NAD⁺ ratio was sufficiently low (Snoep, J.L. et al, 1991, Fems Microbiology Letters, 81, 63-66). Instead of oxygen regulating the expression and function of Pdh, it has been shown that Pdh is regulated by NADH/NAD⁺ ratio (de Graef, M. et al, 1999, Journal of Bacteriology, 181, 2351-57. If the n-butanol pathway expressed in a host cell consumes NADH fast enough to maintain a low NADH/NAD⁺ level inside the cell, an endogenous or heterologously expressed Pdh may remain active and provide NADH sufficient to balance the pathway.

These Pdh enzymes can balance the n-butanol pathway in a recombinant microorganism herein disclosed.

Expression of a Pdh that is functional under anaerobic conditions is expected to increase the moles of NADH obtained per mole of glucose. Kim et al. describe a Pdh that makes available in *E*. *coli* up to four moles of NADH per mole of glucose consumed (Kim, Y. et al.(2007). Appl. Environm. Microbiol., 73, 1766-1771). Yeast cells can also be engineered to express PDH complexes from diverse bacterial sources. For example, Pdh from *Enterococcus faecalis* is similar to the Pdh from *E*. *coli* but is inactivated at much lower NADH/NAD⁺ levels. Additionally, some organisms such as *Bacillus subtilis* and almost all strains of lactic acid bacteria use a Pdh in anaerobic metabolism. Expression of an n-butanol production pathway in a microorganism expressing an Pdh that is anaerobically active is expected to result in n-butanol yields of greater than 1.4% if the n-butanol production pathway can compete with endogenous fermentative pathways.

Alternatively, acetyl-CoA may be produced in the cytosol by overexpressing two bacterial enzymes, a pyruvate formate lyase (e.g., *E*. *coli pflB*) and a formate dehydrogenase (e.g., *Candida boidinii fdh1*). Using this pathway, pyruvate is converted to acetyl-CoA and formate. Formate dehydrogenase then catalyzes the NADH-dependent conversion of formate to carbon dioxide. The net result of these reactions is the same as if pyruvate was converted to acetyl-CoA by pyruvate dehydrogenase complex:

Pyruvate + NAO⁺ → acetyl-CoA + NADH + CO₂.

NADH-dependent formate dehydrogenase (Fdh; EC 1.2.1.2) catalyzes the oxidation of formate to CO₂ and the simultaneous reduction of NAD⁺ to NADH. Fdh can be used in accordance with the present invention to increase the intracellular availability of NADH within the host microorganism and may be used to balance the n-butanol producing pathway with respect to NADH. In particular, a biologically active NADH-dependent Fdh can be activated and in particular overexpressed in the host microorganism. In the presence of this newly introduced formate dehydrogenase pathway, one mole of NADH will is formed when one mole of formate is converted to carbon dioxide. In certain embodiments, in the native microorganism a formate dehydrogenase converts formate to CO₂ and H₂ with no cofactor involvement.

Furthermore any of the genes encoding the foregoing enzymes (or any others mentioned herein (or any of the regulatory elements that control or modulate expression thereof) may be subject to directed evolution using methods known to those of skill in the art. Such action allows those of skill in the art to optimize the enzymes for expression and activity in yeast.

In addition, pyruvate decarboxylase, acetyl-CoA synthetase, and acetaldehyde dehydrogenase genes from other fungal and bacterial species can be expressed for the modulation of this pathway. A variety of organisms could serve as sources for these enzymes, including, but not limited to, *Saccharomyces* sp., including *S. cerevisiae* mutants and *S*. *uvarum, Kluyveromyces,* including *K. thermotolerans, K. lactis,* and *K. marxianus, Pichia, Hansenula,* including *H. polymorpha, Candidia, Trichosporon, Yamadazyma,* including *Y*. *stipitis*, *Torulaspora pretoriensis, Schizosaccharomyce pombe, Cryptococcus* sp., *Aspergillus* sp., *Neurospora* sp. or *Ustilago* sp. Examples of useful pyruvate decarboxylase are those from *Saccharomyces bayanus* (1PYD), *Candida glabrata, K lactis* (KIPDC1), or *Aspergillus nidulans* (PdcA), and acetyl-CoA sythetase from *Candida albicans, Neurospora crassa, A. nidulans,* or *K. lactis* (ACS1), and acetaldehyde dehydrogenase from *Aspergillus niger* (ALDDH), *C. albicans, Cryptococcus neoformans* (alddh). Sources of prokaryotic enzymes that are useful include, but are not limited to, *E*. *coli, Z. mobilis, Bacillus* sp., *Clostridium* sp., *Pseudomonas* sp., *Lactococcus* sp., *Enterobacter* sp. and *Salmonella* sp. Further enhancement of this pathway can be obtained through engineering of these enzymes for enhanced activity by site-directed mutagenesis and other evolution methods (which include techniques known to those of skill in the art).

Prokaryotes such as, but not limited to, *E*. *coli, Z. mobilis, Staphylococcus aureus, Bacillus* sp., *Clostridium* sp., *Corynebacterium* sp., *Pseudomonas* sp., *Lactococcus* sp., *Enterobactersp.,* and *Salmonella* sp., can serve as sources for this enzyme complex. For example, pyruvate dehydrogenase complexes from *E. coli* (aceE, *aceF,* and *IpdA*), *Z. mobilis* (pdhAalpha, pdhAbeta, pdhB, and lpd), *S. aureus* (pdhA, pdhB, pdhC, and pdhC), *Bacillus subtilis, Corynebacterium glutamicum,* and *Pseudomonas aeruginosa,* can be used for this purpose.

Methods to grow and handle yeast are well known in the art. Methods to overexpress, express at various lower levels, repress expression of, and delete genes in yeast cells are well known in the art and any such method is contemplated for use to construct the yeast strains of the present.

Any method can be used to introduce an exogenous nucleic acid molecule into yeast and many such methods are well known to those skilled in the art. For example, transformation, electroporation, conjugation, and fusion of protoplasts are common methods for introducing nucleic acid into yeast cells. See, e.g., Ito et al., J. Bacterol. 153:163-168 (1983); Durrens et al., Curr. Genet. 18:7-12 (1990); and Becker and Guarente, Methods in Enzymology 194:182-187 (1991).

In an embodiment, the integration of a gene of interest into a DNA fragment or target gene occurs according to the principle of homologous recombination. According to this embodiment, an integration cassette containing a module comprising at least one yeast marker gene, with or without the gene to be integrated (internal module), is flanked on either side by DNA fragments homologous to those of the ends of the targeted integration site (recombinogenic sequences). After transforming the yeast with the cassette by appropriate methods, a homologous recombination between the recombinogenic sequences may result in the internal module replacing the chromosomal region in between the two sites of the genome corresponding to the recombinogenic sequences of the integration cassette.

In an embodiment, for gene deletion, the integration cassette may include an appropriate yeast selection marker flanked by the recombinogenic sequences. In an embodiment, for integration of a heterologous gene into the yeast chromosome, the integration cassette includes the heterologous gene under the control of an appropriate promoter and terminator together with the selectable marker flanked by recombinogenic sequences. In an embodiment, the heterologous gene comprises an appropriate native gene desired to increase the copy number of a native gene(s). The selectable marker gene can be any marker gene used in yeast, including, but not limited to, *URA3* gene from *S*. *cerevisiae* or a homologous gene; or hygromycin resistance gene for auxotrophy complementation or antibiotic resistance-based selection of the transformed cells, respectively. The recombinogenic sequences can be chosen at will, depending on the desired integration site suitable for the desired application.

Additionally, in an embodiment, certain introduced marker genes are removed from the genome using techniques well known to those skilled in the art. For example, *URA3* marker loss can be obtained by plating *URA3* containing cells in FOA (5-fluoro-orotic acid) containing medium and selecting for FOA resistant colonies (Boeke, J. et al, 1984, Mol. Gen. Genet, 197, 345-47).

The exogenous nucleic acid molecule contained within a yeast cell of the disclosure can be maintained within that cell in any form. For example, exogenous nucleic acid molecules can be integrated into the genome of the cell or maintained in an episomal state that can stably be passed on ("inherited") to daughter cells. Such extra-chromosomal genetic elements (such as plasmids, etc.) can additionally contain selection markers that ensure the presence of such genetic elements in daughter cells. Moreover, the yeast cells can be stably or transiently transformed. In addition, the yeast cells described herein can contain a single copy, or multiple copies, of a particular exogenous nucleic acid molecule as described above.

Methods for expressing a polypeptide from an exogenous nucleic acid molecule are well known to those skilled in the art. Such methods include, without limitation, constructing a nucleic acid such that a regulatory element promotes the expression of a nucleic acid sequence that encodes the desired polypeptide. Typically, regulatory elements are DNA sequences that regulate the expression of other DNA sequences at the level of transcription. Thus, regulatory elements include, without limitation, promoters, enhancers, and the like. For example, the exogenous genes can be under the control of an inducible promoter or a constitutive promoter. Moreover, methods for expressing a polypeptide from an exogenous nucleic acid molecule in yeast are well known to those skilled in the art. For example, nucleic acid constructs that are capable of expressing exogenous polypeptides within *Kluyveromyces* (*see,* e.g., U.S. Pat. Nos. 4,859,596 and 4,943,529) and *Saccharomyces* (*see*, e.g., Gelissen et al., Gene 190(1):87-97 (1997)) are well known. In another embodiment, heterologous control elements can be used to activate or repress expression of endogenous genes. Additionally, when expression is to be repressed or eliminated, the gene for the relevant enzyme, protein or RNA can be eliminated by known deletion techniques.

As described herein, yeast within the scope of the disclosure can be identified by selection techniques specific to the particular enzyme being expressed, overexpressed or repressed. Methods of identifying the strains with the desired phenotype are well known to those skilled in the art. Such methods include, without limitation, PCR and nucleic acid hybridization techniques such as Northern and Southern analysis, altered growth capabilities on a particular substrate or in the presence of a particular substrate, a chemical compound, a selection agent and the like. In some cases, immunohistochemistry and biochemical techniques can be used to determine if a cell contains a particular nucleic acid by detecting the expression of the encoded polypeptide. For example, an antibody having specificity for an encoded enzyme can be used to determine whether or not a particular yeast cell contains that encoded enzyme. Further, biochemical techniques can be used to determine if a cell contains a particular nucleic acid molecule encoding an enzymatic polypeptide by detecting a product produced as a result of the expression of the enzymatic polypeptide. For example, transforming a cell with a vector encoding acetyl-CoA synthetase and detecting increased cytosolic acetyl-CoA concentrations indicates the vector is both present and that the gene product is active. Methods for detecting specific enzymatic activities or the presence of particular products are well known to those skilled in the art. For example, the presence of acetyl-CoA can be determined as described by Dalluge et al., Anal. Bioanal. Chem. 374(5):835-840 (2002).

Yeast cells of the present invention have reduced enzymatic activity such as reduced alcohol dehydrogenase activity. The term "reduced" as used herein with respect to a cell and a particular enzymatic activity refers to a lower level of enzymatic activity than that measured in a comparable yeast cell of the same species. Thus yeast cells lacking alcohol dehydrogenase activity is considered to have reduced alcohol dehydrogenase activity since most, if not all, comparable yeast strains have at least some alcohol dehydrogenase activity. Such reduced enzymatic activities can be the result of lower enzyme concentration, lower specific activity of an enzyme, or a combination thereof. Many different methods can be used to make yeast having reduced enzymatic activity. For example, a yeast cell can be engineered to have a disrupted enzyme-encoding locus using common mutagenesis or knock-out technology. See, e.g., Methods in Yeast Genetics (1997 edition), Adams, Gottschling, Kaiser, and Stems, Cold Spring Harbor Press (1998).

Alternatively, antisense technology can be used to reduce enzymatic activity. For example, yeast can be engineered to contain a cDNA that encodes an antisense molecule that prevents an enzyme from being made. The term "antisense molecule" as used herein encompasses any nucleic acid molecule that contains sequences that correspond to the coding strand of an endogenous polypeptide. An antisense molecule also can have flanking sequences (e.g., regulatory sequences). Thus antisense molecules can be ribozymes or antisense oligonucleotides. A ribozyme can have any general structure including, without limitation, hairpin, hammerhead, or axhead structures, provided the molecule cleaves RNA.

Yeast having a reduced enzymatic activity can be identified using any method. For example, yeast having reduced alcohol dehydrogenase activity can be easily identified using common methods, for example, by measuring ethanol formation via gas chromatography.

In one embodiment, n-butanol can be produced from one of the metabolically-engineered strains of the present disclosure using a two-step process. Because high levels of butanol (e.g., 1.5% in the media and this generally varies by yeast and strain) can be toxic to the cells, one strategy to obtain large quantities of n-butanol is to grow a strain capable of producing n-butanol under conditions in which no butanol, or only an insignificant, non-toxic amount of butanol, is produced. This step allows accumulation of a large quantity of viable cells, i.e., a significant amount of biomass, which can then be shifted to growth conditions under which n-butanol is produced. Such a strategy allows a large amount of n-butanol to be produced before toxicity problems become significant and slow cell growth. For example, cells can be grown under aerobic conditions (in which n-butanol production is suppressed or absent) then shifted to anaerobic or microaerobic conditions to produce n-butanol (e.g., by activation of the appropriate metabolic pathways that have been engineered into the strain in accordance with the present invention). Alternatively, expression of the relevant enzymes can be under inducible control, e.g., thermal sensitive promoters or other thermal sensitive step (such as the thermostability of the enzyme itself), so the first step takes place with the relevant pathway(s) or enzymes turned off (i.e., inactive), induction takes place (e.g., temperature shift), and n-butanol is produced. Methods for making genes subject to inducible control are well known. Thermostable enzymes are known or can be selected by methods know in the art. As in other processes of the disclosure, once n-butanol is produced, it can be recovered in accordance with an embodiment.

Processes for recovering n-butanol from microorganisms, including yeast are disclosed in U.S. Provisional Application Serial No. 11/949,724, filed December 3, 2007.

### EXAMPLES

Table 1 lists a set of genes that are described in Examples 1-38. The relevant primers (forward and reverse) that may be used to amplify each gene, as well as the sequence of each primer, are given. Genes are listed according to the nomenclature conventions appropriate for each species; certain genes as listed are preceded by two letters, representing the first letter of the genus and species of origin for a given gene. For certain gene names, the suffix "-co" is attached to indicate that a codon-optimized, synthethic gene was constructed using preferred codon usage for either the bacterium *E*. *coli,* or the yeast *S*. *cerevisiae,* as indicated in the text.

**Table 1.**

| **Gene** | **Gene SEQ ID NO:** | **primer name** | **SEQ ID NO:** | **primer sequence** |
|---|---|---|---|---|
| *Cb-hbd* | 155 | Gevo-311 | 42 | GAGGTTGTCGACATGAAAAAGATTTTGTACTTGGAG |
| | | Gevo-175 | 43 | AATTGGATCCTTATTTAGAATAATCATAGAATCCT |
| *Cb-crt* | 156 | Gevo-312 | 44 | GTTCTTGTCGACATGGAATTAAAAAATGTTATTCTTG |
| | | Gevo-171 | 45 | AATTGGATCCTTATTTATTTTGAAAATTCTTTTCTGC |
| *Cb-bcd* | 157 | Gevo-313 | 46 | CAAGAGGTCGACATGAATTTCCAATTAACTAGAGAAC |
| | | Gevo-314 | 47 | GCGTCCGGATCCCTATCTTAAAATGCTTCCTGCG |
| *Cb-etfA* | 158 | Gevo-315 | 48 | CGGAAAGTCGACATGAATATAGCAGATTACAAAGGC |
| | | Gevo-173 | 49 | AATTGGATCCTTATTCAGCGCTCTTTATTTCTTTA |
| *Cb-etfB* | 159 | Gevo-316 | 50 | CAAAATGTCGACATGAATATAGTAGTTTGTGTAAAAC |
| | | Gevo-317 | 51 | TAATTTGGATCCTTAGATGTAGTTTTTCTTTTAAT |
| *Cb-adhA* | 160 | Gevo-319 | 52 | GAACCAGTCGACATGGCACGTTTTACTTTACCAAG |
| | | Gevo-177 | 53 | AATTGGATCCTTACAAATTAACTTTAGTTCCATAG |
| *Cb-aldh* | 161 | Gevo-318 | 54 | TCCATAGTCGACATGAATAAAGACACACTAATACCT |
| | | Gevo-249 | 55 | |
| *Ca-thl* | 162 | Gevo-308 | 56 | GATCGAGTCGACATGAAAGAAGTTGTAATAGCTAG |
| | | Gevo-309 | 57 | GTTATAGGATCCCTAGCACTTTTCTAGCAATATTG |
| *Ca-hbd* | 163 | Gevo-281 | 58 | GTGGATGTCGACATGAAAAAGGTATGTGTTATAGGTG |
| | | Gevo-161 | 59 | AATTGGATCCTTATTTTGAATAATCGTAGAAACCT |
| *Ca-crt* | 164 | Gevo-282 | 60 | TCCTACGTCGACATGGAACTAAACAATGTCATCCT |
| | | Gevo-283 | 61 | TAACTTGGATCCCTATCTAIIIIIGAAGCCTTCAAT |
| *Ca-bcd* | 165 | Gevo-284 | 62 | CAAGAGGTCGACATGGATTTTAATTTAACAAGAGAAC |
| | | Gevo-285 | 63 | CAATAAGGATCCTTATCTAAAAATTTTTCCTGAAATAAC |
| *Ca-etfA* | 166 | Gevo-286 | 64 | CGGGAAGTCGACATGAATAAAGCAGATTACAAGGGC |
| | | Gevo-287 | 65 | GTTCAAGGATCCTTAATTATTAGCAGCTTTAACTTG |
| *Ca-etfB* | 167 | Gevo-288 | 66 | CAAAATTGTCGACATGAATATAGTTGTTTGTTTAAAAC |
| | | Gevo-289 | 67 | GTTTTAGGATCCTTAAATATAGTGTTCTTCTTTTAATTTT |
| | | G | G | G |
| *Ca-adhE2* | 168 | Gevo-292 | 68 | CAAGAAGTCGACATGAAAGTTACAAATCAAAAAGAAC |
| | | Gevo-293 | 69 | |
| *Ca-aad* | 169 | Gevo-290 | 70 | AGGAAAGTCGACATGAAAGTCACAACAGTAAAGGA |
| | | Gevo-291 | 71 | |
| *Ca-bdhA* | 170 | Gevo-294 | 72 | CATAACGTCGACATGCTAAGTTTTGATTATTCAATAC |
| | | Gevo-247 | 73 | AATTGGATCCTTAATAAGATTTTTTAAATATCTCAA |
| *Ca-bdhB* | 171 | Gevo-295 | 74 | CATAACGTCGACATGGTTGATTTCGAATATTCAATAC |
| | | Gevo-159 | 75 | AATTGGATCCTTACACAGATTTTTTGAATATTTGTA |
| *Ca-thl-co* | 1 | Gevo-310 | 76 | GATCGAGAATTCATGAAAGAAGTTGTAATAGCTAG |
| | | Gevo-309 | 77 | GTTATAGGATCCCTAGCACTTTTCTAGCAATATTG |
| *Ca-hbd-co* | 2 | Gevo-296 | 78 | CGGATAGTCGACATGAAAAAGGTATGTGTTATAGGC |
| | | Gevo-297 | 79 | TCCCAAGGATCCTTATTTTGAATAATCGTAGAAACCCT |
| *Ca-crt-co* | 3 | Gevo-282 | 80 | TCCTACGTCGACATGGAACTAAACAATGTCATCCT |
| | | Gevo-283 | 81 | TAACTTGGATCCCTATCTATTTTTGAAGCCTTCAAT |
| *Ca-bcd-co* | 4 | Gevo-284 | 82 | CAAGAGGTCGACATGGATTTTAATTTAACAAGAGAAC |
| | | Gevo-298 | 83 | GTAAAGGGATCCTTAACTAAAATTTTTCCTGAAATG |
| *Ca-eftA-co* | 5 | Gevo-286 | 84 | CGGGAAGTCGACATGAATAAAGCAGATTACAAGGGC |
| | | Gevo-299 | 85 | GTTCAAGGATCCTTAATTATTAGCAGCTTTAACCTG |
| *Ca-eftB-co* | 6 | Gevo-288 | 86 | CAAAATTGTCGACATGAATATAGTTGTTTGTTTAAAAC |
| | | Gevo-300 | 87 | GACTTTGGATCCTTAAATATAGTGTTCTTCTTTCAG |
| *Ca-adhE2-co* | 7 | Gevo-292 | 88 | CAAGAAGTCGACATGAAAGTTACAAATCAAAAAGAAC |
| | | Gevo-301 | 89 | |
| *Me-bcd-co* | 8 | Gevo-302 | 90 | CTTATAGTCGACATGGATTTTAACTTAACAGATATTC |
| | | Gevo-303 | 91 | CCGCCAGGATCCTTAACGTAACAGAGCACCGCCGGT |
| *Me-eftA-co* | 9 | Gevo-304 | 92 | CGGAAAGTCGACATGGATTTAGCAGAATACAAAGGC |
| | | Gevo-305 | 93 | CTTTGTGGATCCTTATGCAATGCCTTTCTGTTTC |
| *Me-eftB-co* | 10 | Gevo-306 | 94 | CAAACTGAATTCATGGAAATATTGGTATGTGTCAAAC |
| | | Gevo-307 | 95 | ACCAACGGATCCTTAAATGATTTTCTGGGCAACCA |
| *ERG10* | 154 | Gevo-273 | 96 | GTTACAGTCGACATGTCTCAGAACGTTTACATTG |
| | | Gevo-274 | 97 | GATAACGGATCCTCATATCTTTTCAATGACAATAG |
| *IpdA* | 20 | Gevo-610 | 119 | ttttGTCGACACTAGTatgagtactgaaatcaaaactcaggtcgtg |
| | | Gevo-611 | 120 | ttttCTCGAGttacttcttcttcgctttcgggttcgg |
| *aceE* | 21 | Gevo-606 | 116 | ttttGTCGACACTAGTatgtcagaacgtttcccaaatgacgtgg |
| | | Gevo-607 | 117 | ttttCTCGAGttacgccagacgcgggttaactttatctg |
| *aceF* | 22 | Gevo-653 | 136 | ttttGTCGACACTAGTatggctatcgaaatcaaagtaccggacatcggg |
| | | Gevo-609 | 118 | ttttCTCGAGttacatcaccagacggcgaatgtcagacag |
| *PDA1* | 23 | Gevo-660 | 143 | ttttCTCGAGactagtATGgcaactttaaaaacaactgataagaagg |
| | | Gevo-661 | 144 | ttttagatctTTAATCCCTAGAGGCAAAACCTTGC |
| *PDB1* | 24 | Gevo-662 | 145 | ttttCTCGAGactagtATGgcggaagaattggaccgtgatgatg |
| | | Gevo-663 | 146 | tttGGATCCTTATTCAATTGACAAGACTTCTTTGACAG |
| *PDX1* | 25 | Gevo-664 | 147 | TtttCTCGAGactagtATGttacttgctgtaaagacattttcaatgcc |
| | | Gevo-665 | 148 | ttttggatccTCAAAATGATTCTAACTCCCTTACGTAATC |
| *LAT1* | 26 | Gevo-656 | 139 | |
| | | Gevo-657 | 140 | ttttGGATCCTCACAATAGCATTTCCAAAGGATTTTCAAT |
| *LPD1* | 27 | Gevo-658 | 141 | |
| | | Gevo-659 | 142 | ttttGGATCCTCAACAATGAATAGCTTTATCATAGG |
| *PDC1* | 28 | Gevo-639 | 129 | ttttctcgagactagtATGTCTGAAATTACTTTGGG |
| | | Gevo-640 | 130 | ttttgpatccTTATTGCTTAGCGTTGGTAGCAGCAG |
| *CUP1 prom* | 178 | Gevo-637 | 127 | ttttGAGCTCgccgatcccattaccgacatttpgg |
| | | Gevo-638 | 128 | |
| *pflA* | 36 | PflA_forw | 98 | cattgaattcatgtcagttattggtcgcattcac |
| | | PflA_Rev | 99 | cattgtcgacttagaacattaccttatgaccgtactg |
| *pflB* | 37 | PflB_forw | 100 | cattgaattcatgtccgagcttaatgaaaagttagcc |
| | | PflB_Rev | 101 | cattgtcgacttacatagattgagtqaaggtacgag |
| *Cb-FDH1* | 38 | fdh1_forw | 102 | cattgaattcatgaagatcgttttagtcttatatggtgc |
| | | fdh1_rev | 103 | cattgtcgacttatttcttatcgtgtttaccgtaagc |
| *KIALD6* | 39 | KIALD6_rig ht3 | 104 | gttaggatccttaatccaacttgatcctgacggccttg |
| | | KIALD6_Lef t5 | 105 | ccaagtcgacatgtcctctacaattgctgagaaattgaacctc |
| *KIACS1* | 40 | KIACS1_Ri ght3 | 106 | gttagcggccgcttataatttcacggaatcgatcaagtgc |
| | | KIACS1_Lef t5 | 107 | ccaagctagcatgtctcctgctgttgataccgcttcc |
| *KIACS2* | 41 | KIACS2_rig ht3 | 108 | ggttggatccttatttcttctgctgactgaaaaattgattttctactgc |
| | | KIACS2_Lef t5 | 109 | ccaagaattcatgtcgtcggataaattgcataagg |
| *ACS1* | 30 | Gevo-479 | 112 | catgcc*gtcga*catgtcgccctctgccgtac |
| | | Gevo-480 | 113 | gattaagcggccgcttacaacttgaccgaatcaattag |
| *ACS2* | 31 | Gevo-483 | 114 | gatgaa*gtcgac*atgacaatcaaggaacataaagtag |
| | | Gevo-484 | 115 | gttaaa*ggatcc*ttatttctttttttgagagaaaaattg |
| *ALD6* | 29 | Gevo-643 | 133 | ccaa*gtcga*catgactaagctacactttgacac |
| | | Gevo-644 | 134 | gtcggtaagagtgttgctgtggactcg |
| *Ca-ter* | 179 | Gevo-345 | 183 | atgtttgtcgacatgatagtaaaagcaaagtttgta |
| | | Gevo-346 | 184 | cttaatgcggccgcttaaggttctaattttcttaataattc |
| *Ah-ter* | 180 | Gevo-343 | 185 | Gcttgagtcgacatgatcattaaaccgaaagttcg |
| | | Gevo-344 | 186 | atttaaggatcctcacagttcqacaacatcaaattta |
| *Eg-ter* | 181 | Gevo-347 | 187 | catcacgtcgacatggccatgttcaccactac |
| | | Gevo-348 | 188 | ctcgcgggatccttactgctgagctgcgctc |
| *Sc-ccr* | 182 | Gevo-341 | 189 | gtcttagtcgacatgaccgtgaaagacattctg |
| | | Gevo-342 | 190 | attggcggatcctcacacattacggaaacggtta |

Table 2 lists a set of plasmid constructs and their relevant features, as described in the Examples. Included in the table are the relevant plasmid name (pGV); the prototrophic marker present, useful for selection and maintenance of the plasmid in an appropriate auxotrophic strain; a promoter sequence (from the given *S*. *cerevisiae* gene region); the gene under control of the aforementioned promoter; additional promoter + gene combinations, if present.

**Table 2: Summary of relevant features of plasmids in Examples.**

| **Name** | **Prototrophic marker** | **Promoter 1** | **GENE 1** | **Promoter 2** | **GENE 2** |
|---|---|---|---|---|---|
| pGV1099 | *HIS3* | TEF1 | (AU1 tag) | | |
| pGV1100 | *TRP1* | TEF1 | (HA tag) | | |
| pGV1101 | *LEU2* | TEF1 | (AU1 tag) | | |
| pGV1102 | *URA3* | TEF1 | (HA tag) | | |
| pGV1103 | *HIS3* | TDH3 | (myc tag) | | |
| pGV1104 | *TRP1* | TDH3 | (myc tag) | | |
| pGV1105 | *LEU2* | TDH3 | (myc tag) | | |
| pGV1106 | *URA3* | TDH3 | (myc tag) | | |
| pGV1208 | *TRP1* | TEF1 | Ca-hbd-co | | |
| pGV1209 | *LEU2* | TEF1 | Ca-crt-co | | |
| pGV1213 | *URA3* | TEF1 | Ca-adhE2-co | | |
| pGV1214 | *HIS3* | TDH3 | Me-bcd-co | | |
| pGV1217 | *TRP1* | TEF1 | Ca-hbd-co | TDH3 | Ca-eftA-co |
| pGV1218 | *LEU2* | TEF1 | Ca-crt-co | TDH3 | Ca-eftB-co |
| pGV1219 | *HIS3* | TEF1 | ScERG10 | TDH3 | Me-bcd-co |
| pGV1220 | *HIS3* | TEF1 | Ca-thl-co | TDH3 | Ca-bcd-co |
| pGV1221 | *TRP1* | TEF1 | Ca-hbd-co | TDH3 | Me-eftA-co |
| pGV1222 | *LEU2* | TEF1 | Ca-crt-co | TDH3 | Me-eftB-co |
| pGV1223 | *HIS3* | TEF1 | ScERG10 | TDH3 | Ca-bcd-co |
| pGV1224 | *HIS3* | TEF1 | Ca-thl-co | TDH3 | Me-bcd-co |
| pGV1225 | *HIS3* | TEF1 | Ca-thl-co | TDH3 | Ca-ter |
| pGV1226 | *HIS3* | TEF1 | Ca-thl-co | TDH3 | Ah-ter |
| pGV1227 | *HIS3* | TEF1 | Ca-thl-co | TDH3 | Eg-ter |
| pGV1228 | *HIS3* | TEF1 | Ca-thl-co | TDH3 | Sc-ccr |
| pGV1262 | *LEU2* | TEF1 | ScACS1 | | |
| pGV1263 | *URA3* | TEF1 | ScACS2 | | |
| pGV1319 | *URA3* | TDH3 | Ca-AdhE2_co | TEF1 | *ACS1* |
| pGV1320 | *URA3* | TDH3 | Ca-AdhE2_co | TEF1 | *ACS2* |
| pGV1321 | *LEU2* | TDH3 | *ALD6* | | |
| pGV1326 | *LEU2* | TEF1 | *ALD6* | | |
| pGV1334 | *HIS3* | TDH3 | *IpdA* | | |
| pGV1339 | *LEU2* | TEF1 | Ca_Crt_co | TDH3 | *ALD6* |
| pGV1379 | *HIS3* | TDH3 | *aceE* | | |
| pGV1380 | *HIS3* | TDH3 | *aceF* | | |
| pGV1381 | *HIS3* | TDH3 | *LAT1* | | |
| pGV1383 | *HIS3* | TDH3 | *PDA1* | | |
| pGV1384 | *HIS3* | TDH3 | *PDB1* | | |
| pGV1385 | *HIS3* | TDH3 | *PDX1* | | |
| pGV1388 | *URA3* | CUP1 | n/a | | |
| pGV1389 | *URA3* | TDH3 | *PDC1* | | |
| pGV1399 | *LEU2* | TEF1 | Ca-hbd-co | TDH3 | *ALD6* |
| pGV1414 | *URA3* | MET3 | n/a | | |
| pGV1428 | *HIS3* | TDH3 | n/a | | |
| pGV1429 | *TRP1* | TDH3 | n/a | | |
| pGV1430 | *LEU2* | TDH3 | n/a | | |
| pGV1483 | *URA3* | MEt3 | n/a | | |
| pGV1603 | *TRP1* | TDH3 | *aceE* | | |
| pGV1604 | *LEU2* | TDH3 | *aceF* | | |
| pGV1605 | *URA3* | TEF1 | adhE2 | TDH3 | *PDC1* |
| 1102Fdh1 | *URA3* | TEF1 | *Cb-FDH1* | | |
| 1103PflA | *HIS3* | TDH3 | *pflA* | | |
| 1104PflB | *TRP1* | TDH3 | *pflB* | | |
| 1208_PflA | *TRP1* | TEF1 | Ca_hbd_co | TDH3 | *pflA* |
| 1208KI | *HIS3* | TEF1 | Ca_hbd_co | | |
| 1208KIALD6 | *HIS3* | TEF1 | Ca_hbd_co | TDH3 | *KIALD6* |
| 1208KIPflA | *HIS3* | TEF1 | Ca_hbd_co | TDH3 | *pflA* |
| 1208KIPflA | *TRP1* | TEF1 | Ca_Crt_co | TDH3 | *pflB* |
| 1208-IpdA | *TRP1* | TEF1 | thl | TDH3 | *-IpdA* |
| 1209_PflB | *LEU2* | TEF1 | Ca_Crt_co | TDH3 | *pflB* |
| 1209-aceE | *LEU2* | TEF1 | crt | TDH3 | *aceE* |
| 1209KI | *TRP1* | TEF1 | Ca_Crt_co | | |
| 1209klACS1 | *LEU2* | TEF1 | Ca_Crt_co | TDH3 | *KIACS1* |
| 1209klACS2 | *LEU2* | TEF1 | Ca_Crt_co | TDH3 | *KIACS2* |
| 1213_Fdh1 | *URA3* | TDH3 | Ca_AdhE2_co | TEF1 | *Cb-FDH1* |
| 1213-aceF | *URA3* | TEF1 | adhE2 | TDH3 | *aceF* |
| 1213KI | *URA3* | TDH3 | Ca_AdhE2_co | | |
| 1213KIPflA | *LEU2* | TEF1 | Ca_thl_co | TDH3 | *Cb-FDH1* |
| 1227KI | *LEU2* | TEF1 | Ca_thl_co | TDH3 | Eg-TER-co |
| 1388-PDC1 | *URA3* | CUP1 | *PDC1* | | |
| 1428-PflA | *HIS3* | TDH3 | *pflA* | | |
| 1428ALD6 | *HIS3* | TDH3 | *KIALD6* | | |
| 1428-IpdA | *HIS3* | TDH3 | *IpdA* | | |
| 1429_PflB | *TRP1* | TDH3 | *pflB* | | |
| 1429-aceE | *TRP1* | TDH3 | *aceE* | | |
| 1429ACS1 | *TRP1* | TDH3 | *KIACS1* | | |
| 1430_Fdh1 | *LEU2* | TDH3 | *Cb-FDH1* | | |
| 1430-aceF | *LEU2* | TDH3 | *aceF* | | |
| 1431ACS2 | *URA3* | TDH3 | *KIACS2* | | |
| pGV1103-lpd1 | *HIS3* | TDH3 | LPD1 | | |

Table 3 describes butanol produced in a yeast, *S. cerevisiae* (strain W303a), carrying various plasmids, and thereby expressing a set of introduced genes, which are as listed.

**Table 3: Butanol production bv Saccharomyces cerevisiae transformants.**

| **Isolate Name** | **Plasmid Combination** | **Introduced Genes** | **Butanol Amount 72 h p.i (µM)** |
|---|---|---|---|
| Gevo 1094; Gevo1095 | pGV1208; pGV1209; pGV1225; pGV1213 | *Ca-hbd-co; Ca-Crt-co; Ca-thl-co+Ca-ter; Ca-adhE2-co* | 129; 145 |
| Gevo 1096; Gevo1097 | pGV1208; pGV1209; pGV1226; pGV1213 | *Ca-hbd; Ca-Crt; Ca-thl-co+Ah-ter; Ca-adhE2-co* | 207; 216 |
| Gevo 1098; Gevo1099 | pGV1208; pGV1209; pGV1227; pGV1213 | *Ca-hbd; Ca-Crt; Ca-thl-co+Eg-ter; Ca-adhE2-co* | 251; 313 |
| Gevo 1100, Gevo1101 | pGV1208; pGV1209; pGV1228; pGV1213 | *Ca-hbd; Ca-Crt; Ca-thl-co+Sc-ter, Ca-adhE2-co* | 109; 109 |
| Gevo 1102, Gevo1103 | pGV1217; pGV1218; pGV1220; pGV1213 | *Ca-hbd-co+ Ca-etfa-co; Ca-Crt-co+Ca-etfb-co; Ca-thl-co+Ca-bcd-co; Ca-adhE2-co* | 317; 332 |
| Gevo 1104, Gevo1105 | pGV1217; pGV1218; pGV1223; pGV1213 | *Ca-hbd-co+ Ca-etfa-co; Ca-Crt-co+Ca-etfb-co; ERG10+Ca-bcd-co; Ca-adhE2-co* | 172; 269 |
| Gevo 1106, Gevo1107 | pGV1221; pGV1222; pGV1224; pGV1213 | *Ca-hbd-co+ Ca-etfa-co; Ca-Crt-co+Ca-etfb-co; Ca-thl-co+Me-bcd-co; Ca-adhE2-co* | 125; 115 |
| Gevo 1108, Gevo1109 | pGV1221; pGV1222; pGV1219; pGV1213 | *Ca-hbd-co+ Ca-etfa-co; Ca-Crt-co+Ca-etfb-co; ERG10+Me-bcd-co; Ca-adhE2-co* | 101; 124 |
| Gevo 1110, Gevo1111 | pGV1099; pGV1100; pGV1101; pGV1106 | N/A | 0; 12 |

All gene cloning and combination procedures were initially carried out in *E. coli* using established methods (Miller, J.H., 1992, Sambrook, J. et. al, 2001).

A set of vectors useful for expression in a yeast, *S*. *cerevisiae,* has been described previously (Mumberg, D., et al. (1995) Gene 156:119-122; Sikorski & Heiter (1989) Genetics 122:19-27). In particular, these publications describe a set of selectable markers (*HIS3, LEU2, TRP1, URA3*) and *S. cerevisiae* replication origins that are also used in many of the vectors listed in Table 2.

### Example 1. Plasmid construction for expression of butanol pathway genes in the yeast, S. cerevisiae.

The *S*. *cerevisiae* thiolase gene, *ERG10,* was cloned by PCR from genomic DNA from the *S. cerevisiae* strain W303a, using primers which introduced a *Sal*I site immediately upstream of the start codon and a *BamH*I site immediately after the stop codon. This PCR product was digested with *Sal*I and *BamH*I and cloned into the same sites of pUC19 (Yanisch-Perron, C., Vieira, J., 1985, Gene, 33, 103-19) to generate pGV1120.

The plasmids pGV1031, pGV1037, pGV1094, and pGV1095 were used as templates for PCR amplification of the *C. acetobutylicum* genes *(Ca-) Ca-thl, Ca-hbd, Ca-crt,* and *Ca-bdhB,* respectively. pGV1090 was used as template for PCR amplification of *Ca-bcd, Ca-etfA,* and *Ca-etfB.* Genomic DNA of *Clostridium* ATCC 824 was used to amplify *Ca-bdhA.* Amplified fragments were digested with *Sal*I and *BamH*I and cloned into the same sites of pUC19. This scheme generated plasmids, pGV1121, pGV1122, pGV1123, pGV1124, pGV1125, pGV1126, pGV1127, pGV1128, which contain the genes, *Ca-thl, Ca-hbd, Ca-crt, Ca-bcd, Ca-etfA, Ca-etfB, Ca-bdhA,* and Ca-*bdhB,* respectively.

The *Clostridium beijerinckii (Cb-)* genes, *Cb-hbd, Cb-crt, Cb-bcd, Cb-etfA, Cb-etfB*, *Cb-aldh,* and *Cb-adhA* were amplified by PCR using primers designed to introduce a *Sal*I site just upstream of the start and a *BamH*I site just downstream of the stop codon. The plasmids pGV1050, pGV1049, pGV1096 and pGV1091 were used as templates for PCR amplification of *Cb-hbd, Cb-crt, Cb-aldh,* and *Cb-adhA,* respectively. Genomic DNA of *Clostridium beijerinckii* ATCC 51743 was used as template for *Cb-bcd, Cb-etfA,* and *Cb-etfB.* The PCR amplified fragments were digested with *Sal*I and *BamH*I and cloned into the same sites of pUC19. This procedure generated plasmids pGV1129, pGV1130, pGV1131, pGV1132, pGV1133, pGV1134, and pGV1135, which contain the genes, *Cb-hbd, Cb-crt, Cb-bcd, Cb-etfA, Cb-etfB, Cb-aldh,* and *Cb-adhA,* respectively.

The C. acetobutylicum and Meghasphaera elsdenii (Me-) genes that were codon optimized (-co) for expression in *E*. *coli* were also cloned. These genes include Ca-thl-co, Ca-hbd-co, Ca-crt-co, Ca-bcd-co, Ca-etfA-co, Ca-etfB-co, Ca-adhE2-co, Me-bcd-co, Me-etfA-co, and Me-etfB-co. These genes, except for Ca-thl-co and Me-etfB-co were amplified using primers designed to introduce a SalI site just upstream of the start codon and a BamHI site just downstream of the stop codon. In the case of Ca-thl-co and Me-etfB-co, primers were designed to introduce an EcoRI site just upstream of the start codon and a BamHI site just downstream of the stop codon. The resulting PCR products were digested using the appropriate restriction enzymes (SalI and BamHI or EcoRI and BamHI) and cloned into the same sites of pUC19 to generate plasmids pGV1197, pGV1198, pGV1199, pGV1200, pGV1201, pGV1202, pGV1203, pGV1205, pGV1206, which contain the genes, Ca-thl-co, Ca-hbd-co, Ca-crt-co, Ca-bcd-co, Ca-etfA-co, Ca-etfB-co, Ca-adhE2-co, Me-etfA-co, and Me-etfB-co, respectively. Me-bcd-co gene was directly cloned into pGV1103 as a SalI-BamHI fragment to generate pGV1214.

The above genes were cloned into high copy yeast expression vectors, pGV1099, pGV1100, pGV1101, pGV1102, pGV1103, pGV1104, pGV1105 and pGV1106. The properties of the vectors used for gene cloning and resulting plasmid constructs are described in Table 2.

The thiolase genes, *ERG10* and *Ca-thl* were released from pGV1120 and pGV1121 using *Sal*I and *Bam*HI and cloned into pGV1099 (carrying a *HIS3* marker) to generate pGV1138 and pGV1139, respectively. The codon-optimized thiolase gene, *Ca-thl-co* was removed from pGV1197 and cloned into pGV1099 using *Eco*RI and *Bam*HI to generate pGV1207. Thus, these genes are cloned in-frame with two copies of the AU1 tag (SEQ ID NO:172) and expressed using the *S*. *cerevisiae TEF1* promoter region (SEQ ID NO:175). The hydroxybutyryl-CoA-dehydrogenase genes, *Ca-hbd* (from pGV1122), *Cb-hbd* (from pGV1129), and *Ca-hbd-co* (from pGV1198) were cloned into pGV1100 (carries *LEU2* marker) using *Sal*I and *BamH*I to generate pGV1140, pGV1141, and pGV1208, respectively. This results in these genes being cloned in-frame with an HA tag (SEQ ID NO:173) and expressed using the *TEF1* promoter. The crotonase genes, *Ca-crt* (from pGV1123), *Cb-crt* (from pGV1130), *Ca-crt-co* (from pGV1199) were cloned into pGV1101 (carries *TRP1* marker) using *Sal*I and *Bam*HI to generate pGV1142, pGV1143, and pGV1209, respectively. Thus, these genes are cloned in-frame with two copies of the AU1 tag and expressed using the *TEF1* promoter.

The butyryl-CoA dehydrogenase and the respective electron transfer genes *etfA* and *etfB* were cloned behind a myc tag (SEQ ID NO:174) expressed using the *TDH3* promoter region from *S. cerevisiae* (SEQ ID NO:176). The *Ca-bcd* (from pGV1124), *Cb-bcd* (from pGV1131), *Ca-bcd-co* (from pGV1200) and *Me-bcd-co* genes were cloned into pGV1103 (carries *HIS3* marker) to generate pGV1144, pGV1145, pGV1210, and pGV1214. The *Ca-etfA* (from pGV1125), *Ca-etfB* (from pGV1126), *Cb-etfA* (from pGV1132), *Cb-etfB* (from pGV1133), *Ca-etfB-co* (from pGV1202), and *Me-etfA-co* (from pGV1205) genes were cloned into pGV1104 (carries *LEU2* marker) to generate pGV1146, pGV1147, pGV1148, pGV1149, pGV1212, and pGV1215, respectively. The *Ca-etfA-co* (from pGV1201) and *Me-effB-co* (from pGV1206) were cloned into pGV1104 (carries *TRP1* marker) to generate pGV1211 and pGV1216, respectively.

The gene for an aldehyde dehydrogenase, *Cb-aldh* (from pGV1134), was cloned into pGV1102 (carries *URA3* marker) to generate pGV1150. The *Cb-aldh* gene is placed in frame with the HA tag (SEQ ID NO:173) expressed using the *TEF1* promoter. The bi-functional aldehyde/alcohol dehydrogenases, *Ca-aad, Ca-adhE2,* and *Ca-adhE2-co,* and the specific alcohol dehydrogenases, *Ca-bdhA, Ca-bdhB,* and *Cb-adhA* were cloned behind a myc-tag expressed under the control of the *TDH3* promoter. *Ca-aad* and *Ca-adhE2* were amplified by PCR using primers designed to introduce a *Sal*I site just upstream of the start codon and a *Not*I site just downstream of the stop codon. The plasmid, pGV1089, was used as a template for *Ca-aad,* and the *C*. *acetobutylicum* genomic DNA was used as a template for *Ca-adhE2.* These PCR products were cloned into pGV1106 (carries *URA3* marker) using *Sal*I and *Not*I to generate pGV1136 (*Ca-aad*) and pGV1137 (*Ca-adhE*2)*.* The codon optimized Ca-*adhE2-co* (from pGV1203) was cloned into pGV1106 using *Sal*I and *Bam*HI to generate pGV1213. The alcohol dehydrogenases, *Ca-bdhA* (from pGV1127), *Ca-bdhB* (from pGV1128), and *Cb-adhA* (from pGV1135), were cloned into pGV1106 using *Sal*I and *BamH*I to generate pGV1151, pGV1152, and pGV1153, respectively.

Therefore, the above described yeast expression genes for butyryl-coA dehydrogenase, electron transfer protein A, electron transfer protein B, and the specific alcohol dehydrogenase were combined with the *TEF1* promoter driven thiolase, hydroxybutyryl-CoA dehydrogenase, crotonase, or the aldehyde dehydrogenase, in pair-wise fashion as summarized in Table 2 above.

For this purpose, the *Eco*ICRI to *Xho*I fragments from pGV1144 *(TDH3* promoter and *Ca-bcd*) and from pGV1145 (*TDH3* promoter and *Cb-bcd*) were cloned into the *Not*I (filled in with Klenow) to *Xhol* sites of pGV1138 to generate pGV1167 (*ERG10 + Ca-bcd*) and pGV1168 (*ERG10* + *Cb-bcd*), respectively. These same *Eco*ICRI to *Xho*I fragments were also similarly cloned into pGV1139 to generate pGV1169 (*Ca-thl* + *Ca-bcd*) and pGV1170 (*Ca-thl* + *Cb-bcd*), respectively. Using the same strategy, the *Eco*ICRI to *Xhol* fragments from pGV1146 (*TDH3* promoter and Ca-*etfA*), pGV1148 (*TDH3* promoter and *Ca-etfB*), pGV1147 (*TDH3* promoter and *Cb-etfA*), and pGV1149 *(TDH3* promoter and *Cb-etfB*) were cloned into the Notl (filled in with Klenow) to *Xhol* sites of pGV1140, pGV1141, pGV1142, pGV1143 to generate pGV1171 (*Ca-hbd* + *Ca-etfA*), pGV1172 (*Ca-crt* + *Ca-etfB*), pGV1173 (*Cb-hbd* + *Cb-etfA*), and pGV1174 (*Cb-crt* + *Cb-etfB*), respectively. The aldehyde dehyrogenase and the alcohol dehydrogenases were combined similarly by cloning the *Eco*ICRI to *Xho*I fragments from pGV1151 *(TDH3* promoter and *Ca-bdhA*), pGV1152 *(TDH3* promoter and *Ca-bdhB*) and pGV1153 *(TDH3* promoter and *Cb-adhA*) into the (filled in with Klenow) to *Xho*I sites of pGV1150 to generate pGV1175 (*Cb-aldh* + *Ca-bdhA*), pGV1176 (*Cb-aldh* + *Ca-bdhB*), and pGV1177 (*Cb-aldh* + *Cb-adhA*), respectively.

In the case of the codon-optimized genes, the *Eco*ICRI to *Xho*I fragments from pGV1210 *(TDH3* promoter and *Ca-bcd-co*), pGV1211 *(TDH3* promoter and Ca-*etfA-co*), pGV1212 *(TDH3* promoter and *Ca-etfB-co*) were cloned into the *Bam*HI (filled in with Klenow) to *Xho*I sites of pGV1207, pGV1208, and pGV1209, respectively to generate pGV1220 (*Ca-thl-co* + *Ca-bcd-co*), pGV1217 (*Ca-hbd-co* + *Ca-etfA-co*), and pGV1218 (*Ca-crt-co* + *Ca-etfB-co*). The *Eco*ICRI to *Xhol* fragments from pGV1214 *(TDH3* promoter and *Me-bcd-co*), pGV1215 (*TDH3* promoter and *Me-etfA-co*), pGV1216 *(TDH3* promoter and *Me-etfB-co*) were also cloned into the same set of vectors, respectively, to generate pGV1224 (*Ca-thl-co* + *Me-bcd-co*), pGV1221 (*Ca-hbd-co + Me-etfA-co*), and pGV1222 (*Ca-crt-co* + *Me-etfB-co*). Furthermore, the *Eco*ICRI to *Xho*I fragments from pGV1210 (*TDH3* promoter and *Ca-bcd-co*) and from pGV1214 (*TDH3* promoter and *Me-bcd-co*) were cloned into the *Bam*HI (filled in with Klenow) to *Xhol* sites of pGV1138 to generate pGV1223 (*ERG10* + *Ca-bcd-co*) and pGV1219 (*ERG10* + *Me-bcd-co*)*.*

In addition to the above pathway, constructs were generated that utilize alternatives to the *bcdletfAletfB* complex, namely trans-enoyl reductase and crotonyl-CoA reductase. Trans-enoyl reductase genes from *C. aetobutylicum* (*Ca-ter*), *Aeromonas hydrophila* (*Ah-ter*), and *Euglena gracilis* (*Eg-ter*) and the crotonyl-coA reductase from *Streptomyces collinus* (*Sc-ccr*) were cloned. *Ca-ter* was PCR amplified from *C. acetobutylicum* genomic DNA using primers designed to introduce a *Sal*I site immediately upstream of the start codon and a *Not*I site just downstream of the stop codon. *Ah-ter*, *Eg-ter*, and Sc-ccrwere PCR amplified from pGV1114, pGV1115, and pGV1166, respectively, using primer designed to introduce a *Sal*I site immediately upstream of the start codon and a *Bam*HI site just downstream of the stop codon. The sequences for these three genes have been codon optimized for expression in *E*. *coli.* Also, the *Eg-ter* sequence encodes for a protein that is missing the N-terminal region which may be involved in mitochondrial localization. The respective PCR products were cloned into pGV1103 using appropriate restriction enzymes to generate pGV1155 (Ca-*ter*), pGV1156 (*Ah-ter*), pGV1157 (*Eg-ter*) and pGV1158 (*Sc-ccr*)*.*

For use in expressing the butanol pathway in yeast, these alternatives to the bcd/etfA/etfB complex were each combined with a thiolase gene on one plasmid. The *Ca-ter, Ah-ter, Eg-ter* and Sc-ccr genes were combined with the *Ca-thl-co* gene by cloning the *Eco*ICR1 to *Xho*I fragment from pGV1155, pGV1156, pGV1157 and pGV1158 into the *Bam*HI (filled in with Klenow) to *Xho*I sites of pGV1207 to generate pGV1225 (*Ca-thl-co* + Ca-ter), pGV1226 (*Ca-thl-co* + *Ah-ter*), pGV1227 (*Ca-thl-co* + *Eg-ter*) and pGV1228 (*Ca-thl-co* + Sc-ccr), respectively.

### Example 2. Yeast extract/Western Blot Analysis.

For analysis of protein expression, crude yeast protein extracts were made by a rapid TCA precipitation protocol. One OD600 equivalent of cells was collected and treated with 200 µL of 1.85N NaOH/7.4% 2-mercaptoethanol on ice for 10 mins. 200 µL of 50% TCA was added and the samples incubated on ice for an additional 10 mins. The precipitated proteins were collected by centrifugation at 25,000 rcf for 2 mins and washed with 1 mL of ice cold acetone. The proteins were again collected by centrifugation at 25,000 rcf for 2 mins. The pellet was then resuspended in SDS Sample Buffer and boiled (99°C) for 10 mins. The samples were centrifuged at maximum in a microcentrifuge for 30 sec to remove insoluble matter.

Samples were separated by a SDS-PAGE and transferred to nitrocellulose. Western analysis was done using the TMB Western Blot Kit (KPL). HA.11, myc (9E10), and AU1 antibodies were obtained from Covance. Westerns were performed as described by manufacturer, except that when the myc antibody was used, detector block solution was used at 0.3x - 0.5x supplemented with 1% detector block powder. Expression of all genes described in Example 1, was verified utilizing this method.

### Example 3. Yeast transformations.

*Saccharomyces cerevisiae* (W303a) transformations were done using lithium acetate method (Gietz, R.D.a.R.A.W., 2002, Methods in Enzymology, 350, 87-96). Briefly, 1 mL of an overnight yeast culture was diluted into 50 mL of fresh YPD medium and incubated in a 30°C shaker for 5-6 hours. The cells were collected, washed with 50 mL sterile water, and washed with 25 mL sterile water. The cells were resuspended using 1 mL 100mM lithium acetate and transferred to a microcentrifuge tube. The cells were pelleted by centrifuging for 10 s. The supernatant was discarded and the cells were resuspended in 4x volume of 100mM lithium acetate. 15 µL of the cells were added to the DNA mix (72 µL 50% PEG, 10 µL 1 M lithium acetate, 3 uL 10mg/ml denatured salmon sperm DNA, 2 µL each of the desired plasmid DNA and sterile water to a total volume of 100 µL). The samples were incubated at 30°C for 30 min and heat shocked at 42°C for 22 min. The cells were then collected by centrifuging for 10 s, resuspended in 100 µL SOS medium (Sambrook, J., Fritsch, E.F., Maniatis, T., 1989), and plated onto appropriate SC selection plates (Kaiser C., M., S. and Mitchel, A, 1994) -without uracil, tryptophan, leucine or histidine.

### Example 4. Production of n-butanol.

Transformants (Table 1 above) expressing different combinations of enzymes related to the proposed butanol production pathway were assessed for n-butanol production. Pre-cultures of the isolates were prepared by inoculating a few colonies from SC agar plates into 3 ml of SC medium (Kaiser C., M., S. and Mitchel, A, 1994) which was shaken under aerobic conditions for 16 hours at 30°C at 250 rpm. The resulting cells were pelleted at 4000xg for 5 minutes and resuspended in 500 µl of SC medium. Cell growth was assessed by absorbance at 600 nm with suitable dilutions. For each isolate tested, cells yielding 15 OD were injected (200 µl) into anaerobic balch tubes containing 5 ml of SC anaerobic medium, previously saturated with N₂ gas to remove dissolved oxygen. The tubes were incubated at 30° C with 250 rpm shaking to prevent cell settling.

The tubes were sampled 10, 26, 44 and 70 hours post-inoculation by removing 500 µl of culture with a sterile syringe. Afterwards, 250 µl of 40% glucose solution was injected into each tube to maintain adequate carbon in the culture medium. At each time point, the recovered samples were centrifuged to pellet the cells and the supernatant was immediately frozen until all the samples were collected.

N-butanol production by the transformants was determined by gas chromotography (GC) analysis. All frozen samples were thawed at room temperature and 400 µl of each sample with 80 µl of 10mM Pentanol added as an internal control was filtered through a 0.2 µm filter. 200 µl of the resulting filtrate was placed in GC vials and subjected to GC analysis.

Samples were run on a Series II Plus gas chromatograph with a flame ionization detector (FID), fitted with a HP-7673 autosampler system. Analytes were identified based on the retention times of authentic standards and quantified using 5-point calibration curves. All samples were injected at a volume of 1 µL. Direct analysis of the n-butanol product was performed on a DB-FFAP capillary column (30 m length, 0.32 mm ID, 0.25 µm film thickness) connected to the FID detector. The temperature program for separating the alcohol products was 225°C injector, 225°C detector, 50°C oven for 0 minutes, then 8°C/minute gradient to 80°C, 13°C/minute gradient to 170°C, 50°C/minute gradient to 220°C, then 220°C for 3 minutes.

For evaluation of butanol production, two independent transformants of each plasmid combination were tested. The results are summarized in Table 3 above. The two Gevo numbers under "Isolate Name" refer to the two independent transformants assessed for each plasmid combination.

The butanol amounts produced over time by the best two producers, transformants Gevo1099 and Gevo1102, relative to the isolates transformed with only the empty vectors, Gevo1110 and Gevo1111 are shown below (Fig. 6). Gevo 1099 and Gevo 1102 displayed an increase in butanol production over time with the butanol concentration increasing from 123µM to 313 µM and 57 µM to 317 µM, respectively, from 24 to 72 hours post inoculation.

### Example 5. Cloning and Expression of E. coli Pyruvate Dehydrogenase subunits in Saccharomyces cerevisiae.

The purpose of this Example is to describe how to clone *aceE, aceF,* and *lpdA* genes from *E*. *coli,* which together comprise the three subunits of the enzyme pyruvate dehydrogenase (PDH) as found in *E*. *coli*. The three genes were amplified from genomic DNA using PCR. This Example also illustrates how the protein products of these three genes were expressed in a host organism, *Saccharomyces cerevisiae.*

The *lpdA* gene from *E*. *coli* was amplified by PCR using *E*. *coli* genomic DNA as a template. To amplify specifically *lpdA,* the primers Gevo-610 and Gevo-611 were used; other PCR amplification reagents were supplied in manufacturer's kits, for example, KOD Hot Start Polymerase (Novagen, Inc., catalog #71086-5), and used according to the manufacturer's protocol. The forward and reverse primers incorporated nucleotides encoding *Sal*I and *Xho*I restriction endonuclease sites, respectively. The resulting PCR product was digested with *Sal*I and *Xhol* and cloned into pGV1103, yielding pGV1334. The inserted *lpdA* DNA was sequenced in its entirety.

The *aceE* and *aceF* genes from *E*. *coli* were inserted into pGV1334 using an approach similar to that described above. The *aceE* gene was amplified from *E*. *coli* genomic DNA using the primers Gevo-606 and Gevo-607, digested with *Sal*I+*Xho*I, and cloned into the vector pGV1334 cut with *Sal*I+*Xho*I, yielding pGV1379. The *aceE* insert was sequenced in its entirety. To obtain a plasmid with a different selectable prototrophic marker suitable for *S. cerevisiae* expression, the aceE insert was cloned out of pGV1379 as a *Sal*I+*Xho*I fragment and cloned into *Sal*I+*Xho*I cut pGV1104 yielding pGV1603.

The *aceF* gene was amplified from *E*. *coli* genomic DNA using the primers Gevo-653 and Gevo-609. The resulting 1.9 kb product was digested with *Sal*I + *Xho*I and cloned into the vector pGV1334, cut with the same enzymes, yielding pGV1380. The *aceF* insert was sequenced in its entirety. To obtain a plasmid with a different selectable marker suitable for S. cerevisiae expression, the *aceF* insert was cloned out of pGV1380 and cloned into pGV1105, yielding pGV1604.

To express these proteins in *S. cerevisiae,* the *S*. *cerevisiae* strain Gevo1187 (CEN.PK) was transformed with any combination of pGV1334, pGV1603, and pGV1604, and transformants selected on appropriate dropout media as described in Example 3. As a control, cells were transformed with the corresponding empty vectors-pGV1103, pGV1104, and pGV1105, respectively. Cultures grown from transformants were assayed for LpdA, AceE, or AceF expression by preparing crude yeast protein extracts and analyzing them by Western blotting (based on detecting the Myc epitope present in each protein) as described in Example 2.

### Example 6. Cloning of S. cerevisiae PDH subunits from genomic DNA, modified to remove endogenous mitochondrial targeting sequences, and their expression in S. cerevisiae cells.

In most eukaryotes, the pyruvate dehydrogenase (PDH) complex is localized inside the mitochondria. The various proteins comprising PDH are directed to enter the mitochondria by virtue of their containing, in their N-terminal region, around 20-40 amino acids commonly known as a mitochondrial targeting sequence. The presence of such a sequence can be determined experimentally or computationally (e.g. by the program MitoProt: http://mips.gsf.de/cgi-bin/proj/medgen/mitofilter). Successful mitochondrial import of the protein is followed by specific proteolytic cleavage and removal of the targeting sequence, resulting in a "cleaved" imported form. It is well known that removing such a sequence from a protein by genetic alteration of its coding sequence causes that protein to become unable to transit into the mitochondria.Thus, an attractive strategy to redirect a normally mitochondrial protein into the cytosol involves expressing only that portion of the gene encoding the "cleaved" portion of the protein remaining after mitochondrial import and subsequent protease cleavage.

The purpose of this Example is to describe the cloning of several of the genes comprising the *S*. *cerevisiae* pyruvate dehydrogenase complex, and the expression and detection of these genes in a culture of *S*. *cerevisiae* cells.

Several of the genes that encode subunits of PDH were cloned by PCR, using essentially the procedure described in Example 5, except the template was *S*. *cerevisiae* genomic DNA. The *S*. *cerevisiae* gene to be amplified and the corresponding primers that were used are shown in Table 1.

To generate genes encoding proteins predicted to be localized in the cytosol, the first primer listed in each pair of primers (listed in Table 1) was designed to amplify a region of each gene downstream of the portion predicted to encode the mitochondrial targeting sequence. The resulting PCR products were cloned into the vector pGV1103 using unique restriction enzyme sites encoded in the primers used to amplify each gene, yielding the plasmids listed in Table 2. Each insert was sequenced in its entirety. To test for expression of each gene, *S*. *cerevisiae* strain Gevo1187 (CEN.PK) was transformed singly with each of pGV1381, pGV1383, pGV1384, or pGV1385, following essentially the procedure as described in Example 3, and selecting HIS+ colonies on SC-his defined dropout media. Protein expression was assayed by lysate preparation and Western blotting (to detect the Myc tag present on each protein) as described (Example 2).

### Example 7. Prophetic. Cloning and expression of the S. cerevisiae subunit LPD1 and its expression in S. cerevisiae cells.

This prophetic Example describes how to clone the gene *LPD1* from *S*. cerevisiae genomic DNA by PCR, and how to detect expression of *LPD1* in a host *S*. *cerevisiae* cell.

The open reading of Lpd1 lacking those nucleotides predicted to encode the mitochondrial targeting sequence are amplified using the primers Gevo-658 plus Gevo-659 in a PCR reaction, essentially as described in Example 5. A 1.5kb product is digested with *Xho*I+*Bam*HI and cloned into pGV1103 cut with the same restriction enzymes. The resulting clone, pGV1103-Ipd1, is transformed into Gevo 1187 and resultant colonies are selected by HIS+ prototrophy, essentially as described in Example 3. A culture of cells containing pGV1103-Ipd1 is grown and *LPD1* expression is detected by harvesting of cells followed by Western blotting (for the Myc tag present on the protein) essentially as described in Example 2.

### Example 8. Prophetic. Cloning of E. coli PDH subunits and their expression in K. lactis

Certain yeasts, especially those known as "Crabtree negative", offer distinct advantages as a production host. Unlike Crabtree-positive strains (e.g. *Saccharomyces cerevisiae*) which ferment excess glucose to ethanol under aerobic conditions, Crabtree-negative strains, such as those of the genus *Kluyveromyces,* will instead metabolize glucose via the TCA cycle to yield biomass. Consequently, Crabtree-negative yeasts are tolerant of inactivation (during aerobic growth) of the so-called PDH-bypass route of glucose dissimilation, which can occur, for example, by deletion of the *KIPDC1* gene.

The following prophetic Example describes how to clone the genes encoding the three subunits of *E*. *coli* PDH into vectors suitable for expression in the yeast *Kluyveromyces lactis,* and also how to detect the expression of those genes.

The *E*. *coli* genes *IpdA, aceE,* and *aceF* are amplified by PCR as described in Example 5. Resulting PCR products are digested with *Sal*I + *Xho*I and cloned into the vectors pGV1428, pGV1429, and pGV1430, respectively, each cut *Sal*I + *Xho*I. These steps yield the plasmids pGV1428-IpdA, pGV1429-aceE, and pGV1430-aceF. Each insert is sequenced in its entiretyA strain of *K. lactis* (e.g Gevo 1287) is transformed with one or any combination of these plasmids according to known methods (e.g. Kooistra R, Hooykaas PJ, Steensma HY. (2004) Yeast. 15;21(9):781-92), and resultant colonies are selected by appropriate prototrophies. Cultures grown from transformants are assayed for LpdA, AceE, or AceF expression using crude yeast protein extracts and Western blot analysis (based on detecting the Myc epitope present in each protein) as described in Example 2.

### Example 9. Prophetic. Measurement of PDH activity in cells overexpressing PDH subunits.

The purpose of this Example is to describe how PDH activity can be measured by means of an in vitro assay.

A method to quantitate PDH activity in a cell lysate Is described in the literature: (Wenzel TJ, et al. (1992). Eur J Biochem 209(2):697-705.) This method utilizes a lysate derived from a cellular fraction enriched in mitochondria. A different embodiment of this method utilizes, as a source of PDH, cell lysates obtained from whole cells. Such lysates are prepared as described previously (Example 2). Another embodiment of this assay method uses a cell lysate derived from a cellular fraction highly enriched for cytosolic (non-mitochondrial) proteins. This biochemical fractionation will reduced the contribution of endogenous mitochondrial PDH in the assay. Methods to prepare such enriched lysates are commerically available and well-known to those skilled in the art; (e.g. Mitochondrial/Cytosol Fractionation Kit, BioVision, Inc., Mountain View, CA).

In another embodiment, PDH activity is immunopurified from cells by virtue of the presence of a Myc epitope tag encoded in one or more of the expression plasmid. Methods to immunopurify epitope-tagged proteins are well-known to those skilled in the art (e.g. Harlow and Lane, Antibodies: A Laboratory Manual,(1988) CSHL Press). The immunopurified PDH complex is thus distinct from endogenous complexes and serves as the source of activity in the aforementioned PDH in vitro assay.

### Example 10. Prophetic. Measurement of increased intracellular acetyl-CoA in cells overexpressing PDH.

The purpose of this example is to describe how intracellular levels of acetyl-CoA, a product of PDH, can be measured in a population of cultured yeast cells.

To measure intracellular acetyl-CoA, those yeast transfromants carrying appropriate plasmid combinations necessary to express the complete set of PDH genes (e.g. pGV1334, pGV1603, and pGV1604) will be assessed for cellular acetyl-CoA levels in comparison to the vector-only control transformants (e.g. pGV1103, pGV1104, and pGV1105). Yeast cells are grown to saturation in appropriate defined dropout media (e.g. SC -His, -Leu, -Trp) in shake flasks. The optical density (OD600) of the culture is determined and cells pelletted by centrifugation at 2800xg for 5 minutes. The cells are lysed using a bead beater and the lysates are utilized for protein determination and analysis for acetyl-CoA determination with established methods (Zhang et al, Connection of Propionyl-CoA Metabolism to Polyketide Biosynthesis in Aspergillus nidulans. Genetics, 168:785-794).

### Example 11. Prophetic. Co-expression of E. coli PDH subunit genes and a butanol production pathway in S. cerevisiae.

The purpose of this Example is to describe how genes encoding the *E*. *coli* PDH subunits will be co-expressed with those genes comprising a butanol production pathway, in the host *Saccharomyces cerevisiae.* Co-expressing PDH with a butanol production pathway will increase the yield of butanol produced relative to merely expressing the butanol pathway without heterologously expressed functional PDH in the cytosol.

The cloned genes *lpdA, aceE* and and *aceF* (see Example 5) are subcloned into butanol pathway gene plasmids, specifically pGV1208, pGV1209 and pGV1213 (Table 2). To do this, pGV1334, pGV1603 and pGV1604 are each digested with the restriction enzymes *Eco*ICRI plus *Xho*I, and the resulting released insert is ligated into pGV1208, pGV1209 and pGV1213 that is digested with *Bam*HI, the overhang filled in by Klenow DNA polymerase, and then digested with *Xhol,* all using standard molecular biology methods (Sambrook, J. Fritsch, E.F., Maniatis, T., 1989). These steps yield pGV1208-IpdA, pGV1209-aceE and pGV1213-aceF, respectively. The resulting plasmids are transformed along with pGV1227 into Gevo 1187 and selected for HIS, LEU, TRP and URA prototrophy, all essentially as described in Example 3. Strains transformed with the parental plasmids pGV1208 plus pGV1209 plus pVG1213 plus pGV1227 are used as controls, to assess the affect of PDH co-expression on butanol production. Production of butanol is performed as described in Example 4. The expected n-butanol yield is greater than 10%.

### Example 12. Prophetic. Generation of a form of PDH that is functional under anaerobic conditions, or under conditions of excess NADH.

The purpose of this Example is to describe the isolation of a mutant form of PDH which is active anaerobically, or is active in the presence of a high [NADH]/[NAD+] ratio relative to the ratio present during normal aerobic growth. Such a mutant form of PDH is desirable in that it may allow for continued PDH enzymatic activity even under microaerobic or anaerobic conditions.

Methods to obtain and identify altered versions of PDH that permit microaerobic or anaerobic activity have been described previously: (Kim, Y. et al. (2007). Appl. Environm. Microbiol., 73, 1766-1771; US Patent Application No. 11/949,724).

### Example 13. Prophetic. Co-expression of E. coli PDH subunit genes and a butanol production pathway in a S. cerevisiae strain with reduced or absent pyruvate decarboxylase activity.

The purpose of this Example is to describe how genes encoding the *E*. *coli* PDH subunits are co-expressed with genes comprising a butanol production pathway, in a host *Saccharomyces cerevisiae* strain with reduced or absent pyruvate decarboxylase (PDC) activity. Both PDC and PDH utilize and therefore compete for available pyruvate pools. Whereas the product of PDH, acetyl-CoA, can be directly utilized by the butanol pathway, the product of PDC, acetaldehyde, can be further reduced to ethanol (via alcohol dehydrogenase), an undesired side-product of butanol fermentation, or can be converted to acetyl-CoA via the concerted action of acetaldehyde dehydrogenase plus acetyl-CoA synthase. Thus, reducing or eliminating PDC activity will increase the yield of butanol from pyruvate in a cell also overexpressing functional PDH in the cytosol.

Generation of a pdc- strain of *S*. *cerevisiae*

Strains of *S*. *cerevisiae* having reduced or absent PDC activity are described in the literature (e.g., Flikweert, M.T., et al., (1996). Yeast 15;12(3):247-57; Flikweert MT, et al., (1999). FEMS Microbiol Lett. 1;174(1):73-9; van Maris AJ, et al., (2004) Appl Environ Microbiol. 70(1): 159-66. and are well-known to those skilled in the art. In one embodiment, a strain of *S*. *cerevisiae* lacking all PDC activity has the genotype pdc-1Δ pdc-5Δ pdc6Δ.Such strains lacks detectable PDC activity and are unable to grow on glucose as a sole carbon source, but can live when the growth media is supplemented with ethanol or acetate as an alternative carbon source. In another embodiment, a derivative of this strain has been evolved to grow on glucose, a convenient and commonly used carbon source. A third embodiment of a strain with greatly reduced PDC activity is a strain of the relevant genotype pdc-2Δ, also described in the literature (Flikweert MT, et al., (1999). Biotechnol Bioeng. 66(1):42-50). Any of these strains can serve as a useful host for the expression of PDH plus a butanol pathway. If necessary, any pdc- mutant strain will be engineered, by means of standard molecular biology and yeast genetic techniques, to make available those auxotrophic markers such that the plasmids pGV1208-IpdA, pGV1209-aceE, and pGV1213-aceF can be selected and stably maintained within a host cell. Such genetic engineering will take place by disruption of the relevant endogenous gene by a *URA3*-based disruption cassette, with subsequent removal of the *URA3* marker by FOA counterselection.

Butanol production in a PDH-overexpressing pdc- strain

The cloned genes *lpdA, aceE* and *aceF* (see Example 5) are subcloned into butanol pathway gene plasmids, specifically pGV1208, pGV1209 and pGV1213 (Table 2), essentially as described in Example 11.

The set of plasmids pGV1208-IpdA plus pGV1209-aceE plus pGV1213-aceF plus pGV1227, or the set pGV1208 plus pGV1209 plus pGV1213 plus pGV1227 as a control, are transformed into the appropriate pdc- mutant yeast strain and resulting colonies grown in liquid culture. Production of butanol is performed as described in Example 4. The expected n-butanol yield is greater than 50%.

It is likely that strains with diminished or absent PDC activity will exhibit a pronounced growth defect, and therefore may have to be supplemented with an additional carbon source (e.g. acetate or ethanol). Since the defect in growth in pdc- *S*. *cerevisiae* arises from their lack of cytoplasmic pools of acetyl-CoA, it is expected that successful expression of PDH in the cytosol will generate sufficient acetyl-CoA to rescue this growth defect. Such restoration of growth can serve as a useful in vivo readout of PDH activity in the cytosol.

### Example 14 (Prophetic). pfl (Pyruvate formate lyase) and FDH1 (Formate dehydrogenase) expression in Saccharomyces cerevisiae.

Cloning of *E*. *coli pflB* (inactive Pyruvate formate lyase) and *pflA* (Pyruvate formate lyase activating enzyme).

For the cloning of *Escherichia coli pflB* and *pflA*, genes are amplified using *E*. *coli* genomic DNA and pflB_forw, PflB_rev and PflA_forw, PflA_rev primers, respectively. For the cloning of the *Candida boidinii FDH1* (*Cb-FDH1)* gene, genomic DNA of *Canida boidinii* is used with fdh_forw and fdh_rev primers. Utilizing the restriction sites, *Sal*I and *Eco*RI incorporated into the forward and reverse gene amplification primers, respectively, the amplified DNA is ligated onto *Sal*I and *Eco*RI digested pGV1103, pGV1104 and pGV1102 yielding pGV1103pflA, pGV1104pflB and pGV1002fdh1. The proteins expressed from the resulting plasmids are tagged with myc, myc and HA tags, respectively.

The resulting plasmids (pGV1103pflA, pGV1104pflB and pGV1002fdh1) and vectors (pGV1103, pGV1104and pGV1102) are utilized to transform yeast strain Gevo 1187 as indicated by example 3 to yield PflA, PflB, Fdh1 expressing (PFL+) and control (PFL-) transformants. Both sets of transformants are chosen by selection for HIS, TRP and URA prototrophy.

The resulting trasformants are evaluated for PflA, PflB and Cb-Fdh1 expression using crude yeast protein extracts and western blot analysis as described in Example 2.

Those yeast transfromants verified to express all three proteins are assessed for cellular acetyl-CoA levels in comparison to the vector only control transformants. For this, PFL+ and PFL- cells are grown in SC-ura, his, trp medium in shake flask format. The optical density (OD600) of the culture determined and cells pelletted by centrifugation at 2800 xrcf for 5 minutes. The cells are lysed using a bead beater and the lysates are utilized for protein determination and analysis for acetyl-CoA determination with established methods (Zhang et al, Connection of Propionyl-CoA Metabolism to Polyketide Biosynthesis in Aspergillus nidulans. Genetics, 168:785-794). Acetyl-CoA amounts are assessed per mg of cellular total protein.

To evaluate the effect of PflA, PflB and Fdh1 expression on n-butanol production, *pflA*, *pflB* and *Cb-FDH1* are subcloned into butanol pathway gene containing pGV1208, pGV1209 and pGV1213 (Table 1). For this, pGV1103pflA, pGV1104pflB and pGV1002fdh1 are digested with *Eco*ICRI+*Xho*I restriction enzymes and ligated into pGV1208, pGV1209 and pGV1213 digested with *Bam*HI (and subsequently blunt ended with Klenow fill-in)+*Xho*I using standard molecular biology methods (Sambrook, J. Fritsch, E.F., Maniatis, T., 1989) to yield pGV1208PflA, pGV1209PflB and pGV1213Fdh1. The resulting plasmids along with pGV1227 are transformed into Gevo 1187 and selected for His, Leu, Trp and Ura prototrophy. Gevo 1110 and Gevo 1111 are used as control isolates (Table 1). Production of butanol is performed as described in Example 4. The expected n-butanol yield is greater than 10%.

### Example 15. (Prophetic) PflA, PflB and Fdh1 Expression in Saccharomyce cerevisiae with reduced or absent pyruvate decarboxylase activity

Cloning of *E*. *coli pflB* (inactive Pyruvate formate lyase) and *pflA* (Pyruvate formate lyase activating enzyme) and *Cb-FDH1* is done as described in Example 14.

The resulting plasmids (pGV1103pflA, pGV1104pflB and pGV1002fdh1) and vectors (pGV1103, pGV1104 and pGV1102) are utilized to transform *S*. *cerevisiae* (relevant genotype: *ura3, trp1, his3 , leu2, pdc1, pdc5, pdc6*) yeast strain as indicated by example 3 to yield PflA, PfIB, Cb-Fdh1 expressing (PFL+) and control (PFL-) transformants. Both sets of transformants are chosen by selection for HIS, TRP and URA prototrophy.

The resulting trasformants will be evaluated for PflA, PflB and Fdh1 expression using crude yeast protein extracts and western blot analysis as described in Example 2.

Those yeast transfromants verified to express all three proteins are assessed for cellular acetyl-CoA levels in comparison to the vector only control transformants as described in Example 14.

To evaluate the effect of expressing PflA, PflB and Fdh1 on n-butanol production, pGV1208PflA, pGV1209PflB and pGV1213Fdh1 along with pGV1227 are transformed into *S. cerevisiae* (MAT A, ura3, trp1, his3, leu2, pdc1, pdc5, pdc6) and selected for His, Leu, Trp and Ura prototrophy. Gevo 1110 and 1111 are used as control isolates (Table 1). Production of butanol is performed as described in Example 4. The expected n-butanol yield is greater than 50%.

### Example 16. (Prophetic) Pfl and Fdh1 Expression in Saccharomyces cerevisiae with reduced or absent ADH1 activity

Cloning of *E. coli pflB* (inactive Pyruvate formate lyase) and *pflA* (Pyruvate formate lyase activating enzyme)

Cloning of *E. coli pflB* (inactive Pyruvate formate lyase) and *pflA* (Pyruvate formate lyase activating enzyme) and *Cb-FDH1* is done as described in Example 14.

The resulting plasmids (pGV1103pflA, pGV1104pflB and pGV1002fdh1)and vectors (pGV1103, pGV1104 and pGV1102) are utilized to transform yeast strain Gevo 1253 (adh1Δ) as described in Example 3 to yield PflA, PflB, Fdh1 expressing (PFL+) and control (PFL-) transformants. Both sets of transformants are chosen by selection for HIS, TRP and URA prototrophy.

The resulting trasformants will be evaluated for PflA, PflB and Fdh1 expression using crude yeast protein extracts and western blot analysis as described in Example 2.

Those yeast transfromants verified to express all three proteins are assessed for cellular acetyl-CoA levels in comparison to the vector only control transformants as described in Example 14.

To evaluate the effect of overexpressing PflA, PflB and Fdh1on n-butanol production, pGV1208PflA, pGV1209PflB and pGV1213Fdh1 along with pGV1227 are transformed into Gevo 1253 and selected for His, Leu, Trp and Ura prototrophy. Gevo 1110 and 1111 are used as control isolates (Table 1). Production of butanol is performed as described in Example 4. The expected n-butanol yield is greater than 10%.

### Example 17. Cloning of PDC1 gene from S. cerevisiae, and its overexpression in S. cerevisiae.

The purpose of this example is to describe the cloning of a gene encoding pyruvate decarboxylase under the control of a constitutively active promoter, and to describe the expression of such a gene in an *S. cerevisiae* host cell.

The complete *PDC1* ORF was amplified from *S. cerevisiae* genomic DNA using primers Gevo-639 plus Gevo-640 in a PCR reaction that was carried out essentially as described (Example 5). The resulting 1.7kb product was digested with XhoI+BamHI and ligated into the vector pGV1106, which was cut *Sal*I+*Bam*HI*,* yielding pGV1389 (see Table 2). The insert was sequenced in its entirety).

To overexpress Pdc1 in *S. cerevisiae,* the *S. cerevisiae* strain Gevo1187 (CEN.PK) was transformed with pGV1389, and transformants selected on SC-ura dropout media as described in Example 3. Cultures grown from transformants were assayed for Pdc1 expression using crude yeast protein extracts and Western blot analysis (based on detecting the Myc epitope present in the recombinant expressed protein) as described in Example 2.

### Example 18. Cloning to permit inducible expression of a pyruvate decarboxylase gene.

The constitutive expression of a gene, for example pyruvate decarboxylase, may be undesirable at certain points during a culture's growth, or may exert an unexpected metabolic or selective pressure on those overexpressing cells. Thus, there is a need to employ a system of regulated gene expression, whereby a gene of interest may be expressed chiefly at an optimal time to maximize culture growth as well as performance in a subsequent fermentation.

The purpose of this example is to describe the cloning of a gene encoding the enzyme pyruvate decarboxylase under the control of an inducibly-regulated promoter, and to describe the expression of such a gene in an *S. cerevisiae* host cell.

The *PDC1* ORF present in pGV1389 (see Example 19) was released as an *Xba*I+*Bam*HI fragment and cloned into the vector pGV1414 which had been digested *Avr*II+*Bam*HI, yielding vector pGV1483. Vector pGV1483 (Table 2) thus features the *S. cerevisiae MET3* gene promoter (SEQ ID NO:177) driving the expression of the *PDC1* gene. The *MET3* promoter is transcriptionally silent in the presence of methionine but becomes active when methionine levels fall below a certain threshold. The plasmid pGV1483 is transformed into Gevo 1187 and resulting transformants are identified by selection on SC-ura media, as described in Example 3. Cultures of Gevo 1187 carrying pGV1483 are grown and assayed for PDC1 expression essentially as described in Example 2.

In another embodiment of this Example, the *PDC1* gene is expressed under the control of the *S. cerevisiae* copper-inducible *CUP1* gene promoter (SEQ ID NO:178). First, the *CUP1* gene promoter was amplified by PCR from *S*. *cerevisiae* genomic DNA using primers in a reaction essentially as described in (Example 5). The PCR product was digested *Sac*I+ *Sal*I and inserted into pGV1106 that was cut *Sac*I+*Sal*I, yielding pGV1388. The inserted *CUP1* promoter sequence was sequenced in its entirety. Next, an *Xba*I+*Bam*HI fragment containing the *PDC1* gene from pGV1389 is inserted into the *Avr*II+*Bam*HI-digested pGV1388, yielding pGV1388-PDC1. Plasmid pGV1388-PDC1 is transformed into Gevo 1187, as described in Example 3, and transformants are identified on SC-ura defined media lacking copper. Cultures of transformed cells are grown in SC-ura media without copper supplementation until they reach an OD600 of > 0.5, at which time copper sulfate is added to a final concentration of 0.5 mM. The cultures are grown for an additional 24 h to 48 h, as desired, and then assayed for expression of Pdc1 by Western blotting, essentially as described (Example 2).

### Example 19. Prophetic. An in vitro assay to measure PDC activity produced in a culture of yeast cells overexpressing a pyruvate decarboxylase enzyme.

The purpose of this Example is to describe an in vitro assay useful for determining the total pyruvate decarboxylase activity present in a cell, and in particular from a population of cells overexpresssing a PDC enzyme.

Assays to measure PDC activity from total cell lysates have been described and are well-known to those skilled in the art ( Maitra PK & Lobo Z. 1971. J Biol Chem. 25;246(2):475-88.; Schmitt HD & Zimmermann FK. 1982. J Bacteriol. 151 (3):1146-52; Eberhardt et al., (1999) Eur. J. Biochem. 262(1),191-201).

In another embodiment of this Example, PDC activity generated by expression of PDC as described in Examples 17 and 18 is measured by first immunoprecipitating PDC, using a specific antibody directed against PDC, or using an antibody directed against the Myc epitope tag, which is present in the overexpressed (but not endogenous) PDC as expressed in Examples RF20 and RF21. Methods to specifically immunoprecipitate proteins present in a complex mixture are well-known to those skilled in the art (e.g., Harlow and Lane, 1988, Antibodies: A Laboratory Manual, CSHL Press). The immunoprecipitated PDC complexes then serve as the source of material to be assayed using the aforementioned assays. This method thus allows the specific assay of heterologous, overexpressed PDC.

### Example 20. Prophetic. Increased butanol productivity resulting from PDC overexpression in S. cerevisiae that also contains a functional butanol production pathway.

The purpose of this Example is to illustrate how PDC overexpression increases butanol productivity in a culture of *Saccharomyces cerevisiae* also expressing a butanol production pathway.

A strain of *S*. *cerevisiae* overexpressing a PDC gene has been described previously (van Hoek et al., (1998). Appl Environ Microbiol. 64(6):2133-40). These experiments revealed that (1) endogenous PDC levels in S. *cerevisiae,* while comprising up to 3.4.% of the total cellular protein, can be further increased by the presence of an overexpression construct; and (2) the fermentative capacity (the maximum specific rate of ethanol production) of PDC-overexpressing cultures at high growth rates was increased relative to that of control strains. These results suggest that **overexpression** of PDC, under certain growth conditions, will increase the flux through a heterologously supplied butanol production pathway.

To overexpress a PDC gene in the presence of a butanol pathway, the *PDC1* gene is excised from pGV1389 by digestion with *Spe*I, the cut DNA overhang is filled in with Klenow DNA polymerase fragment, and the vector then digested with *Xhol.* The fragment is inserted into pGV1213 that is digested with *Bam*HI, the cut ends filled in with Klenow enzyme, and then digested with *Xhol,* yielding plasmid pGV1605. Plasmid pGV1605 or pGV1057 (Mumberg, D., et al. (1995) Gene 156:119-122) is transformed into Gevo 1187 along with plasmids pGV1208, pGV1209, and pGV1213, essentially as described (Example 3) and selected for His, Leu, Trp, and Ura prototrophy. Fermentations are carried out to produce butanol, which is measured as described (Example 4). The inclusion of pGV1605 results in higher butanol productivity (amount of butanol produced per unit time) than does the inclusion of pGV1057 with plasmids pGV1208, pGV1209, and pGV1213 in the aforementioned fermentations. The expected n-butanol yield is greater than 5%.

### Example 21. Prophetic. Increased butanol productivity resulting from PDC overexpression in an S. cerevisiae cell that has reduced alcohol dehydrogenase activity and that also contains a functional butanol production pathway.

The purpose of this Example is to demonstrate how enhanced butanol productivity is obtained by overexpressing a PDC gene in the presence of a butanol production pathway, in a yeast strain deficient in alcohol dehydrogenase (ADH) activity.

Acetaldehyde generated from pyruvate by PDC has two main fates: it can be further metabolized to acetyl-CoA by the action of acetaldehyde dehydrogenase and acetyl-CoA synthase, where it may then be a useful substrate for a butanol synthetic pathway; or, it can be further metabolized by a reductive process to ethanol, by the action of an alcohol dehydrogenase (ADH) enzyme. Therefore, diminishing or removing ADHs, especially those ADH enzymes with a preference for acetaldehye, would reduce or eliminate this undesirable route of acetaldehyde dissimilation and increase available acetyl-CoA pools a butanol pathway.

Plasmids pGV1208, pGV1209, pGV1213, and pGV1605 are simultaneously co-transformed into strain Gevo 1187, which has the relevant genotype *ADH1*⁺, or into strain Gevo1266, which has the relevant genotype *adh1*Δ. Transformed colonies are selected for His, Leu, Trp, and Ura prototrophy, essentially as described in Example 3. Fermentations are carried out to produce butanol, which is measured as described in Example 4. The expected n-butanol yield is greater than 10%. Strain Gevo1266 (*adh1*Δ) exhibits an improved yield of butanol over a parallel fermentation carried out in strain Gevo 1187 (*ADH1*⁺).

### Example 22. Prophetic. Increased butanol yield resulting from PDC overexpression in a K.lactis cell with reduced alcohol dehydrogenase activity and expressing a functional butanol production pathway.

The purpose of this Example is to describe the production of butanol in a *K. lactis* strain with greatly reduced or absent ADH activity. It is predicted that expression of a butanol pathway in such a strain will yield significantly greater yields of butanol per input glucose than would the expression of a butanol pathway in a strain with ADH activity.

Generation of a *Kluyveromyces lactis* strain with reduced alcohol dehydrogenase activity.

Methods to transform cells of and disrupt genes in *Kluyveromyces lactis*-i.e., to replace a functional open reading frame with a selectable marker, followed by the subsequent removal of the marker-have been described previously (Kooistra R, Hooykaas PJ, Steensma HY. (2004) Yeast. 15;21(9):781-92). *Kluyveromyces lactis* has four genes encoding ADH enzymes, two of which, *KIADH1* and *KIADH2*, are localized to the cytoplasm. A mutant derivative of *K. lactis* in which all four genes were deleted (called *K. lactis* adh⁰) has been described in the literature (Saliola, M., et al., (1994) Yeast 10(9):1133-40), as well as the culture conditions required to ideally grow this strain. An alternative version of this approach employs using a marker conferring resistance to the drug G418/geneticin, for example as provided by the kan gene. Such an approach is useful in that it leaves the *URA3* marker available for use as a selectable marker in subsequent transformations.

Expression of a butanol expression pathway in an adh0 strain of *K. lactis*

Plasmids pGV1208, pGV1209, pGV1213, and pGV1605 are simultaneously co-transformed into strain Gevo 1287, which is ADH⁺, or into an adh⁰ strain.Transformed colonies are selected for His, Leu, Trp, and Ura prototroph. Fermentations are carried out to produce butanol, which is measured as described in Example 4. The expected n-butanol yield is greater than 10%. Strain Gevo1287 produces significantly more butanol than does the parallel fermentation carried out in the otherwise isogenic adh⁰ strain.

### Example 23. (Prophetic). ALD6 over-expression in Saccharomyces cerevisiae.

To clone the *ALD6* gene of *S*. *cerevisiae,* a two step fusion PCR method was employed that eliminated an internal *Sal*I restriction enzyme site to facilitate subsequent molecular biology manipulations. Two overlapping PCR products that spans the sequence of the *S*. *cerevisiae ALD6* gene were generated using primers pairs Gevo-643 & Gevo-644 and Gevo-645 & Gevo-646 with *S*. *cerevisiae* genomic DNA as the template. The resulting PCR fragment was digested with *Sal*I+*Bam*HI and ligated into similarly restriction digested pGV1105 and pGV1101 to yield pGV1321 and pGV1326. Subsequently, *ALD6* was subcloned by digestion of pGV1321 and pGV1326 with *Eco*ICRI+*Xho*I and ligation into *Bam*HI(and subsequently blunt ended by Klenow fill-in)+*Xho*I digested pGV1209 and pGV1208 to yield pGV1339 and pGV1399, respectively.

The resulting plasmids (pGV1339 and pGV1399) and vectors (pGV1105 and pGV1101) are utilized to transform yeast strain Gevo 1187 as described in Example 3 to yield *ALD6* over-expressing ("Ald6+") or control transformants, respectively. Both sets of transformants are chosen by selection for TRP and LEU prototrophy appropriate dropout medium.

The resulting trasformants are evaluated for Ald6 expression using crude yeast protein extracts and western blot analysis as described in Example 2.

Those yeast transfromants verified to express Ald6 proteins are assessed for enhanced acetaldehyde dehydrogenase activity in comparison to the vector only control transformants. For this, Ald6+ and control cells are grown in appriate dropout medium in shake flasks. The optical density (OD600) of the culture is determined and cells pelletted by centrifugation at 2800x *g* for 5 minutes. The cells are lysed using a bead beater and the lysates are utilized for protein determination and analysis for aldehyde dehydrogenase activity using established methods (for example, Van Urk et al, Biochim. Biophys. Acta, 191:769).

To evaluate the effect of overexpressing Ald6 on n-butanol production, pGV1339 is transformed into Gevo 1187 along with pGV1208, pGV1227 and pGV1213 and selected for His, Leu, Trp and Ura prototrophy. Gevo 1110 and 1111 are used as control isolates (Table 1). Production of butanol is performed as described in Example 4. The expected n-butanol yield is greater than 5%.

### Example 24. (Prophetic). Ald6 overexpression in a Saccharomyces cerevisiae with no Alcohol dehydrogenase I activity (adh1Δ).

Cloning of *ALD6* gene is carried out as described in Example 23.

The resulting plasmids (pGV1339 and pGV1399) and vectors (pGV1100 and pGV1101) are utilized to transform yeast strain Gevo 1253 as indicated by example 3 to yield Ald6+ overexpressing and control transformants, respectively. Both sets of transformants are chosen on appropriate dropout medium.

The resulting trasformants will be evaluated for Ald6 expression using crude yeast protein extracts and western blot analysis as described in Example 2.

Those yeast transfromants verified to express Ald6 proteins will be assessed for enhanced acetaldehyde dehydrogenase activity as described in Example 23.

To evaluate the consequence of the overexpression of on n-butanol production, pGV1339 will be transformed into Gevo 1253 along with pGV1209, pGV1227 and pGV1213 and selected for His, Leu, Trp and Ura prototrophy. Gevo 1110 and 1111 are used as control isolates (Table 1). Production of butanol is performed as described in Example 4. The expected n-butanol yield is greater than 10%.

### Example 25. (Prophetic). Overexpression of an acetyl-CoA synthase gene in Saccharomyces cerevisiae.

The purpose of this Example is to describe the cloning of a gene encoding acefyl-CoA synthase activity, and the expression of such a gene in a host *S*. *cerevisiae* cell. Specifically, either or both of the *S. cerevisiae* genes *ACS1* or *ACS2* encode acetyl-CoA synthase activity.

For the cloning of *ACS1* and *ACS2* genes, *S. cerevisiae* genomic DNA was utilized as template with Primers Gevo-479 & Gevo-480 (*ACS1*) and Gevo-483 & Gevo-484 (*ACS2*), each set containing *Sal*I and *Bam*HI restriction sites in the forward and reverse primers, respectively. The resulting PCR fragment was digested with *Sal*I+*Bam*HI and ligated into similarly restriction digested pGV1101 and pGV1102 to yield pGV1262 and pGV1263. Subsequently, *ACS1* and *ACS2* were subcloned by digestion of pGV1262 and pGV1263 with *Eco*ICRI+*Xho*I and ligation into *Bam*HI(and subsequently blunt ended with Klenow fill-in)+*Xho*I digested pGV1213 to yield pGV1319 and pGV1320.

The resulting plasmids, pGV1262 and pGV1263, and vectors pGV1101 and pGV1102 are utilized to transform yeast strain Gevo 1187 as described in Example 3 to yield *ACS1*+, *ACS2*+ overexpressing and control transformants, respectively. Both sets of transformants are chosen by selection for LEU, URA prototrophy. The transformants are evaluated for Acs1 or Acs2 expression using crude yeast protein extracts and western blot analysis as described in Example 2.

Those yeast transfromants verified to express Acs1 or Acs2 proteins are assessed for enhanced Acetyl-CoA synthase activity in comparison to the vector only control transformants. For this, ACS1+ or ACS2+ and control cells are grown in SC - LEU, URA medium in shake flask format. The optical density (OD600) of the culture determined and cells pelletted by centrifugation at 2800 x rcf for 5 minutes. The cells are lysed using a bead beater and the lysates are utilized for protein determination and analysis for Acetyl-CoA synthase activity using established methods (Van Urk et al, Biochim. Biophys. Acta, 191:769).

To evaluate of the effect of Acs1 or Acs2 overexpression on n-butanol production, pGV1319 and 1320 will be transformed into Gevo 1187 along with pGV1208, pGV1209 and pGV1227 and selected for His, Leu, Trp and Ura prototrophy. Gevo 1110 and 1111 are used as control isolates (Table 1). Production of butanol is performed as described in Example 4. The expected n-butanol yield is greater than 5%.

### Example 26. (Prophetic). Overexpression of an acetyl-CoA synthase in Saccharomyces cerevisiae cell with no Alcohol dehydrogenase I activity (adh1Δ).

Cloning of *ACS1* and *ACS2* genes of *S. cerevisiae* are as described in Example 25.

The resulting plasmids, pGV1262 and pGV1263, and vectors pGV1101 and pGV1102 are utilized to transform yeast strain Gevo 1253 as indicated by example 3 to yield ACS1+, ACS2+ and overexpressing and control transformants, respectively. Both sets of transformants are chosen by selection for LEU, URA prototrophy. The trasformants are evaluated for Acs1 or Acs2 expression using crude yeast protein extracts and Western blot analysis as described in Example 25.

Those yeast transformants verified to express Acs1 or Acs2 proteins are assessed for enhanced Acetyl-CoA synthase activity as described in Example 26.

To evaluate of the effect of overexpresssing Acs1 or Acs2 on butanol production, pGV1319 and 1320 will be transformed into Gevo 1253 along with pGV1208, pGV1209 and pGV1227 and selected for His, Leu, Trp and Ura prototrophy. Gevo 1110 and 1111 are used as control isolates (Table 1). Production of butanol is performed as described in Example 4. The expected n-butanol yield is greater than 5%.

### Example 27. (Prophetic). ALD6, ACS1 and ACS2 overexpression in Saccharomyces cerevisiae.

*ALD6*, *ACS1* and *ACS2* genes are cloned as described above in Examples 23 and 25.

The resulting plasmids pGV1321 and pGV1262 or pGV1263 and vectors pGV1105 and pGV1102 are utilized to transform yeast strain Gevo 1187 as indicated by Example 3 to yield ALD6+ACS1+, ALD6+ACS2+ over-expressing and control transformants, respectively. Both sets of transformants are chosen by selection for LEU and URA prototrophy.

Transformants ALD6+ACS1+ and ALD6+ACS2+ are assessed for enhanced Acetyl-CoA synthase activity in comparison to the vector-only control transformants. For this, ALD6+ACS1+, ALD6+ACS2+ and control cells are grown in SC - LEU, URA medium in shake flask format and assessed as described in Example 25.

To evaluate the effect of overexpressing Ald6 plus Acs1 or Acs2 results in higher butanol production, Gevo 1187 is transformed with pGV1208, pGV1339, pGV1227 and pGV1319 or 1320 and selected for His, Leu, Trp and Ura prototrophy. Gevo 1110 and 1111 are used as control isolates (Table 1). Production of butanol is assessed as described in Example 4. The expected n-butanol yield is greater than 5%.

### Example 28. (Prophetic). ALD6 plus ACS1 or ACS2 overexpression in Saccharomyces cerevisiae with no Alcohol dehydrogenase I activity (adh1Δ).

*ALD6, ACS1* and *ACS2* genes are cloned as described in Examples 23 and 25.

The resulting plasmids pGV1321 and pGV1262 or pGV1263 and vectors pGV1105 and pGV1102 are utilized to transform yeast strain Gevo 1253 (ΔADH1) as indicated by example 3 to yield ALD6+ACS1+ or ALD6+ACS2+ overexpressing strains or control transformants, respectively. Both sets of transformants are chosen by selection for LEU and URA prototrophy.

Transformants ALD6+ACS1+ or ALD6+ACS2+ are assessed for enhanced Acetyl-CoA synthase activity in comparison to the vector-only control transformants. For this, ALD6+ACS1+ or ALD6+ACS2+ and control cells are grown in SC - LEU, URA medium in shake flask format and assessed as described in Example 25.

To evaluate the effect of overexpressing *SALD6* and *ACS1* or *ACS2* on butanol production, Gevo 1253 is transformed with pGV1208, pGV1339, pGV1227 and pGV1319 or 1320 and selected for HIS, LEU, TRP and URA prototrophy. Gevo 1110 and 1111 are used as control isolates (Table 1). Production of butanol is performed as described in Example 4. The expected n-butanol yield is greater than 10%.

### Example 29. (Prophetic). Cloning of a butanol pathway into vectors for expression in a yeast of the genus Kluyveromyces.

To clone the butanol pathway genes into vectors suitable for expression in the strain *Kluyveromyces lactis,* hbd, Crt, Thl +TER are released from pGV1208, pGV1209 and pGV1227 by digestion with *Sac*I and *Not*I restriction digests and cloned into similarly digested pGV1428, 1429 and 1430 to yield pGV1208K1, pGV1209KI and pGV1227KI. To clone ADHE2 into *Kluyveromyces lactis,* pGV1213 is digested with Mlul and SacI and ligated onto similarly digested pGV1431 to yield pGV1213KI. The resulting plasmids, pGV1208KI, pGV1209KI, pGV1227KI and pGV1213KI are transformed into *K. lactis* (strain Gevo 1287; relevant genotype: MATa, trp1, his3, leu2, ura3) and transformants are selected for TRP, HIS, LEU and URA prototrophy (Kooistra R, Hooykaas PJ, Steensma HY. (2004) Yeast. 15;21(9):781-92). Production of butanol is performed as described in Example 4.

### Example 30. (Prophetic). Pyruvate formate lyaseand Formate dehydrogenase I expression in Kluyveromyces lactis.

Cloning of *E*. *coli pflB* (inactive Pyruvate formate lyase) and *pflA* (Pyruvate formate lyase activating enzyme)

For the cloning of *Escherichia coli pflB* and *pflA*, genes are amplified using *E*. *coli* genomic DNA and pfIB_forw, PflB_rev and PflA_forw, PflA_rev primers , respectively. For the cloning of the *Candida boidinii FDH1* gene, genomic DNA of *Canida boidinii* is used as a template in a PCR reaction with fdh_forw and fdh_rev primers. Utilizing the restriction sites, *Sal*I and *Eco*RI incorporated into the forward and reverse gene amplification primers, respectively, the amplified DNA is ligated onto Sal I and EcoRI digested pGV1428, pGV1429 and pGV1430 yielding pGV1428pflA, pGV1429pflB and pGV1430fdh1. The proteins expressed from the resulting plasmids are tagged with the myc tags for protein expression studies.

The resulting plasmids (pGV1428pflA, pGV1429pflB and pGV1430fdh1) and vectors (pGV1428, pGV1429 and pGV1430) are utilized to transform yeast strain *K. lactis* (Gevo 1287; relevant genotype: MatA, trp1, his3, leu2 and ura3) by known methods (Kooistra R, Hooykaas PJ, Steensma HY. (2004) Yeast. 15;21 (9):781-92) to yield PflA, PflB, Cb-Fdh1 expressing (PFL+) and control (PFL-) transformants. Both sets of transformants are chosen by selection for HIS, TRP and LEU prototrophy.

The resulting trasformants are evaluated for PflA, PflB and Fdh1 expression using crude yeast protein extracts and Western blot analysis as described in Example 2.

Those yeast transfromants verified to express all three proteins are assessed for cellular acetyl-CoA levels in comparison to the vector only control transformants. For this, PFL+ and PFL- cells are grown in SC -LEU, HIS, TRP medium in shake flask format. The optical density (OD600) of the culture determined and cells pelletted by centrifugation at 2800 xrcf for 5 minutes. The cells are lysed using a bead beater and the lysates are utilized for protein determination and analysis for acetyl-CoA determination with established methods (Zhang et al, Connection of Propionyl-CoA Metabolism to Polyketide Biosynthesis in Aspergillus nidulans.Genetics, 168:785-794). Acetyl-CoA amounts are assessed per mg of cellular total protein.

To evaluate the effect of the expression of PflA, PflB and Fdh1 on butanol production, the *pflA*, *pflB* and *Cb-FDH1* are subcloned into butanol pathway gene containing pGV1208KI, pGV1209KI, pGV1227KI and pGV1213KI (Table 1). For this, pGV1428pflA, pGV1429pflB and pGV1002fdh1 are digested with EcoICRI+XhoI restriction enzymes and ligated into pGV1208KI, pGV1209KI and pGV1213KI digested with BamHI (and subsequently blunt ended with Klenow fill-in)+XhoI using standard molecular biology methods (Sambrook, J. Fritsch, E.F., Maniatis, T., 1989) to yield pGV1208KIPflA, pGV1209KIPflB and pGV1213KIFdh1. The resulting plasmids along with pGV1227KI are transformed into a strain of *K. lactis* (MATa, pdc1, trp1, his3, leu2 ura3)) and selected for His, Leu, Trp and Ura prototrophy. *Kluyveromyces lactis* transformants harboring pGV1428, pGV1429, pGV1430 and pGV1431 are used as control isolates Production of butanol is performed as described in Example 4. The expected n-butanol yield is greater than 10%.

### Example 31. (Prophetic). Pyruvate formate lyase and Formate dehydrogenase I expression in Kluyveromyces lactis lacking pyruvate decarboxylase activity.

Cloning of *E*. *coli pflB* (inactive Pyruvate formate lyase) and *pflA* (Pyruvate formate lyase activating enzyme) *Cb-FDH1* are as described in Example 30.

The resulting plasmids (pGV1428pflA, pGV1429pflB and pGV1430fdh1) and vectors (pGV1428, pGV1429 and pGV1430) are utilized to transform yeast strain *K. lactis* (MatA, pdc1, trp1, his3, leu2 and ura3) by known methods (Kooistra R, Hooykaas PJ, Steensma HY. (2004) Yeast. 15;21(9):781-92) to yield PflA, PflB, Cb-Fdh1 expressing (PFL+) and control (PFL-) transformants. Both sets of transformants are chosen by selection for HIS, TRP and LEU prototrophy.

The resulting trasformants are evaluated for PflA, PflB and Cb-Fdh1 expression using crude yeast protein extracts and western blot analysis as described in Example 2.

Those yeast transfromants verified to express all three proteins are assessed for cellular acetyl-CoA levels in comparison to the vector only control transformants. For this, PFL+ and PFL- cells are grown in SC -LEU, HIS, TRP medium in shake flask format and assessed as described in Example 30.

To evaluate how the expression of PflA, PflB and Fdh1 results in higher butanol production, pGV1208KIPflA, pGV1209KIPflB and pGV1213KIFdh1 along with pGV1227KI are transformed into *K. lactis* (MAT a, pdc1Δ, trp1, his3, leu2, ura3) and selected for His, Leu, Trp and Ura prototrophy. *Kluyveromyces lactis* transformants harboring pGV1428, pGV1429, pGV1430 and pGV1431 are used as control isolates. Production of butanol is performed as described in Example 4. The expected n-butanol yield is greater than 50%.

### Example 32. (Prophetic). Pfl (Pyruvate formate lyase) and Fdh1 (Formate dehydrogenase I) expression in a Kluyveromyces lactis devoid of Adh1 activity.

Cloning of *E*. *coli pflB* (inactive Pyruvate formate lyase), *pflA* (Pyruvate formate lyase activating enzyme) and *Cb-FDH1* are described in Example 30.

The resulting plasmids (pGV1428pflA, pGV1429pflB and pGV1430fdh1) and vectors (pGV1428, pGV1429 and pGV1430) are utilized to transform yeast strain *K. lactis* (MAT a, trp1, his3, leu2, ura3) by known methods(Kooistra R, Hooykaas PJ, Steensma HY. (2004) Yeast. 15;21(9):781-92) to yield PflA, PflB, Fdh1 expressing (EcPFL+) and control (EcPFL-) transformants. Both sets of transformants are chosen by selection for HIS, TRP and LEU prototrophy.

The resulting trasformants are evaluated for PflA, PflB and Fdh1 expression using crude yeast protein extracts and western blot analysis as described in Example 2.

Those yeast transfromants verified to express all three proteins are assessed for cellular acetyl-CoA levels in comparison to the vector only control transformants. For this, EcPFL+ and EcPFL- cells are grown in SC -LEU, HIS, TRP medium in shake flask format and assessed as described in Example 30.

To evaluate how the expression of PflA, PflB and Fdh1 results in higher butanol production, pGV1208KIPflA, pGV1209KIPflB and pGV1213KIFdh1 along with pGV1227KI are transformed into *K. lactis* (MAT a, adh1Δ, trp1, his3, leu2, ura3) and selected for His, Leu, Trp and Ura prototrophy. *Kluyveromyces lactis* transformants harboring pGV1428, pGV1429, pGV1430 and pGV1431 are used as control isolates. Production of butanol is performed as described in Example 4. The expected n-butanol yield is greater than 20%.

### Example 33. (Prophetic). KIALD6 overexpression in Kluyveromyces lactis.

To clone *KIALD6,* genomic DNA of *Kluyveromyces lactis* is used as a template in a PCR reaction with primers KIALD6_left5 and KIALD6 right3 (see Table 1), which is otherwise assembled as described in Example 5. The aforementioned primers contain *Sal*I and *Bam*HI restriction sites, respectively, and the resulting PCR fragment is digested with *Sal*I*+Bam*HI and ligated into similarly restriction digested pGV1428 to yield pGV1428KLALD6. Subsequently, *KIALD6* is subcloned by digestion of pGV1428ALD6 with *Eco*ICRI+*Xho*I and ligation into BamHI (and subsequently blunt ended by Klenow fill-in)+*Xh*oI-digested pGV1208KI to yield pGV1208KIALD6.

The resulting plasmid, pGV1428ALD6KI, and vector, pGV1428 are utilized to transform yeast strain *K*. *lactis* (MAT a, trp1, his3, leu2, ura3) by known methods (Kooistra R, Hooykaas PJ, Steensma HY. (2004) Yeast. 15;21(9):781-92) to yield KIALD6+ and KIALD6- over-expressing and control transformants, respectively. Both sets of transformants are chosen by selection for HIS prototrophy.

The resulting trasformants, KIALD6+ and KIALD6- are evaluated for KIAld6 expression using crude yeast protein extracts and Western blot analysis as described in Example 2.

Those *K*. *lactis* transfromants verified to overexpress KIAld6 protein are assessed for enhanced acetaldehyde dehydrogenase activity in comparison to the vector-only control transformants. For this, KIALD6+ and KIALD6- cells are grown in SC - HIS medium in shake flask format and assessed as described in Example 23.

To evaluate how the overexpression of KIALD6 results in higher butanol production, pGV1208KIALD6 is transformed into *K. lactis* (MAT a, trp1, his3, leu2, ura3) along with pGV1209KI, pGV1227KI and pGV1213KI and selected for HIS, LEU, TRP and URA prototrophy Transformants arising from *K. lactis* transformed with pGV1428, pGV1429, pGV1430 and pGV1431 are used as control isolates. Production of butanol is performed as described in Example 4. The expected n-butanol yield is greater than 5%.

### Example 34. (Prophetic). Overexpression of an aldehyde dehydrogenase in Kluyveromyces lactis devoid of Adh1 activity.

Cloning of Kluyveromyces *KIALD6* gene is described in Example 33.

The resulting plasmid, pGV1428ALD6, and vector, pGV1428 are utilized to transform yeast strain *K*. *lactis* (MATa, adh1Δ, trp1, his3, leu2, ura3) by known methods (Kooistra R, Hooykaas PJ, Steensma HY. (2004) Yeast. 15;21(9):781-92) to yield KIALD6+ and KIALD6- over-expressing and control transformants, respectively. Both sets of transformants are chosen by selection for HIS prototrophy.

The resulting trasformants - are evaluated for KIAld6 expression using crude yeast protein extracts and Western blot analysis as described in Example 2.

Those *K. lactis* transfromants verified to express KIAld6 proteins are assessed for enhanced acetaldehyde dehydrogenase activity as described in Example 30.

To evaluate how overexpression of KIAld6 results in higher butanol production, pGV1208KIALD6 is transformed into *K. lactis* (MAT a, adh1Δ, trp1, his3, leu2, ura3) along with pGV1209KI, pGV1227KI and pGV1213KI and selected for HIS, LEU, TRP and URA prototrophy Transformants arising from *K. lactis* transformed with pGV1428, pGV1429, pGV1430 and pGV1431 are used as control isolates. Production of butanol is performed as described in Example 4. The expected n-butanol yield is greater than 10%.

### Example 35. (Prophetic). Overexpression of an acetyl-CoA synthase gene in the yeast Kluyveromyces lactis.

Two paralagous genes, *KIACS1* and *KIACS2,* encode acetyl-CoA activity in the genome of the yeast *Kluyveromyces lactis.* To clone *KIACS1* and *KIACS2, Kluyveromyces lactis* genomic DNA is utilized as template with primers KIACS1_left5 & KIACS2_Right3 (ACS1) and KIACS2_Left5 & KIACS2_Right3 (ACS2) (see Table 1), containing Notl & SalI and SalI & BamHI restriction sites in the forward and reverse primers, respectively. The resulting PCR fragments are digested with appropriate enzymes and ligated into similarly restriction digested pGV1429 and pGV1431 to yield pGV1429ACS1 and pGV1431ACS2. Subsequently, *KIACS1* and *KIACS2* are subcloned by digestion of pGV1429ACS1 and pGV1431ACS2 with *Sac*I & *Not*I and ligation into similarly digested pGV1209KI and pGV1213KI to yield pGV1209KIACS1 and pGVKIACS2.

The resulting plasmids, pGV1429ACS1 and pGV1431ACS2 and empty vectors pGV1429 and pGV1431 are utilized to transform *K. lactis* (MATa, trp1, his3, leu2, ura3) by known methods to yield KIACS1+, KIACS2+ and KIACS- protein over-expressing and control transformants, respectively. Both sets of transformants are chosen by selection for TRP, URA prototrophy. The trasformants are evaluated for KIAcs1 and KIAcs2 expression using crude yeast protein extracts and western blot analysis as described in Example 2.

Those yeast transfromants verified to express KIAcs1 and KIAcs2 proteins are assessed for enhanced acetyl-CoA synthase activity in comparison to the vector only control transformants. For this, KIACS1+, KIACS2+ and KIACS- cells are grown in SC - TRP, URA medium in shake flask format and assessed as described in Example 25.

To evaluate how the overexpression of *KIACS1* and *KIACS2* result in higher butanol production, pGV1209KIACS1 and pGV1209KIACS2 are transformed into strain Gevo 1287 along with pGV1208K1 and pGV1227K1, and transformed cells are selected for His, Leu, Trp and Ura prototrophy. Transformants resulting from a *K. lactis* (MAT a, trp1, his3, leu2, ura3) transformed with pGV1428, pGV1429, pGV1430 and pGV1431 are used as control isolates. Production of butanol is performed as described in Example 4. The expected n-butanol yield is greater than 5%.

### Example 36. (Prophetic). Overexpression of an acetyl-CoA synthase gene in a yeast Kluyveromyces lactis devoid of Adh1 activity.

Cloning of *KIACSI* and *KIACS2* genes of *Kluyveromyces lactis* is described in Example 35.

The resulting plasmids, pGV1429ACS1 and pGV1431ACS2 and empty vectors pGV1429 and pGV1431 are utilized to transform *K. lactis* (MATa, adh1Δ, trp1, his3, leu2, ura3) by known methods to yield *KIACS1*+ and *KIACS2*+ overexpressing and control transformants, respectively. Both sets of transformants are chosen by selection for TRP and URA prototrophy. The trasformants are evaluated for KIAcs1 and KIAcs2 expression using crude yeast protein extracts and Western blot analysis as described in Example 2.

Those yeast transfromants verified to express KIAcs1 and KIAcs2 proteins are assessed for enhanced acetyl-CoA synthase activity as described in Example 25.

To evaluate how the over-expression of *KIACS1* and *KIACS2* result in higher butanol production, pGV1209KIACS1 and pGV1209KIACS2 are transformed into *K. lactis* (MatA, adh1, trp1, his3, leu2 and ura3) along with pGV1208KI and pGV1227KI. Production of butanol is performed as described in Example 4. The expected n-butanol yield is greater than 10%.

### Example 37. (Prophetic). KIALD6 and KIACS1 or KIACS2 over-expression in Kluyveromyces lactis.

*KIALD6, KIACS1* and *KIACS2* genes are cloned as described above in Examples 33 and 35.

The resulting plasmids pGV1428ALD6 and pGV1429ACS1 or pGV1430ACS2 and vectors pGV1428 and pGV1429 or pGV1430 are utilized to transform *K. lactis* (MATa, trp1, his3, leu2, ura3) by known methods to yield KIALD6+KIACS1+, KIALD6+KIACS2+ and KIALD-KIACS-, over-expressing and control transformants, respectively. Both sets of transformants are chosen by selection for HIS, TRP and HIS, LEU prototrophy, respectively.

Transformants KIALD6+KIACS1+ and KIALD6+KIACS2+ are assessed for enhanced Acetyl-CoA synthase activity in comparison to the vector only control transformants (ALD-ACS-). For this, KIALD6+KIACS1+, KIALD6+KIACS2+and KIALD-KIACS- cells are grown in SC - HIS, TRP and HIS,LEU media, respectively, in shake flask format and assessed as described in Example 25.

To evaluate how the overexpression of KIAld6 and KIAcs1 or KIAcs2 result in higher butanol production, *K*. *lactis* (MATa, trp1, his3, leu2 ura3) is transformed with pGV1208KIALD6, pGV1209KIACS1 or pGV1209KIACS2, pGV1227KI, pGV1213KI and selected for HIS, LEU, TRP and URA prototrophy. Transformants resulting from *K. lactis* (MATa, trp1, his3, leu2 ura3) transformed with pGV1428, pGV1429, pGV1430 and pGV1431 are used as control isolates. Production of butanol is performed as described in Example 4. The expected n-butanol yield is greater than 5%.

### Example 38. (Prophetic). KIALD6, KIACS1 and KIACS2 over-expression in Kluyveromyces lactis devoid of KIAdh1 activity (KIadh1Δ).

*KIALD6, KIACS1* and *KIACS2* genes are cloned as described in Examples 33 and 35.

The resulting plasmids pGV1428ALD6 and pGV1429ACS1 or pGV1430ACS2 and vectors pGV1428 and pGV1429 or pGV1430 are utilized to transform *K. lactis* (MATa, Kladh1Δ trp1, his3, leu2 ura3) by known methods to yield KIALD6+KIACS1+, KIALD6+KIACS2+ and KIALD-KIACS-, over-expressing and control transformants, respectively. Both sets of transformants are chosen by selection for HIS, TRP and HIS, LEU prototrophy, respectively.

Transformants, KIALD6+KIACS1+ and KIALD6+KIACS2+ are assessed for cellular acetyl CoA levels as described in Example 14.

To evaluate whether the over-expression of KIAld6 and KIAcs1 or KIAcs2 result in higher butanol production, *K. lactis* (MATa, Kladh1Δ trp1, his3, leu2 ura3) is transformed with pGV1208KIALD6, pGV1209KIACS1 or pGV1209KIACS2, pGV1227KI, pGV1213KI. Production of butanol is performed as described in Example 4. The expected n-butanol yield is greater than 10%.

## Claims

1. A metabolically-engineered yeast, said yeast engineered to:
(a) overexpress one or more the enzymes selected from pyruvate decarboxylase, aldehyde dehydrogenase, and acetyl-CoA synthase; and
(b) comprise at least one heterologous gene encoding an enzyme for a metabolic pathway capable of converting acetyl-CoA to n-butanol selected from acetyl-CoA-acetyltransferase, hydroxybutyryl-CoA dehydrogenase, crotonase, butyryl-CoA dehydrogenase, butyraldehyde dehydrogenase, n-butanol dehydrogenase, and a bifunctional butyraldehyde dehydrogenase/n-butanol dehydrogenase,
wherein said yeast is capable of metabolizing a carbon source to n-butanol and produces an increased amount of cytosolic acetyl-CoA relative to the amount of cytosolic acetyl-CoA produced by a wild-type yeast.

2. The yeast of claim 1, wherein said pyruvate decarboxylase is encoded by a gene selected from the group consisting of the *S*. *cerevisiae PDC1,* the *S. cerevisiae PDC5,* the *S*. *cerevisiae PDC6,* and the *K. lactis KIPDC1.*

3. The yeast of claim 1, wherein said aldehyde dehydrogenase is encoded by a gene selected from the group consisting of the *S*. *cerevisiae ALD6,* and the *K. lactis ALD6.*

4. The yeast of claim 1, wherein said acetyl-CoA synthetase is encoded by a gene selected from the group consisting of the *S*. *cerevisiae ACS1,* the *S*. *cerevisiae ACS2,* the *K. lactis ACS1,* and the *K. lactis ACS2.*

5. A metabolically-engineered yeast, said yeast engineered to:
(a) overexpress a pyruvate dehydrogenase or both a pyruvate formate lyase and a formate dehydrogenase; and
(b) comprise at least one heterologous gene encoding an enzyme for a metabolic pathway capable of converting acetyl-CoA to n-butanol selected from acetyl-CoA-acetyltransferase, hydroxybutyryl-CoA dehydrogenase, crotonase, butyryl-CoA dehydrogenase, butyraldehyde dehydrogenase, n-butanol dehydrogenase, and a bifunctional butyraldehyde dehydrogenase/n-butanol dehydrogenase,
wherein said yeast is capable of metabolizing a carbon source to n-butanol and produces an increased amount of cytosolic acetyl-CoA relative to the amount of cytosolic acetyl-CoA produced by a wild-type yeast.

6. The yeast of claim 5, wherein said pyruvate dehydrogenase is encoded by the *E*. *coli* genes *aceE, aceF,* and *IpdA* or homologs thereof, or the N-terminal mitochondrial targeting signal deleted *S. cerevisiae* genes *PDA1, PDB1, PDX1, LAT1,* and *LPD1.*

7. The yeast of claim 5, wherein said pyruvate formate lyase is encoded by the *E. coli* genes *pflA* and *pflB.*

8. The yeast of claim 5, wherein said formate dehydrogenase is encoded by the *C*. *boidini* gene *FDH1.*

9. The yeast as in any of claims 5 to 8, wherein said yeast is engineered to reduce and/or eliminate pyruvate decarboxylase activity.

10. The yeast as in any of the preceding claims, wherein said yeast is engineered to inactivate an alcohol dehydrogenase gene, whereby alcohol dehydrogenase activity is reduced sufficiently to increase cytosolic acetyl-CoA production in the metabolically-engineered yeast relative to cytosolic acetyl-CoA production in a wild-type yeast.

11. The yeast of claim 10, wherein said alcohol dehydrogenase is selected from the group consisting of the *S. cerevisiae* genes *ADH1, ADH2, ADH3, ADH4,* the *K. lactis* genes *ADHI, ADHII, ADHIII,* and *ADHIV.*

12. The yeast as in any of the preceding claims, wherein said yeast is a species from a genus selected from the group consisting of *Saccharomyces, Kluyveromyces, Dekkera, Pichia, Hansenula, Yarrowia, Pachysolen, Schizosaccharomyces, Candida, Yamadazyma,* and *Torulaspora.*

13. A method of producing n-butanol, the method comprising
(a) providing a metabolically-engineered yeast according to any of the preceding claims; and
(b) culturing the metabolically-engineered yeast for a period of time and under conditions to produce the n-butanol.

## Patentansprüche

1. Metabolisch veränderte Hefe, wobei die Hefe verändert ist, um:
(a) ein oder mehrere Enzyme zu überexprimieren, die aus Pyruvatdecarboxylase, Aldehyddehydrogenase und Acetyl-CoA-Synthase ausgewählt sind; und
(b) zumindest ein heterologes Gen zu umfassen, das für ein Enzym für einen Stoffwechselweg kodiert, der in der Lage ist, Acetyl-CoA in n-Butanol zu überführen, ausgewählt aus Acetyl-CoA-Acetyltransferase, Hydroxybutyryl-CoA-Dehydrogenase, Crotonase, Butyryl-CoA-Dehydrogenase, Butyraldehyddehydrogenase, n-Butanoldehydrogenase und einer bifunktionellen Butyraldehyddehydrogenase/n-Butanoldehydrogenase,
wobei die Hefe in der Lage ist, eine Kohlenstoffquelle zu n-Butanol zu metabolisieren, und im Vergleich zur Menge an zytosolischem Acetyl-CoA, das durch eine Wildtyp-Hefe produziert wird, eine erhöhte Menge an zytosolischem Acetyl-CoA produziert.

2. Hefe nach Anspruch 1, wobei für die Puryvatdecarboxylase ein Gen kodiert, das aus der aus *S. cerevisiae PDC1, S. cerevisiae PDC5, S. cerevisiae PDC6* und *K. lactis KIPDC1* bestehenden Gruppe ausgewählt ist.

3. Hefe nach Anspruch 1, wobei für die Aldehyddehydrogenase ein Gen kodiert, das aus der aus *S. cerevisiae ALD6* und *K. lactis ALD6* bestehenden Gruppe ausgewählt ist.

4. Hefe nach Anspruch 1, wobei für die Acetyl-CoA-Synthetase ein Gen kodiert, das aus der aus *S. cerevisiae ACS1, S. cerevisiae ACS2, K. lactis ACS1* und *K. lactis ACS2* bestehenden Gruppe ausgewählt ist.

5. Metabolisch veränderte Hefe, wobei die Hefe verändert ist, um:
(a) eine Pyruvatdehydrogenase oder sowohl eine Pyruvatfomiatlyase als auch eine Formiatdehydrogenase zu überexprimieren; und
(b) zumindest ein heterologes Gen zu umfassen, das für ein Enzym für einen Stoffwechselweg kodiert, der in der Lage ist, Acetyl-CoA in n-Butanol zu überführen, ausgewählt aus Acetyl-CoA-Acetyltransferase, Hydroxybutyryl-CoA-Dehydrogenase, Crotonase, Butyryl-CoA-Dehydrogenase, Butyraldehyddehydrogenase, n-Butanoldehydrogenase und einer bifunktionellen Butyraldehyddehydrogenase/n-Butanoldehydrogenase,
wobei die Hefe in der Lage ist, eine Kohlenstoffquelle zu n-Butanol zu metabolisieren, und im Vergleich zur Menge an zytosolischem Acetyl-CoA, das durch eine Wildtyp-Hefe produziert wird, eine erhöhte Menge an zytosolischem Acetyl-CoA produziert.

6. Hefe nach Anspruch 5, wobei für die Pyruvatdehydrogenase die *E.-coli*-Gene *aceE, aceF* und *IpdA* oder Homologe davon oder die *S.-cerevisiae-Gene* mit deletiertem N-terminalem mitochondrialem Targeting-Signal *PDA1, PDB1, PDX1, LAT1* und *LPD1* kodieren.

7. Hefe nach Anspruch 5, wobei für die Pyruvatformiatlyase die *E.-coli-Gene pflA* und *pfIB* kodieren.

8. Hefe nach Anspruch 5, wobei für die Formiatdehydrogenase das *C.-boidini-Gen FDH1* kodiert.

9. Hefe nach einem der Ansprüche 5 bis 8, wobei die Hefe so verändert ist, dass sie die Pyruvatdecarboxylaseaktivität verringert und/oder eliminiert.

10. Hefe nach einem der vorangegangenen Ansprüche, wobei die Hefe verändert ist, um ein Alkoholdehydrogenasegen zu inaktivieren, wodurch die Alkoholdehydrogenaseaktivität ausreichend verringert wird, um die Produktion von zytosolischem Acetyl-CoA in der metabolisch veränderten Hefe im Vergleich zur Produktion von zytosolischem CoA in Wildtyp-Hefe zu erhöhen.

11. Hefe nach Anspruch 10, wobei die Alkoholdehydrogenase aus der aus den S.-*cerevisiae-Genen ADH1, ADH2, ADH3, ADH4* und den *K.-lactis*-Genen *ADHI, ADHII, ADHIII und ADHIV* bestehenden Gruppe ausgewählt ist.

12. Hefe nach einem der vorangegangenen Ansprüche, wobei die Hefe eine Spezies einer Gattung ist, die aus der aus *Saccharomyces, Kluyveromyces, Dekkera, Pichia, Hansenula, Yarrowia, Pachysolen, Schizosaccharomyces, Candida, Yamadazyma* und *Torulaspora* bestehenden Gruppe ausgewählt ist.

13. Verfahren zur Herstellung von n-Butanol, wobei das Verfahren Folgendes umfasst:
(a) das Bereitstellen einer metabolisch veränderten Hefe nach einem der vorangegangenen Ansprüche; und
(b) das Kultivieren der metabolisch veränderten Hefe über einen Zeitraum und unter Bedingungen, die zur Herstellung von n-Butanol geeignet sind.

## Revendications

1. Levure métaboliquement modifiée, ladite levure étant modifiée pour :
(a) surexprimer une ou plusieurs enzymes sélectionnées parmi la pyruvate décarboxylase, l'aldéhyde déshydrogénase, et l'acétyl-CoA synthétase ; et
(b) comprendre au moins un gène hétérologue codant pour une enzyme d'une voie métabolique capable de convertir l'acétyl-CoA en n-butanol, sélectionnée parmi l'acétyl-CoA-acétyltransférase, l'hydroxybutyryl-CoA déshydrogénase, la crotonase, la butyryl-CoA déshydrogénase, la butyraldéhyde déshydrogénase, la n-butanol déshydrogénase, et une butyraldéhyde déshydrogénase/n-butanol déshydrogénase bifonctionnelle,
où ladite levure est capable de métaboliser une source de carbone en n-butanol et produit une quantité d'acétyl-CoA cytosolique augmentée par rapport à la quantité d'acétyl-CoA cytosolique produite par une levure de type sauvage.

2. Levure selon la revendication 1, dans laquelle ladite pyruvate décarboxylase est codée par un gène sélectionné dans le groupe consistant en *PDC1* de *S*. *cerevisiae, PDC5* de *S*. *cerevisiae, PDC6* de *S*. *cerevisiae,* et *KIPDC1* de *K*. *lactis.*

3. Levure selon la revendication 1, dans laquelle ladite aldéhyde déshydrogénase est codée par un gène sélectionné dans le groupe consistant en *ALD6* de *S*. *cerevisiae,* et *ALD6* de *K*. *lactis.*

4. Levure selon la revendication 1, dans laquelle ladite acétyl-CoA synthétase est codée par un gène sélectionné dans le groupe consistant en *ACS1* de *S*. *cerevisiae, ACS2* de *S*. *cerevisiae, ACS1* de *K*. *lactis,* et *ACS2* de *K. lactis.*

5. Levure métaboliquement modifiée, ladite levure étant modifiée pour :
(a) surexprimer une pyruvate déshydrogénase ou à la fois une pyruvate formate lyase et une formate déshydrogénase ; et
(b) comprendre au moins un gène hétérologue codant pour une enzyme d'une voie métabolique capable de convertir l'acétyl-CoA en n-butanol, sélectionnée parmi l'acétyl-CoA-acétyltransférase, l'hydroxybutyryl-CoA déshydrogénase, la crotonase, la butyryl-CoA déshydrogénase, la butyraldéhyde déshydrogénase, la n-butanol déshydrogénase, et une butyraldéhyde déshydrogénase/n-butanol déshydrogénase bifonctionnelle,
où ladite levure est capable de métaboliser une source de carbone en n-butanol et produit une quantité d'acétyl-CoA cytosolique augmentée par rapport à la quantité d'acétyl-CoA cytosolique produite par une levure de type sauvage.

6. Levure selon la revendication 5, dans laquelle ladite pyruvate déshydrogénase est codée par les gènes *aceE, aceF,* et *IpdA* d'*E. coli* ou des homologues de ceux-ci, ou les gènes *PDA1, PDB1, PDX1, LAT1,* et *LPD1* de *S*. *cerevisiae* dont le signal de ciblage mitochondrial N-terminal a été supprimé.

7. Levure selon la revendication 5, dans laquelle ladite pyruvate formate lyase est codée par les gènes *pflA* et *pflB* d'*E. coli.*

8. Levure selon la revendication 5, dans laquelle ladite formate déshydrogénase est codée par le gène *FDH1* de *C*. *boidini.*

9. Levure selon l'une quelconque des revendications 5 à 8, où ladite levure est modifiée pour réduire et/ou éliminer l'activité pyruvate décarboxylase.

10. Levure selon l'une quelconque des revendications précédentes, où ladite levure est modifiée pour inactiver un gène de l'alcool déshydrogénase, moyennant quoi l'activité alcool déshydrogénase est suffisamment réduite pour augmenter la production d'acétyl-CoA cytosolique chez la levure métaboliquement modifiée par rapport à la production d'acétyl-CoA cytosolique chez une levure de type sauvage.

11. Levure selon la revendication 10, dans laquelle ladite alcool déshydrogénase est sélectionnée dans le groupe consistant en les gènes *ADH1, ADH2, ADH3, ADH4* de *S*. *cerevisiae,* et les gènes *ADHI, ADHII, ADHIII* et *ADHIV* de *K. lactis.*

12. Levure selon l'une quelconque des revendications précédentes, où ladite levure est une espèce appartenant à un genre sélectionné dans le groupe consistant en *Saccharomyces*, *Kluyveromyces, Dekkera, Pichia, Hansenula, Yarrowia, Pachysolen, Schizosaccharomyces, Candida, Yamadazyma,* et *Torulaspora.*

13. Procédé pour produire du n-butanol, le procédé consistant à
(a) mettre à disposition une levure métaboliquement modifiée selon l'une quelconque des revendications précédentes ; et
(b) cultiver la levure métaboliquement modifiée pendant une période de temps et dans des conditions permettant de produire le n-butanol.
